(19) Europäisches Patentamt
European Patent Office
Office européen des brevets

(11) EP 3 210 998 A1

(12) EUROPEAN PATENT APPLICATION

(43) Date of publication:
**30.08.2017 Bulletin 2017/35**

(21) Application number: **16196765.8**

(22) Date of filing: **29.07.2009**

(51) Int Cl.:
***C07K 14/47*** (2006.01) ***A61P 3/06*** (2006.01)
***A61P 3/10*** (2006.01) ***A61P 5/24*** (2006.01)
***A61P 15/00*** (2006.01) ***A61P 15/06*** (2006.01)
***A61P 15/08*** (2006.01) ***A61P 25/28*** (2006.01)
***A61P 35/00*** (2006.01) ***A61P 35/04*** (2006.01)
***C07K 14/575*** (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **30.07.2008 JP 2008196598**

(62) Document number(s) of the earlier application(s) in accordance with Art. 76 EPC:
**09803004.2 / 2 314 609**

(71) Applicant: **Takeda Pharmaceutical Company Limited**
**Osaka-shi**
**Osaka 541-0045 (JP)**

(72) Inventor: **ASAMI, Taiji**
**Tsukuba-shi Ibaraki, 300-4293 (JP)**

(74) Representative: **Denny, Sophy H.**
**Elkington and Fife LLP**
**Prospect House**
**8 Pembroke Road**
**Sevenoaks, Kent TN13 1XR (GB)**

Remarks:
- This application was filed on 01-11-2016 as a divisional application to the application mentioned under INID code 62.
- Claims filed after the date of receipt of the divisional application (Rule 68(4) EPC).

# (54) METASTIN DERIVATIVE AND USE THEREOF

(57) Disclosed is a stable metastin derivative having excellent biological activities (e.g., a cancer metastasis inhibiting activity, a cancer proliferation inhibiting activity, a gonadotropin secretion promoting activity, a sex hormone secretion promoting activity). A metastin derivative produced by substituting a constituent amino acid in metastin can be improved in stability in the blood and can have an excellent cancer metastasis inhibiting activity, an excellent cancer proliferation inhibiting activity, an excellent gonadotropin secretion inhibiting activity, an excellent sex hormone secretion inhibiting activity and the like by binding the metastin derivative to polyethylene glycol.

EP 3 210 998 A1

## Description

TECHNICAL FIELD

The present invention relates to a metastin derivative modified with a polyethylene glycol and use thereof.

BACKGROUND ART

Human-derived metastin (also known as KiSS-1 peptide) (Patent Document 1) and mouse or rat-derived metastin (Patent Document 2) are known. Sustained release preparations containing metastin are also known (Patent Document 3).

Metastin has an effect of suppressing cancer metastasis and is thus effective for preventing or treating cancers (for example, lung cancer, gastric cancer, liver cancer, pancreatic cancer, colorectal cancer, rectal cancer, colonic cancer, prostate cancer, ovarian cancer, cervical cancer, breast cancer, renal cancer, bladder cancer, brain tumor, etc.); metastin also has an effect of controlling the pancreatic function and is effective for preventing or treating pancreatic diseases (e.g., acute or chronic pancreatitis, pancreatic cancer, etc.); and metastin further has an effect of controlling the placental function and is effective for preventing or treating choriocarcinoma, hydatid mole, invasive mole, miscarriage, fetal hypoplasia, abnormal glucose metabolism, abnormal lipid metabolism or abnormal delivery (Patent Documents 1 to 3).

PRIOR ART DOCUMENTS

PATENT DOCUMENTS

Patent Document 1: WO 00/24890
Patent Document 2: WO 01/75104
Patent Document 3: WO 02/85399

DISCLOSURE OF INVENTION

PROBLEMS TO BE SOLVED BY THE INVENTION

An object of the present invention is to provide a metastin derivative modified with a polyethylene glycol having excellent biological activities (a cancer metastasis suppressing activity, a cancer growth suppressing activity, a gonadotropic hormone secretion promoting activity, a sex hormone secretion promoting activity, a gonadotropic hormone secretion suppressing activity, a sex hormone secretion suppressing activity, etc.).

MEANS FOR SOLVING THE PROBLEMS

The present inventors have made extensive studies to solve the foregoing problems and as a result, have found that a PEG-modified metastin derivative, in which constituent amino acids of metastin are substituted with specific amino acids, and in which the metastin derivative is further chemically modified with a polyethylene glycol (PEG), exhibits improved pharmacokinetics and excellent LH and testosterone release actions. Based on these findings, the present inventors have continued further investigations and come to accomplish the present invention.

More specifically, the present invention provides the following features, and so on.

[1] A metastin derivative modified with a polyethylene glycol or a salt thereof.

[2] The metastin derivative modified with a polyethylene glycol according to [1] above, which is represented by the formula below:

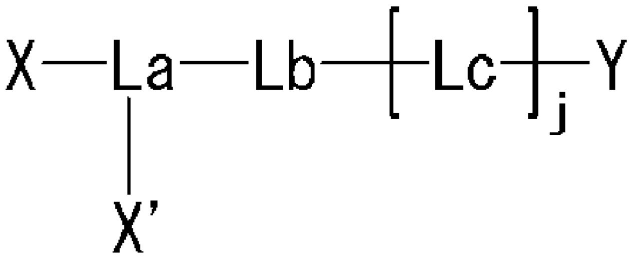

(I)

[wherein

Y represents a metastin derivative;
X represents a polyethylene glycol;
X' is absent, or represents a polyethylene glycol;
La represents a bivalent or trivalent group represented by formula:

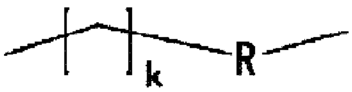

or

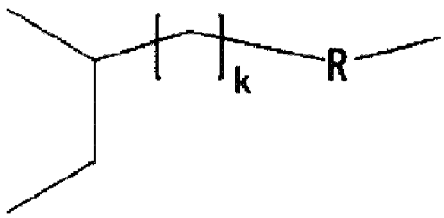

(wherein

R represents a chemical bond, -O-, -CO-O-, -O-CO-, -NH-, -CO-, -S-, -S-S-, -SO-, $-SO_2-$, $-NH-SO_2-$, $-SO_2-NH-$, -C(=O)-NH-N=CH-, -C(=NH)-NH-, $-CO-CH_2-S-$, or

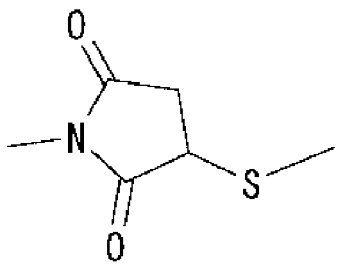

; and
k represents an integer of 0 to 5.);

Lb represents $-(CH_2)_i-$ (wherein i represents an integer of 0 to 5.);
Lc represents a bivalent group represented by

(i) formula: $-B-Q^c-C^b-$
(wherein

B represents a chemical bond, -NH-, -O-, or -S-;
$Q^c$ represents a bivalent group represented by formula: $-(CH_2)_{m1}-Z^c-(CH_2)_{m2}-$
(wherein

m1 represents an integer of 0 to 15;
$Z^c$represents:

(a) a chemical bond; or

(b) a bivalent group selected from -CO-, -O-CO-, -CO-O-, -CO-NH-,-NH-CO-, -CO-NH-CO-, -NH-CO-NH-, $-CH(NH_2)-$, $-CH(-NHR^{zc1})-$, $-CH(R^{zc2}-$,-CH(OH)-, -CH(COOH)-, -C(=NH)-, -S-, -S-S-, -SO-, $-SO_2-$, $-NH-SO_2-$, $-SO_2-NH-$,

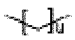 , 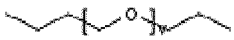 , 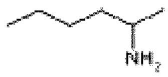 , 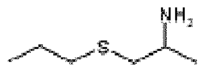 ,

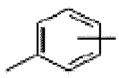 , 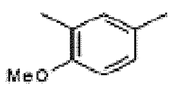 , 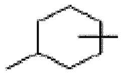 ,

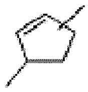 , 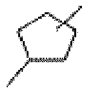 , 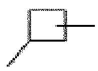 , 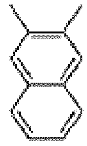 , 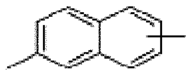 ,

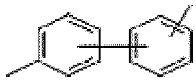 , 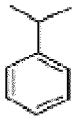 , 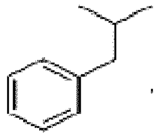 , 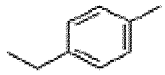 ,

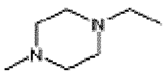 , 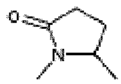 , 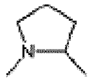 , 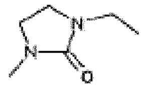 ,

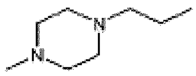 , 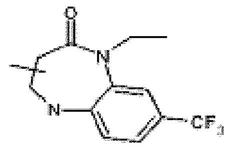 , 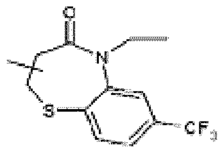 ,

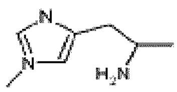 , 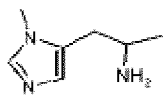

and

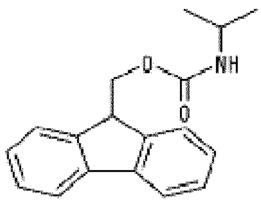

(wherein

u represents an integer of 1 to 18;
v represents an integer of 1 to 12;

$R^{zc1}$ represents an amino-straight $C_{1-5}$ alkyl-carbonyl group, or a X-straight $C_{1-5}$ alkyl group;

$R^{zc2}$ represents an amino-straight $C_{1-5}$ alkyl-carbonyl amino-straight $C_{1-5}$ alkyl group; and

X represents the same as those described above.); and
m2 represents an integer of 0 to 15.); and

$C^b$ represents a chemical bond, -CO-, or $-SO_2-$.); or

(ii) formula: $-Q^{c'}-C^{b'}-$
(wherein

$Q^{c'}$ represents formula: $-(CH_2)_{m1'}-Z^{c'}-(CH_2)_{m2'}-$
(wherein

m1' represents an integer of 0 to 15;
$Z^{c'}$ represents a bivalent group selected from

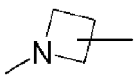
,
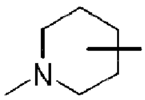
,
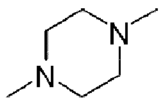
,
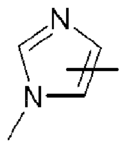
,
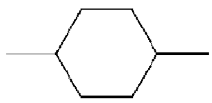
,
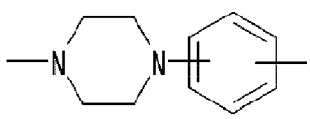
, **and**
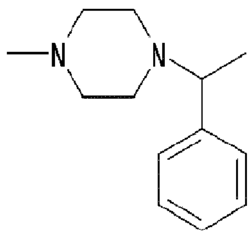
;

and
m2' represents an integer of 0 to 15.); and

$C^{b'}$ represents -CO- or $-SO_2-$.); and
j represents an integer of 1 to 3.]; or a salt thereof.

[3] The metastin derivative modified with a polyethylene glycol or a salt thereof according to [1] or [2] above, wherein the metastin derivative is selected from the group consisting of formulae (1) to (5) described below:

a metastin derivative represented by formula (1):

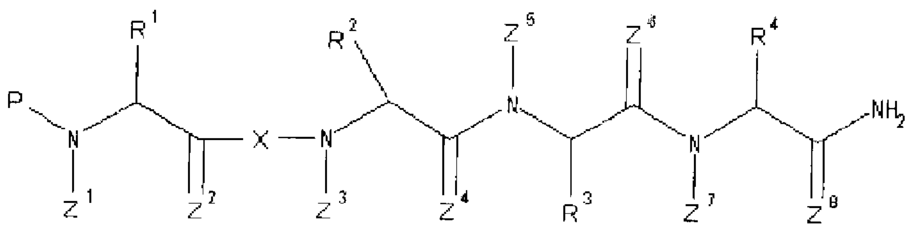
(1)

[wherein

each of $Z^1$, $Z^3$, $Z^5$ and $Z^7$ represents a hydrogen atom or a $C_{1-3}$ alkyl group;
each of $Z^2$, $Z^4$, $Z^6$ and $Z^8$ represents a hydrogen atom, O or S;
$R^1$ represents:

(1) a hydrogen atom; or
(2) a $C_{1-8}$ alkyl group optionally substituted with a substituent selected from the group consisting of an optionally substituted carbamoyl group, an optionally substituted hydroxyl group and an optionally substituted aromatic cyclic group;

$R^2$ represents:

(1) a hydrogen atom; or
(2) a cyclic or linear $C_{1-10}$ alkyl group; or
(3) a $C_{1-10}$ alkyl group consisting of a cyclic alkyl group and a linear alkyl group;

$R^3$ represents:

(1) a $C_{1-8}$ alkyl group having an optionally substituted basic group and optionally having an additional substituent;
(2) an aralkyl group having an optionally substituted basic group and optionally having an additional substituent;
(3) a $C_{1-4}$ alkyl group having a non-aromatic cyclic hydrocarbon group of carbon atoms not greater than 7 having an optionally substituted basic group, and optionally having an additional substituent; or
(4) a $C_{1-4}$ alkyl group having a non-aromatic heterocyclic group of carbon atoms not greater than 7 having an optionally substituted basic group, and optionally having an additional substituent;

$R^4$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and
(6) an optionally substituted non-aromatic heterocyclic group having carbon atoms not greater than 7;

X represents a group represented by formula:

$$-NHCH(Q^1)YQ^2C(=Z^9)-$$

(wherein

$Q^1$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and
(6) an optionally substituted non-aromatic heterocyclic group having carbon atoms not greater than 7;

$Q^2$ represents:

(1) $CH_2$, which may optionally be substituted with a $C_{1-4}$ alkyl group optionally substituted with a substituent selected from the group consisting of a carbamoyl group and a hydroxyl group;
(2) NH, which may optionally be substituted with a $C_{1-4}$ alkyl group optionally substituted with a substituent selected from the group consisting of a carbamoyl group and a hydroxyl group; or
(3) O;

Y represents a group represented by formulae -CONH-, -CSNH-,$-CH_2NH-$, -NHCO-, $-CH_2O-$, $-CH_2S-$ or $-CH_2CH_2-$, which may be substituted with a $C_{1-6}$ alkyl group; and
$Z^9$ represents a hydrogen atom, O or S); and

P represents:

(1) a hydrogen atom;
(2) an optional amino acid residue continuously or discontinuously bound from the C terminus of the 1-48 amino acid sequence represented by SEQ ID NO: 1;
(3) a group represented by formula:

$$J^1\text{-}J^2\text{-}C(J^3)(Q^3)Y^1C(J^4)(Q^4)Y^2C(J^5)(Q^5)Y^3C(J^6)(Q^6)C(=Z^{10})\text{-}$$

(wherein

$J^1$ represents:

(a) a hydrogen atom; or
(b) (i) a $C_{1-15}$ acyl group, (ii) a $C_{1-15}$ alkyl group, (iii) a $C_{6-14}$ aryl group, (iv) a carbamoyl group, (v) a carboxyl group, (vi) a sulfino group, (vii) an amidino group, or (viii) a glyoxyloyl group, which may be substituted with a substituent containing an optionally substituted cyclic group;

$J^2$ represents:

(1) NH optionally substituted with a $C_{1-6}$ alkyl group;
(2) $CH_2$ optionally substituted with a $C_{1-6}$ alkyl group
(3) O; or
(4) S;

each of $J^3$ through $J^6$ represents a hydrogen atom or a $C_{1-3}$ alkyl group;
each of $Q^3$ through $Q^6$ represents, a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7;
(6) an optionally substituted non-aromatic heterocyclic group having carbon atoms not greater than 7;
(7) an optionally substituted amino group;
(8) an optionally substituted guanidino group;
(9) an optionally substituted hydroxyl group;
(10) an optionally substituted carboxyl group;
(11) an optionally substituted carbamoyl group; and

(12) an optionally substituted sulfhydryl group; or a hydrogen atom;

$J^3$ and $Q^3$, $J^4$ and $Q^4$, $J^5$ and $Q^5$, $J^6$ and $Q^6$ may be combined together, or $J^2$ and $Q^3$, $Y^1$ and $Q^4$, $Y^2$ and $Q^5$, or $Y^3$ and $Q^6$ may be combined together to form a ring;
each of $Y^1$ through $Y^3$ represents a group represented by $-CON(J^{13})-$,$-CSN(J^{13})-$, $-C(J^{14})N(J^{13})-$ or $-N(J^{13})CO-$ (each of $J^{13}$ and $J^{14}$ represents a hydrogen atom or a $C_{1-3}$ alkyl group); and
$Z^{10}$ represents a hydrogen atom, O or S);

(4) a group represented by formula:

$$J^1-J^2-C(J^7)(Q^7)Y^2C(J^8)(Q^8)Y^3C(J^9)(Q^9)C(=Z^{10})-$$

(wherein

each of $J^1$ and $J^2$ represents the same as those described above;
each of $J^7$ through $J^9$ represents the same meaning as that of $J^3$;
each of $Q^7$ through $Q^9$ represents the same meaning as that of $Q^3$;
each of $Y^2$ and $Y^3$ represents the same as those described above;
$Z^{10}$ represents the same as those described above; and
$J^7$ and $Q^7$, $J^8$ and $Q^8$, $J^9$ and $Q^9$ may be combined together, or $J^2$ and $Q^7$, $Y^2$ and $Q^8$, or $Y^3$ and $Q^9$ may be combined together to form a ring.);

(5) a group represented by formula:

$$J^1-J^2-C(J^{10})(Q^{10})Y^3C(J^{11})(Q^{11})C(=Z^{10})-$$

(wherein

each of $J^1$ and $J^2$ represents the same as those described above;
each of $J^{10}$ and $J^{11}$ represents the same meaning as that of $J^3$;
each of $Q^{10}$ and $Q^{11}$ represents the same meaning as that of $Q^3$;
$Y^3$ represents the same as those described above;
$Z^{10}$ represents the same as those described above; and
$J^{10}$ and $Q^{10}$, $J^{11}$ and $Q^{11}$ may be combined together, or $J^2$ and $Q^{10}$, $Y^3$ and $Q^{11}$ may be combined together to form a ring.);

(6) a group represented by formula:

$$J^1-J^2-C(J^{12})(Q^{12})C(=Z^{10})-$$

(wherein

each of $J^1$ and $J^2$ represents the same as those described above;
$J^{12}$ represents the same meaning as that of $J^3$;
$Q^{12}$ represents the same meaning as that of $Q^3$;
$Z^{10}$ represents the same as those described above; and
$J^{12}$ and $Q^{12}$ may be combined together, or $J^2$ and $Q^{12}$ may be combined together to form a ring.); or

(7) a group represented by formula $J^1$- ($J^1$ represents the same as those described above).];
a metastin derivative represented by formula (2):

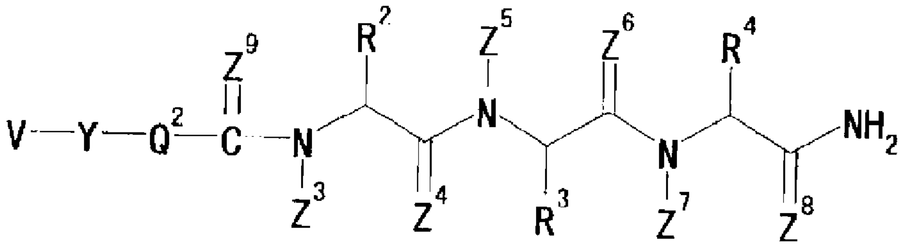 (2)

[wherein V represents a group represented by formula:

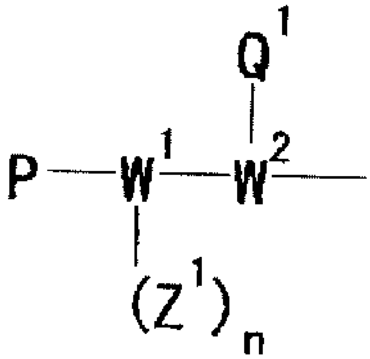

; or
a group represented by formula:

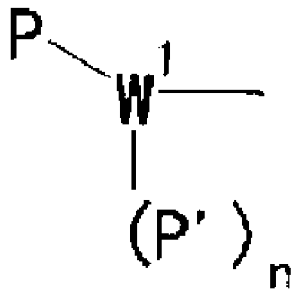

;

n represents 0 or 1;
$W^1$ represents N, CH or O (provided that when $W^1$ is N or CH, n represents 1, and when $W^1$ is O, n represents 0);
$W^2$ represents N or CH;
each of $Z^1$, $Z^3$, $Z^5$ and $Z^7$ represents a hydrogen atom or a $C_{1-3}$ alkyl group;
each of $Z^4$, $Z^6$ and $Z^8$ represents a hydrogen atom, O or S;
$R^2$ represents:

(1) a hydrogen atom; or
(2) a cyclic or linear $C_{1-10}$ alkyl group;
(3) a $C_{1-10}$ alkyl group consisting of a cyclic alkyl group and a linear alkyl group; or
(4) a $C_{1-8}$ alkyl group optionally substituted with a substituent selected from the group consisting of an optionally substituted carbamoyl group, an optionally substituted hydroxyl group and an optionally substituted aromatic cyclic group;

$R^3$ represents:

(1) a $C_{1-8}$ alkyl group having an optionally substituted basic group and optionally having an additional substituent;
(2) an aralkyl group having an optionally substituted basic group and optionally having an additional substituent;
(3) a $C_{1-4}$ alkyl group having a non-aromatic cyclic hydrocarbon group of carbon atoms not greater than 7 having an optionally substituted basic group, and optionally having an additional substituent; or
(4) a $C_{1-4}$ alkyl group having a non-aromatic heterocyclic group of carbon atoms not greater than 7 having an optionally substituted basic group, and optionally having an additional substituent;

$R^4$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and
(6) an optionally substituted non-aromatic heterocyclic group having carbon atoms not greater than 7;

$Q^1$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and
(6) an optionally substituted non-aromatic heterocyclic group having carbon atoms not greater than 7;

$Q^2$ represents:

(1) $CH_2$, which may optionally be substituted with a $C_{1-4}$ alkyl group optionally substituted with a substituent selected from the group consisting of a carbamoyl group and a hydroxyl group;
(2) NH, which may optionally be substituted with a $C_{1-4}$ alkyl group optionally substituted with a substituent selected from the group consisting of a carbamoyl group and a hydroxyl group; or
(3) O;

Y represents a group represented by formulae -CONH-, -CSNH-,$-CH_2NH-$, -NHCO-, $-CH_2O-$, $-CH_2S-$, -COO-, -CSO- or $-CH_2CH_2-$, which may be substituted with a $C_{1-6}$ alkyl group;
$Z^9$ represents a hydrogen atom, O or S; and
P and P' may be the same or different from each other, and P and P' or P and $Q^1$ may be combined together to form a ring, and P and P' represent:

(1) a hydrogen atom;

(2) an optional amino acid residue continuously or discontinuously bound from the C terminus of the 1-48 amino acid sequence represented by SEQ ID NO: 1;

(3) a group represented by formula:

$$J^1\text{-}J^2\text{-}C(J^3)(Q^3)Y^1C(J^4)(Q^4)Y^2C(J^5)(Q^5)Y^3C(J^6)(Q^6)C(=Z^{10})\text{-}$$

(wherein

$J^1$ represents:

(a) a hydrogen atom; or
(b) (i) a $C_{1-15}$ acyl group, (ii) a $C_{1-15}$ alkyl group, (iii) a $C_{6-14}$ aryl group, (iv) a carbamoyl group, (v) a carboxyl group, (vi) a sulfino group, (vii) an amidino group, (viii) a glyoxyloyl group, or (ix) an amino group, which may be substituted with a substituent containing an optionally substituted cyclic group;

$J^2$ represents:

(1) NH optionally substituted with a $C_{1-6}$ alkyl group;
(2) $CH_2$ optionally substituted with a $C_{1-6}$ alkyl group
(3) O; or
(4) S;

each of $J^3$ through $J^6$ represents a hydrogen atom or a $C_{1-3}$ alkyl group;
each of $Q^3$ through $Q^6$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7;
(6) an optionally substituted non-aromatic heterocyclic group having carbon atoms not greater than 7;
(7) an optionally substituted amino group;
(8) an optionally substituted guanidino group;
(9) an optionally substituted hydroxyl group;
(10) an optionally substituted carboxyl group;
(11) an optionally substituted carbamoyl group; and
(12) an optionally substituted sulfhydryl group; or a hydrogen atom;

$J^3$ and $Q^3$, $J^4$ and $Q^4$, $J^5$ and $Q^5$, $J^6$ and $Q^6$ may be combined together, or $J^2$ and $Q^3$, $Y^1$ and $Q^4$, $Y^2$ and $Q^5$, or $Y^3$ and $Q^6$ may be combined together to form a ring;
each of $Y^1$ through $Y^3$ represents a group represented by $-CON(J^{13})-$,$-CSN(J^{13})-$, $-C(J^{14})N(J^{13})-$ or $-N(J^{13})CO-$ (each of $J^{13}$ and $J^{14}$ represents a hydrogen atom or a $C_{1-3}$ alkyl group); and
$Z^{10}$ represents a hydrogen atom, O or S);

(4) a group represented by formula:

$$J^1\text{-}J^2\text{-}C(J^7)(Q^7)Y^2C(J^8)(Q^8)Y^3C(J^9)(Q^9)C(=Z^{10})\text{-}$$

(wherein

each of $J^1$ and $J^2$ represents the same as those described above;
each of $J^7$ through $J^9$ represents the same meaning as that of $J^3$;
each of $Q^7$ through $Q^9$ represents the same meaning as that of $Q^3$;
each of $Y^2$ and $Y^3$ represents the same as those described above;
$Z^{10}$ represents the same as those described above; and
$J^7$ and $Q^7$, $J^8$ and $Q^8$, $J^9$ and $Q^9$ may be combined together, or $J^2$ and $Q^7$, $Y^2$ and $Q^8$, or $Y^3$ and $Q^9$ may be combined together to form a ring.);

(5) a group represented by formula:

$$J^1\text{-}J^2\text{-}C(J^{10})(Q^{10})Y^3C(J^{11})(Q^{11})C(=Z^{10})\text{-}$$

(wherein

each of $J^1$ and $J^2$ represents the same as those described above;
each of $J^{10}$ and $J^{11}$ represents the same meaning as that of $J^3$;
each of $Q^{10}$ and $Q^{11}$ represents the same meaning as that of $Q^3$;
$Y^3$ represents the same as those described above;
$Z^{10}$ represents the same as those described above; and
$J^{10}$ and $Q^{10}$, $J^{11}$ and $Q^{11}$ may be combined together, or $J^2$ and $Q^{10}$, $Y^3$ and $Q^{11}$ may be combined together to form a ring.);

(6) a group represented by formula:

$$J^1\text{-}J^2\text{-}C(J^{12})(Q^{12})C(=Z^{10})\text{-}$$

(wherein

each of $J^1$ and $J^2$ represents the same as those described above;
$J^{12}$ represents the same meaning as that of $J^3$;
$Q^{12}$ represents the same meaning as that of $Q^3$;
$Z^{10}$ represents the same as those described above; and
$J^{12}$ and $Q^{12}$ may be combined together, or $J^2$ and $Q^{12}$ may be combined together to form a ring.); or

(7) a group represented by $J^1$- ($J^1$ represents the same as those described above).];
a metastin derivative represented by formula (3):

$$\text{XX0-XX2-XX3-XX4-XX5-XX6-AzaGly-XX8-XX9-XX10-NH}_2 \qquad (3)$$

[wherein XX0 represents formyl, $C_{1-20}$ alkanoyl, cyclopropanecarbonyl, 6-(acetyl-D-arginylamino)caproyl, 6-((R)-2,3-diaminopropionylamino)caproyl, 6-(D-norleucylamino)caproyl, 4-(D-arginylamino)butyryl, 3-(4-hydroxyphenyl)propionyl, glycyl, tyrosyl, acetylglycyl, acetyltyrosyl, D-tyrosyl, acetyl-D-tyrosyl, pyroglutamyl, 3-(pyridin-3-yl)propionyl, adipoyl, glycoloyl, 6-aminocaproyl, 6-acetylaminocaproyl, 4-[bis-(2-pyridylmethyl)aminomethyl]benzoyl or 4-ureidobenzoyl;
XX2 represents Tyr, D-Tyr, D-Ala, D-Leu, D-Phe, D-Lys, D-Trp or a chemical bond;
XX3 represents:

i) an amino acid selected from Ala, Arg, Asn, Asp, Cys, Gin, Glu, Gly, His, Ile, Leu, Lys, Met, Phe, Ser, Thr, Trp, Tyr and Val, wherein the $\alpha$-amino group may optionally be methylated;
ii) a cyclic amino acid selected from Pro, Aze(2), Aze(3), Pic(2), Pic(3), Hyp, Thz, Abz(2), Abz(3), Pzc(2), Pro(4$NH_2$), Hyp(Bzl), cisHyp, Pro(4F) and Izc;
iii) an amino acid selected from D-Dap, D-Pya(4), DL-Ala(Pip), Orn, Aib and Tyr($PO_3H_2$); or
iv) a chemical bond;

XX4 represents Asn, 2-amino-3-ureidopropionic acid, $N^\beta$-formyl-$\beta$-diaminopropionic acid, $N^\beta$-acetyl-$\beta$-diaminopropionic acid, $N^\omega$-pentylasparagine, $N^\omega$-cyclopropylasparagine, $N^\omega$-benzylasparagine, 2,4-diaminobutanoic acid, 2,3-diaminopropionic acid, His, Gin, Gly, Arg, Cit, Nva, D-Asn or a chemical bond;

XX5 represents Ser, Thr, Val, NMeSer, Gly, Ala, Hyp, D-Ala, D-Thr, D-Pro or a chemical bond;
XX6 represents Phe, Tyr, Trp, Tyr(Me), Thi, Nal(2), Cha, Pya(4), threo-Ser(3Phenyl), erythro-Ser(3Phenyl) or optionally substituted phenylalanine;
AzaGly represents azaglycine;
XX8 represents Leu, Nva, Val or Ala(cPr);
XX9 represents optionally substituted arginine, optionally substituted lysine or optionally substituted ornithine; and
XX10 represents 2-naphthylalanine, 2-thienylalanine, tyrosine, optionally substituted phenylalanine or optionally substituted tryptophan.];

a metastin derivative represented by formula (4):

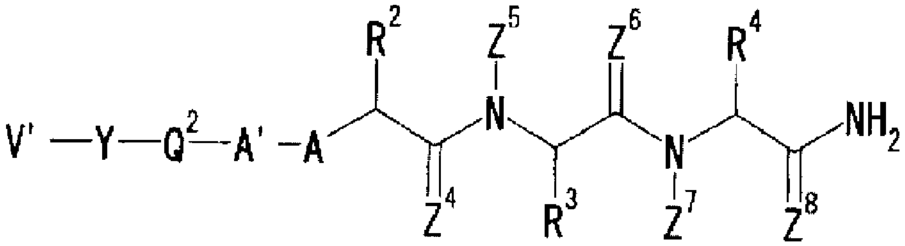

(4)

[wherein V' represents a group represented by formulae:

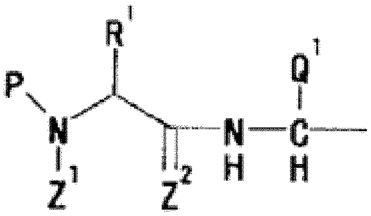

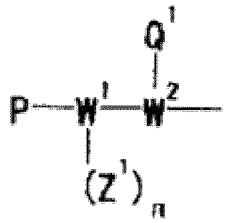

or

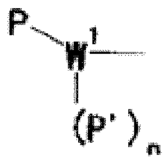

;

n represents 0 or 1;
$W^1$ represents N, CH or O (provided that when $W^1$ is N or CH, n represents 1, and when $W^1$ is O, n represents 0);
$W^2$ represents N or CH;
each of $Z^1$, $Z^5$ and $Z^7$ represents a hydrogen atom or a $C_{1-3}$ alkyl group;
each of $Z^2$, $Z^4$, $Z^6$ and $Z^8$ represents a hydrogen atom, O or S;
$R^1$ represents:

(1) a hydrogen atom;
(2) a $C_{1-8}$ alkyl group optionally substituted with a substituent selected from the group consisting of an optionally substituted carbamoyl group, an optionally substituted hydroxyl group and an optionally substituted aromatic cyclic group;
(3) a cyclic or linear $C_{1-10}$ alkyl group;
(4) a $C_{1-10}$ alkyl group consisting of a cyclic alkyl group and a linear alkyl group; or
(5) an optionally substituted aromatic cyclic group;

$R^2$ represents:

(1) a hydrogen atom; or
(2) a cyclic or linear $C_{1-10}$ alkyl group;
(3) a $C_{1-10}$ alkyl group consisting of a cyclic alkyl group and a linear alkyl group; or
(4) a $C_{1-8}$ alkyl group optionally substituted with a substituent selected from the group consisting of an optionally substituted carbamoyl group, an optionally substituted hydroxyl group and an optionally

substituted aromatic cyclic group;

$R^3$ represents:

(1) a $C_{1-8}$ alkyl group having an optionally substituted basic group and optionally having an additional substituent;
(2) an aralkyl group having an optionally substituted basic group and optionally having an additional substituent;
(3) a $C_{1-4}$ alkyl group having a non-aromatic cyclic hydrocarbon group of carbon atoms not greater than 7 having an optionally substituted basic group, and optionally having an additional substituent; or
(4) a $C_{1-4}$ alkyl group having a non-aromatic heterocyclic group of carbon atoms not greater than 7 having an optionally substituted basic group, and optionally having an additional substituent;

$R^4$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and
(6) an optionally substituted non-aromatic heterocyclic group having carbon atoms not greater than 7;

$Q^1$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and
(6) an optionally substituted non-aromatic heterocyclic group having carbon atoms not greater than 7;

A represents:

(1) a nitrogen atom that is substituted with hydrogen atom or a $C_{1-3}$ alkyl group;
(2) a carbon atom that is substituted with a hydrogen atom or a $C_{1-3}$ alkyl group;
(3) O; or
(4) S;

A' represents:

(1) a carbon atom that may be substituted with a hydrogen atom, O, S, a halogen atom, an optionally halogenated $C_{1-3}$ alkyl group, a carbamoyl group or a hydroxyl group;
(2) a nitrogen atom that may be substituted with a hydrogen atom or an optionally halogenated $C_{1-3}$ alkyl group;
(3) O; or
(4) S;

$Q^2$ represents:

(1) $CH_2$, CO, CS or CH=$CH_2$, which may optionally be substituted with one or two $C_{0-4}$ alkyl groups optionally substituted with a substituent selected from the group consisting of a carbamoyl group, a hydroxyl group, a $C_{1-3}$ alkoxy group, a halogen atom and an amino group,;
(2) NH, which may optionally be substituted with a $C_{1-4}$ alkyl group optionally substituted with a substituent selected from the group consisting of a carbamoyl group and a hydroxyl group; or
(3) O;

Y represents:

(1) a group represented by formulae -CONH-, -CSNH-, -$CH_2$NH-,-NHCO-, -$CH_2$O-, -CO$CH_2$-, -$CH_2$S-, -CS$CH_2$-, -$CH_2$SO-, -$CH_2SO_2$-, -COO-,-CSO-, -$CH_2CH_2$- or -CH=CH-, which may optionally be substituted with a substituent selected from the group consisting of a $C_{1-6}$ alkyl group, a hydroxyl group and a halogen atom;
(2) an optionally substituted $C_{6-7}$ aromatic hydrocarbon group;
(3) an optionally substituted 4- to 7-membered aromatic heterocyclic group consisting of 1 to 5 carbon atoms and hetero atoms selected from the group consisting of nitrogen, oxygen and sulfur atoms;
(4) an optionally substituted non-aromatic cyclic hydrocarbon group having not greater than 5 carbon atoms;
(5) an optionally substituted non-aromatic heterocyclic group having not greater than 5 carbon atoms;

provided that when Y is (2), (3), (4) or (5), $Q^2$ may be a chemical bond; and
P and P' may be the same or different from each other, and P and P' or P and $Q^1$ may be combined together to form a ring, and P and P' represent:

(1) a hydrogen atom;
(2) an optional amino acid residue continuously or discontinuously bound from the C terminus of the 1-48 amino acid sequence represented by SEQ ID NO: 1;
(3) a group represented by formula:

$$J^1\text{-}J^2\text{-}C(J^3)(Q^3)Y^1C(J^4)(Q^4)Y^2C(J^5)(Q^5)Y^3C(J^6)(Q^6)C(=Z^{10})\text{-}$$

(wherein

$J^1$ represents:

(a) a hydrogen atom; or
(b) (i) a $C_{1-20}$ acyl group, (ii) a $C_{1-20}$ alkyl group, (iii) a $C_{6-14}$ aryl group, (iv) a carbamoyl group, (v) a carboxyl group, (vi) a sulfino group, (vii) an amidino group, (viii) a glyoxyloyl group, or (ix) an amino group, which may be substituted with a substituent containing an optionally substituted cyclic group;

$J^2$ represents:

(1) NH optionally substituted with a $C_{1-6}$ alkyl group;
(2) $CH_2$ optionally substituted with a $C_{1-6}$ alkyl group
(3) O; or
(4) S;

each of $J^3$ through $J^6$ represents a hydrogen atom or a $C_{1-3}$ alkyl group;
each of $Q^3$ through $Q^6$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting

of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7;
(6) an optionally substituted non-aromatic heterocyclic group having carbon atoms not greater than 7;
(7) an optionally substituted amino group;
(8) an optionally substituted guanidino group;
(9) an optionally substituted hydroxyl group;
(10) an optionally substituted carboxyl group;
(11) an optionally substituted carbamoyl group; and
(12) an optionally substituted sulfhydryl group; or a hydrogen atom;

$J^3$ and $Q^3$, $J^4$ and $Q^4$, $J^5$ and $Q^5$, $J^6$ and $Q^6$ may be combined together, or $Z^1$ and $R^1$, $J^2$ and $Q^3$, $Y^1$ and $Q^4$, $Y^2$ and $Q^5$, or $Y^3$ and $Q^6$ may be combined together to form a ring;
each of $Y^1$ through $Y^3$ represents a group represented by $-CON(J^{13})-$, $- CSN(J^{13})-$, $-C(J^{14})N(J^{13})-$ or $-N(J^{13})CO-$ (each of $J^{13}$ and $J^{14}$ represents a hydrogen atom or a $C_{1-3}$ alkyl group); and
$Z^{10}$ represents a hydrogen atom, O or S);

(4) a group represented by formula:

$$J^1-J^2-C(J^7)(Q^7)Y^2C(J^8)(Q^8)Y^3C(J^9)(Q^9)C(=Z^{10})-$$

(wherein

each of $J^1$ and $J^2$ represents the same as those described above;
each of $J^7$ through $J^9$ represents the same meaning as that of $J^3$;
each of $Q^7$ through $Q^9$ represents the same meaning as that of $Q^3$;
each of $Y^2$ and $Y^3$ represents the same as those described above;
$Z^{10}$ represents the same as those described above; and
$J^7$ and $Q^7$, $J^8$ and $Q^8$, $J^9$ and $Q^9$ may be combined together, or $J^2$ and $Q^7$, $Y^2$ and $Q^8$ or $Y^3$ and $Q^9$ may be combined together to form a ring.);

(5) a group represented by formula:

$$J^1-J^2-C(J^{10})(Q^{10})Y^3C(J^{11})(Q^{11})C(=Z^{10})-$$

(wherein

each of $J^1$ and $J^2$ represents the same as those described above;
each of $J^{10}$ and $J^{11}$ represents the same meaning as that of $J^3$;
each of $Q^{10}$ and $Q^{11}$ represents the same meaning as that of $Q^3$;
$Y^3$ represents the same as those described above;
$Z^{10}$ represents the same as those described above; and
$J^{10}$ and $Q^{10}$, $J^{11}$ and $Q^{11}$ may be combined together, or $J^2$ and $Q^{10}$, $Y^3$ and $Q^{11}$ may be combined together to form a ring.);

(6) a group represented by formula:

$$J^1-J^2-C(J^{12})(Q^{12})C(=Z^{10})-$$

(wherein

each of $J^1$ and $J^2$ represents the same as those described above;
$J^{12}$ represents the same meaning as that of $J^3$;
$Q^{12}$ represents the same meaning as that of $Q^3$;
$Z^{10}$ represents the same as those described above; and
$J^{12}$ and $Q^{12}$ may be combined together, or $J^2$ and $Q^{12}$ may be combined together to form a ring.); or

(7) a group represented by formula:

$J^1$- ($J^1$ represents the same as those described above);

wherein the bonds between Y-$Q^2$, $Q^2$-A' and A'-A represent independently from each other, a single bond or a double bond.];

a metastin derivative represented by formula (5):

$$XX0\text{-}XX2\text{-}XX3\text{-}XX4\text{-}XX5\text{-}XX6\text{-}AzaGly\text{-}XX8\text{-}XX9\text{-}XX10\text{-}NH_2 \quad (5)$$

[wherein

XX0 represents hydrogen, acetyl, mesyl, 4-fluorobenzoyl, decanoyl, benzylureido, ureido, amidino, dimethylaminoethylureido or isobutylureido;
XX2 represents Tyr, D-Tyr, D-Ala, D-Leu, D-Phe, D-Lys, D-Trp or a chemical bond;
XX3 represents:

i) an amino acid selected from Ala, Arg, Asn, Asp, Cys, Gin, Glu, Gly, His, Ile, Leu, Lys, Met, Phe, Ser, Thr, Trp, Tyr and Val, wherein the α-amino group may optionally be methylated;
ii) a cyclic amino acid selected from Pro, Aze(2), Aze(3), Pic(2), Pic(3), Hyp, Thz, Abz(2), Abz(3), Pzc(2), Pro($4NH_2$), Hyp(Bzl), cisHyp, Pro(4F) and Izc;
iii) an amino acid selected from D-Pya(4), DL-Ala(Pip), Orn, Aib and Tyr($PO_3H_2$); or
iv) a chemical bond;

XX4 represents Asn, 2-amino-3-ureidopropionic acid, $N^β$-formyl-β-diaminopropionic acid, $N^β$-acetyl-β-diaminopropionic acid, $N^ω$-pentylasparagine, $N^ω$-cyclopropylasparagine, $N^ω$-benzylasparagine, 2,4-diaminobutanoic acid, 2,3-diaminopropionic acid, His, Gin, Gly, Arg, Cit, Nva, D-Asn or a chemical bond;
XX5 represents Ser, Thr, Val, NMeSer, Gly, Ala, Hyp, D-Ala, D-Thr, D-Pro or a chemical bond;
XX6 represents Phe, Tyr, Trp, Tyr(Me), Thi, Nal(2), Cha, Pya(4), threo-Ser(3Phenyl), erythro-Ser(3Phenyl) or optionally substituted phenylalanine;
AzaGly represents azaglycine;
XX8 represents Leu, Nva, Val or Ala(cPr);
XX9 represents optionally substituted arginine, optionally substituted lysine or optionally substituted ornithine; and
XX10 represents 2-naphthylalanine, 2-thienylalanine, tyrosine, optionally substituted phenylalanine or optionally substituted tryptophan.]; and

a metastin derivative represented by formula (6):

$$XX0\text{-}XX2\text{-}XX3\text{-}XX4\text{-}XX5\text{-}T\text{-}XX9\text{-}XX10\text{-}NH_2 \quad (6)$$

[wherein

XX0 represents formyl, $C_{1-20}$ alkanoyl, mesyl, optionally substituted ureido, amidino, cyclopropanecarbonyl, 6-(acetyl-D-arginylamino)caproyl, 6-((R)-2,3-diaminopropionylamino)caproyl, 6-(D-norleucylamino)caproyl, 4-(D-arginylamino)butyryl, 3-(4-hydroxyphenyl)propionyl, glycyl, tyrosyl, acetylglycyl, acetyltyrosyl, D-tyrosyl, acetyl-D-tyrosyl, pyroglutamyl, 3-(pyridin-3-yl)propionyl, adipoyl, glycoloyl or 6-aminocaproyl;
XX2 represents Tyr, D-Tyr, D-Ala, D-Leu, D-Phe, D-Lys, D-Trp or a chemical bond;
XX3 represents D-Asp, D-Dap, D-Ser, D-Gln, D-His, D-NMeAla, D-NMePhe, Aze(2), Pic(2), Pic(3), Hyp, Thz, NMeAla, Gly, Aib, Abz(2), Abz(3), Sar, Leu, Lys, Glu, β-alanine, Pzc(2), Orn, His(3Me), Tyr($PO_3H_2$), Pro($4NH_2$), Hyp(Bzl), Trp, Pro, 4-pyridylalanine, Tic, D-Trp, D-Ala, D-Leu, D-Phe, D-Lys, D-Glu, D-2-pyridylalanine, D-3-pyridylalanine, D-4-pyridylalanine, Aad, Pro(4F) or a chemical bond;
XX4 represents Asn, 2-amino-3-ureidopropionic acid, $N^β$-formyldiaminopropionic acid, $N^β$-acetyldiaminopropionic acid, $N^ω$-pentylasparagine, $N^ω$-cyclopropylasparagine, $N^ω$-benzylasparagine, 2,4-diaminobutanoic acid, His, Gin, Cit or D-Asn;

XX5 represents Ser, Thr, Val, NMeSer, Gly, Ala, Hyp, D-Ala, Dap or D-Thr;
T represents formula II:

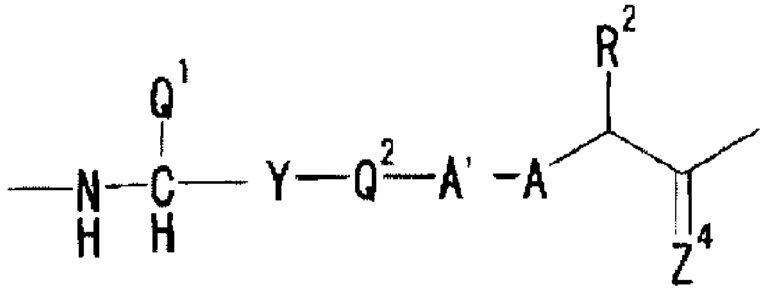

(II)

$Z^4$ represents a hydrogen atom, O or S;
$R^2$ represents:

(1) a hydrogen atom; or
(2) a cyclic or linear $C_{1-10}$ alkyl group;
(3) a $C_{1-10}$ alkyl group consisting of a cyclic alkyl group and a linear alkyl group; or
(4) a $C_{1-8}$ alkyl group optionally substituted with a substituent selected from the group consisting of an optionally substituted carbamoyl group, an optionally substituted hydroxy group and an optionally substituted aromatic cyclic group;

$Q^1$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and
(6) an optionally substituted non-aromatic heterocyclic group having carbon atoms not greater than 7;

A represents:

(1) a nitrogen atom that is substituted with hydrogen atom or a $C_{1-3}$ alkyl group;
(2) a carbon atom that is substituted with a hydrogen atom or a $C_{1-3}$ alkyl group;
(3) O; or
(4) S;

A' represents:

(1) a carbon atom that may be substituted with a hydrogen atom, O, S, a halogen atom, an optionally halogenated $C_{1-3}$ alkyl group, a carbamoyl group or a hydroxy group;
(2) a nitrogen atom that may be substituted with a hydrogen atom or an optionally halogenated $C_{1-3}$ alkyl group,
(3) O; or
(4) S;

$Q^2$ represents:

(1) $CH_2$, CO, CS or $C=CH_2$, which may optionally be substituted with a $C_{0-4}$ alkyl group optionally substituted with a substituent selected from the group consisting of a carbamoyl group, a hydroxyl group, a $C_{1-3}$ alkoxy group, a halogen atom and an amino group;
(2) NH, which may optionally be substituted with a $C_{1-4}$ alkyl group optionally substituted with a substituent selected from the group consisting of a carbamoyl group and a hydroxyl group; or
(3) O;

Y represents:

(1) a group represented by formulae -CONH-, -CSNH-, $-CH_2NH-$,-NHCO-, $-CH_2O-$, $-COCH_2-$, $-CH_2S-$, $-CSCH_2-$, $-CH_2SO-$, $-CH_2SO_2-$, -COO-,-CSO-, $-CH_2CH_2-$ or -CH=CH-, which may optionally be substituted with a substituent selected from the group consisting of a $C_{1-6}$ alkyl group, a hydroxyl group and a halogen atom;
(2) an optionally substituted $C_{6-7}$ aromatic hydrocarbon group;
(3) an optionally substituted 5- to 7-membered aromatic heterocyclic group consisting of 1 to 5 carbon atoms and hetero atoms selected from the group consisting of nitrogen, oxygen and sulfur atoms;
(4) an optionally substituted non-aromatic cyclic hydrocarbon group having not greater than 5 carbon atoms; or
(5) an optionally substituted non-aromatic heterocyclic group having not greater than 5 carbon atoms;

provided that when Y is (2), (3), (4) or (5), $Q^2$ may be a chemical bond; and
the bonds between Y-$Q^2$, $Q^2$-A' and A'-A represent independently from each other, a single bond or a double bond;

XX9 represents Arg, Orn, Arg(Me) or Arg(asym$Me_2$); and
XX10 represents Phe, Trp, 2-naphthylalanine, 2-thienylalanine, tyrosine or 4-fluorophenylalanine].

As the metastin derivative moiety used in the polyethylene glycol-modified metastin derivative of the present invention or a salt thereof, the amino acid moiety of the metastin derivatives selected from the group consisting of formulae (1) to (5) described below is preferably used.

[4] The metastin derivative modified with a polyethylene glycol or a salt thereof according to any one of [1] to [3] above, wherein the molecular weight of the polyethylene glycol is 1 kDa to 1000 kDa.
[5] The metastin derivative modified with a polyethylene glycol or a salt thereof according to any one of [1] to [4] above, wherein -La-Lb-[Lc]j- is $-CO-CH_2CH_2-CO-NH-(CH_2)_5-CO-$, $-CH_2CH_2CH_2-NH-(CH_2)_5-CO-$, or-$CH_2CH_2CH_2CH_2CH_2CO-NH-(CH_2)_5-CO-$.
[6] PEGSK(CS)-CO-Acp-Tyr-Asn-Trp-Asn-Ser-Phe-Gly-Leu-Arg-Phe-$NH_2$,
PEG2K(CS)-CO-Acp-Tyr-Asn-Trp-Asn-Ser-Phe-Gly-Leu-Arg-Phe-$NH_2$,
PEG20K(CS)-CO-Acp-Tyr-Asn-Trp-Asn-Ser-Phe-Gly-Leu-Arg-Phe-NH2,
PEG40K(AL)-$CH_2$-Acp-Tyr-Asn-Trp-Asn-Ser-Phe-Gly-Leu-Arg-Phe-$NH_2$,
PEG20K(HS)-CO-Acp-Tyr-Asn-Trp-Asn-Ser-Phe-Gly-Leu-Arg-Phe-$NH_2$,
PEG40K(HS)-CO-Acp-Tyr-Asn-Trp-Asn-Ser-Phe-Gly-Leu-Arg-Phe-$NH_2$,
PEG30K(AL)-$CH_2$-Acp-Tyr-Asn-Trp-Asn-Ser-Phe-Gly-Leu-Arg-Phe-$NH_2$,
PEG20K(CS)-CO-Acp-D-Tyr-D-Trp-Asn-Thr-Phe-AzaGly-Leu-Arg(Me)-Trp-$NH_2$,
PEG20K(CS)-CO-Acp-D-Tyr-Hyp-Asn-Thr-Phe-AzaGly-Leu-Arg(Me)-Trp-$NH_2$,
PEG20K(CS)-CO-Acp-D-Tyr-Glu-Asn-Thr-Phe(3F)-AzaGly-Leu-Arg(Me)-Trp-$NH_2$, or
PEG20K(CS)-CO-Acp-D-Tyr-Hyp-Asn-Thr-Phe(4F)-AzaGly-Leu-Arg(Me)-Trp-$NH_2$
(wherein the numbers and K mean kDa, which is the molecular weight of PEG, (CS) represents $-CO(CH_2)_2-$, (AL) represents $-(CH_2)_2-$, and (HS) represents $-(CH_2)_5-$.).
[7] A pharmaceutical comprising the metastin derivative modified with a polyethylene glycol or a salt thereof according to [1] above or a prodrug thereof.
[8] The pharmaceutical according to [7] above, which is a cancer metastasis-inhibiting agent or a cancer proliferation-inhibiting agent.
[9] The pharmaceutical according to [7] above, which is a pharmaceutical for preventing or treating cancer.

[10] The pharmaceutical according to [7] above, which is a pharmaceutical for controlling placental function.
[11] The pharmaceutical according to [7] above, which is a pharmaceutical for preventing or treating choriocarcinoma, hydatid mole, invasive mole, miscarriage, fetal hypoplasia, abnormal glucose metabolism, abnormal lipid metabolism or induction of delivery.
[12] The pharmaceutical according to [7] above, which is a pharmaceutical for improving gonadal function.
[13] The pharmaceutical according to [7] above, which is a pharmaceutical for preventing or treating hormone-dependent cancer, infertility, endometriosis, precocious puberty or hysteromyoma.
[14] The pharmaceutical according to [7] above, which is a pharmaceutical for inducing or stimulating ovulation.
[15] The pharmaceutical according to [7] above, which is a pharmaceutical for promoting gonadotropic hormone secretion or sex hormone secretion.
[16] The pharmaceutical according to [7] above, which is a pharmaceutical for preventing or treating Alzheimer's disease, mild cognitive impairment or autism.
[17] The pharmaceutical according to [13] above, which is a down-regulating agent for gonadotropic hormone or sex hormone.
[18] The pharmaceutical according to [13] above, which is a down-regulating agent for human OT7T175 (metastin receptor) protein consisting of the amino acid sequence represented by SEQ ID NO: 9.
[19] The pharmaceutical according to [17] or [18] above, which is a pharmaceutical for preventing or treating hormone-dependent cancer.
[20] A method for preventing or treating cancer, which comprises administering to a mammal, an effective dose of the metastin derivative modified with a polyethylene glycol or a salt thereof according to [1] above.
[21] Use of the metastin derivative modified with a polyethylene glycol or a salt thereof according to [1] above to manufacture a pharmaceutical for preventing or treating cancer.

EFFECTS OF THE INVENTION

In addition to the excellent effects of suppressing cancer metastasis and cancer proliferation, the metastin derivative modified with a polyethylene glycol (PEG) of the present invention or salts thereof or prodrugs thereof have excellent stability in blood, etc., and excellent pharmacokinetics, and are useful as agents for preventing or treating cancers (for example, lung cancer, gastric cancer, liver cancer, pancreatic cancer, colorectal cancer, rectal cancer, colonic cancer, prostate cancer, ovarian cancer, cervical cancer, breast cancer, etc.).

The metastin derivative modified with a polyethylene glycol (PEG) of the present invention or salts thereof or prodrugs thereof have an effect of controlling the pancreatic function and are useful as agents for preventing or treating pancreatic diseases (e.g., acute or chronic pancreatitis, pancreatic cancer, etc.). The metastin derivative of the present invention or salts thereof or prodrugs thereof have an effect of controlling the placental function and are useful as agents for preventing or treating choriocarcinoma, hydatid mole, invasive mole, miscarriage, fetal hypoplasia, abnormal glucose metabolism, abnormal lipid metabolism or induction of delivery.

Also, the metastin derivatives modified with a polyethylene glycol (PEG) of the present invention or salts thereof or prodrugs thereof have effects of increasing blood glucose level, promoting pancreatic glucagon secretion and promoting urine formation, and are useful as agents for preventing or treating obesity, hyperlipemia, type II diabetes mellitus, hypoglycemia, hypertension, diabetic neuropathy, diabetic nephropathy, diabetic retinopathy, edema, urinary disturbances, insulin resistance, unstable diabetes mellitus, fatty atrophy, insulin allergy, insulinoma, arteriosclerosis, thrombotic disorders or lipotoxicity.

Furthermore, the metastin derivatives modified with a polyethylene glycol (PEG) of the present invention or salts thereof or prodrugs thereof have excellent activities of promoting gonadotropic hormone secretion, promoting sex hormone secretion, inducing or stimulating ovulation, and are useful as low toxic and safe agents, e.g., for improving gonadal function, preventing or treating hormone-dependent cancer (e.g., prostate cancer, breast cancer, etc.), infertility, endometriosis, precocious puberty, hysteromyoma, etc., agents for inducing or stimulating ovulation, agents for promoting gonadotropic hormone secretion, contraceptives, agents for promoting sex hormone secretion, or the like.

Furthermore, the compound of the present invention is useful as agents for preventing or treating rheumatic diseases (for example, chronic rheumatoid arthritis, osteoarthritis, gout, etc.), etc.

Furthermore, the metastin derivatives modified with a polyethylene glycol (PEG) of the present invention or salts thereof or prodrugs thereof are useful as agents for preventing or treating Alzheimer's disease, mild cognitive impairment, autism, etc.

The metastin derivatives modified with a polyethylene glycol (PEG) of the present invention or salts thereof or prodrugs thereof are useful as agents for suppressing gonadotropic hormone secretion or sex hormone secretion; down-regulating agents for gonadotropic hormone or sex hormone; down-regulating agents for human OT7T175 (metastin receptor) protein consisting of the amino acid sequence represented by SEQ ID NO: 9; agents for preventing or treating hormone-dependent cancers (e.g., prostate cancer, breast cancer, etc.; particularly, hormone-sensitive prostate cancer,

hormone-sensitive breast cancer, etc.); agents for preventing or treating endometriosis; agents for inhibiting ovarian follicular maturation; menstrual cycle-suspending agents; agents for treating hysteromyoma; agents for treating precocious puberty; contraceptives, etc.

In addition, the metastin derivatives modified with a polyethylene glycol (PEG) of the present invention or salts thereof or prodrugs thereof are useful as immunopotentiators (prophylactic agents for infection after bone-marrow transplant, immunopotentiators for use in cancer); immuostimulants (thymus regeneration, thymic regrowth, enhanced T cell growth); prophylactic/therapeutic agents for bulbospinal muscular atrophy; agents for protecting ovary; prophylactic/therapeutic agents for benign prostate hypertrophy (BPH); prophylactic/therapeutic agents for gender identity disorders; agents for in vitro fertilization (IVF); etc. These derivatives, salts or prodrugs are also useful as prophylactic/therapeutic agents for infertility, hypogonadism, oligospermia, azoospermia, aspermia, asthenospermia, necrospermia, etc. Furthermore, these derivatives, salts or prodrugs are useful for hormone-dependent diseases (e.g., sex hormone-dependent cancers such as prostate cancer, uterine cancer, breast cancer, hypophyseal tumor, etc.), prostate gland enlargement, endometriosis, hysteromyoma, precocious puberty, dysmenorrhea, amenorrhea, premenstrual syndrome, multilocular ovary syndrome, postoperative relapse of the above-mentioned cancers, metastasis of the above-mentioned cancers, hypopituitarism, dwarfism (the case where the secretion of growth hormone is compromised with hyposecretion of pituitary hormone), menopausal disorders, indefinite complaint, sex hormone-dependent disorders such as calcium phosphor bone metabolic disorders. It is also applicable for contraception (or infertility when rebound effects after drug cessation are used), and so on.

Moreover, metastin per se or metastin-encoding DNA, etc. are also useful as agents for suppressing gonadotropic hormone secretion or for suppressing sex hormone secretion; down-regulating agents for gonadotropic hormone or sex hormone; down-regulating agents for human OT7T175 (metastin receptor) protein consisting of the amino acid sequence represented by SEQ ID NO: 9; agents for preventing or treating hormone-dependent cancers (e.g., prostate cancer, breast cancer, etc.; particularly, hormone-sensitive prostate cancer, hormone-sensitive breast cancer, etc.); agents for preventing or treating endometriosis; agents for inhibiting ovarian follicular maturation; menstrual cycle-suspending agents; agents for treating hysteromyoma; agents for treating precocious puberty; contraceptives, etc.

BRIEF DESCRIPTION OF THE DRAWINGS

In Fig. 1, A shows chronological LH concentration in the plasma for compound 1 and metastin (45-54). The vertical axis represents LH concentration in the plasma, and the horizontal axis represents time after initiation of administration. B shows area under the curve (AUC) of LH. The vertical axis represents AUC of LH, and the horizontal axis represents a dose. The symbol * indicates a significant difference ($p \leq 0.025$, unilateral Williams test) in comparison with the control group (0 nmol/kg), and the symbol # indicates a significant difference ($p \leq 0.025$, unilateral Shirley-Williams test) in comparison with the control group.

In Fig. 2, A shows chronological LH concentration in the plasma for compound 3 and metastin (45-54). The vertical axis represents LH concentration in the plasma, and the horizontal axis represents time after initiation of administration. B shows area under the curve (AUC) of LH. The vertical axis represents AUC of LH, and the horizontal axis represents a dose.

Fig. 3 shows area under the curve (AUC) of LH for compounds 8-11 and metastin (45-54). The vertical axis represents AUC of LH, and the horizontal axis represents a dose.

In Fig. 4, A shows chronological total testosterone concentration in the plasma for compound 1 and metastin (45-54). The vertical axis represents total testosterone concentration in the plasma, and the horizontal axis represents time after initiation of administration. B shows area under the curve (AUC) of total testosterone. The vertical axis represents AUC of total testosterone, and the horizontal axis represents a dose. The symbol # indicates a significant difference ($p \leq 0.025$, unilateral Shirley-Williams test) in comparison with the control group.

In Fig. 5, A shows chronological total testosterone concentration in the plasma for compound 3 and metastin (45-54). The vertical axis represents total testosterone concentration in the plasma, and the horizontal axis represents time after initiation of administration. B shows area under the curve (AUC) of total testosterone. The vertical axis represents AUC of total testosterone, and the horizontal axis represents a dose.

Fig. 6 shows area under the curve (AUC) of total testosterone for compounds 8-11 and metastin (45-54). The vertical axis represents AUC of total testosterone, and the horizontal axis represents a dose.

MODE FOR CARRYING OUT THE INVENTION

A metastin derivative in the present invention broadly encompasses a partial peptide of metastin per se and metastin derivatives in which a specific moiety of metastin is substituted or added with other amino acids, etc. For

example, examples of the metastin derivative in the present invention include the metastin derivatives described in WO 2004-063221, WO 2006-001499, WO 2007-072997, WO 2008-050897 or Japanese Patent Application No. 2008-114480.

In addition, furthermore, examples of the metastin derivative in the present invention also include the metastin derivatives described in WO 2004-060264, WO 2007-125619 or PCT/JP2009/05810212.

Specifically, examples of the compounds described in WO 2004/063221 include, for example, metastin derivatives (I) represented by the formula described below or a salt thereof (which corresponds to the metastin derivative represented by formula (1) in the present specification or salt thereof):

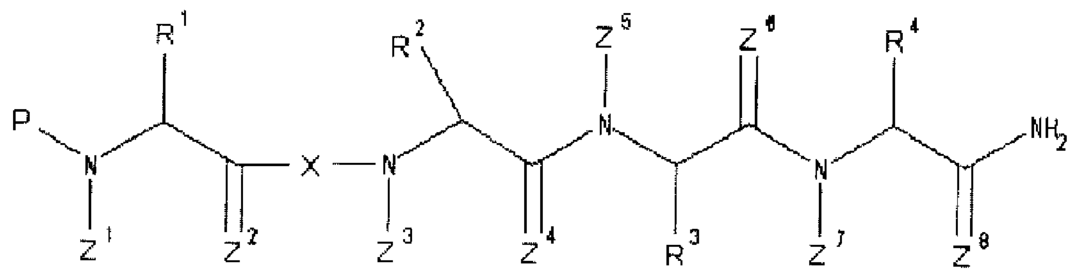

[wherein,

each of $Z^1$, $Z^3$, $Z^5$ and $Z^7$ represents hydrogen atom or a $C_{1-3}$ alkyl group; each of $Z^2$, $Z^4$, $Z^6$ and $Z^8$ represents hydrogen atom, O or S;

$R^1$ represents (1) hydrogen atom, or (2) a $C_{1-8}$ alkyl group optionally substituted with a substituent selected from the group consisting of an optionally substituted carbamoyl group, an optionally substituted hydroxyl group and an optionally substituted aromatic cyclic group;

$R^2$ represents (1) hydrogen atom or (2) a cyclic or linear $C_{1-10}$ alkyl group, or (3) a $C_{1-10}$ alkyl group consisting of a cyclic alkyl group and a linear alkyl group;

$R^3$ represents:

(1) a $C_{1-8}$ alkyl group having an optionally substituted basic group and optionally having an additional substituent,
(2) an aralkyl group having an optionally substituted basic group and optionally having an additional substituent,
(3) a $C_{1-4}$ alkyl group having a non-aromatic cyclic hydrocarbon group of carbon atoms not greater than 7 having an optionally substituted basic group, and optionally having an additional substituent, or,
(4) a $C_{1-4}$ alkyl group having a non-aromatic heterocyclic group of carbon atoms not greater than 7 having an optionally substituted basic group, and optionally having an additional substituent;

$R^4$ represents a $C_{1-4}$ alkyl group, which may optionally be substituted with a substituent selected from the group consisting of:

(1) an optionally substituted $C_{6-12}$ aromatic hydrocarbon group,
(2) an optionally substituted 5- to 14-membered aromatic heterocyclic group consisting of 1 to 7 carbon atoms and hetero atoms selected from the group consisting of nitrogen, oxygen and sulfur atoms,
(3) an optionally substituted $C_{8-14}$ aromatic fused-ring group,
(4) an optionally substituted 5- to 14-membered aromatic fused heterocyclic group consisting of 3 to 11 carbon atoms and hetero atoms selected from the group consisting of nitrogen, oxygen and sulfur atoms,
(5) an optionally substituted non-aromatic cyclic hydrocarbon group having carbon atoms not greater than 7, and,
(6) an optionally substituted non-aromatic heterocyclic group having carbon atoms not greater than 7;

X represents a group shown by formula: $-NHCH(Q^1)YQ^2C(=Z^9)-$ (wherein, $Q^1$ represents a $C_{1-4}$ alkyl group, which may optionally be substituted with a substituent selected from the group consisting of:

(1) an optionally substituted $C_{6-12}$ aromatic hydrocarbon group,
(2) an optionally substituted 5- to 14-membered aromatic heterocyclic group consisting of 1 to 7 carbon atoms and hetero atoms selected from the group consisting of nitrogen, oxygen and sulfur atoms,
(3) an optionally substituted $C_{8-14}$ aromatic fused-ring group,
(4) an optionally substituted 5- to 14-membered aromatic fused heterocyclic group consisting of 3 to 11 carbon atoms and hetero atoms selected from the group consisting of nitrogen, oxygen and sulfur atoms,
(5) an optionally substituted non-aromatic cyclic hydrocarbon group having carbon atoms not greater than 7, and,

(6) an optionally substituted non-aromatic heterocyclic group having carbon atoms not greater than 7;

$Q^2$ represents (1) $CH_2$, which may optionally be substituted with a $C_{1-4}$ alkyl group optionally substituted with a substituent selected from the group consisting of carbamoyl group and hydroxyl group, (2) NH, which may optionally be substituted with a $C_{1-4}$ alkyl group optionally substituted with a substituent selected from the group consisting of carbamoyl group and hydroxyl group, or (3) O;
Y represents a group shown by formula: -CONH-, -CSNH-, $-CH_2NH-$,-NHCO-, $-CH_2O-$, $-CH_2S-$ or $-CH_2CH_2-$, which may optionally be substituted with a $C_{1-6}$ alkyl group; and,
$Z^9$ represents hydrogen atom, O or S); and,
P represents:

(1) hydrogen atom;
(2) an optional amino acid residue continuously or discontinuously bound from the C-terminal end of the 1-48 amino acid sequence in the amino acid sequence represented by SEQ ID NO: 1;
(3) a group represented by formula:

$$J^1\text{-}J^2\text{-}C(J^3)(Q^3)Y^1C(J^4)(Q^4)Y^2C(J^5)(Q^5)Y^3C(J^6)(Q^6)C(=Z^{10})\text{-}$$

(wherein,

$J^1$ represents (a) hydrogen atom or (b) (i) a $C_{1-15}$ acyl group, (ii) a $C_{1-15}$ alkyl group, (iii) a $C_{6-14}$ aryl group, (iv) a carbamoyl group, (v) a carboxyl group, (vi) a sulfino group, (vii) an amidino group or (viii) a glyoxyloyl group, which group may optionally be substituted with a substituent containing an optionally substituted cyclic group;
$J^2$ represents (1) NH optionally substituted with a $C_{1-6}$ alkyl group, (2) $CH_2$ optionally substituted with a $C_{1-6}$ alkyl group, (3) O or (4) S;
each of $J^3$ through $J^6$ represents hydrogen atom or a $C_{1-3}$ alkyl group;
each of $Q^3$ through $Q^6$ represents a $C_{1-4}$ alkyl group, which may optionally be substituted with a substituent selected from the group consisting of:

(1) an optionally substituted $C_{6-12}$ aromatic hydrocarbon group,
(2) an optionally substituted 5- to 14-membered aromatic heterocyclic group consisting of 1 to 7 carbon atoms and hetero atoms selected from the group consisting of nitrogen, oxygen and sulfur atoms,
(3) an optionally substituted $C_{8-14}$ aromatic fused-ring group,
(4) an optionally substituted 5- to 14-membered aromatic fused heterocyclic group consisting of 3 to 11 carbon atoms and hetero atoms selected from the group consisting of nitrogen, oxygen and sulfur atoms,
(5) an optionally substituted non-aromatic cyclic hydrocarbon group having carbon atoms not greater than 7,
(6) an optionally substituted non-aromatic heterocyclic group having carbon atoms not greater than 7,
(7) an optionally substituted amino group,
(8) an optionally substituted guanidino group,
(9) an optionally substituted hydroxyl group,
(10) an optionally substituted carboxyl group,
(11) an optionally substituted carbamoyl group, and
(12) an optionally substituted sulfhydryl group,

or hydrogen atom;
$J^3$ and $Q^3$, $J^4$ and $Q^4$, $J^5$ and $Q^5$ or $J^6$ and $Q^6$ may be combined together, or, $J^2$ and $Q^3$, $Y^1$ and $Q^4$, $Y^2$ and $Q^5$, or $Y^3$ and $Q^6$ may be combined together, to form a ring;
each of $Y^1$ through $Y^3$ represents a group represented by formula: $-CON(J^{13})-$, $-CSN(J^{13})-$, $-C(J^{14})N(J^{13})-$ or $-N(J^{13})CO-$ (wherein each of $J^{13}$ and $J^{14}$ represents hydrogen atom or a $C_{1-3}$ alkyl group); and,
$Z^{10}$ represents hydrogen atom, O or S);

(4) a group represented by formula:

$$J^1\text{-}J^2\text{-}C(J^7)(Q^7)Y^2C(J^8)(Q^8)Y^3C(J^9)(Q^9)C(=Z^{10})\text{-}$$

(wherein,

$J^1$ and $J^2$ have the same significance as described above;
$J^7$ through $J^9$ have the same significance as $J^3$;
$Q^7$ through $Q^9$ have the same significance as $Q^3$;
$Y^2$ and $Y^3$ have the same significance as described above;
$Z^{10}$ has the same significance as described above;
$J^7$ and $Q^7$, $J^8$ and $Q^8$ or $J^9$ and $Q^9$ may be combined together, or, $J^2$ and $Q^7$, $Y^2$ and $Q^8$ or $Y^3$ and $Q^9$ may be combined together, to form a ring);

(5) a group represented by formula:

$$J^1\text{-}J^2\text{-}C(J^{10})(Q^{10})Y^3C(J^{11})(Q^{11})C(=Z^{10})\text{-}$$

(wherein,

$J^1$ and $J^2$ have the same significance as described above;
$J^{10}$ and $J^{11}$ have the same significance as $J^3$;
$Q^{10}$ and $Q^{11}$ have the same significance as $Q^3$;
$Y^3$ has the same significance as described above;
$Z^{10}$ has the same significance as described above; and,
$J^{10}$ and $Q^{10}$ or $J^{11}$ and $Q^{11}$ may be combined together, or $J^2$ and $Q^{10}$ or $Y^3$ and $Q^{11}$ may be combined together, to form a ring);

(6) a group represented by formula: $J^1\text{-}J^2\text{-}C(J^{12})(Q^{12})C(=Z^{10})\text{-}$
(wherein,

$J^1$ and $J^2$ have the same significance as described above;
$J^{12}$ has the same significance as $J^3$;
$Q^{12}$ has the same significance as $Q^3$;
$Z^{10}$ has the same significance as described above; and,
$J^{12}$ and $Q^{12}$ may be combined together, or $J^2$ and $Q^{12}$ may be combined together, to form a ring); or,

(7) a group represented by formula: $J^1$- ($J^1$ represents the same as those described above).]. Among them, preferable metastin derivatives are as follows:

(i) D-Tyr-Asn-Trp-Asn-Ser-Phe-Gly-Leu-Arg(Me)-Phe-$NH_2$ (Compound No. 141),
(ii) D-Tyr-Asn-Trp-Asn-Ser-Phe-Gly-Leu-Arg(Me)-Trp-$NH_2$ (Compound No. 174),
(iii) 3-(3-Indolyl)propionyl-Asn-Ser-Phe-AzaGly-Leu-Arg(Me)-Phe-$NH_2$ (Compound No. 260),
(iv) 3-Phenylpropionyl-Asn-Ser-Phe-AzaGly-Leu-Arg(Me)-Phe-$NH_2$ (Compound No. 269),
(v) 2-(indol-3-yl)ethylcarbamoyl-Asn-Ser-Phe-AzaGly-Leu-Arg(Me)-Phe-$NH_2$ (Compound No. 279),
(vi) D-Tyr-Asn-Pya(4)-Asn-Ser-Phe-AzaGly-Leu-Arg(Me)-Phe-$NH_2$ (Compound No. 286),
(vii) D-Tyr-Asn-Trp-Asn-Ser-Phe$\Psi$(CSNH)Gly-Leu-Arg(Me)-Phe-$NH_2$ (Compound No. 296),
(viii) Tyr$\Psi$($CH_2NH$)Asn-D-Trp-Asn-Ser-Phe-AzaGly-Leu-Arg(Me)-Phe-$NH_2$ (Compound No. 300),
(ix) D-Tyr-D-Asn-Pya(4)-Asn-Ser-Phe-AzaGly-Leu-Arg(Me)-Phe-$NH_2$ (Compound No. 303),
(x) D-Tyr-D-Pya(4)-Asn-Ser-Phe-AzaGly-Leu-Arg(Me)-Phe-$NH_2$ (Compound No. 305),
(xi) D-Tyr-Asn-Trp-Asn-Ser-Phe-AzaGly-Leu-Arg(Me)-Phe(4F)-$NH_2$ (Compound No. 318),
(xii) D-Tyr-Asn-Trp-Asn-Ser-Phe$\Psi$(NHCO)Gly-Leu-Arg(Me)-Phe-$NH_2$ (Compound No. 319),
(xiii) 3-Pyridylpropionyl-Asn-Ser-Phe-AzaGly-Leu-Arg(Me)-Phe-$NH_2$ (Compound No. 322),
(xiv)4-Imidazoleacetyl-Asn-Ser-Phe-AzaGly-Leu-Arg(Me)-Phe-$NH_2$ (Compound No. 323),
(xv) D-Tyr-D-Pya(4)-Asn-Ser-Phe-AzaGly-Leu-Arg(Me)-Trp-$NH_2$ (Compound No. 385), or
(xvi) D-Tyr-D-Pya(4)-Asn-Ser-Phe-AzaGly-Leu-Arg(Me)-Trp-$NH_2$ (Compound No. 386), etc.

Examples of the compounds described in WO 2006/001499 include, for example, metastin derivatives (II) represented by the formula described below or a salt thereof (which corresponds to the metastin derivative represented by formula (2) in the present specification or a salt thereof):

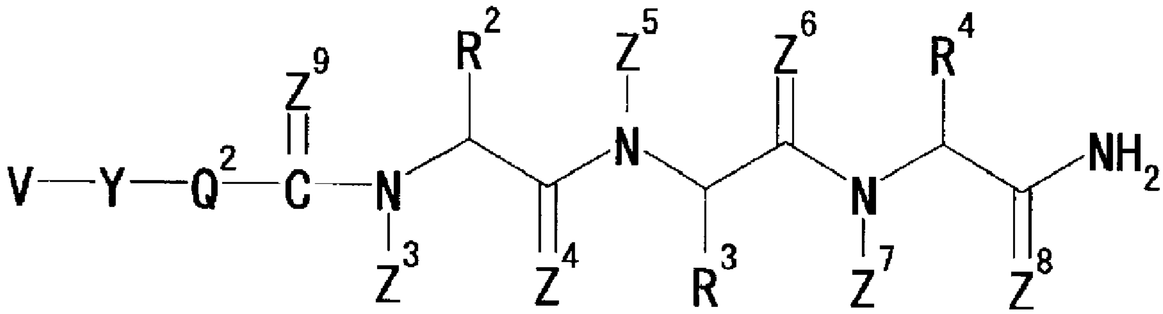

[wherein;

V represents a group represented by formula:

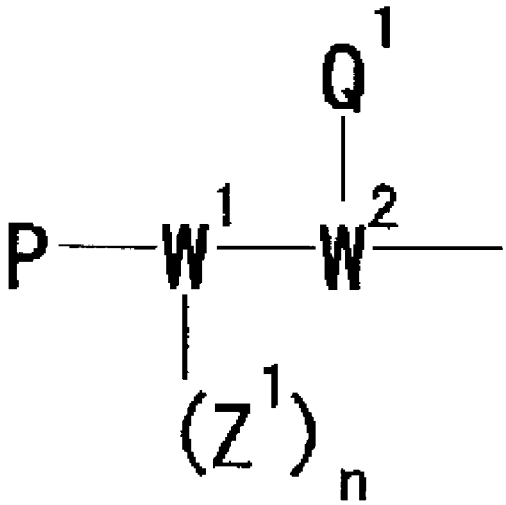

or a group represented by formula:

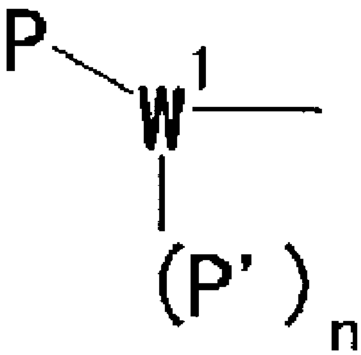

n represents 0 or 1;
$W^1$ represents N, CH or O (provided that when $W^1$ is N or CH, n represents 1 and when $W^1$ is O, n represents 0);

$W^2$ represents N or CH;
$Z^1$, $Z^3$, $Z^5$ and $Z^7$ each represents hydrogen atom or a $C_{1-3}$ alkyl group;
$Z^4$, $Z^6$ and $Z^8$ each represents hydrogen atom, O or S;
$R^2$ represents (1) hydrogen atom or (2) a cyclic or linear $C_{1-10}$ alkyl group, (3) a $C_{1-10}$ alkyl group consisting of a cyclic alkyl group and a linear alkyl group, or (4) a $C_{1-8}$ alkyl group optionally substituted with a substituent selected from the group consisting of an optionally substituted carbamoyl group, an optionally substituted hydroxyl group

and an optionally substituted aromatic cyclic group;
$R^3$ represents (1) a $C_{1-8}$ alkyl group having an optionally substituted basic group and optionally having an additional substituent, (2) an aralkyl group having an optionally substituted basic group and optionally having an additional substituent, (3) a $C_{1-4}$ alkyl group having a non-aromatic cyclic hydrocarbon group of carbon atoms not greater than 7 having an optionally substituted basic group, and optionally having an additional substituent, or (4) a $C_{1-4}$ alkyl group having a non-aromatic heterocyclic group of carbon atoms not greater than 7 having an optionally substituted basic group, and optionally having an additional substituent;
$R^4$ represents a $C_{1-4}$ alkyl group, which may optionally be substituted with a substituent selected from the group consisting of (1) an optionally substituted $C_{6-12}$ aromatic hydrocarbon group, (2) an optionally substituted 5- to 14-membered aromatic heterocyclic group consisting of 1 to 7 carbon atoms and hetero atoms selected from the group consisting of nitrogen, oxygen and sulfur atoms, (3) an optionally substituted $C_{8-14}$ aromatic fused-ring group, (4) an optionally substituted 5- to 14-membered aromatic fused heterocyclic group consisting of 3 to 11 carbon atoms and hetero atoms selected from the group consisting of nitrogen, oxygen and sulfur atoms, (5) an optionally substituted non-aromatic cyclic hydrocarbon group having carbon atoms not greater than 7, and (6) an optionally substituted non-aromatic heterocyclic group having carbon atoms not greater than 7;
$Q^1$ represents a $C_{1-4}$ alkyl group, which may optionally be substituted with a substituent selected from the group consisting of (1) an optionally substituted $C_{6-12}$ aromatic hydrocarbon group, (2) an optionally substituted 5- to 14-membered aromatic heterocyclic group consisting of 1 to 7 carbon atoms and hetero atoms selected from the group consisting of nitrogen, oxygen and sulfur atoms, (3) an optionally substituted $C_{8-14}$ aromatic fused-ring group, (4) an optionally substituted 5- to 14-membered aromatic fused heterocyclic group consisting of 3 to 11 carbon atoms and hetero atoms selected from the group consisting of nitrogen, oxygen and sulfur atoms, (5) an optionally substituted non-aromatic cyclic hydrocarbon group having carbon atoms not greater than 7, and (6) an optionally substituted non-aromatic heterocyclic group having carbon atoms not greater than 7;
$Q^2$ represents (1) $CH_2$, which may optionally be substituted with an optionally substituted $C_{1-4}$ alkyl group with a substituent selected from the group consisting of carbamoyl group and hydroxyl group, (2) NH, which may optionally be substituted with an optionally substituted $C_{1-4}$ alkyl group with a substituent selected from the group consisting of carbamoyl group and hydroxyl group, or (3) O;
Y represents a group represented by formula: -CONH-, -CSNH-,$-CH_2NH-$, -NHCO-, $-CH_2O-$, $-CH_2S-$, -COO-, -CSO- or $-CH_2CH_2-$, which may optionally be substituted with a $C_{1-6}$ alkyl group; and,
$Z^9$ represents hydrogen atom, O or S; and,
P and P', which may be the same or different, each may form a ring by combining P and P' or P and $Q^1$ together and represents:

(1) hydrogen atom;
(2) an optional amino acid residue continuously or discontinuously bound from the C terminus of the 1-48 amino acid sequence in the amino acid sequence represented by SEQ ID NO: 1;
(3) a group represented by formula:

$$J^1\text{-}J^2\text{-}C(J^3)(Q^3)Y^1C(J^4)(Q^4)Y^2C(J^5)(Q^5)Y^3C(J^6)(Q^6)C(=Z^{10})\text{-}$$

(wherein:

$J^1$ represents (a) hydrogen atom or (b) (i) a $C_{1-5}$ acyl group, (ii) a $C_{1-15}$ alkyl group, (iii) a $C_{6-14}$ aryl group, (iv) carbamoyl group, (v) carboxyl group, (vi) sulfino group, (vii) amidino group, (viii) glyoxyloyl group or (ix) amino group, wherein the groups may optionally be substituted with a substituent containing an optionally substituted cyclic group;
$J^2$ represents (1) NH optionally substituted with a $C_{1-6}$ alkyl group, (2) $CH_2$ optionally substituted with a $C_{1-6}$ alkyl group, (3) O or (4) S;
$J^3$ through $J^6$ each represents hydrogen atom or a $C_{1-3}$ alkyl group;
$Q^3$ through $Q^6$ each represents a $C_{1-4}$ alkyl group, which may optionally have a substituent selected from the group consisting of:

(1) an optionally substituted $C_{6-12}$ aromatic hydrocarbon group,
(2) an optionally substituted 5- to 14-membered aromatic heterocyclic group consisting of 1 to 7 carbon atoms and hetero atoms selected from the group consisting of nitrogen, oxygen and sulfur atoms,
(3) an optionally substituted $C_{8-14}$ aromatic fused-ring group,
(4) an optionally substituted 5- to 14-membered aromatic fused heterocyclic group consisting of 3 to 11 carbon atoms and hetero atoms selected from the group consisting of nitrogen, oxygen and sulfur

atoms,

(5) an optionally substituted non-aromatic cyclic hydrocarbon group having carbon atoms not greater than 7,

(6) an optionally substituted non-aromatic heterocyclic group having carbon atoms not greater than 7,

(7) an optionally substituted amino group,

(8) an optionally substituted guanidino group,

(9) an optionally substituted hydroxyl group,

(10) an optionally substituted carboxyl group,

(11) an optionally substituted carbamoyl group, and

(12) an optionally substituted sulfhydryl group,

or hydrogen atom;

$J^3$ and $Q^3$, $J^4$ and $Q^4$, $J^5$ and $Q^5$ or $J^6$ and $Q^6$ may be combined together, or, $J^2$ and $Q^3$, $Y^1$ and $Q^4$, $Y^2$ and $Q^5$, or $Y^3$ and $Q^6$ may be combined together, to form a ring;

$Y^1$ through $Y^3$ each represents a group represented by formula: $-CON(J^{13})-$, $-CSN(J^{13})-$, $-C(J^{14})N(J^{13})-$ or $-N(J^{13})CO-$ (wherein $J^{13}$ and $J^{14}$ each represents hydrogen atom or a $C_{1-3}$ alkyl group); and,

$Z^{10}$ represents hydrogen atom, O or S);

(4) a group represented by formula:

$$J^1\text{-}J^2\text{-}C(J^7)(Q^7)Y^2C(J^8)(Q^8)Y^3C(J^9)(Q^9)C(=Z^{10})\text{-}$$

(wherein:

$J^1$ and $J^2$, each has the same significance as defined above;

$J^7$ through $J^9$ have the same significance as for $J^3$;

$Q^7$ through $Q^9$ have the same significance as for $Q^3$;

$Y^2$ and $Y^3$ each has the same significance as defined above;

$Z^{10}$ has the same significance as defined above;

$J^7$ and $Q^7$, $J^8$ and $Q^8$ or $J^9$ and $Q^9$ may be combined together, or, $J^2$ and $Q^7$, $Y^2$ and $Q^8$ or $Y^3$ and $Q^9$ may be combined together, to form a ring);

(5) a group represented by formula:

$$J^1\text{-}J^2\text{-}C(J^{10})(Q^{10})Y^3C(J^{11})(Q^{11})C(=Z^{10})\text{-}$$

(wherein:

$J^1$ and $J^2$ have the same significance as defined above;

$J^{10}$ and $J^{11}$ have the same significance as for $J^3$;

$Q^{10}$ and $Q^{11}$ have the same significance as for $Q^3$;

$Y^3$ has the same significance as defined above;

$Z^{10}$ has the same significance as defined above; and,

$J^{10}$ and $Q^{10}$ or $J^{11}$ and $Q^{11}$ may be combined together, or $J^2$ and $Q^{10}$ or $Y^3$ and $Q^{11}$ may be combined together, to form a ring);

(6) a group represented by formula:

$$J^1\text{-}J^2\text{-}C(J^{12})(Q^{12})C(=Z^{10})\text{-}$$

(wherein;

$J^1$ and $J^2$ have the same significance as defined above;

$J^{12}$ has the same significance as for $J^3$;

$Q^{12}$ has the same significance as for $Q^3$;

$Z^{10}$ has the same significance as defined above; and,

$J^{12}$ and $Q^{12}$ may be combined together, or $J^2$ and $Q^{12}$ may be combined together, to form a ring); or,

(7) a group represented by formula $J^1$- ($J^1$ represents the same as those described above).]. Among them, preferable metastin derivatives are as follows:

D-Tyr-D-Pya(4)-Asn-Ser-Phe-AzaGly-Leu-Arg(Me)-Phe-$NH_2$ (Compound No. 305),
D-Tyr-D-Pya(4)-Asn-Ser-Phe-AzaGly-Leu-Arg(Me)-Trp-$NH_2$ (Compound No. 385),
D-Tyr-D-Pya(4)-Asn-Thr-Phe-AzaGly-Leu-Arg(Me)-Trp-$NH_2$ (Compound No. 501),
Benzoyl-Asn-Ser-Phe-AzaGly-Leu-Arg(Me)-Trp-$NH_2$ (Compound No. 509),
D-Tyr-D-Trp-Asn-Thr-Phe-AzaGly-Leu-Arg(Me)-Trp-$NH_2$ (Compound No. 512),
Ac-D-Tyr-D-Pya(4)-Asn-Ser-Phe-AzaGly-Leu-Arg(Me)-Phe-$NH_2$ (Compound No. 516),
D-Tyr-D-Trp-Asn-Ser-Phe-AzaGly-Leu-Arg(Me)-Trp-$NH_2$ (Compound No. 540),
D-Arg-Acp-D-Tyr-D-Trp-Asn-Thr-Phe-AzaGly-Leu-Arg(Me)-Trp-$NH_2$ (Compound No. 541),
Benzoyl-Asn-Ser-Phe(4F)-AzaGly-Leu-Arg(Me)-Trp-$NH_2$ (Compound No. 545),
D-Tyr-D-Pya(4)-Asn-Ser-Phe-AzaGly-Leu-Arg(Me)-Phe$\Psi$(CSNH)$NH_2$ (Compound No. 548),
Ac-D-Tyr-D-Trp-Asn-Thr-Phe-AzaGly-Leu-Arg(Me)-Trp-$NH_2$ (Compound No. 550),
Ac-D-Arg-Acp-D-Tyr-D-Trp-Asn-Thr-Phe-AzaGly-Leu-Arg(Me)-Trp-$NH_2$ (Compound No. 551),
D-Dap-Acp-D-Tyr-D-Trp-Asn-Thr-Phe-AzaGly-Leu-Arg(Me)-Trp-$NH_2$ (Compound No. 552),
D-Nle-Acp-D-Tyr-D-Trp-Asn-Thr-Phe-AzaGly-Leu-Arg(Me)-Trp-$NH_2$ (Compound No. 553),
D-Arg-$\gamma$-Abu-D-Tyr-D-Trp-Asn-Thr-Phe-AzaGly-Leu-Arg(Me)-Trp-$NH_2$ (Compound No. 555),
Ac-D-Tyr-D-Trp-Asn-Ser-Phe-AzaGly-Leu-Arg(Me)-Trp-$NH_2$ (Compound No. 558),
3-(4-Hydroxyphenyl)propionyl-D-Trp-Asn-Thr-Phe-AzaGly-Leu-Arg(Me)-Trp-$NH_2$ (Compound No. 559),
Ac-D-Tyr-D-Pya(4)-Asn-Thr-Phe-AzaGly-Leu-Arg(Me)-Trp-$NH_2$ (Compound No. 562),
Ac-D-Tyr-D-Trp-Asn-Val-Phe-AzaGly-Leu-Arg(Me)-Trp-$NH_2$ (Compound No. 564),
Cyclopropanecarbonyl-D-Tyr-D-Trp-Asn-Thr-Phe-AzaGly-Leu-Arg(Me)-Trp-$NH_2$ (Compound No. 566),
Butyryl-D-Tyr-D-Trp-Asn-Thr-Phe-AzaGly-Leu-Arg(Me)-Trp-$NH_2$ (Compound No. 567),
Ac-D-Tyr-D-Trp-Asn-Thr-Phe-AzaGly-Leu-Arg(Me)-Phe-$NH_2$ (Compound No. 571),
Ac-D-Tyr-D-Trp-Alb-Ser-Phe-AzaGly-Leu-Arg(Me)-Trp-$NH_2$ (Compound No. 579),
Ac-D-Tyr-D-Trp-Asn-Ser(Me)-Phe-AzaGly-Leu-Arg(Me)-Trp-$NH_2$ (Compound No. 580),
Ac-D-Tyr-D-Trp-Dap(Ac)-Ser-Phe-AzaGly-Leu-Arg(Me)-Trp-$NH_2$ (Compound No. 584),
Ac-D-Tyr-D-Trp-Dap(For)-Ser-Phe-AzaGly-Leu-Arg(Me)-Trp-$NH_2$ (Compound No. 585),
Ac-D-Tyr-D-Trp-Asn-Thr-Phe-AzaGly-Leu-Arg(Me)-Nal(2)-$NH_2$ (Compound No. 589),
Ac-D-Tyr-D-Trp-Asn-Thr-Phe-AzaGly-Leu-Arg(Me)-Thi-$NH_2$ (Compound No. 590),
Ac-D-Tyr-D-Trp-Asn-Thr-Phe-AzaGly-Leu-Arg(Me)-Tyr-$NH_2$ (Compound No. 591),
Ac-D-Tyr-D-Trp-Asn-Thr-Phe-AzaGly-Leu-Arg(Me)-Phe(4F)-$NH_2$ (Compound No. 592),
Ac-D-Tyr-D-Trp-Asn-Thr-Phe-AzaGly-Leu-Orn-Trp-$NH_2$ (Compound No. 599),
Ac-D-Tyr-D-Trp-Asn-Thr-Phe-AzaGly-Leu-Arg-Trp-NH2 (Compound No. 600),
Ac-D-NMeTyr-D-Trp-Asn-Thr-Phe-AzaGly-Leu-Arg(Me)-Trp-$NH_2$ (Compound No. 602),
Ac-D-Tyr-D-Trp-Asn-Thr-Phe-AzaGly-Leu-Arg(symMe2)-Trp-$NH_2$ (Compound No. 608),
For-D-Tyr-D-Trp-Asn-Thr-Phe-AzaGly-Leu-Arg(Me)-Trp-$NH_2$ (Compound No. 612),
Propionyl-D-Tyr-D-Trp-Asn-Thr-Phe-AzaGly-Leu-Arg(Me)-Trp-$NH_2$ (Compound No. 613),
Ac-Tyr-D-Trp-Asn-Thr-Phe-AzaGly-Leu-Arg(Me)-Trp-$NH_2$ (Compound No. 615),
Ac-D-Ala-D-Trp-Asn-Thr-Phe-AzaGly-Leu-Arg(Me)-Trp-$NH_2$ (Compound No. 616),
Ac-D-Leu-D-Trp-Asn-Thr-Phe-AzaGly-Leu-Arg(Me)-Trp-$NH_2$ (Compound No. 617),
Ac-D-Phe-D-Trp-Asn-Thr-Phe-AzaGly-Leu-Arg(Me)-Trp-$NH_2$ (Compound No. 618),
Ac-D-Lys-D-Trp-Asn-Thr-Phe-AzaGly-Leu-Arg(Me)-Trp-$NH_2$ (Compound No. 621),
Ac-D-Tyr-Trp-Asn-Thr-Phe-AzaGly-Leu-Arg(Me)-Trp-$NH_2$ (Compound No. 623),
Ac-D-Tyr-D-Ala-Asn-Thr-Phe-AzaGly-Leu-Arg(Me)-Trp-$NH_2$ (Compound No. 625),
Ac-D-Tyr-D-Leu-Asn-Thr-Phe-AzaGly-Leu-Arg(Me)-Trp-$NH_2$ (Compound No. 626),
Ac-D-Tyr-D-Phe-Asn-Thr-Phe-AzaGly-Leu-Arg(Me)-Trp-$NH_2$ (Compound No. 627),
Ac-D-Tyr-D-Lys-Asn-Thr-Phe-AzaGly-Leu-Arg(Me)-Trp-$NH_2$ (Compound No. 629),
Ac-D-Tyr-D-Glu-Asn-Thr-Phe-AzaGly-Leu-Arg(Me)-Trp-$NH_2$ (Compound No. 630),
Ac-D-Tyr-D-Trp-Asn-Thr-Pya(4)-AzaGly-Leu-Arg(Me)-Trp-$NH_2$ (Compound No. 635),
Ac-D-Tyr-D-Trp-Asn-Thr-Phe(4F)-AzaGly-Leu-Arg(Me)-Trp-$NH_2$ (Compound No. 637),
Ac-D-Tyr-D-Pya(4)-Asn-Thr-Phe(4F)-AzaGly-Leu-Arg(Me)-Trp-$NH_2$ (Compound No. 638),
Ac-D-Tyr-D-Trp-Asn-Thr-Phe-AzaGly-Val-Arg(Me)-Trp-$NH_2$ (Compound No. 642),
Gly-D-Tyr-D-Trp-Asn-Thr-Phe-AzaGly-Leu-Arg(Me)-Trp-$NH_2$ (Compound No. 648),
Ac-Gly-D-Tyr-D-Trp-Asn-Thr-Phe-AzaGly-Leu-Arg(Me)-Trp-$NH_2$ (Compound No. 649),
D-Tyr-D-Tyr-D-Trp-Asn-Thr-Phe-AzaGly-Leu-Arg(Me)-Trp-$NH_2$ (Compound No. 650),
Ac-D-Tyr-D-Tyr-D-Trp-Asn-Thr-Phe-AzaGly-Leu-Arg(Me)-Trp-$NH_2$ (Compound No. 651),

pGlu-D-Tyr-D-Trp-Asn-Thr-Phe-AzaGly-Leu-Arg(Me)-Trp-$NH_2$ (Compound No. 652), Ac-D-Tyr-Pro-Asn-Thr-Phe-AzaGly-Leu-Arg(Me)-Trp-$NH_2$ (Compound No. 657), Ac-D-Tyr-D-Pya(2)-Asn-Thr-Phe-AzaGly-Leu-Arg(Me)-Trp-$NH_2$ (Compound No. 658), Ac-D-Tyr-D-Pya(3)-Asn-Thr-Phe-AzaGly-Leu-Arg(Me)-Trp-$NH_2$ (Compound No. 660), Ac-D-Tyr-Tic-Asn-Thr-Phe-AzaGly-Leu-Arg(Me)-Trp-$NH_2$ (Compound No. 662), Ac-D-Trp-D-Trp-Asn-Thr-Phe-AzaGly-Leu-Arg(Me)-Trp-$NH_2$ (Compound No. 663), Ac-D-Trp-Asn-Thr-Phe-AzaGly-Leu-Arg(Me)-Trp-$NH_2$ (Compound No. 666), Hexanoyl-D-Trp-Asn-Thr-Phe-AzaGly-Leu-Arg(Me)-Trp-$NH_2$ (Compound No. 667), 3-Pyridinepropionyl-D-Trp-Asn-Thr-Phe-AzaGly-Leu-Arg(Me)-Trp-$NH_2$ (Compound No. 670), Adipoyl-D-Trp-Asn-Thr-Phe-AzaGly-Leu-Arg(Me)-Trp-$NH_2$ (Compound No. 671), Ac-D-Tyr-NMeTrp-Asn-Thr-Phe-AzaGly-Leu-Arg(Me)-Trp-$NH_2$ (Compound No. 672), 6-Aminocaproyl-D-Trp-Asn-Thr-Phe-AzaGly-Leu-Arg(Me)-Trp-$NH_2$ (Compound No. 674), etc.

Examples of the compounds described in WO 2007/072997 include, for example, metastin derivatives represented by the formula described below or a salt thereof (which corresponds to the metastin derivative represented by formula (3) in the present specification or a salt thereof):

XX0-XX2-XX3-XX4-XX5-XX6-AzaGly-XX8-XX9-XX10-$NH_2$

(wherein:

XX0 represents formyl, a $C_{1-20}$ alkanoyl, cyclopropanecarbonyl, 6-(acetyl-D-arginylamino)caproyl, 6-((R)-2,3-diaminopropionylamino)caproyl, 6-(D-norleucylamino)caproyl, 4-(D-arginylamino)butyryl, 3-(4-hydroxyphenyl)propionyl, glycyl, tyrosyl, acetylglycyl, acetyltyrosyl, D-tyrosyl, acetyl-D-tyrosyl, pyroglutamyl, 3-(pyridin-3-yl)propionyl, adipoyl, glycoloyl, 6-aminocaproyl, 6-acetylaminocaproyl, 4-[bis-(2-pyridylmethyl)aminomethyl]benzoyl or 4-ureidobenzoyl;
XX2 represents Tyr, D-Tyr, D-Ala, D-Leu, D-Phe, D-Lys, D-Trp or a chemical bond;
XX3 represents:

i) an amino acid selected from Ala, Arg, Asn, Asp, Cys, Gin, Glu, Gly, His, Ile, Leu, Lys, Met, Phe, Ser, Thr, Trp, Tyr and Val wherein the α-amino group may optionally be methylated;
ii) a cyclic amino acid selected from Pro, Aze(2), Aze(3), Pic(2), Pic(3), Hyp, Thz, Abz(2), Abz(3), Pzc(2), Pro($4NH_2$), Hyp(Bzl), cisHyp, Pro(4F) and Izc;
iii) an amino acid selected from D-Dap, D-Pya(4), DL-Ala(Pip), Orn, Aib and Tyr($PO_3H_2$); or,
iv) a chemical bond;

XX4 represents Asn, 2-amino-3-ureidopropionic acid, $N^{\beta}$-formyl-β-diaminopropionic acid, $N^{\beta}$-acetyl-β-diaminopropionic acid, $N^{\omega}$-pentylasparagine, $N^{\omega}$-cyclopropylasparagine, $N^{\omega}$-benzylasparagine, 2,4-diaminobutanoic acid, 2,3-diaminopropionic acid, His, Gin, Gly, Arg, Cit, Nva, D-Asn or a chemical bond;
XX5 represents Ser, Thr, Val, NMeSer, Gly, Ala, Hyp, D-Ala, D-Thr, D-Pro or a chemical bond;
XX6 represents Phe, Tyr, Trp, Tyr(Me), Thi, Nal(2), Cha, Pya(4), threo-Ser(3Phenyl), erythro-Ser(3Phenyl), or optionally substituted phenylalanine ;
AzaGly represents azaglycine;
XX8 represents Leu, Nva, Val or Ala(cPr);
XX9 represents optionally substituted arginine, optionally substituted lysine or optionally substituted ornithine; and,
XX10 represents 2-naphthylalanine, 2-thienylalanine, tyrosine, optionally substituted phenylalanine or optionally substituted tryptophan). Among them, preferable metastin derivatives are as follows:

Ac-D-Tyr-D-Trp-Asn-Thr-Phe-AzaGly-Leu-D-Arg-Trp-$NH_2$ (Compound No. 708),
Ac-Asn-Thr-Phe-AzaGly-Leu-Arg(Me)-Trp-$NH_2$ (Compound No. 709),
Decanoyl-Asn-Thr-Phe-AzaGly-Leu-Arg(Me)-Trp-$NH_2$ (Compound No. 710),
Acp-Asn-Thr-Phe-AzaGly-Leu-Arg(Me)-Trp-$NH_2$ (Compound No. 712),
Ac-Acp-Asn-Thr-Phe-AzaGly-Leu-Arg(Me)-Trp-$NH_2$ (Compound No. 713),
Ac-D-Tyr-D-Trp-Asp(NHPen)-Thr-Phe-AzaGly-Leu-Arg(Me)-Trp-$NH_2$ (Compound No. 714),
Ac-D-Tyr-D-Trp-Asp(NHcPr)-Thr-Phe-AzaGly-Leu-Arg(Me)-Trp-$NH_2$ (Compound No. 715),
Ac-D-Tyr-D-Trp-Asp(NHBzl)-Thr-Phe-AzaGly-Leu-Arg(Me)-Trp-$NH_2$ (Compound No. 716),
Ac-D-Tyr-D-Trp-Alb-Thr-Phe-AzaGly-Leu-Arg(Me)-Trp-$NH_2$ (Compound No. 717),
Ac-D-Tyr-D-Pya(4)-Alb-Thr-Phe-AzaGly-Leu-Arg(Me)-Trp-$NH_2$ (Compound No. 718),
Ac-D-Tyr-D-Trp-Asn-D-Pro-Phe-AzaGly-Leu-Arg(Me)-Trp-$NH_2$ (Compound No. 719),

Ac-D-Tyr-Aze(2)-Asn-Thr-Phe-AzaGly-Leu-Arg(Me)-Trp-$NH_2$ (Compound No. 720),
Ac-D-Tyr-Pic(2)-Asn-Thr-Phe-AzaGly-Leu-Arg(Me)-Trp-$NH_2$ (Compound No. 721),
Ac-D-Tyr-Pic(3)-Asn-Thr-Phe-AzaGly-Leu-Arg(Me)-Trp-$NH_2$ (Compound No. 722)
Ac-D-Tyr-Hyp-Asn-Thr-Phe-AzaGly-Leu-Arg(Me)-Trp-$NH_2$ (Compound No. 723),
Ac-D-Tyr-Thz-Asn-Thr-Phe-AzaGly-Leu-Arg(Me)-Trp-$NH_2$ (Compound No. 724),
Ac-D-Tyr-NMeAla-Asn-Thr-Phe-AzaGly-Leu-Arg(Me)-Trp-$NH_2$ (Compound No. 725)
Ac-D-Tyr-Gly-Asn-Thr-Phe-AzaGly-Leu-Arg(Me)-Trp-$NH_2$ (Compound No. 726)
Ac-D-Tyr-Aib-Asn-Thr-Phe-AzaGly-Leu-Arg(Me)-Trp-$NH_2$ (Compound No. 727),
Ac-D-Tyr-Abz(2)-Asn-Thr-Phe-AzaGly-Leu-Arg(Me)-Trp-$NH_2$ (Compound No. 728)
Ac-D-Tyr-Aze(3)-Asn-Thr-Phe-AzaGly-Leu-Arg(Me)-Trp-$NH_2$ (Compound No. 730)
Ac-D-Tyr-Sar-Asn-Thr-Phe-AzaGly-Leu-Arg(Me)-Trp-$NH_2$ (Compound No. 731)
Ac-D-Tyr-D-NMeAla-Asn-Thr-Phe-AzaGly-Leu-Arg(Me)-Trp-$NH_2$ (Compound No. 732),
Ac-D-Tyr-Izc-Asn-Thr-Phe-AzaGly-Leu-Arg(Me)-Trp-$NH_2$ (Compound No. 734),
Ac-D-Tyr-D-Asp-Asn-Thr-Phe-AzaGly-Leu-Arg(Me)-Trp-$NH_2$ (Compound No. 735),
Ac-D-Tyr-D-Dap-Asn-Thr-Phe-AzaGly-Leu-Arg(Me)-Trp-$NH_2$ (Compound No. 736),
Ac-D-Tyr-D-Ser-Asn-Thr-Phe-AzaGly-Leu-Arg(Me)-Trp-$NH_2$ (Compound No. 737),
Ac-D-Tyr-D-Gln-Asn-Thr-Phe-AzaGly-Leu-Arg(Me)-Trp-$NH_2$ (Compound No. 738),
Ac-D-Tyr-D-His-Asn-Thr-Phe-AzaGly-Leu-Arg(Me)-Trp-$NH_2$ (Compound No. 739),
Ac-D-Tyr-D-Trp-Dab-Thr-Phe-AzaGly-Leu-Arg(Me)-Trp-$NH_2$ (Compound No. 740),
Ac-D-Tyr-Ala-Asn-Thr-Phe-AzaGly-Leu-Arg(Me)-Trp-$NH_2$ (Compound No. 742),
Ac-D-Tyr-Leu-Asn-Thr-Phe-AzaGly-Leu-Arg(Me)-Trp-$NH_2$ (Compound No. 743),
Ac-D-Tyr-Ser-Asn-Thr-Phe-AzaGly-Leu-Arg(Me)-Trp-$NH_2$ (Compound No. 744),
Ac-D-Tyr-Lys-Asn-Thr-Phe-AzaGly-Leu-Arg(Me)-Trp-$NH_2$ (Compound No. 745),
Ac-D-Tyr-Glu-Asn-Thr-Phe-AzaGly-Leu-Arg(Me)-Trp-$NH_2$ (Compound No. 746),
Ac-D-Tyr-β-Ala-Asn-Thr-Phe-AzaGly-Leu-Arg(Me)-Trp-$NH_2$ (Compound No. 747),
Ac-D-Tyr-D-Trp-Asn-Thr-Phe(4Cl)-AzaGly-Leu-Arg(Me)-Trp-$NH_2$ (Compound No. 748),
Ac-D-Tyr-D-Trp-Asn-Thr-Phe(2F)-AzaGly-Leu-Arg(Me)-Trp-$NH_2$ (Compound No. 749),
Ac-D-Tyr-D-Trp-Asn-Thr-Phe(3F)-AzaGly-Leu-Arg(Me)-Trp-$NH_2$ (Compound No. 750),
Ac-D-Tyr-Lys-Asn-Thr-Phe(2F)-AzaGly-Leu-Arg(Me)-Trp-$NH_2$ (Compound No. 754),
Ac-D-Tyr-Glu-Asn-Thr-Phe(2F)-AzaGly-Leu-Arg(Me)-Trp-$NH_2$ (Compound No. 755),
Ac-D-Tyr-Lys-Asn-Thr-Phe(3F)-AzaGly-Leu-Arg(Me)-Trp-$NH_2$ (Compound No. 756),
Ac-D-Tyr-Glu-Asn-Thr-Phe(3F)-AzaGly-Leu-Arg(Me)-Trp-$NH_2$ (Compound No. 757),
Ac-D-Tyr-Lys-Asn-Thr-Phe(4Cl)-AzaGly-Leu-Arg(Me)-Trp-$NH_2$ (Compound No. 758),
Ac-D-Tyr-Glu-Asn-Thr-Phe(4Cl)-AzaGly-Leu-Arg(Me)-Trp-$NH_2$ (Compound No. 759),
Ac-D-Tyr-Pzc(2)-Asn-Thr-Phe-AzaGly-Leu-Arg(Me)-Trp-$NH_2$ (Compound No. 760),
Ac-D-Tyr-Hyp-Asn-Thr-Phe(2F)-AzaGly-Leu-Arg(Me)-Trp-$NH_2$ (Compound No. 763),
Ac-D-Tyr-Trp-Asn-Thr-Phe(2F)-AzaGly-Leu-Arg(Me)-Trp-$NH_2$ (Compound No. 764),
Ac-D-Tyr-Hyp-Asn-Thr-Phe(3F)-AzaGly-Leu-Arg(Me)-Trp-$NH_2$ (Compound No. 765),
Ac-D-Tyr-Trp-Asn-Thr-Phe(3F)-AzaGly-Leu-Arg(Me)-Trp-$NH_2$ (Compound No. 766),
Ac-D-Tyr-Hyp-Asn-Thr-Phe(4Cl)-AzaGly-Leu-Arg(Me)-Trp-$NH_2$ (Compound No. 767),
Ac-D-Tyr-Trp-Asn-Thr-Phe(4Cl)-AzaGly-Leu-Arg(Me)-Trp-$NH_2$ (Compound No. 768),
Ac-D-Tyr-Gly-Asn-Thr-Phe(4Cl)-AzaGly-Leu-Arg(Me)-Trp-$NH_2$ (Compound No. 769),
Ac-D-Tyr-Aib-Asn-Thr-Phe(4Cl)-AzaGly-Leu-Arg(Me)-Trp-$NH_2$ (Compound No. 770),
Ac-D-Tyr-Orn-Asn-Thr-Phe-AzaGly-Leu-Arg(Me)-Trp-$NH_2$ (Compound No. 771),
Ac-D-Tyr-Thr-Asn-Thr-Phe-AzaGly-Leu-Arg(Me)-Trp-$NH_2$ (Compound No. 772),
Ac-D-Tyr-His(3Me)-Asn-Thr-Phe-AzaGly-Leu-Arg(Me)-Trp-$NH_2$ (Compound No. 773),
Ac-D-Tyr-DL-Ala(Pip)-Asn-Thr-Phe-AzaGly-Leu-Arg(Me)-Trp-$NH_2$ (Compound No. 774),
Ac-D-Tyr-Tyr($PO_3H_2$)-Asn-Thr-Phe-AzaGly-Leu-Arg(Me)-Trp-$NH_2$ (Compound No. 775),
Glycoloyl-D-Tyr-Hyp-Asn-Thr-Phe-AzaGly-Leu-Arg(Me)-Trp-$NH_2$ (Compound No. 776)
Ac-D-Tyr-Asn-Thr-Phe-AzaGly-Leu-Arg(Me)-Trp-$NH_2$ (Compound No. 777),
Ac-D-Tyr-Pro($4NH_2$)-Asn-Thr-Phe-AzaGly-Leu-Arg(Me)-Trp-$NH_2$ (Compound No. 780),
Ac-D-Tyr-Hyp(Bzl)-Asn-Thr-Phe-AzaGly-Leu-Arg(Me)-Trp-$NH_2$ (Compound No. 781),
Ac-D-Tyr-D-NMePhe-Asn-Thr-Phe-AzaGly-Leu-Arg(Me)-Trp-$NH_2$ (Compound No. 782),
Ac-D-Tyr-Gly-Asn-Thr-Phe(2F)-AzaGly-Leu-Arg(Me)-Trp-$NH_2$ (Compound No. 783),
Ac-D-Tyr-Aib-Asn-Thr-Phe(2F)-AzaGly-Leu-Arg(Me)-Trp-$NH_2$ (Compound No. 784),
Ac-D-Tyr-Gly-Asn-Thr-Phe(3F)-AzaGly-Leu-Arg(Me)-Trp-$NH_2$ (Compound No. 785),
Ac-D-Tyr-Aib-Asn-Thr-Phe(3F)-AzaGly-Leu-Arg(Me)-Trp-$NH_2$ (Compound No. 786),
Ac-D-Tyr-Hyp-Asn-Thr-Phe(4F)-AzaGly-Leu-Arg(Me)-Trp-$NH_2$ (Compound No. 787),

Ac-D-Tyr-Glu-Asn-Thr-Phe(4F)-AzaGly-Leu-Arg(Me)-Trp-$NH_2$ (Compound No. 788),
Ac-D-Tyr-Lys-Asn-Thr-Phe(4F)-AzaGly-Leu-Arg(Me)-Trp-$NH_2$ (Compound No. 789),
Ac-D-Tyr-Gly-Asn-Thr-Phe(4F)-AzaGly-Leu-Arg(Me)-Trp-$NH_2$ (Compound No. 790),
Ac-D-Tyr-Aib-Asn-Thr-Phe(4F)-AzaGly-Leu-Arg(Me)-Trp-$NH_2$ (Compound No. 791),
Ac-D-Tyr-Hyp-Asn-Thr-D-Phe-AzaGly-Leu-Arg(Me)-Trp-$NH_2$ (Compound No. 794),
Ac-D-Tyr-Hyp-Asn-Thr-Phe-AzaGly-Leu-Arg-Trp-$NH_2$ (Compound No. 797),
Ac-D-Tyr-Hyp-Alb-Thr-Phe-AzaGly-Leu-Arg(Me)-Trp-$NH_2$ (Compound No. 800),
4-[Bis-(2-Pyridylmethyl)aminomethyl]benzoyl-Phe-AzaGly-Leu-Arg(Me)-Trp-$NH_2$ (Compound No. 801),
Ac-D-Tyr-Hyp-Asn-NMeSer-Phe-AzaGly-Leu-Arg(Me)-Trp-$NH_2$ (Compound No. 809),
Ac-D-Tyr-Hyp-Asn-Hyp-Phe-AzaGly-Leu-Arg(Me)-Trp-$NH_2$ (Compound No. 810),
Ac-D-Tyr-Hyp-Asn-Gly-Phe-AzaGly-Leu-Arg(Me)-Trp-$NH_2$ (Compound No. 813),
Ac-D-Tyr-Hyp-Asn-Ala-Phe-AzaGly-Leu-Arg(Me)-Trp-$NH_2$ (Compound No. 814),
Ac-D-Tyr-Hyp-Asn-D-Ala-Phe-AzaGly-Leu-Arg(Me)-Trp-$NH_2$ (Compound No. 815),
Ac-D-Tyr-Hyp-His-Thr-Phe-AzaGly-Leu-Arg(Me)-Trp-$NH_2$ (Compound No. 816),
Ac-D-Tyr-Hyp-Gln-Thr-Phe-AzaGly-Leu-Arg(Me)-Trp-$NH_2$ (Compound No. 843),
Ac-D-Tyr-Hyp-D-Asn-Thr-Phe-AzaGly-Leu-Arg(Me)-Trp-$NH_2$ (Compound No. 844),
Ac-D-Tyr-Hyp-Cit-Thr-Phe-AzaGly-Leu-Arg(Me)-Trp-$NH_2$ (Compound No. 845),
Ac-D-Tyr-Hyp-Asn-D-Thr-Phe-AzaGly-Leu-Arg(Me)-Trp-$NH_2$ (Compound No. 846),
Ac-D-Tyr-Hyp-Asn-Thr-Phe-AzaGly-Ala(cPr)-Arg(Me)-Trp-$NH_2$ (Compound No. 856),
4-Ureidobenzoyl-Phe-AzaGly-Leu-Arg(Me)-Trp-$NH_2$ (Compound No. 860),
Ac-D-Tyr-Hyp-Arg-Thr-Phe-AzaGly-Leu-Arg(Me)-Trp-$NH_2$ (Compound No. 861),
Ac-D-Tyr-Hyp-Gly-Thr-Phe-AzaGly-Leu-Arg(Me)-Trp-$NH_2$ (Compound No. 862),
Ac-D-Tyr-Hyp-Dap-Thr-Phe-AzaGly-Leu-Arg(Me)-Trp-$NH_2$ (Compound No. 863),
Ac-D-Tyr-Hyp-Dab-Thr-Phe-AzaGly-Leu-Arg(Me)-Trp-$NH_2$ (Compound No. 864),
Ac-D-Tyr-Hyp-Asn-Thr-αMePhe-AzaGly-Leu-Arg(Me)-Trp-$NH_2$ (Compound No. 868),
Ac-D-Tyr-Hyp-Asn-Thr-Phe(2Me)-AzaGly-Leu-Arg(Me)-Trp-$NH_2$ (Compound No. 870),
Ac-D-Tyr-Hyp-Asn-Thr-Phe(3Me)-AzaGly-Leu-Arg(Me)-Trp-$NH_2$ (Compound No. 872),
Ac-D-Tyr-Hyp-Asn-Thr-Phe(4Me)-AzaGly-Leu-Arg(Me)-Trp-$NH_2$ (Compound No. 874),
Ac-D-Tyr-Hyp-Asn-Thr-threo-Ser(3Phenyl)-AzaGly-Leu-Arg(Me)-Trp-$NH_2$ (Compound No. 877),
Ac-D-Tyr-Hyp-Asn-Thr-erythro-Ser(3Phenyl)-AzaGly-Leu-Arg(Me)-Trp-$NH_2$ (Compound No. 882),
Ac-D-Tyr-Hyp-Nva-Thr-Phe-AzaGly-Leu-Arg(Me)-Trp-$NH_2$ (Compound No. 886),
Ac-Hyp-Asn-Thr-Phe-AzaGly-Leu-Arg(Me)-Trp-$NH_2$ (Compound No. 887),
3-(p-Hydroxyphenyl)propionyl-Hyp-Asn-Thr-Phe-AzaGly-Leu-Arg(Me)-Trp-$NH_2$ (Compound No. 888),
pGlu-Asn-Thr-Phe-AzaGly-Leu-Arg(Me)-Trp-$NH_2$ (Compound No. 889),
Ac-D-Tyr-cisHyp-Asn-Thr-Phe-AzaGly-Leu-Arg(Me)-Trp-$NH_2$ (Compound No. 896),
Ac-D-Tyr-Pro(4F)-Asn-Thr-Phe-AzaGly-Leu-Arg(Me)-Trp-$NH_2$ (Compound No. 897),
Ac-Tyr-Hyp-Asn-Thr-Phe-AzaGly-Leu-Arg(Me)-Trp-$NH_2$ (Compound No. 899), or a salt thereof, etc.

Examples of the compounds described in WO 2008/050897 include, for example, metastin derivatives represented by the formula I described below or a salt thereof (which corresponds to the metastin derivative represented by formula (4) in the present specification or a salt thereof):

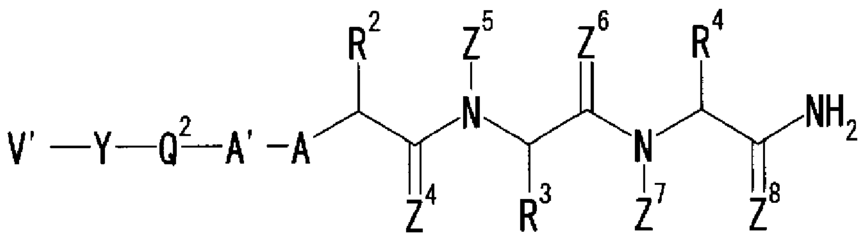

(I)

wherein:

V' represents a group represented by the formula below:

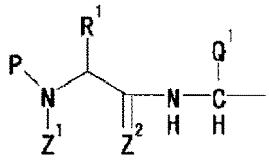,
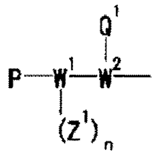
or
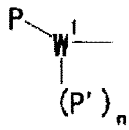

n represents 0 or 1;
$W^1$ represents N, CH or O (provided that when $W^1$ is N or CH, n represents 1 and when $W^1$ is O, n represents 0);
$W^2$ represents N or CH,
each of $Z^1$, $Z^5$ and $Z^7$ represents hydrogen atom or a $C_{1-3}$ alkyl group;
each of $Z^2$, $Z^4$, $Z^6$ and $Z^8$ represents hydrogen atom, O or S;
$R^1$ represents (1) hydrogen atom, (2) a $C_{1-8}$ alkyl group optionally substituted with a substituent selected from the group consisting of an optionally substituted carbamoyl group, an optionally substituted hydroxyl group and an optionally substituted aromatic cyclic group, (3) a cyclic or linear $C_{1-10}$ alkyl group, (4) a $C_{1-10}$ alkyl group consisting of a cyclic alkyl group and a linear alkyl group or (5) an optionally substituted aromatic cyclic group;

$R^2$ represents (1) hydrogen atom or (2) a cyclic or linear $C_{1-10}$ alkyl group, (3) a $C_{1-10}$ alkyl group consisting of a cyclic alkyl group and a linear alkyl group, or (4) a $C_{1-8}$ alkyl group optionally substituted with a substituent selected from the group consisting of an optionally substituted carbamoyl group, an optionally substituted hydroxyl group and an optionally substituted aromatic cyclic group;
$R^3$ represents (1) a $C_{1-8}$ alkyl group having an optionally substituted basic group and optionally having an additional substituent, (2) an aralkyl group having an optionally substituted basic group and optionally having an additional substituent, (3) a $C_{1-4}$ alkyl group having a non-aromatic cyclic hydrocarbon group of carbon atoms not greater than 7 having an optionally substituted basic group, and optionally having an additional substituent, or (4) a $C_{1-4}$ alkyl group having a non-aromatic heterocyclic group of carbon atoms not greater than 7 having an optionally substituted basic group, and optionally having an additional substituent;
$R^4$ represents a $C_{1-4}$ alkyl group, which may optionally be substituted with a substituent selected from the group consisting of (1) an optionally substituted $C_{6-12}$ aromatic hydrocarbon group, (2) an optionally substituted 5- to 14-membered aromatic heterocyclic group consisting of 1 to 7 carbon atoms and hetero atoms selected from the group consisting of nitrogen, oxygen and sulfur atoms, (3) an optionally substituted $C_{8-14}$ aromatic fused-ring group, (4) an optionally substituted 5- to 14-membered aromatic fused heterocyclic group consisting of 3 to 11 carbon atoms and hetero atoms selected from the group consisting of nitrogen, oxygen and sulfur atoms, (5) an optionally substituted non-aromatic cyclic hydrocarbon group having carbon atoms not greater than 7 and (6) an optionally substituted non-aromatic heterocyclic group having carbon atoms not greater than 7;
$Q^1$ represents a $C_{1-4}$ alkyl group, which may optionally be substituted with a substituent selected from the group consisting of (1) an optionally substituted $C_{6-12}$ aromatic hydrocarbon group, (2) an optionally substituted 5- to 14-membered aromatic heterocyclic group consisting of 1 to 7 carbon atoms and hetero atoms selected from the group consisting of nitrogen, oxygen and sulfur atoms, (3) an optionally substituted $C_{8-14}$ aromatic fused-ring group, (4) an optionally substituted 5- to 14-membered aromatic fused heterocyclic group consisting of 3 to 11 carbon atoms and hetero atoms selected from the group consisting of nitrogen, oxygen and sulfur atoms, (5) an optionally substituted non-aromatic cyclic hydrocarbon group having carbon atoms not greater than 7 and (6) an optionally substituted non-aromatic heterocyclic group having carbon atoms not greater than 7;
A represents:

(1) a nitrogen atom substituted with hydrogen atom or a $C_{1-3}$ alkyl group;
(2) a carbon atom substituted with hydrogen atom or a $C_{1-3}$ alkyl group;
(3) O; or
(4) S;

A' represents:

(1) a carbon atom, which may optionally be substituted with hydrogen atom, O, S, a halogen atom, an optionally halogenated $C_{1-3}$ alkyl group, carbamoyl group or hydroxyl group;
(2) a nitrogen atom, which may optionally be substituted with hydrogen atom or an optionally halogenated $C_{1-3}$ alkyl group;
(3) O; or

(4) S;

$Q^2$ represents (1) $CH_2$, CO, CS or $CH=CH_2$, which may optionally be substituted with one or two $C_{0-4}$ alkyl groups optionally substituted with a substituent selected from the group consisting of carbamoyl group, hydroxyl group, a $C_{1-3}$ alkoxy group, a halogen atom and an amino group, (2) NH, which may optionally be substituted with an optionally substituted $C_{1-4}$ alkyl group with a substituent selected from the group consisting of carbamoyl group and hydroxyl group, or, (3) O;
Y represents:

(1) a group represented by formula: -CONH-, -CSNH-, $-CH_2NH-$, -NHCO-, - $CH_2O-$, $-COCH_2-$, $-CH_2S-$, $-CSCH_2-$, $-CH_2SO-$, $-CH_2So_2-$, -COO-, -CSO-, $-CH_2CH_2-$ or -CH=CH-, which may optionally be substituted with a substituent selected from the group consisting of a $C_{1-6}$ alkyl group, hydroxyl group and a halogen atom,
(2) an optionally substituted $C_{6-7}$ aromatic hydrocarbon group,
(3) an optionally substituted 4- to 7-membered aromatic heterocyclic group consisting of 1 to 5 carbon atoms and hetero atoms selected from the group consisting of nitrogen, oxygen and sulfur atoms,
(4) an optionally substituted non-aromatic cyclic hydrocarbon group having carbon atoms not greater than 5, or,
(5) an optionally substituted non-aromatic heterocyclic group having carbon atoms not greater than 5 and when Y is (2), (3), (4) or (5), $Q^2$ may be a chemical bond.

P and P', which may be the same or different, each may form a ring by combining P and P' or P and $Q^1$ together and represents:

(1) hydrogen atom,
(2) an optional amino acid residue continuously or discontinuously bound from the C-terminal end of the 1st-48th amino acid sequence in the amino acid sequence represented by SEQ ID NO: 1,
(3) a group represented by formula: $J^1$-$_J{}^2$-$C(J^3)(Q^3)Y^1C(J^4)(Q^4)Y^2C(J^5)(Q^5)Y^3C(J^6)(Q^6)C(=Z^{10})$-

(wherein $J^1$ represents (a) hydrogen atom or (b) (i) a $C_{1-20}$ acyl group, (ii) a $C_{1-20}$ alkyl group, (iii) a $C_{6-14}$ aryl group, (iv) a carbamoyl group, (v) a carboxyl group, (vi) a sulfino group, (vii) an amidino group, (viii) a glyoxyloyl group or (ix) an amino group, which group may optionally be substituted with a substituent containing an optionally substituted cyclic group; $J^2$ represents (1) NH optionally substituted with a $C_{1-6}$ alkyl group, (2) $CH_2$ optionally substituted with a $C_{1-6}$ alkyl group, (3) O or (4) S; each of $J^3$ through $J^6$ represents hydrogen atom or a $C_{1-3}$ alkyl group;
each of $Q^3$ through $Q^6$ represents a $C_{1-4}$ alkyl group, which may optionally be substituted with a substituent selected from the group consisting of:

(1) an optionally substituted $C_{6-12}$ aromatic hydrocarbon group,
(2) an optionally substituted 5- to 14-membered aromatic heterocyclic group consisting of 1 to 7 carbon atoms and hetero atoms selected from the group consisting of nitrogen, oxygen and sulfur atoms,
(3) an optionally substituted $C_{8-14}$ aromatic fused-ring group,
(4) an optionally substituted 5- to 14-membered aromatic fused heterocyclic group consisting of 3 to 11 carbon atoms and hetero atoms selected from the group consisting of nitrogen, oxygen and sulfur atoms,
(5) an optionally substituted non-aromatic cyclic hydrocarbon group having carbon atoms not greater than 7,
(6) an optionally substituted non-aromatic heterocyclic group having carbon atoms not greater than 7,
(7) an optionally substituted amino group;
(8) an optionally substituted guanidino group;
(9) an optionally substituted hydroxyl group;
(10) an optionally substituted carboxyl group;
(11) an optionally substituted carbamoyl group; and,
(12) an optionally substituted sulfhydryl group;

or hydrogen atom;
$J^3$ and $Q^3$, $J^4$ and $Q^4$, $J^5$ and $Q^5$ or $J^6$ and $Q^6$ may be combined together, or, $Z^1$ and $R^1$, $J^2$ and $Q^3$, $Y^1$ and $Q^4$, $Y^2$ and $Q^5$, or $Y^3$ and $Q^6$ may be combined together, to form a ring;
$Y^1$ through $Y^3$ each represents a group represented by formula: - $CON(J^{13})$-, $-CSN(J^{13})$-, $-C(J^{14})N(J^{13})$- or $-N(J^{13})CO$- (wherein $J^{13}$ and $J^{14}$ each represents hydrogen atom or a $C_{1-3}$ alkyl group); $Z^{10}$ represents hydrogen atom, O or S);

(4) a group represented by formula: $J^1$-$J^2$-C($J^7$)($Q^7$)$Y^2$C($J^8$)($Q^8$)$Y^3$C($J^9$)($Q^9$)C(=$Z^{10}$)- (wherein each of $J^1$ and $J^2$ has the same significance as defined above, $J^7$ through $J^9$ have the same significance as given for $J^3$, $Q^7$ through $Q^9$ have the same significance as given for $Q^3$, $Y^2$ and $Y^3$ have the same significance as defined above, $Z^{10}$ has the same significance as defined above, $J^7$ and $Q^7$, $J^8$ and $Q^8$ or $J^9$ and $Q^9$ may be combined together, or $J^2$ and $Q^7$, $Y^2$ and $Q^8$ or $Y^3$ and $Q^9$ may be combined together, to form a ring.);

(5) a group represented by formula: $J^1$-$J^2$-C($J^{10}$)($Q^{10}$)$Y^3$C($J^{11}$)($Q^{11}$)C(=$Z^{10}$)-(wherein $J^1$ and $J^2$ have the same significance as defined above, $J^{10}$ and $J^{11}$ have the same significance as given for $J^3$, $Q^{10}$ and $Q^{11}$ have the same significance as given for $Q^3$, $Y^3$ has the same significance as defined above, $Z^{10}$ has the same significance as defined above, $J^{10}$ and $Q^{10}$ or $J^{11}$ and $Q^{11}$ may be combined together, or $J^2$ and $Q^{10}$ or $Y^3$ and $Q^{11}$ may be combined together, to form a ring.);

(6) a group represented by formula: $J^1$-$J^2$-C($J^{12}$)($Q^{12}$)C(=$Z^{10}$)- (wherein, $J^1$ and $J^2$ have the same significance as defined above, $J^{12}$ has the same significance as given for $J^3$, $Q^{12}$ has the same significance as given for $Q^3$, and $Z^{10}$ has the same significance as defined above; $J^{12}$ and $Q^{12}$ may be combined together, or $J^2$ and $Q^{12}$ may be combined together, to form a ring.); or,

(7) a group represented by formula: $J^1$- ($J^1$ represents the same as those described above);

wherein the bonds between Y-$Q^2$, $Q^2$-A' and A'-A represent independently from each other, a single bond or a double bond.]. Among them, preferable metastin derivatives are as follows:

Ac-D-Tyr-Hyp-Asn-Thr-Cha-Gly-Ala(cPr)-Arg(Me)-Trp-$NH_2$,
Ac-D-Tyr-Hyp-Asn-Thr-Cha-GlyΨ((E)CH=CH)Leu-Arg(Me)-Trp-$NH_2$,
Ac-D-Tyr-Hyp-Alb-Thr-Cha-GlyΨ((E)CH=CH)Leu-Arg(Me)-Trp-$NH_2$,
Ac-D-Tyr-Hyp-Asn-Thr-Cha-GlyΨ((E)CH=CH)Leu-Arg-Trp-$NH_2$,
Ac-D-Tyr-Hyp-Alb-Thr-Cha-GlyΨ((E)CH=CH)Leu-Arg-Trp-$NH_2$,
Ac-D-Tyr-Hyp-Alb-Thr-Cha-Gly-Ala(cPr)-Arg(Me)-Trp-$NH_2$ and
Ac-D-Tyr-Pro(4F)-Asn-Thr-Cha-Gly-Ala(cPr)-Arg(Me)-Trp-$NH_2$, etc.

Examples of the compounds according to PCT/JP2009/058102 include, for example, metastin derivatives represented by the formula described below or a salt thereof (which corresponds to the metastin derivative represented by formula (5) in the present specification or a salt thereof):

XX0-XX2-XX3-XX4-XX5-XX6-AzaGly-XX8-XX9-XX10-$NH_2$

(wherein:

XX0 represents hydrogen, acetyl, mesyl, 4-fluorobenzoyl, decanoyl, benzylureido, ureido, amidino, dimethylaminoethylureido or isobutylureido;

XX2 represents Tyr, D-Tyr, D-Ala, D-Leu, D-Phe, D-Lys, D-Trp or a chemical bond;

XX3 represents:

i) an amino acid selected from Ala, Arg, Asn, Asp, Cys, Gin, Glu, Gly, His, Ile, Leu, Lys, Met, Phe, Ser, Thr, Trp, Tyr and Val, wherein the α-amino group may optionally be methylated;

ii) a cyclic amino acid selected from Pro, Aze(2), Aze(3), Pic(2), Pic(3), Hyp, Thz, Abz(2), Abz(3), Pzc(2), Pro(4$NH_2$), Hyp(Bzl), cisHyp, Pro(4F) and lzc;

iii) an amino acid selected from D-Pya(4), DL-Ala(Pip), Orn, Aib and Tyr($PO_3H_2$); or,

iv) a chemical bond;

XX4 represents Asn, 2-amino-3-ureidopropionic acid, $N^\beta$-formyl-β-diaminopropionic acid, $N^\beta$-acetyl-β-diaminopropionic acid, $N^\omega$-pentylasparagine, $N^\omega$-cyclopropylasparagine, $N^\omega$-benzylasparagine, 2,4-diaminobutanoic acid, 2,3-diaminopropionic acid, His, Gin, Gly, Arg, Cit, Nva, D-Asn or a chemical bond;

XX5 represents Ser, Thr, Val, NMeSer, Gly, Ala, Hyp, D-Ala, D-Thr, D-Pro or a chemical bond;

XX6 represents Phe, Tyr, Trp, Tyr(Me), Thi, Nal(2), Cha, Pya(4), threo-Ser(3Phenyl), erythro-Ser(3Phenyl) or an optionally substituted phenylalanine;

AzaGly represents azaglycine;

XX8 represents Leu, Nva, Val or Ala(cPr);

XX9 represents an optionally substituted arginine, an optionally substituted lysine or an optionally substituted ornithine; and,

XX10 represents 2-naphthylalanine, 2-thienylalanine, tyrosine, an optionally substituted phenylalanine or an optionally substituted tryptophan); and

a metastin derivative represented by the formula described below or a salt thereof (which corresponds to the metastin derivative represented by formula (6) in the present specification or a salt thereof):

$$XX0\text{-}XX2\text{-}XX3\text{-}XX4\text{-}XX5\text{-}T\text{-}XX9\text{-}XX10\text{-}NH_2$$

wherein:

XX0 represents formyl, a $C_{1-20}$ alkanoyl, mesyl, an optionally substituted ureido, amidino, cyclopropanecarbonyl, 6-(acetyl-D-arginylamino)caproyl, 6-((R)-2,3-diaminopropionylamino)caproyl, 6-(D-norleucylamino)caproyl, 4-(D-arginylamino)butyryl, 3-(4-hydroxyphenyl)propionyl, glycyl, tyrosyl, acetylglycyl, acetyltyrosyl, D-tyrosyl, acetyl-D-tyrosyl, pyroglutamyl, 3-(pyridin-3-yl)propionyl, adipoyl, glycoloyl or 6-aminocaproyl;
XX2 represents Tyr, D-Tyr, D-Ala, D-Leu, D-Phe, D-Lys, D-Trp or a chemical bond;
XX3 represents D-Asp, D-Dap, D-Ser, D-Gln, D-His, D-NMeAla, D-NMePhe, Aze(2), Pic(2), Pic(3), Hyp, Thz, NMeAla, Gly, Aib, Abz(2), Abz(3), Sar, Leu, Lys, Glu, β-alanine, Pzc(2), Orn, His(3Me), Tyr($PO_3H_2$), Pro($4NH_2$), Hyp(Bzl), Trp, Pro, 4-pyridylalanine, Tic, D-Trp, D-Ala, D-Leu, D-Phe, D-Lys, D-Glu, D-2-pyridylalanine, D-3-pyridylalanine, D-4-pyridylalanine, Aad, Pro(4F) or a chemical bond;
XX4 represents Asn, 2-amino-3-ureidopropionic acid, $N^{\beta}$-formyldiaminopropionic acid, $N^{\beta}$-acetyl-diaminopropionic acid, $N^{\omega}$-pentylasparagine, $N^{\omega}$-cyclopropylasparagine, $N^{\omega}$-benzylasparagine, 2,4-diaminobutanoic acid, His, Gin, Cit or D-Asn;
XX5 represents Ser, Thr, Val, NMeSer, Gly, Ala, Hyp, D-Ala, Dap or D-Thr;
T represents formula II:

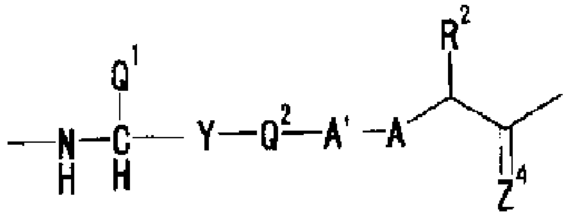

(II)

$Z^4$ represents hydrogen atom, O or S;
$R^2$ represents (1) hydrogen atom or (2) a cyclic or linear $C_{1-10}$ alkyl group, (3) a $C_{1-10}$ alkyl group consisting of a cyclic alkyl group and a linear alkyl group, or (4) a $C_{1-8}$ alkyl group which may optionally be substituted with a group selected from the group consisting of an optionally substituted carbamoyl group, an optionally substituted hydroxy group and an optionally substituted aromatic cyclic group;
$Q^1$ represents a $C_{1-4}$ alkyl group, which may optionally be substituted with a substituent selected from the group consisting of (1) an optionally substituted $C_{6-12}$ aromatic hydrocarbon group, (2) an optionally substituted 5- to 14-membered aromatic heterocyclic group consisting of 1 to 7 carbon atoms and hetero atoms selected from the group consisting of nitrogen, oxygen and sulfur atoms, (3) an optionally substituted $C_{8-14}$ aromatic fused-ring group, (4) an optionally substituted 5- to 14-membered aromatic fused heterocyclic group consisting of 3 to 11 carbon atoms and hetero atoms selected from the group consisting of nitrogen, oxygen and sulfur atoms, (5) an optionally substituted non-aromatic cyclic hydrocarbon group having not greater than 7 carbon atoms and (6) an optionally substituted non-aromatic heterocyclic group having not greater than 7 carbon atoms;
A represents:

(1) a nitrogen atom substituted with hydrogen atom or a $C_{1-3}$ alkyl group;
(2) a carbon atom substituted with hydrogen atom or a $C_{1-3}$ alkyl group;
(3) O; or,
(4) S;

A' represents:

(1) a carbon atom which may optionally be substituted with hydrogen atom, O, S, a halogen atom, an

optionally halogenated $C_{1-3}$ alkyl group, carbamoyl or hydroxy;

(2) a nitrogen atom which may optionally be substituted with hydrogen atom or an optionally halogenated $C_{1-3}$ alkyl group;

(3) O; or,

(4) S;

$Q^2$ represents:

(1) $CH_2$, CO, CS or $C=CH_2$, which may optionally be substituted with a $C_{0-4}$ alkyl group optionally substituted with a substituent selected from the group consisting of carbamoyl, hydroxy, a $C_{1-3}$ alkoxy group, a halogen atom and amino;

(2) NH which may optionally be substituted with a $C_{1-4}$ alkyl group optionally substituted with a substituent selected from the group consisting of carbamoyl and hydroxy; or,

(3) O;

Y represents:

(1) a group represented by formula: -CONH-, -CSNH-, $-CH_2NH-$,-NHCO-, $-CH_2O-$, $-COCH_2-$, $-CH_2S-$, $-CSCH_2-$, $-CH_2SO-$, $-CH_2SO_2-$, -COO-,-CSO-, $-CH_2CH_2-$ or -CH=CH-, which may optionally be substituted with a substituent selected from the group consisting of a $C_{1-6}$ alkyl group, hydroxy and a halogen atom;

(2) an optionally substituted $C_{6-7}$ aromatic hydrocarbon group;

(3) an optionally substituted 5- to 7-membered aromatic heterocyclic group consisting of 1 to 5 carbon atoms and hetero atoms selected from the group consisting of nitrogen, oxygen and sulfur atoms;

(4) an optionally substituted non-aromatic cyclic hydrocarbon group having not greater than 5 carbon atoms;

(5) an optionally substituted non-aromatic heterocyclic group having not greater than 5 carbon atoms;

provided that when Y is (2), (3), (4) or (5), $Q^2$ may be a chemical bond;
the bonds between $Y-Q^2$, $Q^2$-A' and A'-A each independently represents a single bond or a double bond;
XX9 represents Arg, Orn, Arg(Me) or Arg($asymMe_2$); and,
XX10 represents Phe, Trp, 2-naphthylalanine, 2-thienylalanine, tyrosine or 4-fluorophenylalanine;. Among them, the metastin derivative is preferably selected from:

Ms-D-Tyr-Hyp-Asn-Thr-Phe-AzaGly-Leu-Arg(Me)-Trp-$NH_2$ (Compound No. 963),
4-Fluorobenzoyl-D-Tyr-Hyp-Asn-Thr-Phe-AzaGly-Leu-Arg(Me)-Trp-$NH_2$ (Compound No. 964),
Decanoyl-D-Tyr-Hyp-Asn-Thr-Phe-AzaGly-Leu-Arg(Me)-Trp-$NH_2$ (Compound No. 965),
Benzylureido-D-Tyr-Hyp-Asn-Thr-Phe-AzaGly-Leu-Arg(Me)-Trp-$NH_2$ (Compound No. 966),
Ureido-D-Tyr-Hyp-Asn-Thr-Phe-AzaGly-Leu-Arg(Me)-Trp-$NH_2$ (Compound No. 967),
Ac-D-Tyr-Hyp-Asn-Thr-Cha-AzaGly-Leu-Arg(Me)-Trp-$NH_2$ (Compound No. 969),
Ac-D-Tyr-Pro(4F)-Asn-Thr-Cha-AzaGly-Leu-Arg(Me)-Trp-$NH_2$ (Compound No. 970),
Amidino-D-Tyr-Hyp-Asn-Thr-Phe-AzaGly-Leu-Arg(Me)-Trp-$NH_2$ (Compound No. 971),
Dimethylaminoethylureido-D-Tyr-Hyp-Asn-Thr-Phe-AzaGly-Leu-Arg(Me)-Trp-$NH_2$ (Compound No. 972),
iso-Butylureido-D-Tyr-Hyp-Asn-Thr-Phe-AzaGly-Leu-Arg(Me)-Trp-$NH_2$ (Compound No. 973) and
Hyp-Asn-Thr-Phe-AzaGly-Leu-Arg(Me)-Trp-$NH_2$ (Compound No. 977); and
Ac-D-Tyr-Hyp-Asn-Thr-D-Cha-Gly-Ψ[(E)-CH=CH]-Leu-Arg(Me)-Trp-$NH_2$,
Ac-D-Tyr-Hyp-Asn-Thr-Cha-Gly-Ψ[(E)-CH=CH]-D-Leu-Arg(Me)-Trp-$NH_2$,
Ac-D-Tyr-Hyp-Asn-Thr-Cha-Gly-Ψ[(E)-CMe=CH]-Leu-Arg(Me)-Trp-$NH_2$,
Ac-D-Tyr-Hyp-Asn-Thr-Cha-Gly-Ψ[(E)-CF=CH]-Leu-Arg(Me)-Trp-$NH_2$,
Ac-D-Tyr-Hyp-Asn-Thr-Phe-Ψ(CSNH)-Gly-Leu-Arg(Me)-Trp-$NH_2$,
Ac-D-Tyr-Hyp-Asn-Thr-Phe-Ψ(NHCO)-Gly-Leu-Arg(Me)-Trp-$NH_2$,
Ac-D-Tyr-Hyp-Asn-Thr-Phe-Ψ($CH_2NHCOCONH$)-Leu-Arg(Me)-Trp-$NH_2$,
Ac-D-Tyr-Hyp-Asn-Thr-Phe-Ψ($CH_2NH$)-AzaGly-Leu-Arg(Me)-Trp-NH2, and
Ac-D-Tyr-Hyp-Asn-Thr-Phe-Gly-Ψ(CSNH)-Leu-Arg(Me)-Trp-$NH_2$, etc.
Among those mentioned above, the metastin derivative of the present invention is most preferably for example,
Ac-D-Tyr-D-Trp-Asn-Thr-Phe-AzaGly-Leu-Arg(Me)-Trp-$NH_2$ (Compound No. 550),
Ac-D-Tyr-Hyp-Asn-Thr-Phe-AzaGly-Leu-Arg(Me)-Trp-$NH_2$ (Compound No. 723),
Ac-D-Tyr-Glu-Asn-Thr-Phe(3F)-AzaGly-Leu-Arg(Me)-Trp-$NH_2$ (Compound No. 757),
Ac-D-Tyr-Hyp-Asn-Thr-Phe(4F)-AzaGly-Leu-Arg(Me)-Trp-$NH_2$ (Compound No. 787), etc.

Among them, preferable metastin derivatives are those represented by the formula (2) and the formula (3) described above.

Among them, the metastin derivative is further preferably a metastin derivative represented by the formula (2):

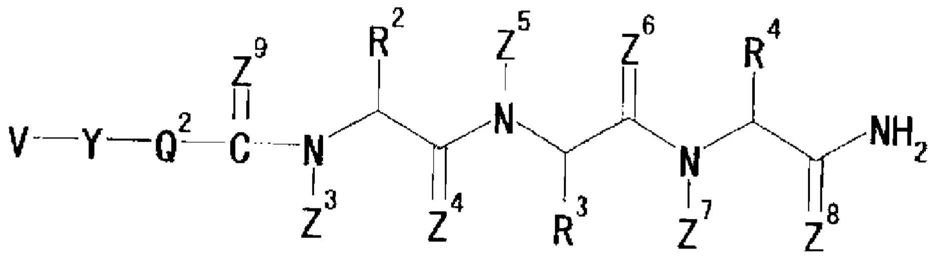

(2)

[wherein V represents a group represented by formula:

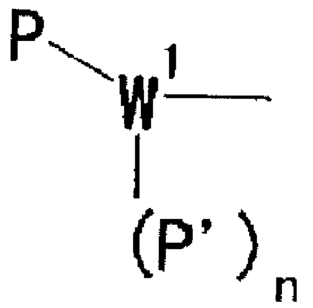

;

n represents 0 or 1;

$W^1$ represents N, CH or O (provided that when $W^1$ is N or CH, n represents 1, and when $W^1$ is O, n represents 0);

each of $Z^3$, $Z^5$ and $Z^7$ represents a hydrogen atom or a $C_{1-3}$ alkyl group;
each of $Z^4$, $Z^6$ and $Z^8$ represents a hydrogen atom, O or S;
$R^2$ represents a $C_{1-8}$ alkyl group;
$R^3$ represents (1) a $C_{1-8}$ alkyl group having an optionally substituted basic group and optionally having an additional substituent;
$R^4$ represents:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group; or
(4) an optionally substituted 5- to 14-membered aromatic fused heterocyclic group consisting of 3 to 11 carbon atoms and hetero atoms selected from the group consisting of nitrogen, oxygen and sulfur atoms;

$Q^2$ represents $CH_2$, which may optionally be substituted with a $C_{1-4}$ alkyl group optionally substituted with a substituent selected from the group consisting of a carbamoyl group and a hydroxyl group;
Y represents a group represented by formulae $-CONH-$, $-CSNH-$,$-CH_2NH-$, $-NHCO-$, $-CH_2O-$, $-CH_2S-$, $-COO-$, $-CSO-$ or $-CH_2CH_2-$, which may be substituted with a $C_{1-6}$ alkyl group;
$Z^9$ represents a hydrogen atom, O or S;
P and P', which may be the same or different from each other, represent:

(1) a hydrogen atom;
(2) an optional amino acid residue continuously or discontinuously bound from the C terminus of the 1-48 amino acid sequence represented by SEQ ID NO: 1; or
(3) a $C_{1-15}$ alkyl group which may be substituted with an optionally substituted cyclic group.]; or

a metastin derivative represented by the formula (3):

$$XX0\text{-}XX2\text{-}XX3\text{-}XX4\text{-}XX5\text{-}XX6\text{-}AzuGly\text{-}XX8\ XX9\text{-}XX10\text{-}NH_2 \qquad (3)$$

[wherein

XX0 represents formyl, or $C_{1-20}$ alkanoyl;
XX2 represents Tyr, D-Tyr, D-Ala, D-Leu, D-Phe, D-Lys, D-Trp or a chemical bond;
XX3 represents:

i) an amino acid selected from Ala, Arg, Asn, Asp, Cys, Gin, Glu, Gly, His, Ile, Leu, Lys, Met, Phe, Ser, Thr, Trp, Tyr and Val, wherein the $\alpha$-amino group may optionally be methylated;
ii) a cyclic amino acid selected from Pro, Aze(2), Aze(3), Pic(2), Pic(3), Hyp, Thz, Abz(2), Abz(3), Pzc(2), Pro(4$NH_2$), Hyp(Bzl), cisHyp, Pro(4F) and Izc;
iii) an amino acid selected from D-Trp, D-Dap, D-Pya(4), DL-Ala(Pip), Orn, Aib and Tyr($PO_3H_2$); or
iv) a chemical bond;

XX4 represents Asn or a chemical bond;
XX5 represents Ser, Thr, Val, NMeSer, Gly, Ala, Hyp, D-Ala, D-Thr, D-Pro or a chemical bond;
XX6 represents Phe, Tyr, Trp, Tyr(Me), Thi, Nal(2), Cha, Pya(4), threo-Ser(3Phenyl), erythro-Ser(3Phenyl) or optionally substituted phenylalanine;
AzaGly represents azaglycine;
XX8 represents Leu, Nva, Val or Ala(cPr);
XX9 represents optionally substituted arginine; and
XX10 represents optionally substituted phenylalanine or optionally substituted tryptophan.].

The metastin derivatives of the present invention can be prepared by per se publicly known methods for peptide synthesis. As the methods for peptide synthesis, for example, either solid phase synthesis or liquid phase synthesis may be used. More specifically, the partial peptide or amino acids that can constitute the peptide of the present invention are repeatedly condensed with the remaining part to give the product having a desired sequence. Where the product has protecting groups, these protecting groups are removed to give the desired peptide. Publicly known methods for condensation and removal of the protecting groups are described, for example, in (1) to (5) below:

(1) M. Bodanszky & M.A. Ondetti: Peptide Synthesis, Interscience Publishers, New York (1966);
(2) Schroeder & Luebke: The Peptide, Academic Press, New York (1965);
(3) Nobuo Izumiya, et al.: Peptide Gosei-no-Kiso to Jikken (Basics and Experiments of Peptide Synthesis), published by Maruzen Co. (1975);
(4) Haruaki Yajima & Shunpei Sakakibara: Seikagaku Jikken Koza (Biochemical Experiment) 1, Tanpakushitsu no Kagaku (Chemistry of Proteins) IV, 205 (1977); and
(5) Haruaki Yajima, ed.: Zoku Iyakuhin no Kaihatsu (A sequel to Development of Pharmaceuticals), Vol. 14, Peptide Synthesis, published by Hirokawa Shoten.

After completion of the reaction, the peptide of the present invention may be purified and isolated by a combination of conventional purification methods such as solvent extraction, distillation, column chromatography, liquid chromatography and recrystallization. When the peptide obtained by the above methods is in a free form, the peptide can be converted into an appropriate salt by a publicly known method; conversely when the peptide is obtained in a salt form, it can be converted into its free form by publicly known methods.

For condensation of the protected amino acids or peptides, a variety of activation reagents for peptide synthesis may be used, and trisphosphonium salts, tetramethyluronium salts, carbodiimides, etc. are particularly preferred. Examples of the trisphosphonium salts include benzotriazol-1-yloxytris(pyrrolidino)phosphonium hexafluorophosphate (PyBOP), bromotris(pyrrolidino)phosphonium hexafluorophosphate (PyBroP) and 7-azabenzotriazol-1-yloxytris(pyrrolidino)phosphonium hexafluorophosphate (PyAOP); examples of the tetramethyluronium salts include 2-(1H-benzotriazol-1-yl)-1,1,3,3-hexafluorophosphate (HBTU), 2-(7-azabenzotriazol-1-yl)-1,1,3,3-hexafluorophosphate (HATU), 2-(1H-benzotriazol-1-yl)-1,1,3,3-tetramethyluronium tetrafluoroborate (TBTU), 2-(5-norbornene-2,3-dicarboxyimido)-1,1,3,3-tetramethyluronium tetrafluoroborate (TNTU) and O-(N-succinimidyl)-1,1,3,3-tetramethyluronium tetrafluoroborate (TSTU); and examples of the carbodiimides include DCC, N,N'-diisopropylcarbodiimide (DIPCDI), N-ethyl-N'-(3-dimethylaminopropyl)carbodiimide hydrochloride (EDCI·HCl), etc. For condensation using these reagents, a racemization inhibitor (e.g., HONB, HOBt, HOAt, HOOBt, etc.) is preferably added. Solvents used in condensation may be appropriately chosen from solvents that are known to be usable for peptide condensation reaction. For example, acid amides such

as anhydrous or hydrous N,N-dimethylformamide, N,N-dimethylacetamide, N-methylpyrrolidone, etc., halogenated hydrocarbons such as methylene chloride, chloroform, etc., alcohols such as trifluoroethanol, phenol, etc., sulfoxides such as dimethyl sulfoxide, etc., tertiary amines such as pyridine, etc., ethers such as dioxane, tetrahydrofuran, etc., nitriles such as acetonitrile, propionitrile, etc., esters such as methyl acetate, ethyl acetate, etc., or suitable mixtures thereof, etc. are used. The reaction temperature is appropriately chosen from the range known to be applicable to peptide binding reactions and is normally suitably chosen from the range of about -20°C to 50°C. The activated amino acid derivatives are used generally in 1.5 to 6 times excess. In the case of solid phase synthesis, the condensation is examined using the ninhydrin reaction; when the condensation is insufficient, the condensation can be completed by repeating the condensation reaction without removal of the protecting groups. When the condensation is yet insufficient even after repeating the reaction, the unreacted amino acids are acylated with acetic anhydride or acetylimidazole to cancel any adverse effect on the subsequent reaction.

Examples of the protecting groups used to protect amino groups in the starting amino acids include Z, Boc, tert-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, trityl, etc. Examples of protecting groups for a carboxyl group include a $C_{1-6}$ alkyl group, a $C_{3-8}$ cycloalkyl group and a $C_{7-14}$ aralkyl group, which have been described above for R, and in addition, allyl, 2-adamantyl, 4-nitrobenzyl, 4-methoxybenzyl, 4-chlorobenzyl, phenacyl group, benzyloxycarbonylhydrazide, tert-butoxycarbonylhydrazide, tritylhydrazide, etc.

The hydroxyl group of serine and threonine can be protected, for example, by esterification or etherification. Examples of the group suitable for this esterification include a group derived from organic acids, e.g. a lower ($C_{2-4}$) alkanoyl group such as an acetyl group, an aroyl group such as a benzoyl group, etc. Examples of the group suitable for the etherification include a benzyl group, a tetrahydropyranyl group, a tert-butyl group, a trytyl (Trt) group, etc.

Examples of groups for protecting the phenolic hydroxyl group of tyrosine include Bzl, 2,6-dichlorobenzyl, 2-nitrobenzyl, Br-Z, tert-butyl, etc.

Examples of groups used to protect the imidazole moiety of histidine include Tos, 4-methoxy-2,3,6-trimethylbenzenesulfonyl (Mtr), DNP, Bom, Bum, Boc, Trt, Fmoc, etc.

Examples of protecting groups for the guanidino group in arginine include Tos, Z, 4-methoxy-2,3,6-trimethylbenzenesulfonyl (Mtr), p-methoxybenzenesulfonyl (MBS), 2,2,5,7,8-pentamethylchroman-6-sulfonyl (Pmc), mesitylene-2-sulfonyl (Mts), 2,2,4,6,7-pentamethyldihydrobenzofuran-5-sulfonyl (Pbf), Boc, Z, $NO_2$, etc.

Examples of protecting groups for side chain amino group of lysine include Z, Cl-Z, trifluoroacetyl, Boc, Fmoc, Trt, Mtr, 4,4-dimethyl-2,6-dioxocyclohexylidenethyl (Dde), etc.

Examples of protecting groups for the indolyl of tryptophan include formyl (For), Z, Boc, Mts, Mtr, etc.

Examples of protecting groups for asparagine and glutamine include Trt, xanthyl (Xan), 4,4'-dimethoxybenzhydryl (Mbh), 2,4,6-trimethoxybenzyl (Tmob), etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, 1-hydroxybenzotriazole (HOBt) or 1-hydroxy-7-azabenzotriazole (HOAt)], etc. Examples of amino acids in which the amino groups in the starting material are activated, include the corresponding phosphorous amides.

Examples of a method of eliminating (removing) the protecting groups include catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, trimethylsilane bromide (TMSBr), trimethylsilyl trifluoromethanesulfonate, tetrafluoroboric acid, tris(trifluoro)boron, boron tribromide or a mixed solution thereof, a base treatment with diisopropylethylamine, triethylamine, piperidine, piperazine, etc., and reduction with sodium in liquid ammonia. The elimination of protecting groups by the acid treatment described above is carried out generally at a temperature of -20°C to 40°C. In the acid treatment, it is efficient to add a cation scavenger such as anisole, phenol, thioanisole, m-cresol, p-cresol, etc., dimethylsulfide, 1,4-butanedithiol, 1,2-ethanedithiol, etc. Furthermore, a 2,4-dinitrophenyl group that is used as a protecting group for the imidazole of histidine is removed by a treatment with thiophenol. A formyl group that is used as a protecting group of the indole of tryptophan is removed by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, removal of the protecting groups and activation of functional groups involved in the reaction may be appropriately chosen from publicly known groups or publicly known means.

In another method for obtaining the amides of the peptide, for example, solid phase synthesis is carried out using a resin for amide synthesis, or the α-carboxyl group of the carboxyl terminal amino acid is converted to amide; the peptide chain is then extended from the amino group side to a desired length. Thereafter, prepared are a peptide (or an amino acid) in which only the protecting group of the N-terminal α-amino group in the peptide chain has been removed from the peptide; and a peptide (or an amino acid) in which only the protecting group of the C-terminal carboxyl

group has been eliminated. The two peptides are condensed in a mixture of the solvents described above. The details of the condensation reaction are the same as those described above. After purification of the protected peptide obtained by the condensation, all the protecting groups are removed by the method described above to give the desired crude peptide. This crude peptide is purified by various known purification means. Lyophilization of the major fraction gives the amide of the desired peptide.

The modified metastin of the present invention can be prepared according to a per se publicly known method for chemically modifying a peptide. Examples of the method for chemically modifying a peptide that may be used include, for example, a method of conjugatively binding a polyethylene glycol (PEG) to the amino-terminal of a peptide, Cys, Asp, Glu or Lys residues, or the carboxy-terminal of an amino acid. Furthermore, examples of the method for chemically modifying a peptide that may be used include, for example, a method described in Roberts, M., et al., Advanced Drug Delivery Reviews, 54, 457-476 (2002) or modifications of these methods. The binding of PEG to the carboxy-terminal can be carried out by enzymatical coupling using recombinant GLP-1 peptide, or by a per se publicly known method described in US Patent No. 4,343,898, or International Journal of Peptide & Protein Research, 43, 127-138 (1994), etc., or by modifications of these methods.

More specifically, an intended modified metastin derivative may be prepared by binding a site, which is modifiable with PEG (for example, lysine residue, etc.), and a polyethylene glycol to a part of the metastin derivative of the present invention by a per se publicly known chemical modification method; or by binding a desired part of a metastin derivative to a desired part of a polyethylene glycol through a linker by chemical modification; or by binding a polyethylene glycol to a linker having a specific structure in advance, and binding the polyethylene glycol having the linker to a metastin derivative by a chemical modification method, etc.

The molecular weight of the PEG that is used in modification of the metastin derivative of the present invention may be appropriately selected depending on a degree of drug efficacy duration, a degree of blood stability, etc. The molecular weight of one PEG molecule is 1 to 1000 kDa, preferably 10 to 500 kDa. The molecular configuration of the PEG that is used in the present invention may be appropriately selected from a straight type, a branched type or a stellar type, etc.

Examples of the modified metastin that is used in the present invention include, for example, a modified metastin represented by formula:

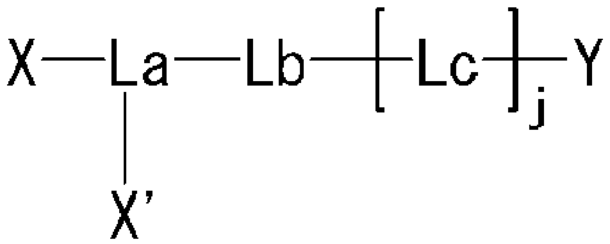

(I)

, etc.

X represents a polyethylene glycol.
X' is absent, or represents a polyethylene glycol.

The "polyethylene glycols" represented by X and X' may be in a straight form, or in a branched form. The molecular weights (or average molecular weights) of the "polyethylene glycols" are not particularly limited, but are preferably about 1 to about 1000 kDa, preferably 10 to 500 kDa, about 2,000 dalton to about 40,000 dalton, about 20,000 dalton to about 40,000 dalton, more preferably about 25,000 dalton to about 35,000 dalton, and further preferably about 30,000 dalton, respectively.

The "polyethylene glycols" are represented by formula: $MeO-(CH_2-CH_2-O)_h-$. Herein, h represents the polymerization degree (or average polymerization degree), and is preferably about 350 to about 1,350, and more preferably about 550 to about 1,350.

The moiety represented by the partial structure:

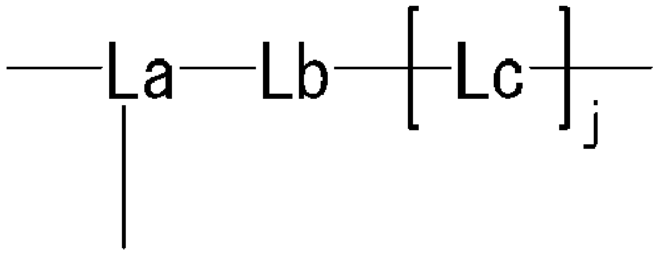

in the formula (I), is a linker that links the "polyethylene glycols" represented by X and X', and a polypeptide represented by Y. The linker is not particularly limited, but a linker that is used widely in PEG-modification (pegylation) of a polypeptide, etc. may be used.

La is a bivalent or trivalent group represented by formula:

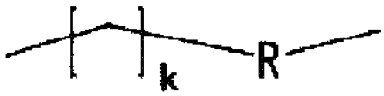

or

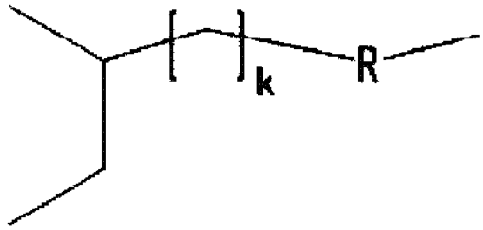

(wherein

R represents a chemical bond, -O-, -CO-O-, -O-CO-, -NH-, -CO-, -S-, -S-S-, -SO-, $-SO_2-$, $-NH-SO_2-$, $-SO_2-NH-$, -C(=O)-NH-N=CH-, -C(=NH)-NH-, $-CO-CH_2-S-$, or

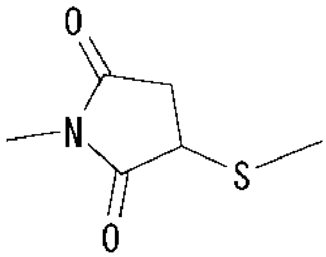

;

and

k represents an integer of 0 to 5.), and

La is preferably divalent or trivalent group represented by formula:

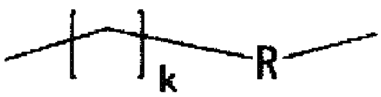

or

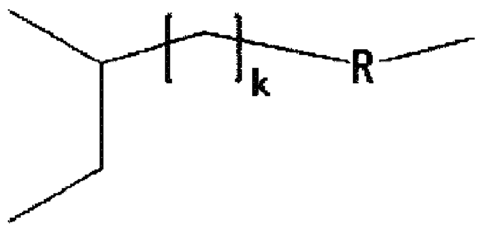

(wherein R represents -CO-, and k represents an integer of 0. Alternatively, R represents a chemical bond, and k represents an integer of 2 to 5.).

La is preferably -CO-, $-CH_2CH_2-$, $-(CH_2)_3-$ or $-(CH_2)_5-$.

Lb represents $-(CH_2)_i-$
(wherein i represents an integer of 0 to 5.).
Lb is preferably $-(CH_2)_i-$
(wherein i represents an integer of 0 to 2.). More specifically, Lb is preferably a chemical bond, $-CH_2-$, or $-CH_2CH_2-$.
Lc represents:

(i) a bivalent group represented by formula: $-B-Q^c-C^b-$
(wherein

B represents a chemical bond, or -NH-;
$Q^c$ represents a bivalent group represented by formula:

$$-(CH_2)_{m1}-Z^c-(CH_2)_{m2}-$$

(wherein

m1 represents an integer of 0 to 15;
$Z^c$ represents:

(a) a chemical bond; or
(b) a bivalent group selected from -CO-, -O-CO-, -CO-O-, -CO-NH-,-NH-CO-, -CO-NH-CO-, -NH-CO-NH-, $-CH(NH_2)-$, $-CH(-NHR^{zc1})-$, $-CH(R^{zc2})-$,-CH(OH)-, -CH(COOH)-, -C(=NH)-, -S-, -S-S-, -SO-, $-SO_2-$, $-NH-SO_2-$, $-SO_2-NH-$,

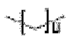, 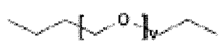, 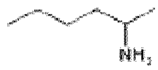, 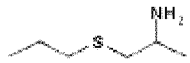,

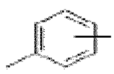, 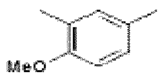, 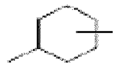,

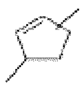, 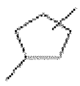, 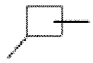, 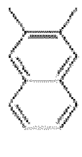, 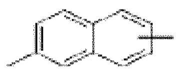,

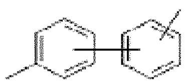, 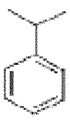, 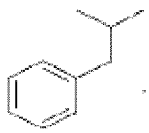, 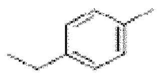,

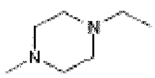, 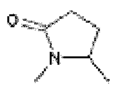, 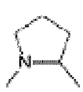, 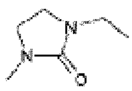,

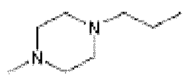, 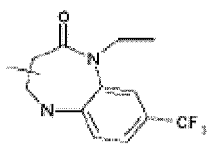, 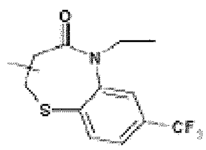.

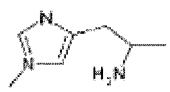, 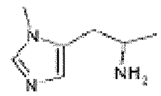

and

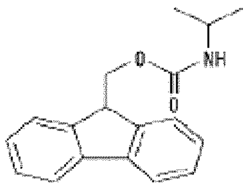

(wherein

u represents an integer of 1 to 18,
v represents an integer of 1 to 12,

$R^{zc1}$ represents an amino-straight $C_{1-5}$ alkyl-carbonyl group, or a X-straight $C_{1-5}$ alkyl group,
$R^{zc2}$ represents an amino-straight $C_{1-5}$ alkyl-carbonyl amino-straight $C_{1-5}$ alkyl group, and

X represents the same as those described above.), and
m2 represents an integer of 0 to 15.); and

$C^b$ represents a chemical bond, -CO-, or $-SO_2-$.); or

(ii) formula: $-Q^{c'}-C^{b'}-$
(wherein

$Q^{c'}$ represents formula: $-(CH_2)_{m1'}-Z^{c'}-(CH_2)_{m2'}-$
(wherein

m1' represents an integer of 0 to 15,
$Z^{c'}$ represents a bivalent group selected from

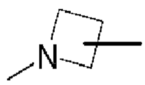, 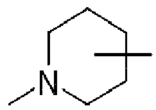, 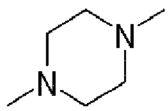, 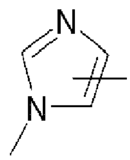,

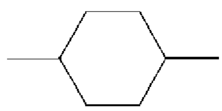, 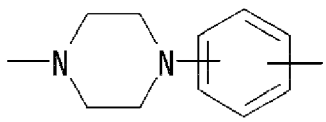, and 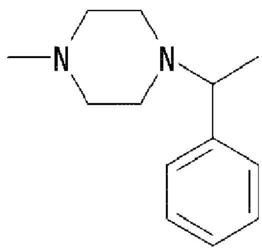

, and
m2' represents an integer of 0 to 15.); and

$C^{b'}$ represents -CO- or $-SO_2-$.).

Lc preferably represents:

(i) a bivalent group represented by formula: $-B-Q^c-C^b-$
(wherein

B represents a chemical bond, -NH-, -O-, or -S-,
$Q^c$ represents a bivalent group represented by formula:

$$-(CH_2)_{m1}-Z^c-(CH_2)_{m2}-$$

(wherein

m1 represents an integer of 0,
$Z^c$ represents:

(a) a chemical bond; or
(b) a bivalent group selected from: -CO-, $-CH(-NHR^{zc1})-$, $-CH(R^{zc2})-$,

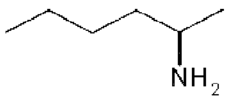
,
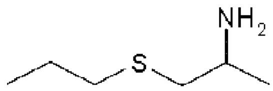
,
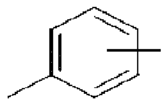

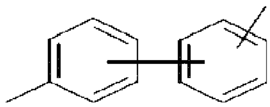
,
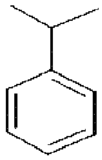
,
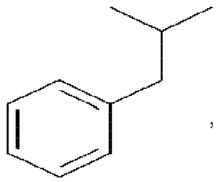
,

, and

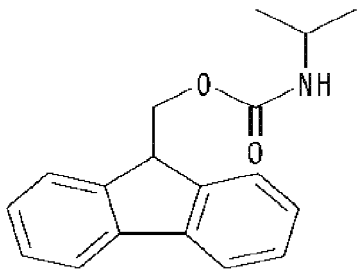

(wherein

u represents an integer of 1 to 18,
v represents an integer of 1 to 12,

$R^{zc1}$ represents an amino-straight $C_{1-5}$ alkyl-carbonyl group, or a X-straight $C_{1-5}$ alkyl group (X represents the same as those described above.), and
$R^{zc2}$ represents an amino-straight $C_{1-5}$ alkyl-carbonyl amino-straight $C_{1-5}$ alkyl group.), and

m2 represents an integer of 0 to 10.); and

$C^b$ represents a chemical bond, -CO-, or $-SO_2-$.); or

(ii) a bivalent group represented by formula: $-Q^{c'}-C^{b'}-$
(wherein

$Q^{c'}$ represents formula: $-(CH_2)_{m1'}-Z^{c'}-(CH_2)_{m2'}-$
(wherein

m1' represents 0,
$Z^{c'}$ represents a bivalent group selected from

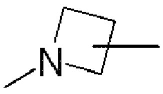 , 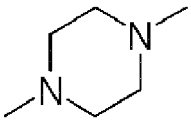 , and 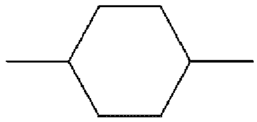 ,

and
m2' represents an integer of 0 to 2.); and

$C^{b'}$ represents -CO- or $-SO_2-$.).

j represents an integer of 1 to 3.
j is preferably an integer of 1 or 2.

Preferably, Lc is a bivalent group represented by

(i) formula: $-NH-Q^c-C^b-$
[wherein

$Q^c$ represents a bivalent group represented by formula: $-(CH_2)_{m1}-$ (m1 is an integer of 0 to 15), and
$C^b$ represents a chemical bond, -CO-, or $-SO_2-$.].

The nitrogen atom of NH in the formula: $-NH-Q^c-C^b-$ is a nitrogen atom to which asterisk (*) is attached below.

$$-N^*H-Q^c-C^b-$$

Furthermore, Lc is preferably a bivalent group represented by

(i) formula: $-B-Q^c-C^b-$
[wherein

B represents a chemical bond, -NH-, -O-, or -S-;
$Q^c$ represents a bivalent group selected from formula:

$$-(CH_2)_{m1}-Z^c-(CH_2)_{m2}-$$

(wherein

m1 represents an integer of 0 to 10,
$Z^c$ represents a chemical bond, -CO-, -O-CO-, -CO-O-, -CO-NH-, -NH-CO-, -CO-NH-CO-, -NH-CO-NH-, $-CH(NH_2)-$, $-CH(-NHR^{zc1})-$, $-CH(R^{zc2})-$,-CH(OH)-, -CH(COOH)-, -C(=NH)-, -S-, -S-S-, -SO-, $-SO_2-$, $-NH-SO_2-$, $-SO_2-NH-$,

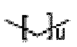 , 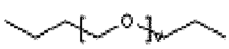 , 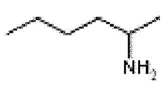 , 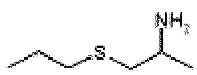 ,

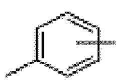 , 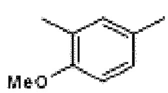 , 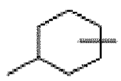 ,

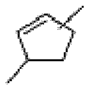,
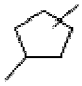,
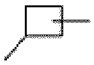,
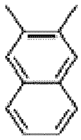,
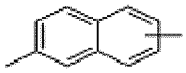,
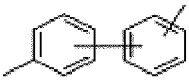,
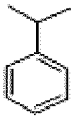,
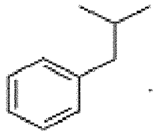,
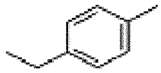,
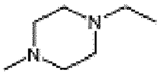,
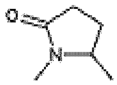,
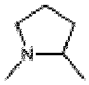,
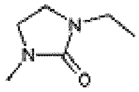,
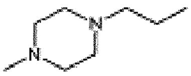,
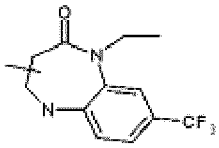,
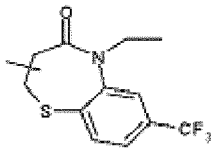,
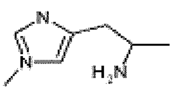,
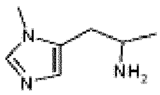,

**and**

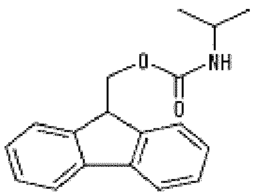

(wherein

u represents an integer of 1 to 10,
v represents an integer of 1 to 10,

$R^{zc1}$ represents an amino-straight $C_{1-5}$ alkyl-carbonyl group, or a X-straight $C_{1-5}$ alkyl group (X represents the same as those described above.),
$R^{zc2}$ represents an amino-straight $C_{1-5}$ alkyl-carbonyl amino-straight $C_{1-5}$ alkyl group), and
m2 represents an integer of 0 to 5.); and

$C^b$ represents a chemical bond, -CO-, or $-SO_2-$.].

Furthermore, Lc is preferably a bivalent group represented by

(ii) formula:

$-Q^{c'}-C^{b'}-$

[wherein

$Q^{c'}$ represents formula:

$$-(CH_2)_{m1}'-Z^{c'}-(CH_2)_{m2}'-$$

(wherein

m1' represents an integer of 0 to 15,
$Z^{c'}$ represents

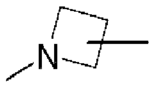 , 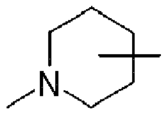 , 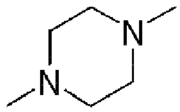 , 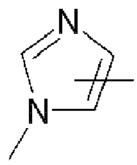 ,

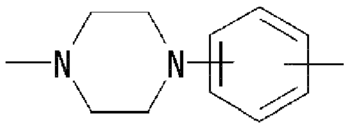 , or 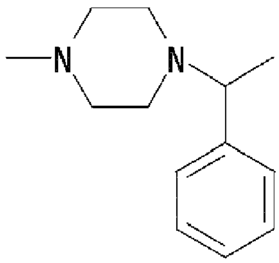

, and
m2' represents an integer of 0 to 15.); and

$C^{b'}$ represents a chemical bond, -CO- or $-SO_2-$.

Particularly, Lc is preferably a bivalent group represented by formula:

$$-NH-Q^c-C^b-$$

[wherein

$Q^c$ represents a bivalent group represented by formula:

$$-(CH_2)_{m1}-Z^c-(CH_2)_{m2}-$$

(wherein

m1 represents 0,
$Z^c$ represents a chemical bond, and
m2 represents 5.); and
$C^b$ represents -CO-.].

$(Lc)_j$ is preferably

(a) a chemical bond, or
(b) a bivalent group selected from:

$$-NH-(CH_2)_{mc1}-CO-,\ NH-(CH_2)_{mc2}-CO-NH-(CH_2)_{mc3}-CO-,$$

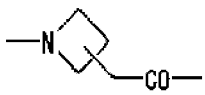 , 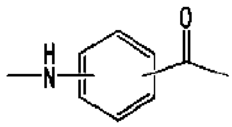 , 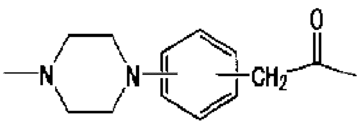 ,

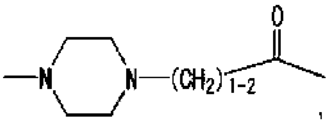 , 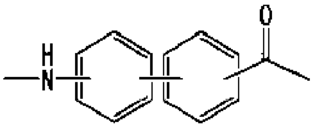 , 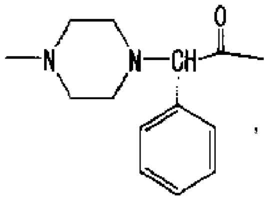 ,

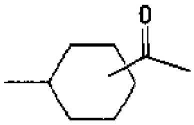 ,

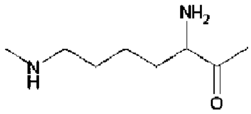 ,

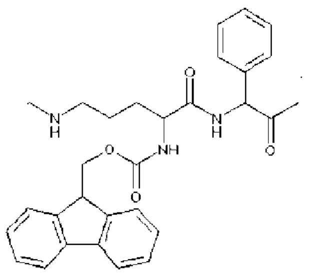 , 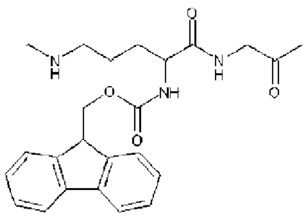 ,

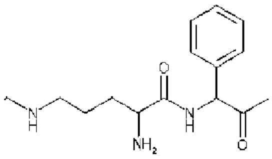 , 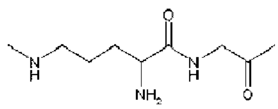 ,

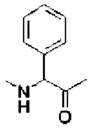 , 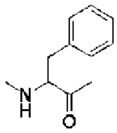 , 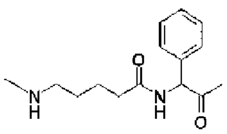 ,

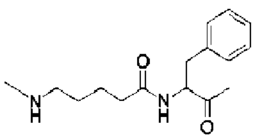 , 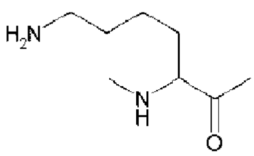 ,

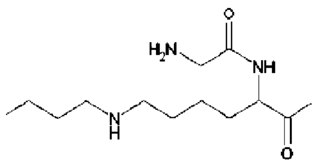 , 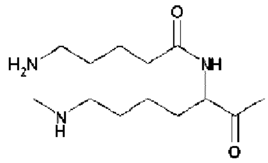 ,

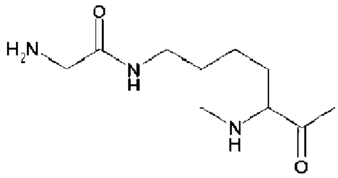 , 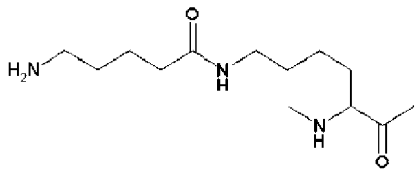 ,

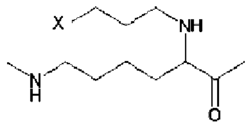

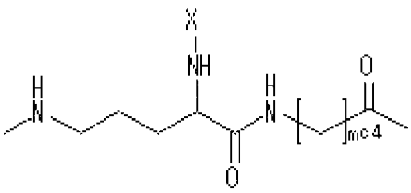 , 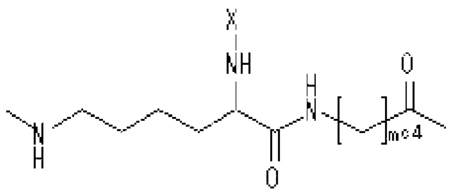 ,

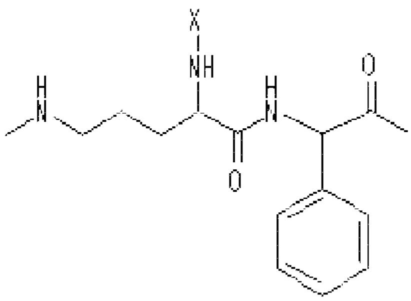 , and 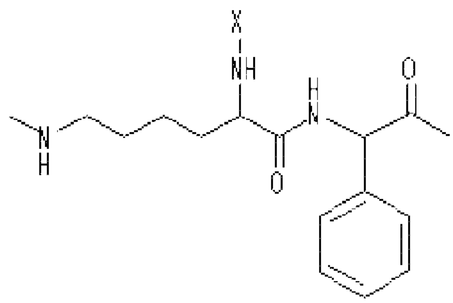

(wherein mc1 represents an integer of 1 to 11, each of mc2 and mc3 represents an integer of 1 to 5 (provided that the sum of mc2 and mc3 is preferably 4 to 7.), mc4 represents an integer of 1 to 5, and X represents the same as those described above.).

Furthermore, Lb is preferably a chemical bond, and $(Lc)_j$ is preferably

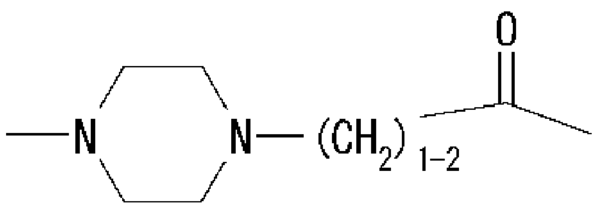

The moiety represented by the partial structure:

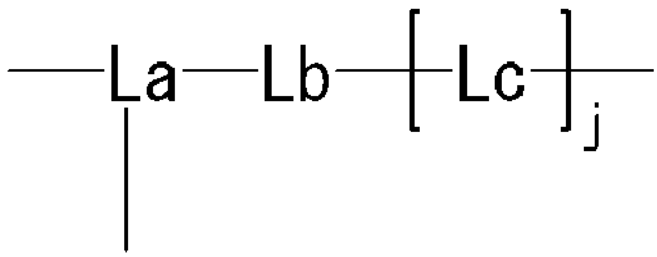

is preferably

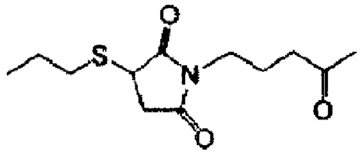,

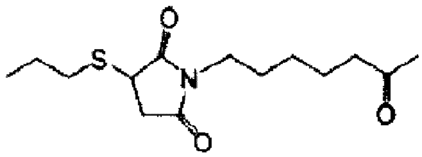

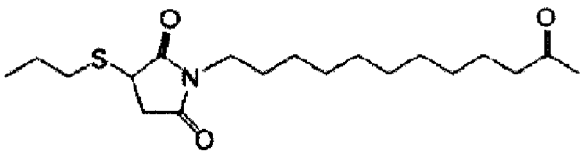.

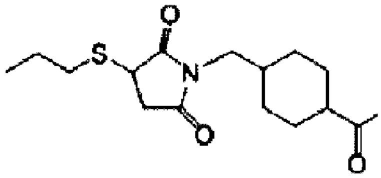

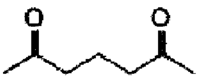,

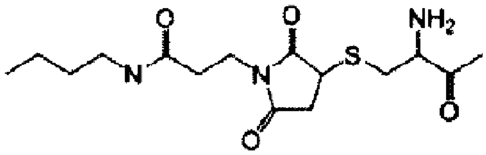,

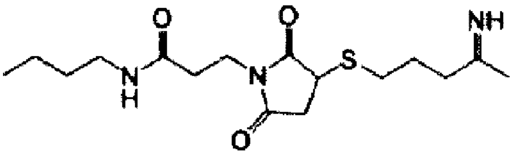,

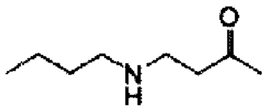

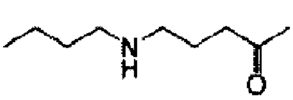.

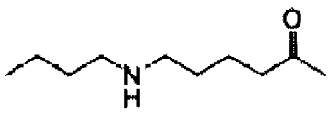

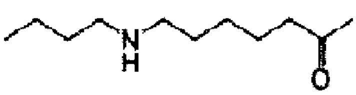.

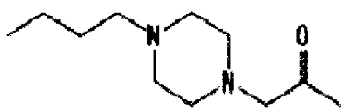

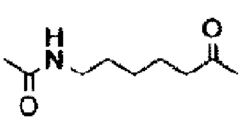,

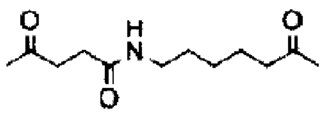,

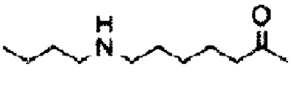,

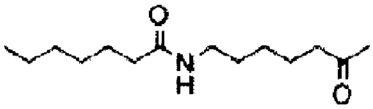,

$CO(CH_2)_2CO$, $(CH_2)_5CO$, $(CH_2)_3$, $CO(CH_2)_2CONH(CH_2)_5CH_2$, $(CH_2)_5CONH(CH_2)_5CH_2$ or $(CH_2)_3NH(CH_2)_5CH_2$.

The moiety represented by the partial structure:

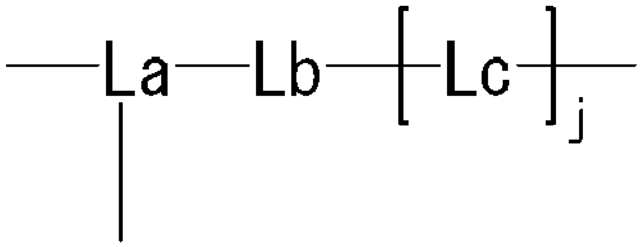

is particularly preferably $-CO-CH_2CH_2-CO-NH-(CH_2)_5-CO-$(also described as (CS)-CO-Acp- in the present specification), $-CH_2CH_2CH_2-NH-(CH_2)_5-CO-$(similarly, also described as (AL)-$CH_2$-Acp-), and $-CH_2CH_2CH_2CH_2CH_2CO-NH-(CH_2)_5-CO-$ (similarly, also described as (HS)-CO-Acp-), etc.

Furthermore, after the reaction, the PEG-modified metastin can be purified and isolated by a conventional purification method, for example, combination of solvent extraction, distillation, column chromatography, liquid chromatography, recrystallization and two phase distribution of PEG/dextran. When the PEG-modified metastin obtained by the above methods is in a free form, the PEG-modified metastin can be converted into an appropriate salt by a publicly known method; conversely when the PEG-modified metastin is obtained in a salt form, it can be converted into its free form by publicly known methods.

When the modified metastin of the present invention is present in the form of a configurational isomer, diastereomer, conformer, or the like, each can be isolated by the separating and purifying means described above, if desired. In addition, when the compound of the present invention is racemic, it can be separated into an S isomer and an R isomer by a conventional optical resolution means.

When steric isomers exist for the metastin derivative of the present invention, the present invention encompasses both of these isomers alone and isomers present as a mixture thereof.

In addition, the modified metastin of the present invention may also be hydrate or anhydrate. The modified metastin of the present invention may also be labeled with an isotope (e.g., $^{3}H$, $^{14}C$, $^{35}S$), etc.

As used herein, the peptides are represented in accordance with the conventional way of describing peptides, that is, the N-terminus (amino terminus) at the left hand and the C-terminus (carboxyl terminus) at the right hand. In the peptides, the C-terminus is usually in the form of an amide ($-CONH_2$), a carboxyl group (-COOH), a carboxylate ($-COO^-$), an alkyl amide (-CONHR) or an ester (-COOR) and the amide ($-CONH_2$) is particularly preferred. Examples of the ester or alkyl amide as R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, $\alpha$-naphthyl, etc.; a $C_{7-14}$ aralkyl group such as a phenyl-$C_{1-2}$-alkyl group(e.g., benzyl, phenethyl, benzhydryl, etc), or an a-naphthyl-$C_{1-2}$-alkyl group(e.g. $\alpha$-naphthylmethyl, etc.); pivaloyloxymethyl group, which are widely used as an ester for oral use, and the like.

Examples of the salts of the modified metastin of the present invention include metal salts, ammonium salts, salts with organic bases, salts with inorganic acids, salts with organic acids, salts with basic or acidic amino acids, and the like. Preferred examples of the metal salts include alkali metal salts such as sodium salts, potassium salts, etc.; alkaline earth metal salts such as calcium salts, magnesium salts, barium salts, etc.; aluminum salts; and the like. Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc. Preferred examples of the salts with inorganic acids include salts with hydrochloric acid, hydrobromic acid, nitric acid, sulfuric acid, phosphoric acid, etc. Preferred examples of salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Preferred examples of salts with basic amino acids include salts with arginine, lysine, ornithine, etc., and preferred examples of salts with acidic amino acids include salts with aspartic acid, glutamic acid, etc.

In particular, pharmaceutically acceptable salts are preferred. For example, when the compound has an acidic functional group therein, inorganic salts such as alkali metal salts (e.g., sodium salt, potassium salt, etc.), alkaline earth metal salts (e.g., calcium salt, magnesium salt, barium salt, etc.), ammonium salts, etc. are preferable. When the compound has a basic functional group therein, salts with inorganic acids with hydrochloric acid, hydrobromic acid, nitric acid, sulfuric acid, phosphoric acid, etc., and salts with organic acids such as acetic acid, phthalic acid, fumaric acid, oxalic acid, tartaric acid, maleic acid, citric acid, succinic acid, methanesulfonic acid, p-toluenesulfonic acid, etc. are preferred.

A prodrug of the metastin derivative modified with a polyethylene glycol (PEG) of the present invention or a salt thereof (hereinafter, sometimes simply referred to as the modified metastin of the present invention) refers to such

a modified metastin that is converted into the modified metastin of the present invention through a reaction with an enzyme, gastric acid, etc., in the living body under physiological conditions. In other words, the prodrug of the present invention refers to a modified metastin that undergoes enzymatic oxidation, reduction, hydrolysis, etc. to be converted into the modified metastin of the present invention, or a modified metastin that undergoes hydrolysis, etc. by gastric acid, etc. to be converted into the modified metastin of the present invention.

Examples of the prodrug of the modified metastin of the present invention include modified metastins wherein the amino group of the modified metastin of the present invention is acylated, alkylated, phosphorylated, etc. (e.g., modified metastins wherein the amino group of the modified metastin of the present invention is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, tert-butylated, etc); modified metastins wherein the hydroxyl group of the modified metastin of the present invention is acylated, alkylated, phosphorylated, borated, etc. (e.g., modified metastins wherein the hydroxyl group of the modified metastin of the present invention is acetylated, palmitoylated, propanoylated, pivaloylated, succinylated, fumarylated, alanylated, dimethylaminomethylcarbonylated, etc.); and modified metastins wherein the carboxy group of the metastin derivative of the present invention is esterified, amidated, etc. (e.g., modified metastins wherein the carboxy group of the modified metastin of the present invention is the ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified, methylamidated, etc); and the like. These prodrugs of the modified metastins can be produced from the modified metastin of the present invention by per se known methods.

The prodrugs of the modified metastin of the present invention may be those that are converted into the modified metastin of the present invention under the physiological conditions as described in "Iyakuhin no Kaihatsu (Pharmaceutical Research and Development)", Vol. 7 (Bunshisekkei (Drug Design)), pages 163-198, published 1990 by Hirokawa Publishing Co.

The modified metastin of the present invention or its salts or prodrugs (hereinafter sometimes simply referred to as the compound of the present invention) possess a cancer metastasis suppressing activity or a cancer growth suppressing activity. Thus, the modified metastinis useful for medicaments such as agents for preventing or treating all types of cancer (e.g., lung cancer, gastric cancer, liver cancer, pancreatic cancer, colorectal cancer, rectal cancer, colonic cancer, prostate cancer, ovarian cancer, cervical cancer, breast cancer, etc.), and the like.

The compound of the present invention also possesses the effect of controlling pancreatic function and is thus useful as a pharmaceutical such as an agent for preventing or treating various pancreatic diseases (e.g., acute or chronic pancreatitis, pancreatic cancer, etc.).

Furthermore, the compound of the present invention possesses the effect of controlling placental function and is thus useful as a pharmaceutical such as an agent for preventing or treating choriocarcinoma, hydatid mole, invasive mole, miscarriage, fetal hypoplasia, abnormal glucose metabolism, abnormal lipid metabolism or induction of delivery, etc.

Moreover, the compound of the present invention possesses the effects of increasing blood glucose level, promoting pancreatic glucagon secretion and promoting urine formation and is thus useful as hyperglycemic agents, pancreatic glucagon secretagogue agents or agents for promoting urine formation, and as a pharmaceutical such as agents for preventing or treating obesity, hyperlipemia, type II diabetes mellitus, hypoglycemia, hypertension, diabetic neuropathy, diabetic nephropathy, diabetic retinopathy, edema, urinary disturbances, insulin resistance, unstable diabetes mellitus, fatty atrophy, insulin allergy, insulinoma, arteriosclerosis, thrombotic disorders or lipotoxicity; and the like.

In addition, the compound of the present invention also possesses the effects of promoting gonadotropic hormone (e.g., FSH, LH, etc.) secretion, promoting sex hormone [e.g., androgens (e.g., testosterone, androstenedione, etc.), estrogens (e.g., estradiol, estrone, etc.), progesterones, etc.] secretion, improving gonadal function and inducing or stimulating ovulation, as well as a sexual maturation effect, etc., and hence, can be used as a pharmaceutical for improving gonadal function, a pharmaceutical for inducing or stimulating ovulation, a pharmaceutical for promoting gonadotropic hormone secretion or sex hormone secretion, or a pharmaceutical for preventing or treating hormone-dependent cancers [e.g., prostate cancer, breast cancer, etc.], infertility [e.g., irregular menstruation, dysmenorrhea, amenorrhea, weight loss-induced amenorrhea, secondary amenorrhea, anovulation, hypoovarianism, hypogonadism, spermatogenetic failure, hypogonadism (e.g., impotence, etc.), genital atrophy, testicular atrophy, testicular function disorder, azoospermia, hypoandrogenemia, etc.], endometriosis, precocious puberty, hysteromyoma, etc.

Furthermore, the prodrug of the metastin derivative of the present invention or its salt is useful as a pharmaceutical for preventing or treating Alzheimer's disease, moderate cognitive impairment, autism, etc.

Moreover, the compound of the present invention is useful as a pharmaceutical for preventing or treating rheumatic diseases (e.g., rheumatoid arthritis, osteoarthritis, gout, etc.).

The compound of the present invention is also useful as a pharmaceutical for preventing or treating autism, an immunostimulator (thymus regeneration, thymic regrowth, enhanced T cell growth), a pharmaceutical for preventing or treating hypertension, diabetic neuropathy, diabetic nephropathy, diabetic retinopathy, thrombotic disorders or lipotoxicity;

a pharmaceutical for preventing or treating hyperlipemia, type II diabetes mellitus, hypertension, diabetic neuropathy, diabetic nephropathy or diabetic retinopathy; an antianxiety agent; an antistress agent; an anti-insomnia agent; an antimanic-depressive agent; a pharmaceutical for preventing or treating hypertension (e.g., essential hypertension, renal hypertension, salt sensitive hypertension, etc.), angina pectoris (e.g., stable angina, unstable angina, etc.), myocardial infarction, cerebrovascular disorders (e.g., asymptomatic cerebrovascular disorder, transient ischemic attack, apoplexy, cerebrovascular dementia, hypertensive encephalopathy, cerebral infarction, etc.), venous insufficiency, obliterative peripheral circulatory disturbances, Raynaud's disease, arteriosclerosis including atherosclerosis (e.g., aneurysm, coronary arteriosclerosis, cerebral arteriosclerosis, peripheral arteriosclerosis, etc.), vascular thickening or occlusion and organ impairments after intervention (e.g., percutaneous coronary intervention, stent placement, coronary thrombolytic therapy, etc.), portal hypertension, respiratory disorders (e.g., asthma, pulmonary hypertension, etc.); a pharmaceutical for preventing or treating impaired glucose tolerance (IGT); an insulin secretagogue, an inhibitor for transition from IGT to diabetes; and,

a pharmaceutical for preventing or treating diabetic complications [e.g., neuropathy, nephropathy, retinopathy, cataract, macroangiopathy, osteopenia, hyperosmolar diabetic coma, infectious diseases (e.g., respiratory infection, urinary tract infection, digestive tract infection, skin soft-tissue infection, lower leg infection), diabetic gangrene, xerostomia, hypacusis, cerebrovascular disorder, peripheral circulatory disturbance], osteoporosis, cachexia (e.g., cancerous cachexia, tuberculous cachexia, diabetic cachexia, blood disease cachexia, endocrine disease cachexia, infectious disease cachexia or cachexia due to acquired immunodeficiency syndrome), fatty liver, polycystic ovary syndrome, renal diseases (e.g., diabetic nephropathy, glomerulonephritis, glomerulosclerosis, nephrotic syndrome, hypertensive nephrosclerosis, end stage kidney disease), muscular dystrophy, cardiac infarction, angina pectoris, cerebrovascular disorders (e.g., cerebral infarction, apoplexy), Alzheimer's disease, Parkinson's disease, dementia, insulin resistance syndrome, Syndrome X, metabolic syndrome, hyperinsulinemia, hyperinsulinemia-induced sensory disorders, tumors (e.g., leukemia, skin cancer), irritable bowel syndrome, acute or chronic diarrhea, inflammatory diseases (e.g., spondylitis deformans, arthritis deformans, lumbago, gout, postoperative or posttraumatic inflammation, swelling, neuralgia, pharyngolaryngitis, cystitis, hepatitis (including nonalcoholic steatohepatitis), pneumonia, enteritis, inflammatory bowel disorder (including inflammatory bowel diseases), ulcerative colitis, gastric mucosal injury (including gastric mucosal injury induced by aspirin)), small intestinal mucosal injury, malabsorption, testis function disorder, visceral obesity syndrome, etc.; and,

The compound of the present invention is also useful for reduction of visceral adiposity, inhibition of visceral fat accumulation, improvement of glucose metabolism, improvement of lipid metabolism, suppression of oxidized LDL production, improvement of lipoprotein metabolism, improvement of coronary artery metabolism, prevention or treatment of cardiovascular complications, prevention or treatment of heart failure complications, decrease in blood remnant, prevention or treatment of anovulation, prevention or treatment of hirsutism, or prevention or treatment of hyperandrogenemia; as a pharmaceutical for improving pancreatic (β cell) function, a pharmaceutical for regeneration of the pancreas (β cells), a pharmaceutical for stimulating pancreatic (β cell) regeneration, an appetite regulator, etc.

These compounds can also be used in combination with drugs other than the compounds described above.

As drugs (hereinafter sometimes simply referred to as concomitant drugs) that can be used in combination with these compounds, the medicaments described above as drugs that can be used in combination with the compound of the present invention can be used in a similar manner.

The compound of the present invention can be used in combination with medicaments, e.g., chemotherapeutic agents for treating cancer, hormonal therapeutic agents, immunotherapeutic agents, drugs for inhibiting the actions of cell growth factors and their receptors, etc. (hereinafter simply referred to as concomitant drugs). Examples of the "chemotherapeutic agents" are alkylating agents, metabolic antagonists, anticancer antibiotics, plant-derived anticancer agents, etc. Specifically, the drugs described above can be used.

Besides, the compound of the present invention has excellent blood stability and pharmacokinetics, as compared to native metastin such as metastin 54 (1-54) or metastin 10 (45-54). The metastin derivative modified with PEG of the present invention or its salt or prodrug, the metastin per se, DNA encoding metastin, etc. are useful as a pharmaceutical for suppressing gonadotropic hormone (e.g., FSH, LH) secretion or sex hormone [e.g., androgen (e.g., testosterone, androstenedione), estrogen (e.g., estradiol, estrone), progesterone] secretion; in particular, it is useful for suppressing gonadotropic hormone secretion or sex hormone secretion through down-regulation of gonadotropic hormone or sex hormone (wherein, the down-regulation of gonadotropic hormone or sex hormone may be pulse loss of LHRH or depletion of LHRH) or down-regulation of human OT7T175 (metastin receptor) protein consisting of the amino acid sequence represented by SEQ ID NO: 9; particularly useful as a pharmaceutical for preventing or treating hormone-dependent cancers (e.g., prostate cancer, breast cancer, etc.; especially hormone-sensitive prostate cancer, hormone-sensitive breast cancer, etc.); a pharmaceutical for preventing or treating endometriosis; a pharmaceutical for inhibiting ovarian follicular maturation; a menstrual cycle-suspending agent; a pharmaceutical for treating hysteromyoma; a pharmaceutical for treating precocious puberty; or as a contraceptive, etc. Where the metastin derivative of the present invention or its salt or prodrug, metastin per se, or DNA encoding metastin, etc. have normal agonist activity, an effective dose of the metastin derivative sufficient to suppress the secretion of gonadotropic hormone or sex hormone is administered at the

site or tissue where the therapeutic effects are to be exerted, so that the metastin derivative is present in a dose more than required (i.e., the metastin derivative is administered in an excess over the normal effective dose, at which the metastin derivative exerts the effects of suppressing cancer metastasis, suppressing cancer growth, etc.; or the gonadotropic hormone secretion agent, the effect of promoting sex hormone secretion, etc.) to exhibit the effects of suppressing gonadotropic hormone secretion or sex hormone secretion. Specific examples include sustained or continuous administration of the normal effective dose (including an administration technique to gradually release the pharmaceutical ingredients by bolus administration); and the like. Further when the metastin derivative of the present invention or its salt or the prodrug thereof, etc. has a sufficient agonist activity more than required (a super-agonist activity), it becomes possible to sustain the activities more than exhibited by the necessary dose at the site or tissue where the therapeutic effect are to be exhibited. It is therefore sufficient even by normal effective dose administration to suppress the secretion of gonadotropic hormone or sex hormone, whereby the effect of suppressing gonadotropic hormone secretion or sex hormone secretion is exhibited.

In other words, an effective dose of the metastin derivative of the present invention or its salt or prodrug, metastin per se, or DNA encoding metastin, etc. sufficient to suppress the secretion of gonadotropic hormone or sex hormone is administered, so that the metastin derivative is present in a dose more than required at the site or tissue where the therapeutic effects are to be exerted, or its activities can be sustained more than required, which enables to exhibit the effects of suppressing gonadotropic hormone secretion or suppressing sex hormone secretion.

The pharmaceutical comprising the compound of the present invention is low toxic. Therefore, the compound of the present invention can be safely administered either directly as it is or as a mixture with pharmacologically acceptable carriers, orally or parenterally (e.g., topically, rectally, intravenously, etc.), in the form of pharmaceutical preparations such as tablets (including dragees and film-coated tablets), powders, granules, capsules (including soft capsules), liquid dosage forms, injections, suppositories, sustained release dosage forms, etc., in accordance with publicly known means generally used in process for producing pharmaceutical preparations.

The compound of the present invention is contained in the pharmaceutical preparation of the present invention in about 0.01 to about 100 wt%, based on the total weight of the preparation.

A dose of the compound of the present invention may vary depending upon subject to be administered, target organ, conditions, route of administration, etc., and in oral administration, the compound is generally administered to the patient with cancer (as 60 kg body weight) in a daily dose of about 0.01 to about 100 mg, preferably about 0.1 to about 50 mg and more preferably about 0.1 to about 20 mg. In parenteral administration, a single dose of the compound may vary depending upon subject to be administered, target organ, conditions, route of administration, etc., and in the form of an injectable dosage form, it is advantageous to administer the compound to the patient with cancer (as 60 kg body weight) via intravenous, subcutaneous or intramuscular injection generally in a daily dose of about 0.001 to about 30 mg, preferably about 0.01 to about 20 mg, and more preferably about 0.01 to about 10 mg. For other animal species, the corresponding dose as converted per 60 kg weight can be administered.

Pharmacologically acceptable carriers, which may be used to manufacture the pharmaceutical of the present invention, include various organic or inorganic carrier substances conventionally used as materials for pharmaceutical preparations. These substances include, e.g., an excipient, a lubricant, a binder and a disintegrating agent in a solid dosage form, and a solvent, a dissolution aid, a suspending agent, an isotonizing agent, a buffer, a soothing agent, etc. in a liquid dosage form. In addition, conventional additives such as a preservative, an antioxidant, a colorant, a sweetener, an adsorbent, a wetting agent, etc. can be appropriately used in suitable amounts, if necessary.

Examples of excipients include lactose, saccharose, D-mannitol, starch, cornstarch, crystalline cellulose, light anhydrous silicic acid, etc. Examples of lubricants include magnesium stearate, calcium stearate, talc, colloidal silica, etc.

Examples of binders include crystalline cellulose, saccharose, D-mannitol, dextrin, hydroxypropylcellulose, hydroxypropylmethylcellulose, polyvinylpyrrolidone, starch, sucrose, gelatin, methylcellulose, sodium carboxymethylcellulose, etc.

Examples of disintegrating agents include starch, carboxymethylcellulose, carboxymethylcellulose calcium, sodium carboxymethyl starch, L-hydroxypropylcellulose, etc.

Examples of solvents include water for injection, alcohol, propylene glycol, Macrogol, sesame oil, corn oil, olive oil, etc.

Examples of dissolution aids include polyethylene glycol, propylene glycol, D-mannitol, benzyl benzoate, ethanol, trisaminomethane, cholesterol, triethanolamine, sodium carbonate, sodium citrate, etc.

Examples of suspending agents include surfactants such as stearyltriethanolamine, sodium laurylsulfate, lauryl aminopropionate, lecithin, benzalkonium chloride, benzethonium chloride, glycerin monostearate, etc.; hydrophilic polymers such as polyvinyl alcohol, polyvinyl pyrrolidone, sodium carboxymethylcellulose, methylcellulose, hydroxymethylcellulose, hydroxyethylcellulose, hydroxypropylcellulose, etc.

Examples of isotonizing agents include glucose, D-sorbitol, sodium chloride, glycerin, D-mannitol, etc.

Examples of buffers include buffering solutions of a phosphate, acetate, carbonate, citrate, etc.

Examples of soothing agents include benzyl alcohol, etc.

Examples of preservatives include p-hydroxybenzoates, chlorobutanol, benzyl alcohol, phenethyl alcohol, dehydroacetic acid, sorbic acid, etc.

Examples of antioxidants include a sulfite, ascorbic acid, α-tocopherol, etc.

In addition, the compound of the present invention can be used in combination with drugs other than the compound of the present invention.

The drugs that can be used in combination with the compound of the present invention (hereinafter sometimes simply referred to as concomitant drugs) include medicaments such as chemotherapeutic agents for treating cancer, hormonal therapeutic agents, immunotherapeutic agents, drugs for inhibiting the actions of cell growth factors and their receptors, etc. (hereinafter simply referred to as concomitant drugs).

The "chemotherapeutic agent" includes, for example, an alkylating agent, a metabolic antagonist, an anticancer antibiotic, a plant-derived anticancer agent, etc.

Examples of the "alkylating agent" include nitrogen mustard, nitrogen mustard-N-oxide hydrochloride, chlorambutyl, cyclophosphamide, ifosfamide, thiotepa, carboquone, improsulfan tosylate, busulfan, nimustine hydrochloride, mitobronitol, melphalan, dacarbazine, ranimustine, estramustine sodium phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulphan, trophosphamide, zinostatin stimalamer, carboquone, adozelesin, cystemustine, bizelesin, etc.

Examples of the "metabolic antagonist" include mercaptopurine, 6-mercaptopurine riboside, thioinosine, methotrexate, enocitabine, cytarabine, cytarabine ocfosfate, ancitabine hydrochloride, 5-FU drugs (e.g., fluorouracil, tegafur, UFT, doxifluridine, carmofur, gallocitabine, emmitefur, etc.), aminopterin, leucovorin calcium, tabloid, butocine, folinate calcium, levofolinate calcium, cladribine, emitefur, fludarabine, gemcitabine, hydroxycarbamide, pentostatin, piritrexim, idoxuridine, mitoguazone, thiazophrine, ambamustine, etc.

Examples of the "anticancer antibiotic" include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarcomycin, carzinophilin, mitotane, zorubicin hydrochloride, mitoxantrone hydrochloride, idarubicin hydrochloride, etc.

Examples of the "plant-derived anticancer agent" include etoposide, etoposide phosphate, vinblastine sulfate, vincristine sulfate, vindesine sulfate, teniposide, paclitaxel, docetaxel, vinorelbine, etc.

Examples of the "hormonal therapeutic agent" include fosfestrol, diethylstylbestrol, chlorotrianisene, medroxyprogesterone acetate, megestrol acetate, chlormadinone acetate, cyproterone acetate, danazol, allylestrenol, gestrinone, mepartricin, raloxifene, ormeloxifene, levormeloxifene, antiestrogens (e.g., tamoxifen citrate, toremifene citrate, etc.), pill dosage forms, mepitiostane, testrolactone, aminoglutethimide, LH-RH agonists (e.g., goserelin acetate, buserelin, leuprorelin, etc.), droloxifene, epitiostanol, ethinylestradiol 1 sulfonate, aromatase inhibitors (e.g., fadrozole hydrochloride, anastrozole, retrozole, exemestane, vorozole, formestane, etc.), anti-androgens (e.g., flutamide, bicartamide, nilutamide, etc.), 5α-reductase inhibitors (e.g., finasteride, epristeride, etc.), adrenocorticohormone drugs (e.g., dexamethasone, prednisolone, betamethasone, triamcinolone, etc.), androgen synthesis inhibitors (e.g., abiraterone, etc.), retinoid and drugs that retard retinoid metabolism (e.g., liarozole, etc.), ER downregulator (e.g. fulvestrant (Faslodex (trademark), etc.), etc., and among others, LH-RH agonists (e.g., goserelin acetate, buserelin, leuprorelin, etc.) are preferred.

Examples of the "immunotherapeutic agent (BRM)" include picibanil, krestin, sizofiran, lentinan, ubenimex, interferons, interleukins, macrophage colony-stimulating factor, granulocyte colony-stimulating factor, erythropoietin, lymphotoxin, BCG vaccine, Corynebacterium parvum, levamisole, polysaccharide K, procodazole, etc.

The "cell growth factor" in the "drugs for inhibiting the actions of cell growth factors and their receptors" can be any substance so long as it is a substance capable of stimulating the cell growth and, normally, peptides which have a molecular weight of 20,000 or less and bind to their receptors to exhibit the actions in a lower level can be used as the factor. Specific examples are (1) EGF (epidermal growth factor) or a substance having substantially the same activity as EGF [e.g., EGF, hereglin, etc.], (2) insulin or a substance having substantially the same activity as insulin [e.g., insulin, IGF (insulin-like growth factor)-1, IGF-2, etc.], (3) FGF (fibroblast growth factor) or a substance having substantially the same activity as FGF [e.g., acidic FGF, basic FGF, KGF (keratinocyte growth factor), FGF-10, etc.], (4) other cell growth factors [e.g., CSF (colony stimulating factor), EPO (erythropoietin), IL-2 (interleukin-2), NGF (nerve growth factor), PDGF (platelet-derived growth factor), TGFβ (transforming growth factor β), HGF (hepatocyte growth factor), VEGF (vascular endothelial growth factor), etc.] and the like.

The "receptor of the cell growth factor" can be any receptor as long as it is capable of binding to the cell growth factors described above, and specific examples are EGF receptor, hereglin receptor (HER2), insulin receptor, IGF receptor, FGF receptor-1 or FGF receptor-2, etc.

The "drug for inhibiting the actions of cell growth factors" includes HER2 antibody (trastuzumab (Herceptin (trademark)), etc.), imatinib mesylate, ZD1839 or EGFR antibody (cetuximab (Erbitux (trademark)), etc.), antibody against VEGF (e.g., bevacizumab (Avastin (trademark))), VEGFR antibody, VEGFR inhibitor, EGFR inhibitor (erlotinib (Tarceva

(trademark)) and gefitinib (Iressa (trademark)), etc.).

In addition to the medicaments described above, there are also used L-asparginase, aceglatone, procarbazine hydrochloride, protoporphyrin-cobalt complex, mercury-hematoporphyrin sodium, topoisomerase I inhibitor (e.g., irinotecan, topotecan, etc.), topoisomerase II inhibitor (e.g., sobzoxan, etc.), differentiation-inducing agent (e.g., retinoid, vitamin D group, etc.), angiogenesis inhibitor (e.g., thalidomide, SU11248, etc.), $\alpha$-blocker (e.g., tamsulosin hydrochloride, naftopidil, urapidil, alfuzosin, terazosin, prazosin, silodosin, etc.), serine-threonine kinase inhibitor, endothelin receptor antagonist (e.g., atrasentan, etc.), proteasome inhibitor (e.g., bortezomib, etc.), Hsp90 inhibitor (e.g., 17-AAG, etc.), spironolactone, minoxidil, 11$\alpha$-hydroxyprogesterone, bone resorption inhibitor or bone metastasis suppressor (e.g., zoledronic acid, alendronic acid, pamidronic acid, etidronic acid, ibandronic acid, clodronic acid), etc.

Use of the compound of the present invention in combination with the concomitant drug exhibits the following excellent effects.

(1) The dose can be reduced as compared to the dose when the compound of the present invention or the concomitant drug is administered alone.
(2) A drug concomitantly administered with the compound of the present invention can be chosen depending on the condition (mild, severe, etc.) of a patient.
(3) The concomitant drug, whose functional mechanism is different from that of the compound of the present invention, can be chosen so that a treatment period can be set longer.
(4) The concomitant drug, whose functional mechanism is different from that of the compound of the present invention, can be chosen so that sustained therapeutic effects can be achieved.
(5) Synergistic effects can be obtained by the use of the compound of the present invention in combination with the concomitant drug.

Hereinafter, the use of the compound of the present invention in combination with the concomitant drug is referred to as "the concomitant preparation of the present invention."

When the concomitant preparation of the present invention is used, a dosing period of the compound of the present invention and the concomitant drug is not limited; the compound of the present invention or its pharmaceutical composition and the concomitant drug or its pharmaceutical composition may be administered to the subject to be administered either simultaneously or at certain time intervals. The dose of the concomitant drug may be modified according to the dose used clinically and may be appropriately chosen depending upon subject to be administered, route for administration, disease, combination, etc.

A mode for administration of the concomitant preparation of the present invention is not particularly limited, but it is sufficient that the compound of the present invention is used in combination with the concomitant drug at the time of administration. For such mode of administration, there are, for example, (1) administration of a single dosage form obtained by mixing the compound of the present invention and the concomitant drug together at the same time, (2) simultaneous administration of two dosage forms prepared separately from the compound of the present invention and the concomitant drug through the same route for administration, (3) administration of two dosage forms prepared separately from the compound of the present invention and the concomitant drug at certain time intervals through the same route for administration, (4) simultaneous administration of two dosage forms prepared separately from the compound of the present invention and the concomitant drug through different routes for administration, (5) administration of two dosage forms prepared separately from the compound of the present invention and the concomitant drug at certain time intervals (e.g., administration of the compound of the present invention and the concomitant drug in this order, or administration in a reversed order) through different routes for administration, etc.

The concomitant preparation of the present invention is low toxic and thus can be safely administered orally or parenterally (e.g., topically, rectally, intravenously, etc.) in the form of pharmaceutical preparations such as tablets (including dragees and film-coated tablets), powders, granules, capsules (including soft capsules), liquid dosage forms, injections, suppositories, sustained release dosage forms, etc., which are obtained by mixing the compound of the present invention or (and) the concomitant drug described above with pharmacologically acceptable carriers. Injectable dosage forms can be administered intravenously, intramuscularly or subcutaneously, into the organ, or directly at the focus.

Pharmacologically acceptable carriers, which may be used to manufacture the concomitant preparation of the present invention, include various organic or inorganic carrier substances conventionally used as materials for pharmaceutical preparations. These substances include, e.g., an excipient, a lubricant, a binder and a disintegrating agent in a solid dosage form, and a solvent, a dissolution aid, a suspending agent, an isotonizing agent, a buffer, a soothing agent, etc. in a liquid dosage form. In addition, conventional additives such as a preservative, an antioxidant, a colorant, a sweetener, an adsorbent, a wetting agent, etc. can be appropriately used in suitable amounts, if necessary.

Examples of excipients include lactose, saccharose, D-mannitol, starch, cornstarch, crystalline cellulose, light anhydrous silicic acid, etc.

Examples of lubricants include magnesium stearate, calcium stearate, talc, colloidal silica, etc.

Examples of binders include crystalline cellulose, saccharose, D-mannitol, dextrin, hydroxypropylcellulose, hydroxypropylmethylcellulose, polyvinylpyrrolidone, starch, sucrose, gelatin, methylcellulose, sodium carboxymethylcellulose, etc.

Examples of disintegrating agents include starch, carboxymethylcellulose, carboxymethylcellulose calcium, sodium carboxymethyl starch, L-hydroxypropylcellulose, etc.

Examples of solvents include water for injection, alcohol, propylene glycol, Macrogol, sesame oil, corn oil, olive oil, etc.

Examples of dissolution aids include polyethylene glycol, propylene glycol, D-mannitol, benzyl benzoate, ethanol, trisaminomethane, cholesterol, triethanolamine, sodium carbonate, sodium citrate, etc.

Examples of suspending agents include surfactants such as stearyltriethanolamine, sodium laurylsulfate, lauryl aminopropionate, lecithin, benzalkonium chloride, benzethonium chloride, glycerin monostearate, etc.; hydrophilic polymers such as polyvinyl alcohol, polyvinyl pyrrolidone, sodium carboxymethylcellulose, methylcellulose, hydroxymethylcellulose, hydroxyethylcellulose, hydroxypropylcellulose, etc.

Examples of isotonizing agents include glucose, D-sorbitol, sodium chloride, glycerin, D-mannitol, etc.

Examples of buffers include buffering solutions of a phosphate, acetate, carbonate, citrate, etc.

Examples of soothing agents include benzyl alcohol, etc.

Examples of preservatives include p-hydroxybenzoates, chlorobutanol, benzyl alcohol, phenethyl alcohol, dehydroacetic acid, sorbic acid, etc.

Examples of antioxidants include a sulfite, ascorbic acid, $\alpha$-tocopherol, etc.

In the concomitant preparation of the present invention, a ratio of the compound of the present invention to the concomitant drug may be appropriately chosen depending upon subject to be administered, route for administration, disease, etc.

For example, the amount of the compound of the present invention contained in the concomitant preparation of the present invention varies depending on the dosage form of the preparation, but is usually about 0.01 to 100% by weight, preferably about 0.1 to 50% by weight, and more preferably about 0.5 to 20% by weight, based on the total weight of the preparation.

The amount of the concomitant drug contained in the concomitant preparation of the present invention varies depending on the dosage form of the preparation, but is usually about 0.01 to 100% by weight, preferably about 0.1 to 50% by weight, and more preferably about 0.5 to 20% by weight, based on the total weight of the preparation.

The amount of additives such as a carrier, etc. contained in the concomitant preparation of the present invention varies depending on the dosage form of the preparation, and is usually about 1 to 99.99% by weight, preferably about 10 to 90% by weight, based on the total weight of the preparation.

These amounts may be the same, also when the compound of the present invention and the concomitant drug are separately prepared.

These preparations can be manufactured by per se publicly known methods conventionally used in the step of manufacturing pharmaceutical preparations.

For example, an injectable dosage form can be prepared into an aqueous injection of the compound of the present invention or the concomitant drug together with a dispersing agent (e.g., Tween 80 (manufactured by Atlas Powder Company, USA), HCO 60 (manufactured by Nikko Chemicals Co., Ltd.), polyethylene glycol, carboxymethyl cellulose, sodium alginate, hydroxypropylmethyl cellulose, dextrin, etc.), a stabilizer (e.g., ascorbic acid, sodium pyrosulfite), a surfactant (e.g., polysorbate 80, macrogol, etc.), a solubilizing agent (e.g., glycerin, ethanol, etc.), a buffering agent (e.g., phosphoric acid or its alkali metal salt, citric acid or its alkali metal salt, etc.), an isotonizing agent (e.g., sodium chloride, potassium chloride, mannitol, sorbitol, glucose, etc.), a pH adjusting agent (e.g., hydrochloric acid, sodium hydroxide, etc.), a preservative (e.g., ethyl p-oxybenzoate, benzoic acid, methylparabene, propylparabene, benzyl alcohol, etc.), a solubilizer (e.g., concentrated glycerin, meglumine, etc.), a dissolution aid (e.g., propylene glycol, saccharose, etc.), a soothing agent (e.g., glucose, benzyl alcohol, etc.), and the like; or, by dissolving, suspending or emulsifying the compound of the present invention or the concomitant drug in a vegetable oil such as olive oil, sesame oil, cottonseed oil, corn oil, etc. and a dissolution aid such as propylene glycol or the like to dissolve, suspend or emulsify and prepare into an oily injection; which can be used as the injectable dosage form.

An oral dosage form can be produced in a conventional manner by adding to the compound of the present invention or the concomitant drug, for example, an excipient (e.g., lactose, saccharose, starch, etc.), a disintegrating agent (e.g., starch, calcium carbonate, etc.), a binder (e.g., starch, gum arabic, carboxymethyl cellulose, polyvinylpyrrolidone, hydroxypropyl cellulose, etc.), a lubricant (e.g., talc, magnesium stearate, polyethylene glycol 6000, etc.) and other additives, compressing the resulting mixture and, if necessary, coating the compressed product for the purpose of taste masking, enteric degradation or sustained release by techniques per se publicly known. Coating agents for this purpose include, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose

phthalate, hydroxymethyl cellulose acetate succinate, Eudragit (manufactured by Rohm Company, Germany, methacrylic acid-acrylic acid copolymer) and pigments (e.g., colcothar, titanium dioxide). The oral dosage form may be either an immediate release dosage form or a sustained release dosage form.

For example, to make a suppository, the compound of the present invention or the concomitant drug is prepared into an oily or aqueous solid, semisolid or liquid suppository composition by techniques per se publicly known. Oily bases used for the composition described above include glycerides of higher fatty acids [e.g., cacao butter, Witepsols (manufactured by Dynamite Nobel Company, Germany), etc.], moderate fatty acids [e.g., miglyols (manufactured by Dynamite Nobel Company, Germany), etc.], vegetable oils (e.g., sesame oil, soybean oil, cottonseed oil, etc.), and the like. Aqueous bases include, for example, polyethylene glycols and propylene glycol. Aqueous gel bases include, for example, natural rubbers, cellulose derivatives, vinyl polymers, acrylic polymers, etc.

The sustained release dosage form above includes sustained release microcapsules, and the like.

Sustained release microcapsules can be obtained by per se publicly known methods, and are preferably prepared in the form of, e.g., a sustained release dosage form by the method [2] shown below for administration.

Preferably, the compound of the present invention is prepared into a dosage form for oral administration such as a solid dosage form (e.g., powders, granules, tablets, capsules) or into a dosage form for rectal administration such as a suppository, etc. A dosage form for oral administration is particularly preferred.

The concomitant drug can be prepared into the dosage form described above, depending on kind of the drug.

Hereinafter, [1] an injectable preparation of the compound of the present invention or the concomitant drug and its production, [2] a sustained release or immediate release preparation of the compound of the present invention or the concomitant drug and its production and [3] a sublingual, buccal or rapid oral disintegrating preparations of the compound of the present invention or the concomitant drug and its production are specifically described below.

Injectable Preparation and its Production

[1] An injectable preparation obtained by dissolving the compound of the present invention or the concomitant drug in water is preferred. The injectable preparation may contain a benzoate and/or a salicylate.

The injectable preparation is obtained by dissolving the compound of the present invention or the concomitant drug and optionally a benzoate and/or a salicylate in water.

Examples of the benzoate and/or salicylate described above include an alkali metal salt such as sodium and potassium salts, etc., an alkaline earth metal salt such as calcium and magnesium salts, etc., an ammonium salt, a meglumine salt, a salt of an organic acid such as trometamol, and the like.

The concentration of the compound of the present invention or the concomitant drug in the injectable preparation is 0.05 to 50 w/v %, preferably about 0.3 to 20 w/v %. The concentration of the benzoate and/or salicylate is 0.5 to 50 w/v %, preferably 3 to 20 w/v %.

Furthermore, additives generally used in an injectable preparation such as a stabilizer (ascorbic acid, sodium pyrosulfite, etc.), a surfactant (polysorbate 80, macrogol, etc.), a solubilizing agent (glycerin, ethanol, etc.), a buffering agent (phosphoric acid and its alkali metal salts, citric acid and its alkali metal salts, etc.), an isotonizing agent (sodium chloride, potassium chloride, etc.), a dispersing agent (hydroxypropylmethyl cellulose, dextrin), a pH adjusting agent (hydrochloric acid, sodium hydroxide, etc.), a preservative (ethyl p-oxybenzoate, benzoic acid, etc.), a solubilizer (concentrated glycerin, meglumine, etc.), a dissolution aid (propylene glycol, saccharose, etc.), a soothing agent (glucose, benzyl alcohol, etc.) may be appropriately added to the preparation. Any of these additives is added in an amount generally used in an injectable preparation.

The injectable preparation is adjusted to pH of 2 to 12, preferably 2.5 to 8.0 by adding a pH adjusting agent.

The injectable preparation can be obtained by dissolving both the compound of the present invention or the concomitant drug and optionally a benzoate and/or salicylate, and, if necessary, the above additives in water. These ingredients may be dissolved in any order and the dissolution may be appropriately performed according to the same procedures for preparing a conventional injectable preparation.

An aqueous solution for the injectable preparation is preferably warmed, and used as the injectable preparation after filtration sterilization by filtration or autoclaved as in a conventional injectable preparation to provide for the injectable preparation.

An aqueous solution for the injectable preparation is preferably autoclaved, e.g., at 100 to 121°C for 5 to 30 minutes.

Moreover, the preparation may be in a solution form to which antibacterial activity is imparted to be usable as a multiple dosage form in divided dosing.

[2] Sustained Release or Immediate Release Preparation and its Production

A preferred sustained release preparation comprises a core comprising the compound of the present invention

or the concomitant drug, which is optionally coated with a water-insoluble material or a swelling polymer. For example, a sustained release preparation for oral administration of a once-daily dosage form is preferred.

Examples of the water-insoluble material used for the coating agent include cellulose ethers such as ethyl cellulose, butyl cellulose, etc., cellulose esters such as cellulose acetate, cellulose propionate, etc., polyvinyl esters such as polyvinyl acetate, polyvinyl butyrate, etc., acrylic acid polymers such as an acrylic acid/methacrylic acid copolymer, a methyl methacrylate copolymer, an ethoxyethyl methacrylate/cinnamoethyl methacrylate/aminoalkyl methacrylate copolymer, a polyacrylic acid, a polymethacrylic acid, a methacrylic acid alkylamide copolymer, a poly(methyl methacrylate), a polymethacrylate, a polymethacrylamide, an aminoalkyl methacrylate copolymer, a poly(methacrylic anhydride), a glycidyl methacrylate copolymer, in particular, a series of Eudragits (Rohm & Pharma) such as Eudragit RS-100, RL-100, RS-30D, RL-30D, RL-PO and RS-PO (ethyl acrylate/methyl methacrylate/chlorotrimethyl methacrylate/ethyl ammonium copolymer) and Eudragit NE-30D (methyl methacrylate/ethyl acrylate copolymer), etc., hydrogenated oils such as hydrogenated castor oil (e.g., LUBRI WAX (Freund Industrial Co., Ltd.), etc.), waxes such as carnauba wax, a fatty acid glycerin ester, paraffin, etc., polyglycerin fatty acid esters, etc.

The swelling polymer is preferably a polymer having an acidic removable group and exhibiting pH-dependent swelling. It is preferred that a polymer has an acidic removable group and undergoes a less swelling at an acidic pH such as in the stomach but is swollen extensively at a neutral pH such as in the small and large intestines.

Examples of such a polymer having an acidic removable group and exhibiting pH-dependent swelling include a crosslinked polyacrylic acid polymer such as Carbomers 934P, 940, 941, 974P, 980, 1342, etc., polycarbophil and calcium polycarbophil (all manufactured by BF Goodrich Chemicals), Hivis Wakos 103, 104, 105 and 304 (all manufactured by Wako Pure Chemical Industries, Ltd.), etc.

The coating agent used in the sustained release preparation may further contain a hydrophilic material.

Examples of the hydrophilic material include a polysaccharide which may have a sulfate group, such as pullulan, dextrin, an alkali metal alginate, etc., a polysaccharide having a hydroxyalkyl group or a carboxyalkyl group such as hydroxypropyl cellulose, hydroxypropylmethyl cellulose, sodium carboxymethylcellulose, etc., methyl cellulose, polyvinyl pyrrolidone, polyvinyl alcohol, polyethylene glycol, etc.

The amount of the water-insoluble material contained in the coating agent of the sustained release preparation is about 30 to about 90% (w/w), preferably about 35 to about 80% (w/w), more preferably about 40 to about 75% (w/w), and the swelling polymer content is about 3 to about 30% (w/w), preferably about 3 to about 15% (w/w). The coating agent may further contain a hydrophilic material, and the amount of the hydrophilic material contained in the coating agent is about 50% (w/w) or less, preferably about 5 to about 40% (w/w), more preferably about 5 to about 35% (w/w). As used herein, the term % (w/w) above is used to mean % by weight based on the coating agent composition, which is the remainder of the coating agent solution after removing any solvent (e.g., water, a lower alcohol such as methanol, ethanol, etc.).

The sustained release preparation is manufactured by preparing a core containing a drug as illustratively shown below, followed by coating the resulting core with a coating agent solution obtained by heat-melting a water-insoluble material or a swelling polymer or by dissolving or dispersing such a material in a solvent.

I. Production of Drug-Containing Core

The shape of a core containing a drug to be coated with a coating agent (hereinafter sometimes simply referred to as a core) is not specifically limited but preferably prepared into a particulate shape such as granules, fine granules, or the like.

When the core is granules or fine granules, they have a mean particle size of preferably about 150 to about 2,000 $\mu$m, more preferably about 500 to about 1,400 $\mu$m.

The core can be prepared in a conventional manner. For example, a drug is blended with a suitable excipient, binder, disintegrating agent, lubricant, stabilizer, etc., which is then subjected to wet extrusion granulation, fluidized bed granulation, or the like.

The drug content in the core is about 0.5 to about 95% (w/w), preferably about 5.0 to about 80% (w/w), more preferably about 30 to about 70% (w/w).

Examples of the excipient contained in the core include a saccharide such as saccharose, lactose, mannitol, glucose, etc., starch, crystalline cellulose, calcium phosphate, cornstarch, etc. Among others, crystalline cellulose and cornstarch are preferred.

Examples of the binder used include polyvinyl alcohol, hydroxypropyl cellulose, polyethylene glycol, polyvinyl pyrrolidone, Pluronic F68, gum arabic, gelatin, starch, etc. Examples of the disintegrating agent include calcium carboxymethyl cellulose (ECG505), sodium croscarmellose (Ac-Di-Sol), crosslinked polyvinyl pyrrolidone (crospovidone), a low substituted hydroxypropyl cellulose (L-HPC), etc. Among them, hydroxypropyl cellulose, polyvinyl pyrrolidone and a low substituted hydroxypropyl cellulose are preferred. Examples of the lubricant and the anticoagulant include talc, magnesium stearate and its inorganic salts, and examples of the lubricant include polyethylene glycol, etc. Examples

of the stabilizer include an acid such as tartaric acid, citric acid, succinic acid, fumaric acid, maleic acid, etc.

In addition to the procedures described above, the core can be prepared by using other techniques such as an tumbling granulation technique, a pan coating technique, a fluidized bed coating technique and a melt granulation technique, wherein a drug or a mixture of the drug with an excipient, a lubricant, etc. is portionwise added to inert carrier particles as seeds for the core, while spraying a binder dissolved in a suitable solvent such as water, a lower alcohol (e.g., methanol, ethanol, etc.) or the like. Examples of the inert carrier particles include those prepared from saccharose, lactose, starch, crystalline cellulose and waxes, and, preferably, these carriers have a mean particle size of about 100 μm to about 1,500 μm.

In order to separate the drug contained in the core from a coating agent, the surface of the core may be covered with a protective material. Examples of the protective material include the hydrophilic material described above and water-insoluble material. The preferred protective material is polyethylene glycol or a polysaccharide having a hydroxyalkyl group or a carboxyalkyl group, more preferably, hydroxypropylmethyl cellulose and hydroxypropyl cellulose. The protective material may contain, as a stabilizer, an acid such as tartaric acid, citric acid, succinic acid, fumaric acid, maleic acid, etc., and a lubricant such as talc. When the protective material is used, the amount thereof to be coated is about 1 to about 15% (w/w), preferably about 1 to about 10% (w/w), more preferably about 2 to about 8% (w/w) based on the core.

The protective material can be coated by a conventional coating method. Specifically, the core is spray-coated with the protective material by a fluidized bed coating technique, a pan coating technique, etc.

II. Coating of Core with Coating Agent

The core obtained in I above is coated with a solution of the coating agent prepared by melt-heating the water-insoluble material and pH-dependent swelling polymer described above and a hydrophilic material or by dissolving or dispersing them in a solvent to obtain a sustained release preparation.

A coating method of the core with the coating agent solution includes, for example, spray-coating, etc.

The composition ratio of the water-insoluble material, swelling polymer and hydrophilic material in the coating agent solution can be appropriately chosen to be within the amounts of the respective ingredients contained in the coating.

The amount of the coating agent is about 1 to about 90% (w/w), preferably about 5 to about 50% (w/w), more preferably about 5 to about 35% (w/w) based on the core (excluding the amount of the protective material coating).

As the solvent for the coating agent solution, water and an organic solvent can be used singly or as a mixture thereof. When a mixture is used, the ratio of water and the organic solvent (water/organic solvent: a weight ratio) may vary with the range of 1 to 100%, and is preferably 1 to about 30%. The organic solvent is not particularly limited so far as it can dissolve the water-insoluble material, and examples of the solvent include a lower alcohol such as methyl alcohol, ethyl alcohol, isopropyl alcohol, n-butyl alcohol, etc., a lower alkanone such as acetone, etc,, acetonitrile, chloroform, methylene chloride, etc. Among others, a lower alcohol is preferred, with ethyl alcohol and isopropyl alcohol being more preferred. Water and a mixture of water and an organic solvent are used preferably as solvents for the coating agent solution. In this case, an acid such as tartaric acid, citric acid, succinic acid, fumaric acid, maleic acid, etc. may be added to the coating agent solution, if necessary, for the purpose of stabilizing the coating agent solution.

The coating through spray coating can be performed using a conventional coating method. Specifically, the core is sprayed with a coating agent solution by a fluidized bed coating technique, a pan coating technique, or the like. Upon coating, a lubricant such as talc, titanium oxide, magnesium stearate, calcium stearate, light silicic anhydride, etc., and a plasticizer such as glycerin fatty ester, hardened castor oil, triethyl citrate, cetyl alcohol, stearyl alcohol, etc. may also be added.

After coating with the coating agent, an antistatic agent such as talc may also be admixed, if necessary.

The immediate release preparation may be a liquid (solution, suspension, emulsion, etc.) or a solid (particles, pills, tablets, etc.). An oral preparation and a parenteral preparation such as an injectable preparation may be used, and an oral preparation is preferred.

The immediate release preparation may usually contain a carrier, additives and an excipient (hereinafter sometimes abbreviated as excipients) which are conventionally used in the pharmaceutical field, in addition to a drug which is an active ingredient. The pharmaceutical excipients are not specifically limited so long as they are excipients conventionally used in the pharmaceutical field. Examples of the excipient for an oral solid preparation include lactose, starch, cornstarch, crystalline cellulose (Avicel PH101, manufactured by Asahi Kasei Corporation, etc.), powdered sugar, granulated sugar, mannitol, light silicic anhydride, magnesium carbonate, calcium carbonate, L-cysteine, etc., preferably, cornstarch and mannitol. Any of these excipients may be employed alone or in combination with each other. The amounts of the excipients are, for example, about 4.5 to about 99.4 w/w %, preferably about 20 to about 98.5 w/w %, more preferably about 30 to about 97 w/w %, based on the total weight of the immediate release preparation.

The content of drug in the immediate release preparation may appropriately be selected from the range of about 0.5% to about 95%, preferably about 1% to about 60% to the whole amount of the immediate release preparation.

When the immediate release preparation is an oral solid preparation, the preparation contains a disintegrating agent in addition to the ingredients described above. Examples of the disintegrating agent include calcium carboxymethylcellulose (ECG505 manufactured by GOTOKU CHEMICAL Co., Ltd.), sodium croscarmellose (for example, Ac-Di-Sol manufactured by Asahi Kasei Corporation), crospovidone (for example, COLIDON CL manufactured by BASF), low-substituted hydroxypropyl cellulose (Shin-Etsu chemical Co., Ltd.), carboxymethyl starch (MATSUTANI CHEMICAL INDUSTRY Co., Ltd.), sodium carboxymethyl starch (EXPLOTAB manufactured by KIMURA SANGYO), partial $\alpha$ starch (PCS manufactured by Asahi Kasei Corporation), etc. For example, the disintegrating agent that disintegrates granules by water absorption or swelling upon contact with water, or forming a channel between the active ingredient comprising the core and an excipient can be used. Any of these disintegrating agents can be used alone or in combination with each other. The amount of the disintegrating agent used may be appropriately chosen depending upon the type and the amount of the drug used or a particular preparation design for the intended release performance. For example, the amount is about 0.05 to about 30 w/w %, preferably about 0.5 to about 15 w/w % based on the total weight of the immediate release preparation.

When the immediate release preparation is an oral solid preparation, the oral solid preparation may optionally contain additives conventionally used in a solid preparation, in addition to the ingredients described above. Examples of the additives include binders (for example, sucrose, gelatin, powdery gum arabic, methyl cellulose, hydroxypropyl cellulose, hydroxypropylmethyl cellulose, carboxymethylcellulose, polyvinyl pyrrolidone, pullulan, dextrin, etc.), lubricants (polyethylene glycol, magnesium stearate, talc, light silicic anhydride (for example, Aerosil (NIPPON AEROSIL)), surfactants (for example, anionic surfactants such as sodium alkylsulfate, nonionic surfactants such as polyoxyethylene fatty ester, polyoxyethylene sorbitan fatty ester, polyoxyethylene castor oil derivatives, etc.), colorants (for example, tar colorants, caramel, colcothar, titanium oxide, riboflavins), if necessary, corrigents (for example, sweeteners, flavors, etc.), adsorbents, preservatives, wetting agents, antistatic agents, etc. Furthermore, an organic acid such as tartaric acid, citric acid, succinic acid, fumaric acid or the like can also be added as a stabilizer.

As the binder above, hydroxypropyl cellulose, polyethylene glycol and polyvinyl pyrrolidone, etc. are preferably used.

The immediate release preparation can be prepared by mixing the ingredients described above and kneading the mixture, if necessary, and then molding according to a conventional technique for making pharmaceutical preparations. The mixing above can be carried out in a conventional manner, e.g., by mixing, kneading, etc. Specifically, where the immediate release preparation is in the form of particles, the preparation can be prepared by mixing ingredients with a vertical granulator, a multi-purpose kneader (HATA IRON WORKS CO., LTD), a fluidized bed granulator FD-5S (POWREX CORPORATION) or the like, and then granulating the resulting by wet extrusion granulation or fluidized bed granulation by a technique similar to that for preparing the core of the sustained release preparation described above.

The immediate release preparation and the sustained release preparation thus obtained can be formulated, as they are, or, together with appropriate pharmaceutical excipients, in pharmaceutical preparations separately in a conventional manner to prepare respective preparations for administering in combination with each other simultaneously or at certain time intervals. Alternatively, both preparations may be formulated in a single dosage form for oral administration (e.g., granules, fine granules, tablets, capsules) as they are, or, together with appropriate pharmaceutical excipients. Both preparations in the form of granules or fine granules may also be filled in a single capsule for oral administration.

[3] Sublingual, Buccal or Rapid Oral Disintegrating Preparation and its Production

A sublingual, buccal or rapid oral disintegrating preparation may be in the form of a solid preparation such as a tablet, or may be in the form of an oral mucosal patch (film), or an oral disintegrating film.

The sublingual, buccal or rapid oral disintegrating preparation is preferably a preparation containing the compound of the present invention or the concomitant drug and an excipient. The preparation may also contain auxiliary agents such as a lubricant, an isotonizing agent, a hydrophilic carrier, a water-dispersible polymer, a stabilizer, etc. Further for the purpose of promoting the absorption and enhancing the bioavailability, the preparation may also contain $\beta$-cyclodextrin or $\beta$-cyclodextrin derivatives (e.g., hydroxypropyl-$\beta$-cyclodextrin, etc.), and the like.

Examples of the above excipient include lactose, saccharose, D-mannitol, starch, crystalline cellulose, light silicic anhydride, etc. Examples of the lubricant include magnesium stearate, calcium stearate, talc, colloidal silica, etc., preferably, magnesium stearate and colloidal silica. Examples of the isotonizing agent include sodium chloride, glucose, fructose, mannitol, sorbitol, lactose, saccharose, glycerin and urea, particularly preferably, mannitol. As the hydrophilic carrier, there are, for example, swelling hydrophilic carriers such as crystalline cellulose, ethyl cellulose, crosslinked polyvinyl pyrrolidone, light silicic anhydride, silicic acid, dicalcium phosphate, calcium carbonate, etc., preferably, crystalline cellulose (e.g., microcrystalline cellulose, etc.). As the water-dispersible polymer, there are, for example, a gum (e.g., tragacanth gum, acacia gum, guar gum), alginate (e.g., sodium alginate), cellulose derivatives (e.g., methyl cellulose, carboxymethylcellulose, hydroxymethyl cellulose, hydroxypropyl cellulose, hydroxypropylmethyl cellulose), gelatin, water-soluble starch, polyacrylic acid (e.g., carbomer), polymethacrylic acid, polyvinyl alcohol, polyethylene glycol, polyvinyl pyrrolidone, polycarbophil, ascorbate palmitate salt, etc., preferably, hydroxypropylmethyl cellulose, polyacrylic acid, alginate, gelatin, carboxymethylcellulose, polyvinyl pyrrolidone and polyethylene glycol. Hydroxypropylmethyl cellulose is particularly preferred. As the stabilizer, there are, for example, cysteine, thiosorbitol, tartatic acid, citric acid, sodium carbonate, ascrobic acid, glycine, sodium sulfite, etc., particularly preferably citric acid and ascorbic acid.

The sublingual, buccal or rapid oral disintegrating preparation can be prepared by blending the compound of the present invention or the concomitant drug and an excipient by a method per se known. Furthermore, if desired, the auxiliary agents described above, such as the lubricant, isotonizing agent, hydrophilic carrier, water-dispersible polymer, stabilizer, colorant, sweetener, preservative, etc. may also be blended therewith. After blending the ingredients described above simultaneously or at certain time intervals, the mixture is compressed into tablets to obtain the sublingual, buccal or oral quick disintegration tablet. In order to obtain a suitable hardness, a solvent such as water, an alcohol, etc. can be used to moisturize or wet the ingredients before or after tabletting, followed by drying.

In preparing the oral mucosal patch (film), the compound of the present invention or the concomitant drug and the water-dispersible polymer (preferably, hydroxypropyl cellulose, hydroxypropylmethyl cellulose), excipient, etc. described above are dissolved in a solvent such as water, etc. and then the resulting solution is cast into a film. In addition, additives such as a plasticizer, a stabilizer, an antioxidant, a preservative, a colorant, a buffering agent, a sweeteners, etc. may be added to the preparation. A glycol such as polyethylene glycol, propylene glycol, etc. may be added to impart an appropriate elasticity to a film, and a bioadhesive polymer (e.g., polycarbophile, carbopol) may also be added to enhance the adhesion of the film to the oral mucosal lining. The casting can be carried out by pouring a solution onto a non-adhesive surface, spreading the solution using a coater such as a doctor blade in a uniform thickness (preferably, approximately 10 to 1000 microns), and then drying the solution to form a film. The film thus formed is dried at room temperature or while warming, and then cut into pieces each having a desired surface area.

A preferred rapid oral disintegrating preparation is, for example, a rapid diffusion preparation in a solid network form, which comprises the compound of the present invention or the concomitant drug and a water-soluble or water-diffusible carrier inert to the compound of the present invention or the concomitant drug. The network is formed by sublimating a solvent from the solid composition comprising a solution of the compound of the present invention or the concomitant drug in a suitable solvent.

In addition to the compound of the present invention or the concomitant drug, the composition of the rapid oral disintegrating preparation may preferably contain a matrix-forming agent and secondary ingredients.

Examples of the matrix-forming agent include gelatins, dextrins and animal or vegetable proteins from soybean, wheat, psyllium seed, etc.; gummy materials such as gum arabic, guar gum, agar, xanthane gum, etc.; polysaccharides; alginates; carboxymethylcelluloses; carrageenans; dextrans; pectins; synthetic polymers such as polyvinyl pyrrolidone; materials derived from gelatin-gum arabic complexes, etc. The matrix-forming agent further includes saccharides such as mannitol, dextrose, lactose, galactose, trehalose, etc.; cyclic saccharides such as cyclodextrins, etc.; inorganic salts such as sodium phosphate, sodium chloride, aluminum silicate, etc.; amino acids having 2 to 12 carbon atoms such as glycine, L-alanine, L-aspartic acid, L-glutamic acid, L-hydroxyproline, L-isoleucine, L-leucine, L-phenylalanine, etc.

One or more matrix-forming agents can be incorporated into a solution or suspension before solidification. The matrix-forming agents may be present in addition to a surfactant, or may be present in the absence of a surfactant. The matrix-forming agents serve not only to form a matrix itself, but also assist to maintain diffusion of the compound of the present invention or the concomitant drug in the solution or suspension.

The composition may contain a secondary ingredient such as a preservative, an antioxidant, a surfactant, a thickening agent, a colorant, pH adjusting agent, a flavor, a sweetener, a taste masking agent, etc. As the suitable colorant, there are, for example, iron oxide red, black and yellow, FD & C dyes available from ERIS & EVERALD such as FD & C Blue No. 2 and FD & C Red No. 40, etc. Examples of the suitable flavor include mint, raspberry, licorice, orange, lemon, grape fruit, caramel, vanilla, cherry, grape flavor and a combination thereof. Examples of the suitable pH adjusting agent include citric acid, tartaric acid, phosphoric acid, hydrochloric acid and maleic acid. Examples of the suitable sweetener include aspartame, acesulfame K and thaumatine. Examples of the suitable taste masking agent include sodium bicarbonate, ion exchange resins, cyclodextrin inclusion compounds, adsorbents and microencapsulated apomorphine.

The preparation generally contains the compound of the present invention or the concomitant drug in an amount of about 0.1 to about 50% by weight, preferably about 0.1 to about 30% by weight and, preferably, the preparation (the sublingual tablet, buccal, etc. described above) allows 90% or more of the compound of the present invention or the concomitant drug to be dissolved (in water) within a time period of about 1 to about 60 minutes, preferably about 1 minute to about 15 minutes, more preferably about 2 minutes to about 5 minutes, or is a rapid oral disintegrating preparation which disintegrates within about 1 to about 60 seconds, preferably about 1 to about 30 seconds, more preferably about 1 to about 10 seconds, after being placed in the oral cavity.

The amount of the above excipient is about 10 to about 99% by weight, preferably about 30 to about 90% by weight based on the total weight of the preparation. The amount of β-cyclodextrin or β-cyclodextrin derivative is 0 to

about 30% by weight based on the total weight of the preparation. The amount of the lubricant is about 0.01 to about 10% by weight, preferably about 1 to about 5% by weight based on the total weight of the preparation. The amount of the isotonizing agent is about 0.1 to about 90% by weight, preferably about 10 to about 70% by weight based on the total weight of the preparation. The amount of the hydrophilic carrier is about 0.1 to about 50% by weight, preferably about 10 to about 30% by weight based on the total weight of the preparation. The amount of the water-dispersible polymer is about 0.1 to about 30% by weight, preferably about 10 to about 25% by weight based on the total weight of the preparation. The amount of the stabilizer is about 0.1 to about 10% by weight, preferably about 1 to about 5% by weight based on the total weight of the preparation. If necessary, the preparation described above may further contain additives such as a colorant, a sweetener, a preservative, etc.

A dose of the concomitant preparations of the present invention varies depending upon kind of the compound of the present invention, age, body weight, conditions, dosage form, route for administration, dosing period, etc.

A dose of the compound of the present invention may vary depending upon subject to be administered, target organ, conditions, route of administration, etc., and in oral administration, the compound is generally administered to the patient (as 60 kg body weight) with cancer in a daily dose of about 0.01 to 100 mg, preferably about 0.1 to 50 mg and more preferably about 0.1 to 20 mg. In parenteral administration, a single dose of the compound may vary depending upon subject to be administered, target organ, conditions, route of administration, etc., and in the form of an injectable dosage form, it is advantageous to intravenously administer the compound to the patient (as 60 kg body weight) with cancer generally in a daily dose of about 0.001 to 30 mg, preferably about 0.01 to 20 mg, and more preferably about 0.01 to 10 mg. For other animal species, the corresponding dose as converted per 60 kg weight can be administered. Of course, the dose may vary depending on individual conditions as described above; in such a case, a dose less than the dose given above may be sufficient, or may be higher than the range above.

It is possible to set any range of a dose for the concomitant drug, so long as it causes no adverse side effects. A daily dose of the concomitant drug may vary depending on the severity of disease, the age, sex, body weight and susceptibility of the subject, dosing period and intervals, the properties, formulation, type and active ingredients of the pharmaceutical preparation, etc. and is not particularly limited. For example, in oral administration, the dose is about 0.001 to 2000 mg, preferably about 0.01 to 500 mg, and more preferably about 0.1 to 100 mg in terms of a drug, per kg body weight of a mammal; usually, this dose is administered by dividing 1 to 4 times per day.

When the pharmaceutical of the present invention is administered, it may be administered concomitantly. Alternatively, the concomitant drug is first administered and then the compound of the present invention is administered, or the compound of the present invention is first administered and then the concomitant drug is administered. When they are administered at certain time intervals, the intervals vary depending on the active ingredient to be administered, dosage form and route of administration; for example, when the concomitant drug is first administered, the compound of the present invention may be administered within 1 minute to 3 days, preferably 10 minutes to 1 day, more preferably 15 minutes to 1 hour after the administration of the concomitant drug. When the compound of the present invention is first administered, the concomitant drug may be administered within 1 minute to 1 day, preferably 10 minutes to 6 hours, more preferably 15 minutes to 1 hour after the administration of the compound of the present invention.

As a preferred dosing method, for example, about 0.001 to 200 mg/kg of the concomitant drug in the form of an oral dosage preparation is administered orally and, after about 15 minutes, about 0.005 to 0.5 mg/kg of the compound of the present invention in the form of a parenteral preparation is administered parenterally as a daily dose.

As the metastins, there are used, for example, human metastin described in WO 00/24890, mouse or rat metastin described in WO 01/75104, etc.

Specific examples of human metastin include a peptide comprising the N-terminal 47th-54th amino acid sequence in the amino acid sequence represented by SEQ ID NO: 1 and consisting of 8 to 54 amino acid residues, and the like.

The "peptide comprising the N-terminal 47th-54th amino acid sequence in the amino acid sequence represented by SEQ ID NO: 1 and consisting of 8 to 54 amino acid residues" may be any peptide, as far as it is a peptide comprising the N-terminal 47th-54th amino acid sequence in the amino acid sequence represented by SEQ ID NO: 1 and consisting of 8 to 54 amino acid residues, but means that these peptides have substantially the same physiological activity (e.g., a receptor binding activity, a signal transduction action, a blood glucose level elevating action, a pancreatic glucagon secretion promoting action, a urine formation promoting action, etc.). Specifically, there are used (i) a peptide having the amino acid sequence represented by SEQ ID NO: 1, (ii) a peptide comprising the N-terminal 47th-54th amino acid sequence at the C terminus in the amino acid sequence represented by SEQ ID NO: 1 and consisting of 8 to 15 amino acid residues, etc.

More specifically, human metastin used includes (i) a peptide consisting of the amino acid sequence represented by SEQ ID NO: 1 (human metastin 54 (1-54)), (ii) a peptide consisting of the N-terminal 40th-54th amino acid sequence in the amino acid sequence represented by SEQ ID NO: 1 (human metastin 15 (40-54); SEQ ID NO: 15), (iii) a peptide consisting of the N-terminal 45th-54th amino acid sequence in the amino acid sequence represented by SEQ ID NO: 1 (human metastin 10 (45-54); SEQ ID NO: 16), (iv) a peptide consisting of the N-terminal 46th-54th amino acid sequence

in the amino acid sequence represented by SEQ ID NO: 1 (human metastin 9 (46-54); SEQ ID NO: 17), (v) a peptide consisting of the N-terminal 47th-54th amino acid sequence in the amino acid sequence represented by (human metastin 8 (47-54); SEQ ID NO: 18), etc.

As mouse metastin (A), there are used, for example, (i) a peptide comprising the N-terminal 134th-141st amino acid sequence in the amino acid sequence represented by SEQ ID NO: 3 and consisting of 8 to 52 amino acid residues. Specific examples of mouse metastin (A) used include (i) a peptide consisting of the N-terminal 90th-141st amino acid sequence in the amino acid sequence represented by SEQ ID NO: 3, (ii) a peptide consisting of the N-terminal 132nd-141st amino acid sequence in the amino acid sequence represented by SEQ ID NO: 3, (iii) a peptide consisting of the N-terminal 127th-141 st amino acid sequence in the amino acid sequence represented by SEQ ID NO: 3, and the like.

As mouse metastin (B), there are used, for example, a peptide comprising the N-terminal 138th-145th amino acid sequence in the amino acid sequence represented by SEQ ID NO: 5 and consisting of 8 to 52 amino acid residues. Specific examples of mouse metastin (B) used include a peptide consisting of the N-terminal 94th-145th amino acid sequence in the amino acid sequence represented by SEQ ID NO: 5, and the like.

As rat metastin, there are used, for example, a peptide comprising the N-terminal 112th-119th amino acid sequence in the amino acid sequence represented by SEQ ID NO: 7 and consisting of 8 to 52 amino acid residues. Specific examples of rat metastin used include (i) a peptide consisting of the N-terminal 68th-119th amino acid sequence in the amino acid sequence represented by SEQ ID NO: 7, (ii) a peptide consisting of the N-terminal 110th-119th amino acid sequence in the amino acid sequence represented by SEQ ID NO: 7, (iii) a peptide consisting of the N-terminal 105th-119th amino acid sequence in the amino acid sequence represented by SEQ ID NO: 7, and the like.

As used herein, the metastins are represented in accordance with the conventional way of describing peptides, that is, the N-terminus (amino end) at the left hand and the C-terminus (carboxyl end) at the right hand. In the peptide represented by SEQ ID NO: 1, the C-terminus may be in any form of a carboxyl group (-COOH), a carboxylate (-COO-), an amide (-$CONH_2$) and an ester (-COOR). Herein, examples of the ester or alkylamide group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, $\alpha$-naphthyl, etc.; a $C_{7-14}$ aralkyl such as a phenyl-$C_{1-2}$ alkyl group such as benzyl, phenethyl, etc., an $\alpha$-naphthyl-$C_{1-2}$ alkyl group such as $\alpha$-naphthylmethyl, etc.; pivaloyloxymethyl group, which are widely used as an ester for oral use, and the like.

Furthermore, the metastins include peptides, wherein the amino at the N-terminal methionine residue is protected with a protecting group (e.g., a $C_{1-6}$ acyl group such as formyl, a $C_{2-6}$ alkanoyl group such as acetyl, etc.); those wherein the N-terminal region is cleaved in vivo and the glutamyl group thus formed is pyroglutaminated; those wherein a substituent (e.g., -OH, -SH, -COOH, amino, imidazole, indole, guanidino, etc.) on the side chain of an amino acid in the molecule is protected with a suitable protecting group (e.g., a $C_{1-6}$ acyl group such as formyl, a $C_{2-6}$ alkanoyl group such as acetyl, etc.), or conjugated peptides such as glycopeptides bound to sugar chains.

Salts of the metastin of the present invention used are salts with physiologically acceptable bases (e.g., alkali metal salts) or acids (e.g., organic acids or inorganic acids), especially physiologically acceptable acid addition salts are preferred. Examples of such salts include salts with inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

As the DNA encoding metastins, there are used, for example, a DNA encoding human metastin described in WO 00/24890, a DNA encoding mouse or rat metastin described in WO 01/75104, etc.

The DNAs encoding the metastins may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

The DNA encoding human metastin, mouse metastin precursor (A), mouse metastin precursor (B) or rat metastin precursor may be any DNA, so long as each is a DNA containing a nucleotide sequence represented by SEQ ID NO: 2, SEQ ID NO: 4, SEQ ID NO: 6 or SEQ ID NO: 8, or a DNA containing a nucleotide sequence hybridizable to the nucleotide sequence represented by any nucleotide sequence represented by SEQ ID NO: 2, SEQ ID NO: 4, SEQ ID NO: 6 or SEQ ID NO: 8 under highly stringent conditions and encoding the human metastin, mouse metastin (A), mouse metastin (B) or rat metastin described above.

Specific examples of the DNA hybridizable to the nucleotide sequence represented by any of SEQ ID NO: 2, SEQ ID NO: 4, SEQ ID NO: 6 or SEQ ID NO: 8 include a DNA containing a nucleotide sequence having at least about 70% homology, preferably at least about 80% homology, more preferably at least about 90% homology and the most preferably at least about 95% homology, to the nucleotide sequence represented by any of SEQ ID NO: 2, SEQ ID NO: 4, SEQ ID NO: 6 or SEQ ID NO: 8.

Homology in the nucleotide sequence can be measured under the following conditions (an expectation value

= 10; gaps are allowed; filtering = ON; match score = 1; mismatch score = -3) using the homology scoring algorithm NCBI BLAST (National Center for Biotechnology Information Basic Local Alignment Search Tool).

The hybridization can be carried out by per se publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration of about 19 mM at a temperature of about 65°C are most preferred.

Specifically, as the DNA encoding the human metastin comprising the amino acid sequence represented by SEQ ID NO: 1, the DNA consisting of the nucleotide sequence represented by SEQ ID NO: 2 can be used. Accordingly, for the nucleotide sequence encoding the human metastin consisting of the various amino acid sequences described above, a nucleotide sequence corresponding to each of the partial amino acid sequences in the amino acid sequence represented by SEQ ID NO: 1 may be chosen from the nucleotide sequence represented by SEQ ID NO: 2.

As the DNA encoding the mouse metastin precursor (A) comprising the amino acid sequence represented by SEQ ID NO: 3, there can be employed a DNA consisting of the nucleotide sequence represented by SEQ ID NO: 4. Accordingly, for the nucleotide sequence encoding the mouse metastin precursor (A) consisting of the various amino acid sequences described above, a nucleotide sequence corresponding to each of the partial amino acid sequences in the amino acid sequence represented by SEQ ID NO: 3 may be chosen from the nucleotide sequence represented by SEQ ID NO: 4.

As the DNA encoding the mouse metastin precursor (B) comprising the amino acid sequence represented by SEQ ID NO: 5, there can be employed a DNA consisting of the nucleotide sequence represented by SEQ ID NO: 6. Accordingly, for the nucleotide sequence encoding the mouse metastin precursor (B) comprising of the various amino acid sequences described above, a nucleotide sequence corresponding to each of the partial amino acid sequences in the amino acid sequence represented by SEQ ID NO: 5 may be chosen from the nucleotide sequence represented by SEQ ID NO: 6.

As the DNA encoding the rat metastin comprising the amino acid sequence represented by SEQ ID NO: 7, there can be employed a DNA consisting of the nucleotide sequence represented by SEQ ID NO: 8. Accordingly, for the nucleotide sequence encoding the rat metastin comprising the various amino acid sequences described above, a nucleotide sequence corresponding to each of the partial amino acid sequences in the amino acid sequence represented by SEQ ID NO: 7 may be chosen from the nucleotide sequence represented by SEQ ID NO: 8.

More specifically, for the peptide comprising the amino acid sequence represented by SEQ ID NO: 1 (human metastin 54 (1-54)), a DNA comprising the nucleotide sequence represented by SEQ ID NO: 2, etc. is used.

As a DNA encoding the peptide consisting of the N-terminal 40th-54th amino acid sequence in the amino acid sequence represented by SEQ ID NO: 1 (human metastin 15 (40-54); SEQ ID NO: 15), a DNA comprising the nucleotide sequence represented by SEQ ID NO: 19, etc. is used.

As a DNA encoding the peptide consisting of the N-terminal 45th-54th amino acid sequence in the amino acid sequence represented by SEQ ID NO: 1 (human metastin 10 (45-54); represented by SEQ ID NO: 16), a DNA comprising the nucleotide sequence represented by SEQ ID NO: 20, etc. is used.

As a DNA encoding the peptide consisting of the N-terminal 46th-54th amino acid sequence in the amino acid sequence represented by SEQ ID NO: 1 (human metastin 9 (46-54); represented by SEQ ID NO: 17), a DNA comprising the nucleotide sequence represented by SEQ ID NO: 21, etc. is used.

As a DNA encoding the peptide consisting of the N-terminal 47th-54th amino acid sequence in the amino acid sequence represented by SEQ ID NO: 1 (human metastin 8 (47-54); represented by SEQ ID NO: 18), a DNA containing the nucleotide sequence represented by SEQ ID NO: 22, etc. is used.

As the metastin receptor, its partial peptides or salts thereof, there are used, for example, a human metastin receptor, its partial peptides or salts thereof described in WO 00/24890, a mouse or rat metastin receptor, its partial peptides or salts thereof described in WO 01/75104, etc.

Specifically, the metastin receptor includes a protein comprising the same or substantially the same amino acid sequence as the amino acid sequence represented by SEQ ID NO: 9, SEQ ID NO: 11 or SEQ ID NO: 13, etc.

The amino acid sequence which has substantially the same amino acid sequence as the amino acid sequence represented by SEQ ID NO: 9, SEQ ID NO: 11 or SEQ ID NO: 13 includes, for example, an amino acid sequence having at least about 70% homology, preferably at least about 80% homology, more preferably at least about 90% homology, and most preferably at least about 95% homology, to the amino acid sequence represented by SEQ ID NO: 9, SEQ ID NO: 11 or SEQ ID NO: 13.

Homology of the amino acid sequences can be determined under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

As the protein comprising substantially the same amino acid sequence as the amino acid sequence represented by SEQ ID NO: 9, SEQ ID NO: 11 or SEQ ID NO: 13, preferred is a protein comprising substantially the same amino acid sequence as the amino acid sequence represented by SEQ ID NO: 9, SEQ ID NO: 11 or SEQ ID NO: 13 and having the activity of the same nature as that of a protein having the amino acid sequence represented by SEQ ID NO: 9, SEQ ID NO: 11 or SEQ ID NO: 13, etc.

The activity of substantially the same nature includes, for example, a ligand binding activity, a signal transduction activity, and the like. The "substantially the same nature" is used to mean that the nature of these activities is equivalent in terms of quality. Thus, the activities such as a ligand binding activity, a signal transduction activity, etc. are preferably equivalent (e.g., about 0.01 to 100 times, preferably about 0.5 to 10 times, more preferably 0.5 to 2 times), but differences in degree such as a level of these activities, quantitative factors such as a molecular weight of the protein may be present and allowable.

The activities such as a ligand binding activity, a signal transduction activity, etc. can be assayed by per se publicly known methods with modifications and may be determined according to methods for determining a ligand or screening methods described in, e.g., WO 00/24890 or WO 01/75104.

Examples of the metastin receptor used include proteins comprising (i) the amino acid sequence represented by SEQ ID NO: 9, SEQ ID NO: 11 or SEQ ID NO: 13, of which at least 1 or 2 (preferably about 1 to about 30, more preferably about 1 to about 10 and most preferably several (1 or 2)) amino acids are deleted; (ii) the amino acid sequence represented by SEQ ID NO: 9, SEQ ID NO: 11 or SEQ ID NO: 13, to which at least 1 or 2 (preferably about 1 to about 30, more preferably about 1 to about 10 and most preferably several (1 or 2)) amino acids are added; (iii) the amino acid sequence represented by SEQ ID NO: 9, SEQ ID NO: 11 or SEQ ID NO: 13, in which at least 1 or 2 (preferably about 1 to about 30, more preferably about 1 to about 10 and most preferably several (1 or 2)) amino acids are substituted by other amino acids; or (iv) a combination of these amino acid sequences; and the like.

As used herein, the metastin receptors are represented in accordance with the conventional way of describing peptides, that is, the N-terminus (amino end) at the left hand and the C-terminus (carboxyl end) at the right hand. In the metastin receptors including the metastin receptor represented by SEQ ID NO: 9, SEQ ID NO: 11 or SEQ ID NO: 13, the C-terminus may be in any form of a carboxyl group (-COOH), a carboxylate (-COO-), an amide ($-CONH_2$) and an ester (-COOR). Herein, examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, $\alpha$-naphthyl, etc.; a $C_{7-14}$ aralkyl such as a phenyl-$C_{1-2}$ alkyl group such as benzyl, phenethyl, etc., an $\alpha$-naphthyl-$C_{1-2}$ alkyl group such as $\alpha$-naphthylmethyl, etc.; and pivaloyloxymethyl group, which are widely used as an ester for oral use, and the like.

Where the metastin receptors contain a carboxyl group (or a carboxylate) at a position other than the C-terminus, the carboxyl group may be amidated or esterified and such amides or esters are also included within the receptor protein of the present invention. In this case, the ester group used may be the same group as the C-terminal esters described above.

Furthermore, the metastin receptors include those wherein the amino group at the N-terminal methionine residue is protected with a protecting group (e.g., a $C_{1-6}$ acyl group such as formyl, a $C_{2-6}$ alkanoyl group such as acetyl, etc.); those wherein the N-terminal region is cleaved in vivo and the glutamyl group thus formed is pyroglutaminated; those wherein a substituent (e.g., -OH, -SH, amino, imidazole, indole, guanidino, etc.) on the side chain of an amino acid in the molecule is protected with a suitable protecting group (e.g., a $C_{1-6}$ acyl group such as formyl, a $C_{2-6}$ alkanoyl group such as acetyl, etc.), or conjugated proteins such as glycoproteins bound to sugar chains.

Specific examples of the metastin receptors include human metastin receptor consisting of the amino acid sequence represented by SEQ ID NO: 9, rat metastin receptor consisting of the amino acid sequence represented by SEQ ID NO: 11, mouse metastin receptor consisting of the amino acid sequence represented by SEQ ID NO: 13, etc.

The partial peptide of the metastin receptor (hereinafter sometimes simply referred to as the partial peptide) may be any peptide, so long as it is a partial peptide of the metastin receptor described above; there are used those such as protein molecules of the metastin receptor, which are the sites exposed outside the cell membrane, and having a ligand binding activity.

Specifically, the partial peptide of the metastin receptor consisting of the amino acid sequence represented by SEQ ID NO: 9, SEQ ID NO: 11 or SEQ ID NO: 13 is a peptide containing the parts analyzed to be extracellular domains (hydrophilic domains) in the hydrophobic plotting analysis. A peptide containing a hydrophobic domain in part can be used as well. In addition, the peptide may contain each domain separately or a plurality of domains together.

In the metastin receptor, preferred partial peptides are those having the number of amino acids of at least 20, preferably at least 50, and more preferably at least 100, in the amino acid sequence described above, which constitutes the metastin receptor.

The partial peptide may be a peptide having the amino acid sequence described above, of which at least 1 or 2 (preferably about 1 to about 10 and more preferably several (1 or 2)) amino acids are deleted; to which at least 1 or 2 (preferably about 1 to about 20, more preferably about 1 to about 10 and much more preferably several (1 or 2)) amino

acids are added; or, in which at least 1 or 2 (preferably about 1 to about 10 and more preferably several (1 or 2)) amino acids are substituted by other amino acids.

In the partial peptide, the C terminus may be any form of a carboxyl group (-COOH), a carboxylate (-COO-), an amide ($-CONH_2$) and an ester (-COOR), as in the metastin receptor described above.

Furthermore, the partial peptides include peptides, wherein the amino group at the N-terminal methionine residue is protected with a protecting group; those wherein the N-terminal region is cleaved in vivo and Gin thus formed is pyroglutaminated; those wherein a substituent on the side chain of an amino acid in the molecule is protected with a suitable protecting group, or conjugated peptides such as glycopeptides bound to sugar chains, as in the metastin receptors described above.

Salts of the metastin receptor or the partial peptide used are salts with physiologically acceptable bases or acids, especially physiologically acceptable acid addition salts are preferred. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The DNA encoding the metastin receptor or its partial peptides used includes, for example, a DNA encoding the human metastin receptor or its partial peptides described in WO 00/24890, a DNA encoding the mouse or rat metastin receptor or its partial peptides described in WO 01/75104, etc.

The DNAs encoding the metastin receptor or its partial peptides may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding human metastin receptor, mouse metastin receptor or rat metastin receptor may be any DNA, so long as it is a DNA comprising each nucleotide sequence represented by SEQ ID NO: 10, SEQ ID NO: 12 or SEQ ID NO: 14, or a DNA comprising a nucleotide sequence hybridizable to the nucleotide sequence represented by SEQ ID NO: 10, SEQ ID NO: 12 or SEQ ID NO: 14 under highly stringent conditions and encoding a receptor having the activity of substantially the same nature (e.g., a ligand binding activity, a signal transduction activity, etc.) as that of the human metastin receptor, mouse metastin receptor or rat metastin receptor consisting of the amino acid sequence represented by SEQ ID NO: 10, SEQ ID NO: 12 or SEQ ID NO: 14.

Examples of the DNA hybridizable to the nucleotide sequence represented by any of SEQ ID NO: 10, SEQ ID NO: 12 or SEQ ID NO: 14 include DNAs containing a nucleotide sequence having at least about 70% homology, preferably at least about 80% homology, more preferably at least about 90% homology and the most preferably at least about 95% homology, to the nucleotide sequence represented by any of SEQ ID NO: 10, SEQ ID NO: 12 or SEQ ID NO: 14.

Homology in the nucleotide sequence can be measured under the following conditions (an expectation value = 10; gaps are allowed; filtering = ON; match score = 1; mismatch score = -3) using the homology scoring algorithm NCBI BLAST (National Center for Biotechnology Information Basic Local Alignment Search Tool).

The hybridization can be carried out by per se publicly known methods or by modifications of these methods, for example, according to the method described in Molecular Cloning, 2nd (J. Sambrook et al., Cold Spring Harbor Lab. Press, 1989), etc. A commercially available library may also be used according to the instructions of the attached manufacturer's protocol. Preferably, the hybridization can be carried out under highly stringent conditions.

The highly stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration of about 19 mM at a temperature of about 65°C are most preferred.

More specifically, as the DNA encoding the human metastin receptor consisting of the amino acid sequence represented by SEQ ID NO: 9, the DNA consisting of the nucleotide sequence represented by SEQ ID NO: 10 is used.

As the DNA encoding the rat metastin receptor consisting of the amino acid sequence represented by SEQ ID NO: 11, the DNA consisting of the nucleotide sequence represented by SEQ ID NO: 12 is used.

As the DNA encoding the mouse metastin receptor consisting of the amino acid sequence represented by SEQ ID NO: 13, the DNA consisting of the nucleotide sequence represented by SEQ ID NO: 14 is used.

The metastin receptors, their partial peptides or salts thereof and the DNAs encoding the metastin receptors or their partial peptides can be obtained or produced by the methods described in WO 00/24890 or WO 01/75104.

The present invention will be described in detail by referring to EXAMPLES, FORMULATION EXAMPLES and TEST EXAMPLES, but is not deemed to be limited thereto, and any modification may be made without departing from the scope of the present invention.

In the following EXAMPLES, the term "room temperature" normally means a temperature of about 10°C to about 35°C. In percentages, the yield is shown by mol/mol% and the solvent used in chromatography by vol%, and the remaining by wt%. In proton NMR spectra, data on OH, NH protons, etc. that are broad and unidentified are not shown.

The other abbreviations used in the specification mean as follows.

Abbreviation Description

10Ψ,CSNH : C-terminal-$CONH_2$ at 10-position is substituted with -$CSNH_2$.
1Ψ2,$CH_2NH$ : The -CONH- bond between 1- and 2-positions is substituted with the -$CH_2NH$- bond.
2Ψ3,$CH_2NH$ : The -CONH- bond between 2- and 3-positions is substituted with the -$CH_2NH$- bond.
3Ψ4,$CH_2NH$ : The -CONH- bond between 3- and 4-positions is substituted with the -$CH_2NH$- bond.
4Ψ5,$CH_2NH$ : The -CONH- bond between 4- and 5-positions is substituted with the -$CH_2NH$- bond.
6Ψ7,CSNH : The -CONH- bond between 6- and 7-positions is substituted with the -CSNH- bond.
6Ψ7,NHCO : The -CONH- bond between 6- and 7-positions is substituted with the -NHCO- bond.
6Ψ7,$CH_2NH$ : The -CONH- bond between 6- and 7-positions is substituted with the -$CH_2NH$- bond.
6Ψ7,$CH_2O$ : The -CONH- bond between 6- and 7-positions is substituted with the -$CH_2O$- bond.
7Ψ8,$CH_2NH$ : The -CONH- bond between 7- and 8-positions is substituted with the -$CH_2NH$- bond.
8Ψ9,$CH_2NH$ : The -CONH- bond between 8- and 9-positions is substituted with the -$CH_2NH$- bond.
9Ψ10,$CH_2NH$ : The -CONH- bond between 9- and 10-positions is substituted with the -$CH_2NH$- bond.

Aad : 2-aminoadipic acid
Abu : 2-aminobutanoic acid
Abz(2) : 2-aminobenzoic acid
Abz(3) : 3-aminobenzoic acid
Ac : acetyl
AcONB : N-acetoxy-5-norbornene-2,3-dicarboxyimide
Acp : 6-aminocaproic acid
AcOEt : ethyl acetate
AcOH : acetic acid
Aib : α-aminoisobutanoic acid
Ala(2-Qui) : 2-quinolylalanine
Ala(3-Bzt) : 3-benzothienylalanine
Ala(cBu) : cyclobutylalanine
Ala(cPr) : cyclopropylalanine
Ala(Pip) : (4-piperidin-1-yl)alanine
Alb : albizziin 2-amino-3-ureidopropionic acid
Ambz(4) : 4-aminomethylbenzoyl
Arg(Ac) : $N^{\omega}$-acetylarginine
Arg($Boc_2$,Me) : $N^{\omega,\omega'}$-bis-tert-butoxycarbonyl-$N^{\omega}$-methylarginine
Arg(Et) : $N^{\omega}$-ethylarginine
Arg(Me) : $N^{\omega}$-methylarginine
Arg(asy$Me_2$) or Arg($Me_2$)asym : asymmetric-$N^{\omega,\omega}$-dimethylarginine
Arg(sym$Me_2$) or Arg($Me_2$)sym : symmetric-$N^{\omega,\omega}$-dimethylarginine
Arg($NO_2$) : $N^{\omega}$-nitroarginine
Arg(Pbf) : $N^{\omega}$-2,2,4,6,7-pentamethyldihydrobenzofuransulfonylarginine
Arg(n-Pr) : $N^{\omega}$-propylarginine
Arg(Tos) : $N^{\omega}$-tosylarginine
Asp(NHMe) : $N^{\omega}$-methylasparagine
Asp($NMe_2$) : $N^{\omega,\omega}$-dimethylasparagine
Asp(NHPen) : $N^{\omega}$-pentylasparagine
Asp(NHcPr) : $N^{\omega}$-cyclopropylasparagine
Asp(NHBzl) :$N^{\omega}$-benzylasparagine
AzaGly : azaglycine
AzaPhe : azaphenylalanine
Aze(2) : azetidine-2-carboxylic acid
β-Ala : β-alanine
Boc : tert-butoxycarbonyl
$Boc_2O$ : di-tert-butyl dicarbonate
Br-Z : 2-bromobenzyloxycarbonyl
$Bu^t$ : tert-butyl
Bzl : benzyl

CDI : 1,1'-carbonyldiimidazole
Cha : cyclohexylalanine
CIP : 2-chloro-1,3-dimethylimidazoliumtetrafluoroborate
Cit : citrulline
Clt resin : 2-chlorotrytyl resin
Cl-Z : 2-chlorobenzyloxycarbonyl
Dab : 2,4-diaminobutanoic acid
Dap : 2,3-diaminopropionic acid
Dap(Ac) : $N^{\beta}$-acetyl-β-diaminopropionic acid
Dap(For) : $N^{\beta}$-formyl-β-diaminopropionic acid
Dap(Gly) : $N^{\beta}$-glycyl-β-diaminopropionic acid
Dap(GnGly) : $N^{\beta}$-(N-guanidinoglycyl)-β-diaminopropionic acid
DCM : dichloromethane
DEA : diethylamine
DIEA : N,N-diisopropylethylamine
DIPCDI : 1,3-diisopropylcarbodiimide
DMAP : 4-dimethylaminopyridine
DMF : N,N-dimethylformamide
EDC : 1-ethyl-3-(3-dimethylaminopropyl)carbodiimide
EDT : 1,2-ethanedithiol
Fmoc : 9-fluorenylmethoxycarbonyl
For : formyl
γ-Abu : 4-aminobutanoic acid
γ-MeLeu, Leu(Me) : γ-methylleucine
Glyψ[(E)CH=CH]Leu : The -CONH- bond between Gly and Leu is substituted with (E)-alkene.
Glyψ($CH_2CH_2$)Leu : The -CONH- bond between Gly and Leu is substituted with -$CH_2CH_2$- bond.
Glyψ($CH_2S$)Leu : The -CONH- bond between Gly and Leu is substituted with -$CH_2S$- bond.
Gn : guanidino
GuAmb : 4-guanidinomethylbenzoyl
Har : homoarginine
Har(Me) : $N^{\omega}$-methylhomoarginine
His(3Me) : 3-methylhistidine π-methylhistidine
HOAt : 1-hydroxy-7-azabenzotriazole
HOBt : 1-hydroxybenzotriazole
HOOBt : 3,4-dihydro-3-hydroxy-4-oxo-1,2,3-benzotriazine
HONB : N-hydroxy-5-norbornene-2,3-dicarboxamide
Hph : homophenylalanine
Hyp : trans-4-hydroxyproline
Hyp(Bzl) : O-benzyl-trans-4-hydroxyproline
IndPr : 3-(indol-3-yl)propionyl
Izc : imidazolidine-2-carboxylic acid
Lys($Me_2$) : $N^{\varepsilon,\varepsilon}$-dimethyllysine
MBHA : p-methylbenzhydrylamine
MeOH : methanol
Mtt : 4-methyltrytyl
N($(CH_2)_3$Gn)Gly : N-(3-guanidinopropyl)glycine
Nal(1) : 1-naphthylalanine
Nal(2) : 2-naphthylalanine
Nar : norarginine
Nar(Me) : $N^{\omega}$-methylnorarginine
Nle : norleucine
NMeAla : $N^{\alpha}$-methylalanine
NMeArg : $N^{\alpha}$-methylarginine
NMeAsn : $N^{\alpha}$-methylasparagine
NMeLeu : $N^{\alpha}$-methylleucine
NMePhe : $N^{\alpha}$-methylphenylalanine
NMeSer : $N^{\alpha}$-methylserine
NMeTrp : $N^{\alpha}$-methyltryptophan

NMeTyr : $N^{\alpha}$-methyltyrosine
Nva : Norvaline
$OBu^t$ : tert-butoxy
Orn : ornithine
Orn(Mtt) : $N^{\delta}$-(4-methyltrytyl)ornithine
PAL : 5-(4-(9-fluorenylmethoxycarbonyl)aminomethyl-3,5-dimethoxyphenoxy)valeric acid
Pbf : 2,2,4,6,7-pentamethyldihydrobenzofuran-5-sulfonyl

PEG2K(CS)-CO: PEG2K(CS)-CO represents methoxypolyethylene glycol that has 2 kDa, and binds to $-CO(CH_2)_2-$ represented by (CS), to have a structure of the formula (a) below.

PEG5K(CS)-CO: PEG5K(CS)-CO represents methoxypolyethylene glycol that has 5 kDa, and binds to $-CO(CH_2)_2-$ represented by (CS), to have a structure of the formula (a) below.

PEG20K(CS)-CO: PEG20K(CS)-CO represents methoxypolyethylene glycol that has 20 kDa, and binds to $-CO(CH_2)_2-$ represented by (CS), to have a structure of the formula (a) below.

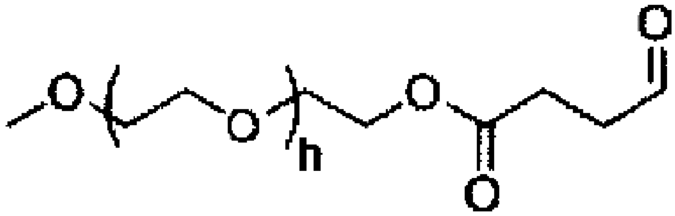

(a)

PEG20K(HS)-CO: PEG20K(HS)-CO represents methoxypolyethylene glycol that has 20 kDa, and binds to $-(CH_2)_5-$ represented by (HS), to have a structure of the formula (b) below.

PEG40K(HS)-CO: PEG40K(HS)-CO represents methoxypolyethylene glycol that has 40 kDa, and binds to $-(CH_2)_5-$ represented by (HS), to have a structure of the formula (b) below.

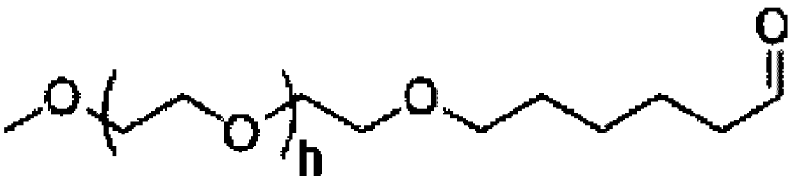

(b)

PEG30K(AL)-$CH_2$-: PEG30K(AL)-$CH_2$- represents methoxypolyethylene glycol that has 30 kDa, and binds to $-(CH_2)_2-$ represented by (AL), to have a structure of the formula (c) below.

PEG40K(AL)-$CH_2$-: PEG40K(AL)-$CH_2$- represents methoxypolyethylene glycol that has 40 kDa, and binds to $-(CH_2)_2-$ represented by (AL), to have a structure of the formula (c) below.

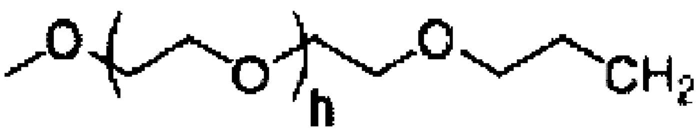

(c)

pGlu : pyroglutamic acid
Phe(2Cl) : 2-chlorophenylalanine
Phe(2F) : 2-fluorophenylalanine
Phe(3,4$Cl_2$) : 3,4-dichlorophenylalanine
Phe(3,4$F_2$) : 3,4-difluorophenylalanine
Phe(3$CF_3$) : 3-trifluoromethylphenylalanine
Phe(3Cl) : 3-chlorophenylalanine
Phe(3F) : 3-fluorophenylalanine
Phe(4Cl) : 4-chlorophenylalanine
Phe(4CN) : 4-cyanophenylalanine
Phe(4F) : 4-fluorophenylalanine
Phe(4Gn) : 4-guanidinophenylalanine

Phe($4NH_2$) : 4-aminophenylalanine
Phe($4NO_2$) : 4-nitrophenylalanine
Phe(4CN) : 4-cyanophenylalanine
Phe($F_5$) : pentafluorophenylalanine
Phe(2Me) : 2-methylphenylalanine
Phe(3Me) : 3-methylphenylalanine
Phe(4Me) : 4-methylphenylalanine
Phe$\psi$($CH_2CH_2$)AzaGly : The -CONH- bond between Phe and AzaGly is substituted with -$CH_2CH_2$- bond.
Phe$\psi$[(E)CH=CH]Gly : The -CONH- bond between Phe and Gly is substituted with (E)-alkene.
Phe$\psi$($CH_2CH_2$)Gly : The -CONH- bond between Phe and Gly is substituted with -$CH_2CH_2$- bond.
Phe$\psi$($CH_2$S)Gly : The -CONH- bond between Phe and Gly is substituted with -$CH_2$S- bond.
Phe$\psi$((R)CH(OH)-(E)CH=)Gly : The -CONH- bond between Phe and Gly is substituted with -CH(OH)-CH- bond, the -CH(OH)- position is in (R) configuration, and the bond between the carbon atom of -CH- position and the $\alpha$-carbon atom of Gly is (E) alkene.
Phe$\psi$((S)CH(OH)-(E)CH=)Gly : The -CONH- bond between Phe and Gly is substituted with -CH(OH)-CH- bond, the -CH(OH)- position is in (S) configuration, and the bond between the carbon atom of -CH- position and the $\alpha$-carbon atom of Gly is (E) alkene.
Phe$\psi$((R)CH(OH)-$CH_2$)Gly : The -CONH- bond between Phe and Gly is substituted with -CH(OH)-$CH_2$- bond, and the -CH(OH)- position is in (R) configuration.
Phe$\psi$((S)CH(OH)-$CH_2$)Gly : The -CONH- bond between Phe and Gly is substituted with -CH(OH)-$CH_2$- bond, and the -CH(OH)- position is in (S) configuration.
Phe$\psi$($CH_2$O)Gly : The -CONH- bond between Phe and Gly is substituted with -$CH_2$O- bond.
Phe$\psi$($COCH_2$)Gly : The -CONH- bond between Phe and Gly is substituted with -$COCH_2$- bond.
Phe$\psi$(CSNH)-$NH_2$ : The C terminal phenylalanylamide is substituted with phenylalanylthioamide.
Phg : phenylglycine
PhOH : phenol
PhSMe : thioanisole
Pic(3) : 3-piperidinecarboxylic acid

Pip(2) : pipecolinic acid
Pip : pipecolinic acid
Pro : proline
Pro(4F) : trans-4-fluoroproline
Pro($4NH_2$) : cis-4-aminoproline
Pya(2) : 2-pyridylalanine
Pya(3) : 3-pyridylalanine
Pya(4) : 4-pyridylalanine
PyAOP : (7-azabenzotriazole-1-yloxy)-tris(pyrrolidino)phosphonium hexafluorophosphate
PyBOP : (benzotriazole-1-yloxy)-tris(pyrrolidino)phosphonium hexafluorophosphate
PyBrop : bromo-tris(pyrrolidino)phosphonium hexafluorophosphate
Pzc(2) : piperazine-2-carboxylic acid
Sar : N-methylglycine
Ser(Ac) : O-acetylserine
Ser(Me) : O-methylserine
Thi : 2-thienylalanine
Thz : thioproline
Tic : 1,2,3,4-tetrahydroisoquinoline-3-carboxylic acid
TIS : triisopropylsilane
Tie : tert-leucine
Tos :tosyl
Trp(For) : $N^{in}$-formyltryptophan
Trt : trytyl
Tyr(Me) : O-methyltyrosine
Tyr($PO_3H_2$) : O-phosphotyrosine
Tyr$\Psi$($CH_2$NH)Asn : The -CONH- bond between Tyr and Asn is substituted with the -$CH_2$NH- bond.
TFA : trifluoroacetic acid
TFE : trifluoroethanol
Z : benzyloxycarbonyl

In the specification and drawings, where the codes of nucleotides and amino acids are denoted by abbreviations, they are based on the abbreviations in accordance with the IUPAC-IUB Commission on Biochemical Nomenclature or the common codes in the art, examples of which are shown below. For amino acids that may have the optical isomer, L form is presented unless otherwise indicated.

| | |
|---|---|
| DNA | : deoxyribonucleic acid |
| cDNA | : complementary deoxyribonucleic acid |
| A | : adenine |
| T | : thymine |
| G | : guanine |
| C | : cytosine |
| Y | : thymine or cytosine |
| N | : thymine, cytosine, adenine or guanine |
| R | : adenine or guanine |
| M | : cytosine or adenine |
| W | : thymine or adenine |
| S | : cytosine or guanine |
| RNA | : ribonucleic acid |
| mRNA | : messenger ribonucleic acid |
| dATP | : deoxyadenosine triphosphate |
| dTTP | : deoxythymidine triphosphate |
| dGTP | : deoxyguanosine triphosphate |
| dCTP | : deoxycytidine triphosphate |
| ATP | : adenosine triphosphate |
| EDTA | : ethylenediaminetetraacetic acid |
| SDS | : sodium dodecyl sulfate |
| TFA | : trifluoroacetic acid |
| EIA | : enzyme immunoassay |
| Gly or G | : glycine |
| Ala or A | : alanine |
| Val or V | : valine |
| Leu or L | : leucine |
| Ile or I | : isoleucine |
| Ser or S | : serine |
| Thr or T | : threonine |
| Cys or C | : cysteine |
| Met or M | : methionine |
| Glu or E | : glutamic acid |
| Asp or D | : aspartic acid |
| Lys or K | : lysine |
| Arg or R | : arginine |
| His or H | : histidine |
| Phe or F | : phenylalanine |
| Tyr or Y | : tyrosine |
| Trp or W | : tryptophan |
| Pro or P | : pro line |
| Asn or N | : asparagine |
| Gin or Q | : glutamine |
| pGlu | : pyroglutamic acid |

The sequence identification numbers in the sequence listing of the specification indicates the following sequence, respectively.

[SEQ ID NO: 1]
This shows the amino acid sequence of human-derived metastin (metastin).
[SEQ ID NO: 2]
This shows the nucleotide sequence of DNA encoding human metastin.
[SEQ ID NO: 3]

This shows the amino acid sequence of mouse metastin precursor (A).
[SEQ ID NO: 4]
This shows the nucleotide sequence of DNA encoding mouse metastin precursor (A), which is the nucleotide sequence contained in plasmid pCMV-mKiSS-1 harbored on transformant Escherichia coli DH10B/pCMV-mKiSS-1.
[SEQ ID NO: 5]
This shows the amino acid sequence of mouse metastin precursor (B).
[SEQ ID NO: 6]
This shows the nucleotide sequence of DNA encoding mouse metastin precursor (B), which is the nucleotide sequence contained in plasmid pCR2.1-mKiSS-1.4A harbored on transformant Escherichia coli DH5α/pCR2.1-mKiSS-1.4A.
[SEQ ID NO: 7]
This shows the amino acid sequence of rat-derived metastin precursor.
[SEQ ID NO: 8]
This shows the nucleotide sequence of DNA encoding rat metastin precursor.
[SEQ ID NO: 9]
This shows the amino acid sequence of human OT7T175 (metastin receptor).
[SEQ ID NO: 10]
This shows the nucleotide sequence of DNA encoding human OT7T175 (metastin receptor).
[SEQ ID NO: 11]
This shows the amino acid sequence of rat OT7T175 (metastin receptor).
[SEQ ID NO: 12]
This shows the nucleotide sequence of DNA encoding rat OT7T175 (metastin receptor).
[SEQ ID NO: 13]
This shows the amino acid sequence of mouse OT7T175 (metastin receptor).
[SEQ ID NO: 14]
This shows the nucleotide sequence of DNA encoding mouse OT7T175 (metastin receptor).
[SEQ ID NO: 15]
This shows the amino acid sequence of human metastin 15 (40-54).
[SEQ ID NO: 16]
This shows the amino acid sequence of human metastin 10 (45-54) (MS10).
[SEQ ID NO: 17]
This shows the amino acid sequence of human metastin 9 (46-54).
[SEQ ID NO: 18]
This shows the amino acid sequence of human metastin 8 (47-54).
[SEQ ID NO: 19]
This shows the nucleotide sequence of DNA encoding human metastin 15 (40-54).
[SEQ ID NO: 20]
This shows the nucleotide sequence of DNA encoding human metastin 10 (45-54).
[SEQ ID NO: 21]
This shows the nucleotide sequence of DNA encoding human metastin 9 (46-54).
[SEQ ID NO: 22]
This shows the nucleotide sequence of DNA encoding human metastin 8 (47-54).

The transformant *Escherichia coli* DH10B/pCMV-mKiSS-1 has been on deposit since January 24, 2000 with International Patent Organisms Depository, National Institute of Advanced Industrial Science and Technology (the former Ministry of International Trade and Industry, Agency of Industrial Science and Technology, National Institute of Bioscience and Human Technology (NIBH)), located at Central 6, 1-1-1 Higashi, Tsukuba, Ibaraki (postal code 305-8566), Japan, under the Accession Number FERM BP-7003 and since December 16, 1999 with Institute for Fermentation (IFO), located at 2-17-85, Juso-Honmachi, Yodogawa-ku, Osaka-shi, Osaka, Japan, under the Accession Number IFO 16348.

The transformant *Escherichia coli* DH5α/pCR2.1-mKiSS-1.4A has been on deposit since March 6, 2000 with International Patent Organisms Depository, National Institute of Advanced Industrial Science and Technology (the former Ministry of International Trade and Industry, Agency of Industrial Science and Technology, National Institute of Bioscience and Human Technology (NIBH)), located at Central 6, 1-1-1 Higashi, Tsukuba, Ibaraki (postal code 305-8566), Japan, under the Accession Number FERM BP-7073 and since February 16, 2000 with Institute for Fermentation (IFO), located at 2-17-85 Juso-Honmachi, Yodogawa-ku, Osaka-shi, Osaka, Japan, under the Accession Number IFO 16360.

[Reference Example 1] H-Tyr(Bu$^t$)-Asn(Trt)-Trp(Boc)-Asn(Trt)-Ser(Bu$^t$)-Phe-Gly-Leu-Arg(Pbf)-Phe-Rink amide-PEG resin

To 242 mg (0.15 mmol) ofNovaPEG Rink amide resin as a starting material were introduced sequentially Phe, Arg(Pbf), Leu, Gly, Phe, Ser(Bu$^t$), Asn(Trt), Trp(Boc), Asn(Trt) and Tyr(Bu$^t$) according to ABI 433A (Fmoc/DCC/HOBt 0.25 mmol protocol), and then the mixture was dried under reduced pressure to give the intended H-Tyr(Bu$^t$)-Asn(Trt)-Trp(Boc)-Asn(Trt)-Ser(Bu$^t$)-Phe-Gly-Leu-Arg(Pbf)-Phe-Rink amide-PEG resin (482 mg, loading Calcd.: 0.276 mmol/g).

EXAMPLE 1

Synthesis a PEG5K(CS)-CO-Acp-Tyr-Asn-Trp-Asn-Ser-Phe-Gly-Leu-Arg-Phe-$NH_2$ (compound 1)

H-Tyr(Bu$^t$)-Asn(Trt)-Trp(Boc)-Asn(Trt)-Ser(Bu$^t$)-Phe-Gly-Leu-Arg(Pbf)-Phe-Rink amide-PEG resin (0.276 mmol/g, 36.2 mg, 0.01 mmol) obtained in Reference Example 1 was swollen in DMF, and then the resultant was added with Fmoc-Acp-OH (14.1 mg, 0.04 mmol), HOAt in DMF (0.5 M, 80 $\mu$L, 0.04 mmol) and DIPCDI (6.36 $\mu$L, 0.04 mmol) along with DMF, and treated at room temperature for 4 hours. The resin was washed with DMF, and the same procedure was repeated for 12 hours. The resin was washed with DMF, and then added with $Ac_2O$ (3.65 $\mu$L, 0.04 mmol) and DIEA (6.97 $\mu$L, 0.04 mmol) along with DMF, and treated for 30 minutes. The resin was washed with DMF, and then treated with 20% piperidine/DMF solution for 20 minutes. The resin was washed with DMF, and then treated with ME-050CS (NOF CORPORATION, average molecular weight: 5325, 107 mg, 0.02 mmol) and triethylamine (2.78 $\mu$L, 0.02 mmol) in DMF:$H_2O$ (10:1) for 2 days. The resin was washed with DMF and MeOH, and then dried under reduced pressure. The dried, obtained resin (69.0 mg) was added with 1.5 mL of TFA/PhSMe/m-cresol/$H_2O$/TIS/EDT (80/5/5/5/2.5/2.5) and stirred at room temperature for 4 hours. Diethylether was added to the reaction solution and the resulting precipitate was separated by centrifugation, and then the supernatant was removed. This procedure was repeated twice followed by washing. The residue was extracted with a 50% aqueous solution of acetic acid, and the resin was removed by filtration. Then, linear concentration gradient elution (60 minutes) was performed at a flow rate of 8 ml/min using eluant A: 0.1 % TFA-water and eluant B: 0.1 % TFA-containing acetonitrile with A/B: 60.0/40.0 to 45.0/55.0, on preparative HPLC using Daisopak-SP100-5-ODS-P (2 × 25 cm). The fractions containing the product were collected and lyophilized to give 9.4 mg of white powders.

Mass spectrum: $(M+H)^+$ 6021-7680 (calculated molecular weight, calculated from PEG average molecular weight: about 6626)
Elution time on HPLC: 15.8 mins.
Elution conditions:

Column: SHISEIDO CAPCELL PAK C18 MGII (4.6 × 100 mm)
Linear concentration gradient elution using eluant A: 0.1% TFA-water and eluant B: 0.1% TFA-containing acetonitrile, with A/B = 80/20 to 30/70 (25 mins.)
Flow rate: 1.0 mL/min.

EXAMPLE 2

Synthesis b PEG2K(CS)-CO-Acp-Tyr-Asn-Trp-Asn-Ser-Phe-Gly-Leu-Arg-Phe-$NH_2$ (compound 2)

H-Tyr(Bu$^t$)-Asn(Trt)-Trp(Boc)-Asn(Trt)-Ser(Bu$^t$)-Phe-Gly-Leu-Arg(Pbf)-Phe-Rink amide-PEG resin (0.276 mmol/g, 36.2 mg, 0.01 mmol) was swollen in DMF, and then the resultant was added with Fmoc-Acp-OH (14.1 mg, 0.04 mmol), HOAt in DMF (0.5 M, 80 $\mu$L, 0.04 mmol) and DIPCDI (6.36 $\mu$L, 0.04 mmol) along with DMF, and treated at room temperature for 4 hours. The resin was washed with DMF, and the same procedure was repeated for 12 hours. The resin was washed with DMF, and then added with $Ac_2O$ (3.6 $\mu$L, 0.04 mmol) and DIEA (6.97 $\mu$L, 0.04 mmol) along with DMF, and treated for 30 minutes. The resin was washed with DMF, and then treated with 20% piperidine/DMF solution for 20 minutes. The resin was washed with DMF, and then treated with ME-020CS (NOF CORPORATION, average molecular weight: 2322, 46.4 mg, 0.02 mmol) and triethylamine (2.78 $\mu$L, 0.02 mmol) in DMF:$H_2O$ (10:1) for 2 days. The resin was washed with DMF and MeOH, and then dried under reduced pressure. The dried, obtained resin (40.3 mg) was added with 1.5 mL of TFA/PhSMe/m-cresol/$H_2O$/TIS/EDT(80/5/5/5/2.5/2.5), and stirred at room temperature for 4 hours. Diethylether was added to the reaction solution and the resulting precipitate was separated by centrifugation, and then the supernatant was removed. This procedure was repeated twice followed by washing. The residue was extracted with a 50% aqueous solution of acetic acid, and the resin was removed by filtration. Then, linear concentration

gradient elution (60 minutes) was performed at a flow rate of 8 mL/min using eluant A: 0.1% TFA-water and eluant B: 0.1% TFA-containing acetonitrile with A/B: 62.0/38.0 to 47.0/53.0 on preparative HPLC using Daisopak-SP100-5-ODS-P (2 × 25 cm). The fractions containing the product were collected and lyophilized to give 1.5 mg of white powders.

Mass spectrum: $(M+H)^+$ 3292-4105 (calculated molecular weight, calculated from PEG average molecular weight: about 3623)
Elution time on HPLC: 14.9 mins
Elution conditions:

Column: SHISEIDO CAPCELL PAK C18 MGII (4.6 × 100 mm)
Linear concentration gradient elution using eluant A: 0.1% TFA-water and eluant B: 0.1% TFA-containing acetonitrile, with A/B = 80/20 to 30/70 (25 mins.)
Flow rate: 1.0 mL/min

[Reference Example 2]

Synthesis c Acp-Tyr-Asn-Trp-Asn-Ser-Phe-Gly-Leu-Arg-Phe-$NH_2$ (compound A)

H-Tyr($Bu^t$)-Asn(Trt)-Trp(Boc)-Asn(Trt)-Ser($Bu^t$)-Gly-Leu-Arg(Pbf)-Phe-Rink amide-PEG resin (0.276 mmol/g, 283 mg, 0.0781 mmol) obtained in Reference Example 1 was swollen in DMF, and then the resultant was added with Fmoc-Acp-OH (168 mg, 0.469 mmol), HOAt in DMF (0.5 M, 938 μL, 0.469 mmol) and DIPCDI (74.6 μL, 0.469 mmol), and shook at room temperature overnight. The resin was washed with DMF, and then treated with 20% piperidine/DMF solution for 20 minutes to remove the Fmoc group, and then washed with DMF and MeOH sequentially and dried under reduced pressure. The whole amount of the obtained, dried resin was suspended in 5 mL of TFA/thioanisole/m-cresol/$H_2O$/EDT/TIS (80:5:5:5:2.5:2.5), and stirred for 240 mins. The reaction solution was dropped to diethylether while the resin was removed by filtration to induce precipitation, and the resulting precipitate was separated by centrifugation, and the supernatant was removed. This procedure was repeated twice followed by washing. The residue was extracted with an aqueous solution of acetic acid, and purified separately twice on preparative HPLC using YMC Pack R&D-ODS-5-B S-5, 120A column (30 × 250 mm). Linear concentration gradient elution (60 minutes) was performed with elution conditions of eluant A: 0.1% TFA-water and eluant B: 0.1% TFA-containing acetonitrile with A/B: 72/28 to 62/38 at a flow rate of 15 mL/min. The fractions containing the product were collected and lyophilized to give 25.9 mg of the product TFA salt as white powders.

Mass spectrum: $(M+H)^+$ 1416.1 (Calcd. 1415.7)
Elution time on HPLC: 11.5 mins
Elution conditions:

Column: SHISEIDO CAPCELL PAK C18 MGII (4.6 × 100 mm)
Linear concentration gradient elution using eluant A: 0.1 % TFA-water and eluant B: 0.1% TFA-containing acetonitrile, with A/B = 80/20 to 30/70 (25 mins.)
Flow rate: 1.0 mL/min

EXAMPLE 3

Synthesis d PEG20K(CS)-CO-Acp-Tyr-Asn-Trp-Asn-Ser-Phe-Gly-Leu-Arg-Phe-$NH_2$ (compound 3)

H-Acp-Tyr-Asn-Trp-Asn-Ser-Phe-Gly-Leu-Arg-Phe-$NH_2$ TFA salt obtained in Reference Example 2 (5 mg) and ME-200CS (NOF CORPORATION, average molecular weight: 20687, 65.7 mg, 0.00318 mmol) were dissolved in DMF/$H_2O$ (0.5/0.2 mL), and the obtained solution was added with $Et_3N$ (1.47 μL, 0.0106 mmol), and stirred overnight at room temperature. The reaction solution was added to diethylether and the precipitate formed was separated by centrifugation, and then the supernatant was removed followed by washing.
The residue was extracted with water. The solution was pre-treated with a membrane filter, and then linear concentration gradient elution (60 minutes) was performed using eluant A: 0.1 % TFA-water and eluant B: 0.1 % TFA-containing acetonitrile with A/B: 50/50 to 40/60 on preparative HPLC using YMC Pack R&D-ODS-5-B S-5, 120A column (30 × 250 mm) at a flow rate of 15 mL/min. The fractions containing the product were collected and lyophilized to give 29.6 mg of the product TFA salt as white powders.

Mass spectrum: $(M+H)^+$ 19700 to 25600 (Calcd. 21988)

Elution time on HPLC: 16.6 mins
Elution conditions:

Column: SHISEIDO CAPCELL PAK C18 MGII (4.6 × 100 mm)
Linear concentration gradient elution using eluant A: 0.1% TFA-water and eluant B: 0.1% TFA-containing acetonitrile, with A/B = 80/20 to 30/70 (25 mins.)
Flow rate: 1.0 mL/min

Compounds 5 and 6 were synthesized by Synthesis d in the same manner.

EXAMPLE 4

Synthesis e PEG40K(AL)-$CH_2$-Acp-Tyr-Asn-Trp-Asn-Ser-Phe-Gly-Leu-Arg-Phe-$NH_2$ (compound 4)

H-Acp-Tyr-Asn-Trp-Asn-Ser-Phe-Gly-Leu-Arg-Phe-$NH_2$ (3.6 mg) obtained in Reference Example 2 and ME-400 AL2 (NOF CORPORATION, average molecular weight: 41384, 78.8 mg, 0.00191 mmol) were dissolved in DMF (3 mL). The reaction solution was added with AcOH (30 μL), and $NaBH(OAc)_3$ (10 mg) sequentially, and then stirred at room temperature for 2 hours. The reaction solution was added to diethylether and the precipitate formed was separated by centrifugation. Then, the supernatant was removed followed by washing. The residue was extracted with water. The solution was pre-treated with a membrane filter, and then linear concentration gradient elution (60 minutes) was performed using eluant A: 0.1 % TFA-water and eluant B: 0.1 % TFA-containing acetonitrile with A/B: 60/40 to 30/70 on preparative HPLC using YMC Pack R&D-ODS-5-B S-5, 120A column (30 × 250 mm) at a flow rate of 15 mL/min. The fractions containing the product were collected and lyophilized to give 57.6 mg of the product TFA salt as white powders.

Mass spectrum: $(M+H)^+$ 41000 to 47000 (calculated molecular weight, calculated from PEG average molecular weight: about 42784)
Elution time on HPLC: 16.5 mins
Elution conditions:

Column: SHISEIDO CAPCELL PAK C18 MGII (4.6 × 100 mm)
Linear concentration gradient elution using eluant A: 0.1% TFA-water and eluant B: 0.1% TFA-containing acetonitrile, with A/B = 80/20 to 30/70 (25 mins.)
Flow rate: 1.0 mL/min

Compound 7 was synthesized by Synthesis e in the same manner.

[Reference Example 3]

Synthesis of Fmoc-Arg(Me,$Boc_2$)-Trp(Boc)-Rink amide MBHA resin

Rink amide MBHA resin (0.45 mmol/g, 2.22 g, 1.00 mmol) was swollen in DMF, and then Trp(Boc) and Orn(Mtt) were sequentially introduced according to Fmoc-SPPS using Fmoc-AA-OH/DIPCDI/HOOBt (4eq.) (75 min) in condensation and 20% piperidine/DMF in removing the Fmoc group (1+20 min), to give Fmoc-Orn(Mtt)-Trp(Boc)-Rink amide MBHA resin. The Fmoc-Orn(Mtt)-Trp(Boc)-Rink amide MBHA resin was washed with MeOH, and then swollen in toluene. The toluene was filtered, and then a procedure of treating the resin with TFA/TIS/toluene (1:5:94) was repeated until the color reaction (yellow) was not recognized for the reaction solution (30 min × 4 + 3hr). The resin was washed with toluene, and then neutralized with 5% DIEA-toluene solution. The obtained resin was added with N-methyl-N,N'-bis-Boc-1-guanylpyrazole (0.973 g, 3 mmol) in toluene/TFE (4:1), and adjusted to pH 10 or so with DIEA, and treated at room temperature overnight. The resin was washed with toluene, and then it was confirmed that a Kaiser test was negative. The resin was further washed with DMF and MeOH sequentially, and then dried with a desiccator to give Fmoc-Arg(Me,$Boc_2$)-Trp(Boc)-Rink amide MBHA resin.
Yield: 2.682 g (substitution rate: 0.269 mmol/g)

[Reference Example 4]

Measurement of the resin substitution rate by quantitative determination of Fmoc group

1.374 mg of Fmoc-Arg(Me,$Boc_2$)-Trp(Boc)-Rink amide MBHA resin was weighed and put into a 20 mL measuring

flask. 4 mL piperidine was added thereto, and then the mass was increased with DMF 20 mL. The measuring flask was tumbled and mingled, and then allowed to stand for 30 minutes. These procedures were conducted for a measuring flask with no addition of the resin, which was taken as a blank solution. The absorbance at 301 nm was measured for each of the solutions. As a result, $Abs_{sample}$ was 0.324 and $Abs_{blank}$ was 0.180. With use of these values, the substitution rate was calculated by the equation below.

$$\text{Substitution rate (mmol/g)} = (Abs_{sample} - Abs_{blank}) \times 20\ \text{(mL)}/7800 \times \text{resin amount (g)}$$

[Reference Example 5]

Synthesis of Fmoc-AzaGly-Leu-Arg(Me,$Boc_2$)-Trp(Boc)-Rink amide MBHA resin

Fmoc-Arg(Me,$Boc_2$)-Trp(Boc)-Rink amide MBHA resin (0.269 mmol/g, 2.37 g, 0.636 mmol) was swollen in DMF, and then treated with 20% piperidine/DMF for 20 minutes to remove the Fmoc group. The resin was washed with DMF, and then treated with Fmoc-Leu-OH (899 mg, 2.54 mmol), HOOBt (415 mg, 2.54 mmol) and DIPCDI (404 μL, 2.54 mmol) in DMF for 75 minutes. The resin was washed with DMF, and then treated with 20% piperidine/DMF for 20 minutes to remove the Fmoc group, and then washed with DMF, to give H-Leu-Arg(Me,$Boc_2$)-Trp(Boc)-Rink amide MBHA resin. On the other hand, a solution of Fmoc-$NHNH_2$ (739 mg, 2.54 mmol) in DMF (10 mL) was added with a solution of CDI (392 mg, 2.54 mmol) in THF (10 mL), and stirred at room temperature for 1 hour. The H-Leu-Arg(Me,$Boc_2$)-Trp(Boc)-Rink amide MBHA resin (DMF swollen: 0.636 mmol) was added with the above-mentioned solution, and shook at room temperature overnight. The resin was washed with DMF, and it was confirmed that a Kaiser test was positive. Therefore, the resin was capping-treated with decanoic anhydride (234 μL, 0.636 mmol) and DIEA (111 μL, 0.636 mmol) in DMF at room temperature for 30 minutes, and then it was confirmed that a Kaiser test was negative. The obtained Fmoc-AzaGly-Leu-Arg(Me,$Boc_2$)-Trp(Boc)-Rink amide MBHA resin was washed with MeOH and then dried under reduced pressure (yield: 2.66 g, 0.636 mmol).

[Reference Example 6]

Synthesis f H-Acp-D-Tyr-D-Trp-Asn-Thr-Phe-AzaGly-Leu-Arg(Me)-Trp-$NH_2$ (compound B)

Fmoc-AzaGly-Leu-Arg(Me,$Boc_2$)-Trp(Boc)-Rink amide MBHA resin (0.378 mmol/g, 159 mg, 0.06 mmol) was swollen in DMF, and then Phe, Thr($Bu^t$), Asn(Trt), D-Trp(Boc), D-Tyr($Bu^t$) and Acp were sequentially condensed according to manual Fmoc-SPPS using Fmoc-AA-OH/DIPCDI/HOAt (4eq.) (75 mins) in condensation and 20% piperidine/DMF in removing the Fmoc group (1+20 minutes) to give H-Acp-D-Tyr($Bu^t$)-D-Trp-Asn-Thr-Phe-AzaGly-Leu-Arg(Me,$Boc_2$)-Trp(Boc)-Rink amide MBHA resin. The resin was sequentially washed with DMF and MeOH, and then dried under reduced pressure. 207 mg of the obtained, dried resin was suspended in 2 mL of TFA/thioanisole/m-cresol/$H_2O$/EDT/TIS (80:5:5:5:2.5:2.5) and stirred for 180 minutes. Diethylether was added to the reaction solution and the precipitate formed was separated by centrifugation, and then the supernatant was removed. This procedure was repeated twice followed by washing. The residue was extracted with an aqueous solution of acetic acid, and the resin was removed by filtration, and then purified separately twice on preparative HPLC using YMC Pack R&D-ODS-5-B S-5, 120A column (30 × 250 mm). Linear concentration gradient elution (60 minutes) was performed with elution conditions of eluant A: 0.1 % TFA-water and eluant B: 0.1 % TFA-containing acetonitrile at a flow rate of 15 mL/min with A/B: 71.5/28.5 to 61.5/38.5 or 70/30 to 60/40. The fractions containing the product were collected and lyophilized to give 7.1 mg of the product TFA salt as white powders.

Mass spectrum: $(M+H)^+$ 1370.1 (Calcd. 1369.7)
Elution time on HPLC: 10.8 mins
Elution conditions:

Column: SHISEIDO CAPCELL PAK C18 MGII (4.6 × 100 mm)
Linear concentration gradient elution using eluant A: 0.1 % TFA-water and eluant B: 0.1% TFA-containing acetonitrile, with A/B = 80/20 to 30/70 (25 mins.)
Flow rate: 1.0 mL/min

EXAMPLE 5

Synthesis g

PEG20K(CS)-CO-Acp-D-Tyr-D-Trp-Asn-Thr-Phe-AzaGly-Leu-Arg(Me)-Trp-$NH_2$ (compound 8)

H-Acp-D-Tyr-D-Trp-Asn-Thr-Phe-AzaGly-Leu-Arg(Me)-Trp-$NH_2$TFA salt (3 mg) obtained in Reference Example 6 and ME-200CS (NOF CORPORATION, average molecular weight: 19865, 34.8 mg, 0.00175 mmol) were dissolved in DMF (1.5 mL), and the reaction solution was added with $Et_3N$ (1.52 μL, 0.011 mmol), and stirred at room temperature overnight. The reaction solution was added to diethylether and the precipitate formed was separated by centrifugation, and then the supernatant was removed followed by washing.
The residue was extracted with water. The solution was pre-treated with a membrane filter, and then linear concentration gradient elution (60 minutes) was performed using Eluant A: 0.1 % TFA-water and Eluant B: 0.1 % TFA-containing acetonitrile with A/B: 60/40 to 30/70 on preparative HPLC using YMC Pack R&D-ODS-5-B S-5, 120A column (30 × 250 mm) at a flow rate of 15 mL/min. The fractions containing the product were collected and lyophilized to give 18.8 mg of the product TFA salt as white powders.

Mass spectrum: $(M+H)^+$ 20000 to 29000 (calculated molecular weight, calculated from PEG average molecular weight: about 21119)
Elution time on HPLC: 16.9 mins
Elution conditions:

Column: SHISEIDO CAPCELL PAK C18 MGII (4.6 × 100 mm)
Linear concentration gradient elution using eluant A: 0.1 % TFA-water and eluant B: 0.1% TFA-containing acetonitrile, with A/B = 80/20 to 30/70 (25 mins.).
Flow rate: 1.0 mL/min

Compounds 9 to 11 were synthesized by Synthesis g in the same manner.
The structures and the physical property values of compounds 5 to 7 and 9 to 11 are shown in Table 1. In Table 1, (obs.) indicates an actual value, (cal.) indicates a calculated molecular weight, and (min.) indicates minutes.

[Table 1]

| Compound No. | Structure | $M+H^+$ (obs.) | $M+H^+$ (cal.) | HPLC (min.) |
|---|---|---|---|---|
| 5 | PEG20K(HS)-CO-Acp-Tyr-Asn-Trp-Asn-Ser-Phe-Gly-Leu-Arg-Phe-$NH_2$ | 20000-25000 | 22676 | 16.3 |
| 6 | PEG40K(HS)-CO-Acp-Tyr-Asn-Trp-Asn-Ser-Phe-Gly-Leu-Arg-Phe-$NH_2$ | 42000-48000 | 44132 | 16.5 |
| 7 | PEG30K(AL)-$CH_2$-Acp-Tyr-Asn-Trp-Asn-Ser-Phe-Gly-Leu-Arg-Phe-$NH_2$ | 31000-36000 | 32775 | 17.0 |
| 9 | PEG20K(CS)-CO-Acp-D-Tyr-Hyp-Asn-Thr-Phe-AzaGly-Leu-Arg(Me)-Trp-$NH_2$ | 20000-29000 | 21046 | 16.3 |
| 10 | PEG20K(CS)-CO-Acp-D-Tyr-Glu-Asn-Thr-Phe(3F)-AzaGly-Leu-Arg(Me)-Trp-$NH_2$ | 19000-29000 | 21080 | 16.5 |
| 11 | PEG20K(CS)-CO-Acp-D-Tyr-Hyp-Asn-Thr-Phe(4F)-AzaGly-Leu-Arg(Me)-Trp-$NH_2$ | 19000-28000 | 21064 | 16.3 |

Test Example 1 LH And Testosterone Release Actions In Male Rat

For male Crl: CD (SD) rats (8 or 10 weeks old), compounds 1 (10 weeks old), 3, 8, 9, 10 and 11 (8 weeks old) (1, 10 or 100 nmol/kg), and metastin (45-54) (PEPTIDE INSTITUTE, INC., 10 or 100 nmol/kg), which were dissolved in physiological saline, were administered subcutaneously to the dorsum. For the control group, a physiological saline was administered subcutaneously to the dorsum. About 0.4 mL of the blood was collected from the tail vein before the administration (0 hour) and after 0.5, 1, 2, 4 hours of the administration of compounds 1, 3, 8, 9, 10 and 11, and metastin

(45-54), or physiological saline. The collected blood was well mixed with 12 μL of 10 w/v% EDTA-2Na-containing trasylol solution (Bayer AG), and then separated by centrifugation at 12,000 rpm for 10 minutes, and the supernatant was recovered as the plasma sample. The concentrations of luteinizing hormone (LH) and total testosterone in the plasma were measured with radioimmunoassay (rat LH RIA Immuno-reagent set, National Hormone and Peptide Program, available from Dr. A. F. Parlow, The National Institute of Diabetes and Digestive and Kidney Diseases, and DPC Total Testosterone Kit, Mitsubishi Chemical Formula Iatron, Inc.).

The change of LH concentration in the rat plasma is shown in Fig. 1, and the change of total testosterone concentration in the plasma is shown in Fig. 2. When the area under the curves (AUC) from 0 hour to 4 hours after the administration were compared, the compound 1 showed significant increase in the LH concentration and the total testosterone concentration as well at 10 nmol/kg or more. On the other hand, metastin (45-54) showed significant increase only at 100 nmol/kg. Furthermore, in comparison to metastin (45-54), compound 1 had greater degree of increase in LH and total testosterone concentrations in the plasma. In the same manner, compound 3 showed significant increase in the LH concentration and the total testosterone concentration as well at 10 nmol/kg or more, and compounds 8, 9, 10 and 11 showed increase at 1 nmol/kg or more.

INDUSTRIAL APPLICABILITY

The present invention provides stable metastin derivatives modified with a polyethylene glycol having excellent biological activities (a cancer metastasis suppressing activity, a cancer growth suppressing activity, a gonadotropic hormone secretion promoting activity, a sex hormone secretion promoting activity, a gonadotropic hormone secretion suppressing activity, a sex hormone secretion suppressing activity, etc.).

Embodiments of the invention include the following:

[1] A metastin derivative modified with a polyethylene glycol or a salt thereof.

[2] The metastin derivative modified with a polyethylene glycol in accordance with [1], which is represented by the formula below:

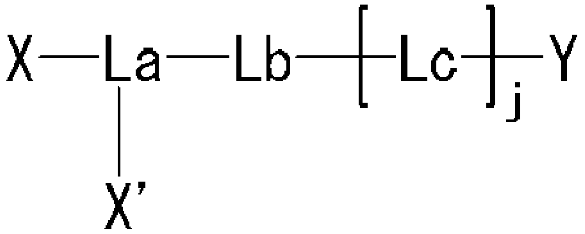

(I)

[wherein

Y represents a metastin derivative;
X represents a polyethylene glycol;
X' is absent, or represents a polyethylene glycol;
La represents a bivalent or trivalent group represented by formula:

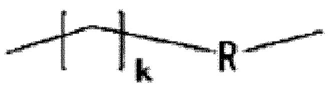

or

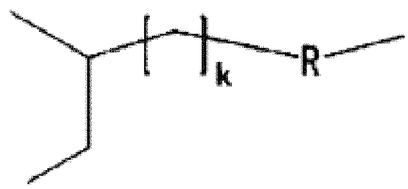

(wherein

R represents a chemical bond, -O-, -CO-O-, -O-CO-, -NH-, -CO-, -S-, -S-S-, -SO-, $-SO_2-$, $-NH-SO_2-$, $-SO_2-NH-$, -C(=O)-NH-N=CH-, -C(=NH)-NH-, $-CO-CH_2-S-$, or

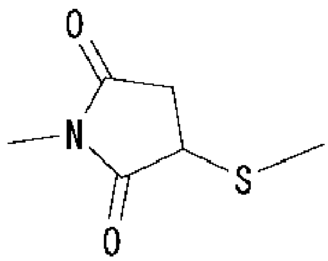

;

and

k represents an integer of 0 to 5.);

Lb represents $-(CH_2)_i-$ (wherein i represents an integer of 0 to 5.);
Lc represents a bivalent group represented by

(i)

formula: $-B-Q^c-C^b-$

(wherein

B represents a chemical bond, -NH-, -O-, or -S-;
$Q^c$ represents a bivalent group represented by

formula: $-(CH_2)_{m1}-Z^c-(CH_2)_{m2}-$

(wherein

m1 represents an integer of 0 to 15;
$Z^c$ represents:

(a) a chemical bond; or
(b) a bivalent group selected from -CO-, -O-CO-, -CO-O-, -CO-NH-, - NH-CO-, -CO-NH-CO-, -NH-CO-NH-, $-CH(NH_2)-$, $-CH(-NHR^{zc1})-$, $-CH(R^{zc2})-$,-CH(OH)-, -CH(COOH)-, -C(=NH)-, -S-, -S-S-, -SO-, $-SO_2-$, $-NH-SO_2-$, $-SO_2-NH-$,

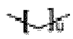
,
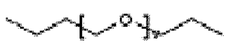
,
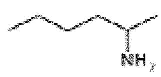
,
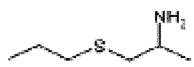
,

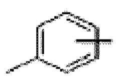
,
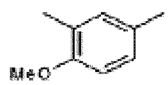
,
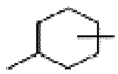
,

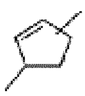
,
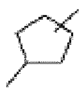
,
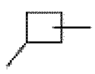
,
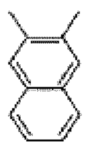
,
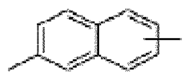
,

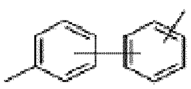, 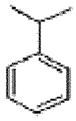, 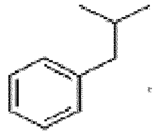, 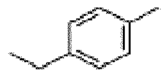,

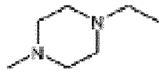, 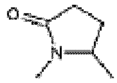, 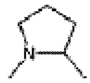, 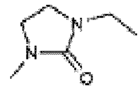,

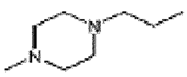, 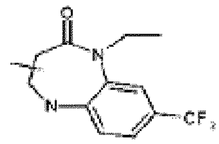, 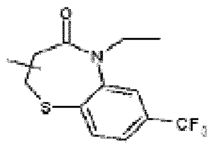,

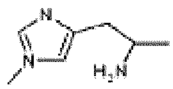, 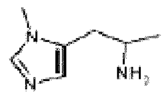

and

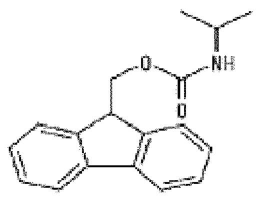

(wherein

u represents an integer of 1 to 18;
v represents an integer of 1 to 12;
$R^{zc1}$ represents an amino-straight $C_{1-5}$ alkyl-carbonyl group, or a X-straight $C_{1-5}$ alkyl group;
$R^{zc2}$ represents an amino-straight $C_{1-5}$ alkyl-carbonyl amino-straight $C_{1-5}$ alkyl group; and
X represents the same as those described above.); and
m2 represents an integer of 0 to 15.); and
$C^b$ represents a chemical bond, -CO-, or $-SO_2-$.); or

(ii)

formula: $-Q^{c'}-C^{b'}-$

(wherein

$Q^{c'}$ represents formula: $-(CH_2)_{m1'}-Z^{c'}-(CH_2)_{m2'}-$
(wherein

m1' represents an integer of 0 to 15;
$Z^{c'}$ represents a bivalent group selected from

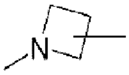 , 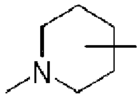 , 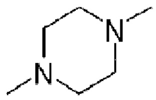 , 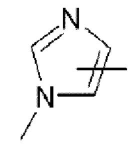 ,

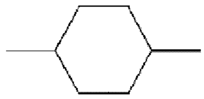 , 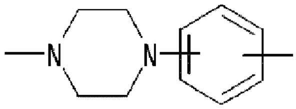 , and 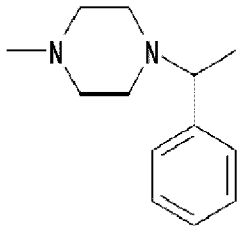 ;

and
m2' represents an integer of 0 to 15.); and
$C^{b'}$ represents -CO- or $-SO_2-$.); and
j represents an integer of 1 to 3.]; or a salt thereof.

[3] The metastin derivative modified with a polyethylene glycol or a salt thereof in accordance with [1] or [2], wherein the metastin derivative is selected from the group consisting of formulae (1) to (5) described below:

a metastin derivative represented by formula (1):

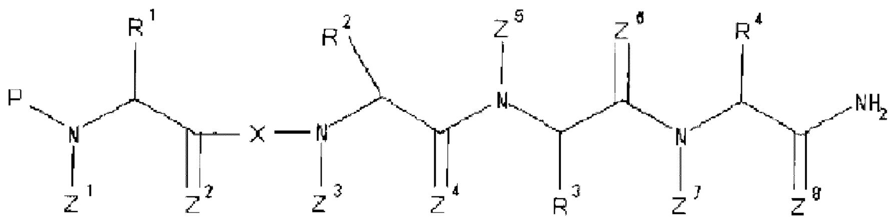

(1)

[wherein

each of $Z^1$, $Z^3$, $Z^5$ and $Z^7$ represents a hydrogen atom or a $C_{1-3}$ alkyl group;
each of $Z^2$, $Z^4$, $Z^6$ and $Z^8$ represents a hydrogen atom, O or S;
$R^1$ represents:

(1) a hydrogen atom; or
(2) a $C_{1-8}$ alkyl group optionally substituted with a substituent selected from the group consisting of an optionally substituted carbamoyl group, an optionally substituted hydroxyl group and an optionally substituted aromatic cyclic group;

$R^2$ represents:

(1) a hydrogen atom; or
(2) a cyclic or linear $C_{1-10}$ alkyl group; or
(3) a $C_{1-10}$ alkyl group consisting of a cyclic alkyl group and a linear alkyl group;

$R^3$ represents:

(1) a $C_{1-8}$ alkyl group having an optionally substituted basic group and optionally having an additional substituent;
(2) an aralkyl group having an optionally substituted basic group and optionally having an additional substituent;

(3) a $C_{1-4}$ alkyl group having a non-aromatic cyclic hydrocarbon group of carbon atoms not greater than 7 having an optionally substituted basic group, and optionally having an additional substituent; or
(4) a $C_{1-4}$ alkyl group having a non-aromatic heterocyclic group of carbon atoms not greater than 7 having an optionally substituted basic group, and optionally having an additional substituent;

$R^4$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and
(6) an optionally substituted non-aromatic heterocyclic group having carbon atoms not greater than 7;

X represents a group represented by formula:

$$-NHCH(Q^1)YQ^2C(=Z^9)-$$

(wherein

$Q^1$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and
(6) an optionally substituted non-aromatic heterocyclic group having carbon atoms not greater than 7;

$Q^2$ represents:

(1) $CH_2$, which may optionally be substituted with a $C_{1-4}$ alkyl group optionally substituted with a substituent selected from the group consisting of a carbamoyl group and a hydroxyl group;
(2) NH, which may optionally be substituted with a $C_{1-4}$ alkyl group optionally substituted with a substituent selected from the group consisting of a carbamoyl group and a hydroxyl group; or
(3) O;

Y represents a group represented by formulae $-CONH-$, $-CSNH-$,$-CH_2NH-$, $-NHCO-$, $-CH_2O-$, $-CH_2S-$ or $-CH_2CH_2-$, which may be substituted with a $C_{1-6}$ alkyl group; and
$Z^9$ represents a hydrogen atom, O or S); and
P represents:

(1) a hydrogen atom;
(2) an optional amino acid residue continuously or discontinuously bound from the C terminus of the 1-48 amino acid sequence represented by SEQ ID NO: 1;
(3) a group represented by formula:

$$J^1\text{-}J^2\text{-}C(J^3)(Q^3)Y^1C(J^4)(Q^4)Y^2C(J^5)(Q^5)Y^3C(J^6)(Q^6)C(=Z^{10})\text{-}$$

(wherein

$J^1$ represents:

(a) a hydrogen atom; or
(b) (i) a $C_{1-15}$ acyl group, (ii) a $C_{1-15}$ alkyl group, (iii) a $C_{6-14}$ aryl group, (iv) a carbamoyl group, (v) a carboxyl group, (vi) a sulfino group, (vii) an amidino group, or (viii) a glyoxyloyl group, which may be substituted with a substituent comprising an optionally substituted cyclic group;

$J^2$ represents:

(1) NH optionally substituted with a $C_{1-6}$ alkyl group;
(2) $CH_2$ optionally substituted with a $C_{1-6}$ alkyl group
(3) O; or
(4) S;

each of $J^3$ through $J^6$ represents a hydrogen atom or a $C_{1-3}$ alkyl group;
each of $Q^3$ through $Q^6$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7;
(6) an optionally substituted non-aromatic heterocyclic group having carbon atoms not greater than 7;
(7) an optionally substituted amino group;
(8) an optionally substituted guanidino group;
(9) an optionally substituted hydroxyl group;
(10) an optionally substituted carboxyl group;
(11) an optionally substituted carbamoyl group; and
(12) an optionally substituted sulfhydryl group; or a hydrogen atom;

$J^3$ and $Q^3$, $J^4$ and $Q^4$, $J^5$ and $Q^5$, $J^6$ and $Q^6$ may be combined together, or $J^2$ and $Q^3$, $Y^1$ and $Q^4$, $Y^2$ and $Q^5$, or $Y^3$ and $Q^6$ may be combined together to form a ring;
each of $Y^1$ through $Y^3$ represents a group represented by $-CON(J^{13})-$,$-CSN(J^{13})-$, $-C(J^{14})N(J^{13})-$ or $-N(J^{13})CO-$ (each of $J^{13}$ and $J^{14}$ represents a hydrogen atom or a $C_{1-3}$ alkyl group); and
$Z^{10}$ represents a hydrogen atom, O or S);

(4) a group represented by formula:

$$J^1\text{-}J^2\text{-}C(J^7)(Q^7)Y^2C(J^8)(Q^8)Y^3C(J^9)(Q^9)C(=Z^{10})\text{-}$$

(wherein

each of $J^1$ and $J^2$ represents the same as those described above;
each of $J^7$ through $J^9$ represents the same meaning as that of $J^3$;

each of $Q^7$ through $Q^9$ represents the same meaning as that of $Q^3$;
each of $Y^2$ and $Y^3$ represents the same as those described above;
$Z^{10}$ represents the same as those described above; and
$J^7$ and $Q^7$, $J^8$ and $Q^8$, $J^9$ and $Q^9$ may be combined together, or $J^2$ and $Q^7$, $Y^2$ and $Q^8$, or $Y^3$ and $Q^9$ may be combined together to form a ring.);

(5) a group represented by formula:

$$J^1\text{-}J^2\text{-}C(J^{10})(Q^{10})Y^3C(J^{11})(Q^{11})C(=Z^{10})\text{-}$$

(wherein

each of $J^1$ and $J^2$ represents the same as those described above;
each of $J^{10}$ and $J^{11}$ represents the same meaning as that of $J^3$;
each of $Q^{10}$ and $Q^{11}$ represents the same meaning as that of $Q^3$;
$Y^3$ represents the same as those described above;
$Z^{10}$ represents the same as those described above; and
$J^{10}$ and $Q^{10}$, $J^{11}$ and $Q^{11}$ may be combined together, or $J^2$ and $Q^{10}$, $Y^3$ and $Q^{11}$ may be combined together to form a ring.);

(6) a group represented by formula:

$$J^1\text{-}J^2\text{-}C(J^{12})(Q^{12})C(=Z^{10})\text{-}$$

(wherein

each of $J^1$ and $J^2$ represents the same as those described above;
$J^{12}$ represents the same meaning as that of $J^3$;
$Q^{12}$ represents the same meaning as that of $Q^3$;
$Z^{10}$ represents the same as those described above; and
$J^{12}$ and $Q^{12}$ may be combined together, or $J^2$ and $Q^{12}$ may be combined together to form a ring.); or

(7) a group represented by formula $J^1$- ($J^1$ represents the same as those described above).];
a metastin derivative represented by formula (2):

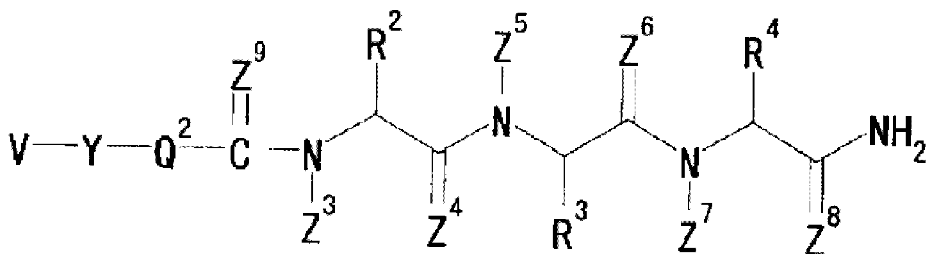

(2)

[wherein V represents a group represented by formula:

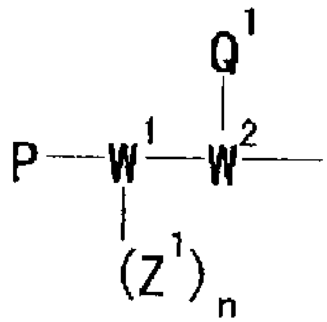

;

or

a group represented by formula:

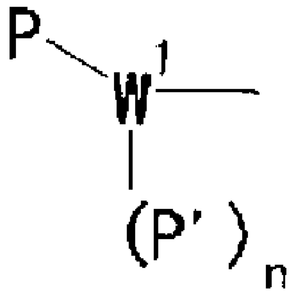

;

n represents 0 or 1;

$W^1$ represents N, CH or O (provided that when $W^1$ is N or CH, n represents 1, and when $W^1$ is O, n represents 0);

$W^2$ represents N or CH;

each of $Z^1$, $Z^3$, $Z^5$ and $Z^7$ represents a hydrogen atom or a $C_{1-3}$ alkyl group;

each of $Z^4$, $Z^6$ and $Z^8$ represents a hydrogen atom, O or S;

$R^2$ represents:

(1) a hydrogen atom; or
(2) a cyclic or linear $C_{1-10}$ alkyl group;
(3) a $C_{1-10}$ alkyl group consisting of a cyclic alkyl group and a linear alkyl group; or
(4) a $C_{1-8}$ alkyl group optionally substituted with a substituent selected from the group consisting of an optionally substituted carbamoyl group, an optionally substituted hydroxyl group and an optionally substituted aromatic cyclic group;

$R^3$ represents:

(1) a $C_{1-8}$ alkyl group having an optionally substituted basic group and optionally having an additional substituent;
(2) an aralkyl group having an optionally substituted basic group and optionally having an additional substituent;
(3) a $C_{1-4}$ alkyl group having a non-aromatic cyclic hydrocarbon group of carbon atoms not greater than 7 having an optionally substituted basic group, and optionally having an additional substituent; or
(4) a $C_{1-4}$ alkyl group having a non-aromatic heterocyclic group of carbon atoms not greater than 7 having an optionally substituted basic group, and optionally having an additional substituent;

$R^4$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and
(6) an optionally substituted non-aromatic heterocyclic group having carbon atoms not greater than 7;

$Q^1$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and
(6) an optionally substituted non-aromatic heterocyclic group having carbon atoms not greater than 7;

$Q^2$ represents:

(1) $CH_2$, which may optionally be substituted with a $C_{1-4}$ alkyl group optionally substituted with a substituent selected from the group consisting of a carbamoyl group and a hydroxyl group;
(2) NH, which may optionally be substituted with a $C_{1-4}$ alkyl group optionally substituted with a substituent selected from the group consisting of a carbamoyl group and a hydroxyl group; or
(3) O;

Y represents a group represented by formulae -CONH-, -CSNH-,$-CH_2NH-$, -NHCO-, $-CH_2O-$, $-CH_2S-$, -COO-, -CSO- or $-CH_2CH_2-$, which may be substituted with a $C_{1-6}$ alkyl group;
$Z^9$ represents a hydrogen atom, O or S; and
P and P' may be the same or different from each other, and P and P' or P and $Q^1$ may be combined together to form a ring, and P and P' represent:

(1) a hydrogen atom;
(2) an optional amino acid residue continuously or discontinuously bound from the C terminus of the 1-48 amino acid sequence represented by SEQ ID NO: 1;
(3) a group represented by formula:

$$J^1\text{-}J^2\text{-}C(J^3)(Q^3)Y^1C(J^4)(Q^4)Y^2C(J^5)(Q^5)Y^3C(J^6)(Q^6)C(=Z^{10})\text{-}$$

(wherein

$J^1$ represents:

(a) a hydrogen atom; or
(b) (i) a $C_{1-15}$ acyl group, (ii) a $C_{1-15}$ alkyl group, (iii) a $C_{6-14}$ aryl group, (iv) a

carbamoyl group, (v) a carboxyl group, (vi) a sulfino group, (vii) an amidino group, (viii) a glyoxyloyl group, or (ix) an amino group, which may be substituted with a substituent comprising an optionally substituted cyclic group;

$J^2$ represents:

(1) NH optionally substituted with a $C_{1-6}$ alkyl group;
(2) $CH_2$ optionally substituted with a $C_{1-6}$ alkyl group
(3) O; or
(4) S;

each of $J^3$ through $J^6$ represents a hydrogen atom or a $C_{1-3}$ alkyl group;
each of $Q^3$ through $Q^6$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7;
(6) an optionally substituted non-aromatic heterocyclic group having carbon atoms not greater than 7;
(7) an optionally substituted amino group;
(8) an optionally substituted guanidino group;
(9) an optionally substituted hydroxyl group;
(10) an optionally substituted carboxyl group;
(11) an optionally substituted carbamoyl group; and
(12) an optionally substituted sulfhydryl group; or a hydrogen atom;

$J^3$ and $Q^3$, $J^4$ and $Q^4$, $J^5$ and $Q^5$, $J^6$ and $Q^6$ may be combined together, or $J^2$ and $Q^3$, $Y^1$ and $Q^4$, $Y^2$ and $Q^5$, or $Y^3$ and $Q^6$ may be combined together to form a ring;
each of $Y^1$ through $Y^3$ represents a group represented by $-CON(J^{13})-$,$-CSN(J^{13})-$, $-C(J^{14})N(J^{13})-$ or $-N(J^{13})CO-$ (each of $J^{13}$ and $J^{14}$ represents a hydrogen atom or a $C_{1-3}$ alkyl group); and
$Z^{10}$ represents a hydrogen atom, O or S);

(4) a group represented by formula:

$$J^1-J^2-C(J^7)(Q^7)Y^2C(J^8)(Q^8)Y^3C(J^9)(Q^9)C(=Z^{10})-$$

(wherein

each of $J^1$ and $J^2$ represents the same as those described above;
each of $J^7$ through $J^9$ represents the same meaning as that of $J^3$;
each of $Q^7$ through $Q^9$ represents the same meaning as that of $Q^3$;
each of $Y^2$ and $Y^3$ represents the same as those described above;
$Z^{10}$ represents the same as those described above; and
$J^7$ and $Q^7$, $J^8$ and $Q^8$, $J^9$ and $Q^9$ may be combined together, or $J^2$ and $Q^7$, $Y^2$ and $Q^8$, or $Y^3$ and $Q^9$ may be combined together to form a ring.);

(5) a group represented by formula:

$$J^1-J^2-C(J^{10})(Q^{10})Y^3C(J^{11})(Q^{11})C(=Z^{10})-$$

(wherein

each of $J^1$ and $J^2$ represents the same as those described above;
each of $J^{10}$ and $J^{11}$ represents the same meaning as that of $J^3$;
each of $Q^{10}$ and $Q^{11}$ represents the same meaning as that of $Q^3$;
$Y^3$ represents the same as those described above;
$Z^{10}$ represents the same as those described above; and
$J^{10}$ and $Q^{10}$, or $J^{11}$ and $Q^{11}$ may be combined together, or $J^2$ and $Q^{10}$, or $Y^3$ and $Q^{11}$ may be combined together to form a ring.);

(6) a group represented by formula:

$$J^1\text{-}J^2\text{-}C(J^{12})(Q^{12})C(=Z^{10})\text{-}$$

(wherein

each of $J^1$ and $J^2$ represents the same as those described above;
$J^{12}$ represents the same meaning as that of $J^3$;
$Q^{12}$ represents the same meaning as that of $Q^3$;
$Z^{10}$ represents the same as those described above; and
$J^{12}$ and $Q^{12}$ may be combined together, or $J^2$ and $Q^{12}$ may be combined together to form a ring.); or

(7) a group represented by $J^1$- ($J^1$ represents the same as those described above).];

a metastin derivative represented by formula (3):

$$\text{XX0-XX2-XX3-XX4 XX5-XX6-AzaGly-XX8-XX9-XX10-NH}_2 \quad (3)$$

[wherein

XX0 represents formyl, $C_{1-20}$ alkanoyl, cyclopropanecarbonyl, 6-(acetyl-D-arginylamino)caproyl, 6-((R)-2,3-diaminopropionylamino)caproyl, 6-(D-norleucylamino)caproyl, 4-(D-arginylamino)butyryl, 3-(4-hydroxyphenyl)propionyl, glycyl, tyrosyl, acetylglycyl, acetyltyrosyl, D-tyrosyl, acetyl-D-tyrosyl, pyroglutamyl, 3-(pyridin-3-yl)propionyl, adipoyl, glycoloyl, 6-aminocaproyl, 6-acetylaminocaproyl, 4-[bis-(2-pyridylmethyl)aminomethyl]benzoyl or 4-ureidobenzoyl;
XX2 represents Tyr, D-Tyr, D-Ala, D-Leu, D-Phe, D-Lys, D-Trp or a chemical bond;
XX3 represents:

i) an amino acid selected from Ala, Arg, Asn, Asp, Cys, Gin, Glu, Gly, His, Ile, Leu, Lys, Met, Phe, Ser, Thr, Trp, Tyr and Val, wherein the α-amino group may optionally be methylated;
ii) a cyclic amino acid selected from Pro, Aze(2), Aze(3), Pic(2), Pic(3), Hyp, Thz, Abz(2), Abz(3), Pzc(2), Pro($4NH_2$), Hyp(Bzl), cisHyp, Pro(4F) and lzc;
iii) an amino acid selected from D-Dap, D-Pya(4), DL-Ala(Pip), Orn, Aib and Tyr($PO_3H_2$); or
iv) a chemical bond;

XX4 represents Asn, 2-amino-3-ureidopropionic acid, $N^{\beta}$-formyl-β-diaminopropionic acid, $N^{\beta}$-acetyl-β-diaminopropionic acid, $N^{\omega}$-pentylasparagine, $N^{\omega}$-cyclopropylasparagine, $N^{\omega}$-benzylasparagine, 2,4-diaminobutanoic acid, 2,3-diaminopropionic acid, His, Gin, Gly, Arg, Cit, Nva, D-Asn or a chemical bond;
XX5 represents Ser, Thr, Val, NMeSer, Gly, Ala, Hyp, D-Ala, D-Thr, D-Pro or a chemical bond;
XX6 represents Phe, Tyr, Trp, Tyr(Me), Thi, Nal(2), Cha, Pya(4), threo-Ser(3Phenyl), erythro-Ser(3Phenyl) or optionally substituted phenylalanine;

AzaGly represents azaglycine;
XX8 represents Leu, Nva, Val or Ala(cPr);
XX9 represents optionally substituted arginine, optionally substituted lysine or optionally substituted ornithine; and
XX10 represents 2-naphthylalanine, 2-thienylalanine, tyrosine, optionally substituted phenylalanine or optionally substituted tryptophan.];

a metastin derivative represented by formula (4):

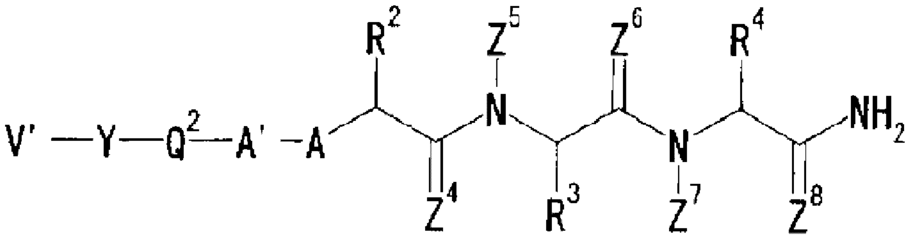

(4)

[wherein V' represents a group represented by formulae:

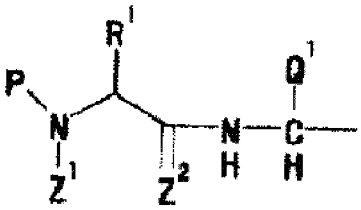

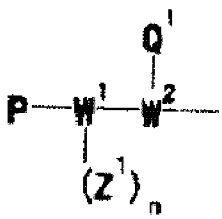

or

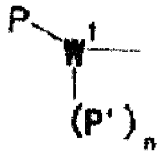

;

n represents 0 or 1;
$W^1$ represents N, CH or O (provided that when $W^1$ is N or CH, n represents 1, and when $W^1$ is O, n represents 0);
$W^2$ represents N or CH;
each of $Z^1$, $Z^5$ and $Z^7$ represents a hydrogen atom or a $C_{1-3}$ alkyl group;
each of $Z^2$, $Z^4$, $Z^6$ and $Z^8$ represents a hydrogen atom, O or S;
$R^1$ represents:

(1) a hydrogen atom;
(2) a $C_{1-8}$ alkyl group optionally substituted with a substituent selected from the group consisting of an optionally substituted carbamoyl group, an optionally substituted hydroxyl group and an optionally substituted aromatic cyclic group;
(3) a cyclic or linear $C_{1-10}$ alkyl group;
(4) a $C_{1-10}$ alkyl group consisting of a cyclic alkyl group and a linear alkyl group; or
(5) an optionally substituted aromatic cyclic group;

$R^2$ represents:

(1) a hydrogen atom; or
(2) a cyclic or linear $C_{1-10}$ alkyl group;
(3) a $C_{1-10}$ alkyl group consisting of a cyclic alkyl group and a linear alkyl group; or
(4) a $C_{1-8}$ alkyl group optionally substituted with a substituent selected from the group consisting of an optionally substituted carbamoyl group, an optionally substituted hydroxyl group and an optionally substituted aromatic cyclic group;

$R^3$ represents:

(1) a $C_{1-8}$ alkyl group having an optionally substituted basic group and optionally having an additional substituent;

(2) an aralkyl group having an optionally substituted basic group and optionally having an additional substituent;
(3) a $C_{1-4}$ alkyl group having a non-aromatic cyclic hydrocarbon group of carbon atoms not greater than 7 having an optionally substituted basic group, and optionally having an additional substituent; or
(4) a $C_{1-4}$ alkyl group having a non-aromatic heterocyclic group of carbon atoms not greater than 7 having an optionally substituted basic group, and optionally having an additional substituent;

$R^4$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and
(6) an optionally substituted non-aromatic heterocyclic group having carbon atoms not greater than 7;

$Q^1$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and
(6) an optionally substituted non-aromatic heterocyclic group having carbon atoms not greater than 7;

A represents:

(1) a nitrogen atom that is substituted with hydrogen atom or a $C_{1-3}$ alkyl group;
(2) a carbon atom that is substituted with a hydrogen atom or a $C_{1-3}$ alkyl group;
(3) O; or
(4) S;

A' represents:

(1) a carbon atom that may be substituted with a hydrogen atom, O, S, a halogen atom, an optionally halogenated $C_{1-3}$ alkyl group, a carbamoyl group or a hydroxyl group;
(2) a nitrogen atom that may be substituted with a hydrogen atom or an optionally halogenated $C_{1-3}$ alkyl group;
(3) O; or
(4) S;

$Q^2$ represents:

(1) $CH_2$, CO, CS or $CH=CH_2$, which may optionally be substituted with one or two $C_{0-4}$ alkyl groups optionally substituted with a substituent selected from the group consisting of a carbamoyl group, a hydroxyl group, a $C_{1-3}$ alkoxy group, a halogen atom and an amino group;
(2) NH, which may optionally be substituted with a $C_{1-4}$ alkyl group optionally substituted with a substituent selected from the group consisting of a carbamoyl group and a hydroxyl group; or
(3) O;

Y represents:

(1) a group represented by formulae -CONH-, -CSNH-, $-CH_2NH-$,-NHCO-, $-CH_2O-$, $-COCH_2-$, $-CH_2S-$, $-CSCH_2-$, $-CH_2SO-$, $-CH_2SO_2-$, -COO-,-CSO-, $-CH_2CH_2-$ or -CH=CH-, which may optionally be substituted with a substituent selected from the group consisting of a $C_{1-6}$ alkyl group, a hydroxyl group and a halogen atom;
(2) an optionally substituted $C_{6-7}$ aromatic hydrocarbon group;
(3) an optionally substituted 4- to 7-membered aromatic heterocyclic group consisting of 1 to 5 carbon atoms and hetero atoms selected from the group consisting of nitrogen, oxygen and sulfur atoms;
(4) an optionally substituted non-aromatic cyclic hydrocarbon group having not greater than 5 carbon atoms; or
(5) an optionally substituted non-aromatic heterocyclic group having not greater than 5 carbon atoms;

provided that when Y is (2), (3), (4) or (5), $Q^2$ may be a chemical bond; and
P and P' may be the same or different from each other, and P and P' or P and $Q^1$ may be combined together to form a ring, and P and P' represent:

(1) a hydrogen atom;
(2) an optional amino acid residue continuously or discontinuously bound from the C terminus of the 1-48 amino acid sequence represented by SEQ ID NO: 1;
(3) a group represented by formula:

$$J^1\text{-}J^2\text{-}C(J^3)(Q^3)Y^1C(J^4)(Q^4)Y^2C(J^5)(Q^5)Y^3C(J^6)(Q^6)C(=Z^{10})\text{-}$$

(wherein

$J^1$ represents:

(a) a hydrogen atom; or
(b) (i) a $C_{1-20}$ acyl group, (ii) a $C_{1-20}$ alkyl group, (iii) a $C_{6-14}$ aryl group, (iv) a carbamoyl group, (v) a carboxyl group, (vi) a sulfino group, (vii) an amidino group, (viii) a glyoxyloyl group, or (ix) an amino group, which may be substituted with a substituent comprising an optionally substituted cyclic group;

$J^2$ represents:

(1) NH optionally substituted with a $C_{1-6}$ alkyl group;
(2) $CH_2$ optionally substituted with a $C_{1-6}$ alkyl group
(3) O; or
(4) S;

each of $J^3$ through $J^6$ represents a hydrogen atom or a $C_{1-3}$ alkyl group;
each of $Q^3$ through $Q^6$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7;
(6) an optionally substituted non-aromatic heterocyclic group having carbon atoms not greater than 7;
(7) an optionally substituted amino group;
(8) an optionally substituted guanidino group;
(9) an optionally substituted hydroxyl group;
(10) an optionally substituted carboxyl group;
(11) an optionally substituted carbamoyl group; and
(12) an optionally substituted sulfhydryl group; or a hydrogen atom;

$J^3$ and $Q^3$, $J^4$ and $Q^4$, $J^5$ and $Q^5$, $J^6$ and $Q^6$ may be combined together, or $Z^1$ and $R^1$, $J^2$ and $Q^3$, $Y^1$ and $Q^4$, $Y^2$ and $Q^5$, or $Y^3$ and $Q^6$ may be combined together to form a ring;
each of $Y^1$ through $Y^3$ represents a group represented by $-CON(J^{13})-$,$-CSN(J^{13})-$, $-C(J^{14})N(J^{13})-$ or $-N(J^{13})CO-$ (each of $J^{13}$ and $J^{14}$ represents a hydrogen atom or a $C_{1-3}$ alkyl group); and
$Z^{10}$ represents a hydrogen atom, O or S);

(4) a group represented by formula:

$$J^1-J^2-C(J^7)(Q^7)Y^2C(J^8)(Q^8)Y^3C(J^9)(Q^9)C(=Z^{10})-$$

(wherein

each of $J^1$ and $J^2$ represents the same as those described above;
each of $J^7$ through $J^9$ represents the same meaning as that of $J^3$;
each of $Q^7$ through $Q^9$ represents the same meaning as that of $Q^3$;
each of $Y^2$ and $Y^3$ represents the same as those described above;
$Z^{10}$ represents the same as those described above; and
$J^7$ and $Q^7$, $J^8$ and $Q^8$, $J^9$ and $Q^9$ may be combined together, or $J^2$ and $Q^7$, $Y^2$ and $Q^8$, or $Y^3$ and $Q^9$ may be combined together to form a ring.);

(5) a group represented by formula:

$$J^1-J^2-C(J^{10})(Q^{10})Y^3C(J^{11})(Q^{11})C(=Z^{10})-$$

(wherein

each of $J^1$ and $J^2$ represents the same as those described above;
each of $J^{10}$ and $J^{11}$ represents the same meaning as that of $J^3$;
each of $Q^{10}$ and $Q^{11}$ represents the same meaning as that of $Q^3$;
$Y^3$ represents the same as those described above;
$Z^{10}$ represents the same as those described above; and
$J^{10}$ and $Q^{10}$, $J^{11}$ and $Q^{11}$ may be combined together, or $J^2$ and $Q^{10}$, $Y^3$ and $Q^{11}$ may be combined together to form a ring.);

(6) a group represented by formula:

$$J^1-J^2-C(J^{12})(Q^{12})C(=Z^{10})-$$

(wherein

each of $J^1$ and $J^2$ represents the same as those described above;
$J^{12}$ represents the same meaning as that of $J^3$;
$Q^{12}$ represents the same meaning as that of $Q^3$;
$Z^{10}$ represents the same as those described above; and
$J^{12}$ and $Q^{12}$ may be combined together, or $J^2$ and $Q^{12}$ may be combined together to form a ring.); or

(7) a group represented by formula:
$J^1$- ($J^1$ represents the same as those described above);
wherein the bonds between Y-$Q^2$, $Q^2$-A' and A'-A represent independently from each other, a single bond or a double bond.];
a metastin derivative represented by formula (5):

XXO-XX2 XX3-XX4-XX5-XX6-AzaGly-XX8-XX9-XX10-$NH_2$ (5)

[wherein

XX0 represents hydrogen, acetyl, mesyl, 4-fluorobenzoyl, decanoyl, benzylureido, ureido, amidino, dimethylaminoethylureido or isobutylureido;
XX2 represents Tyr, D-Tyr, D-Ala, D-Leu, D-Phe, D-Lys, D-Trp or a chemical bond;
XX3 represents:

i) an amino acid selected from Ala, Arg, Asn, Asp, Cys, Gin, Glu, Gly, His, Ile, Leu, Lys, Met, Phe, Ser, Thr, Trp, Tyr and Val, wherein the $\alpha$-amino group may optionally be methylated;
ii) a cyclic amino acid selected from Pro, Aze(2), Aze(3), Pic(2), Pic(3), Hyp, Thz, Abz(2), Abz(3), Pzc(2), Pro($4NH_2$), Hyp(Bzl), cisHyp, Pro(4F) and lzc;
iii) an amino acid selected from D-Pya(4), DL-Ala(Pip), Orn, Aib and Tyr($PO_3H_2$); or
iv) a chemical bond;

XX4 represents Asn, 2-amino-3-ureidopropionic acid, $N^\beta$-formyl-$\beta$-diaminopropionic acid, $N^\beta$-acetyl-$\beta$-diaminopropionic acid, $N^\omega$-pentylasparagine, $N^\omega$-cyclopropylasparagine, $N^\omega$-benzylasparagine, 2,4-diaminobutanoic acid, 2,3-diaminopropionic acid, His, Gin, Gly, Arg, Cit, Nva, D-Asn or a chemical bond;
XX5 represents Ser, Thr, Val, NMeSer, Gly, Ala, Hyp, D-Ala, D-Thr, D-Pro or a chemical bond;
XX6 represents Phe, Tyr, Trp, Tyr(Me), Thi, Nal(2), Cha, Pya(4), threo-Ser(3Phenyl), erythro-Ser(3Phenyl) or optionally substituted phenylalanine;
AzaGly represents azaglycine;
XX8 represents Leu, Nva, Val or Ala(cPr);
XX9 represents optionally substituted arginine, optionally substituted lysine or optionally substituted ornithine; and
XX10 represents 2-naphthylalanine, 2-thienylalanine, tyrosine, optionally substituted phenylalanine or optionally substituted tryptophan.]; and

a metastin derivative represented by formula (6):

XX0-XX2-XX3-XX4-XX5-T-XX9-XX10 $NH_2$ (6)

[wherein

XX0 represents formyl, $C_{1-20}$ alkanoyl, mesyl, optionally substituted ureido, amidino, cyclopropanecarbonyl, 6-(acetyl-D-arginylamino)caproyl, 6-((R)-2,3-diaminopropionylamino)caproyl, 6-(D-norleucylamino)caproyl, 4-(D-arginylamino)butyryl, 3-(4-hydroxyphenyl)propionyl, glycyl, tyrosyl, acetylglycyl, acetyltyrosyl, D-tyrosyl, acetyl-D-tyrosyl, pyroglutamyl, 3-(pyridin-3-yl)propionyl, adipoyl, glycoloyl or 6-aminocaproyl;
XX2 represents Tyr, D-Tyr, D-Ala, D-Leu, D-Phe, D-Lys, D-Trp or a chemical bond;
XX3 represents D-Asp, D-Dap, D-Ser, D-Gln, D-His, D-NMeAla, D-NMePhe, Aze(2), Pic(2), Pic(3), Hyp, Thz, NMeAla, Gly, Aib, Abz(2), Abz(3), Sar, Leu, Lys, Glu, β-alanine, Pzc(2), Orn, His(3Me), Tyr($PO_3H_2$), Pro($4NH_2$), Hyp(Bzl), Trp, Pro, 4-pyridylalanine, Tic, D-Trp, D-Ala, D-Leu, D-Phe, D-Lys, D-Glu, D-2-pyridylalanine, D-3-pyridylalanine, D-4-pyridylalanine, Aad, Pro(4F) or a chemical bond;
XX4 represents Asn, 2-amino-3-ureidopropionic acid, $N^{\beta}$-formyldiaminopropionic acid, $N^{\beta}$-acetyldiaminopropionic acid, $N^{\omega}$-pentylasparagine, $N^{\omega}$-cyclopropylasparagine, $N^{\omega}$-benzylasparagine, 2,4-diaminobutanoic acid, His, Gin, Cit or D-Asn;
XX5 represents Ser, Thr, Val, NMeSer, Gly, Ala, Hyp, D-Ala, Dap or D-Thr;
T represents formula II:

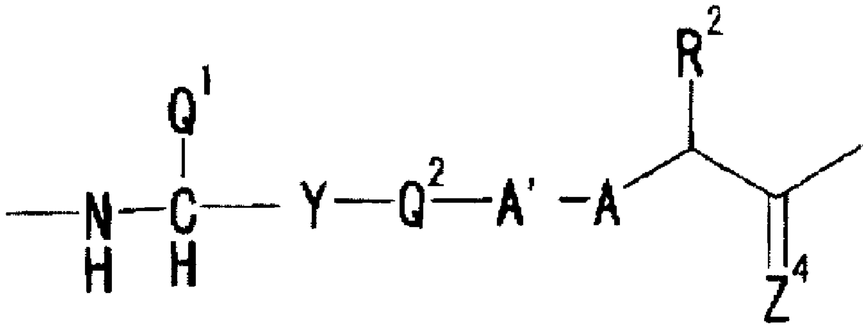

(II)

$Z^4$ represents a hydrogen atom, O or S;
$R^2$ represents:

(1) a hydrogen atom; or
(2) a cyclic or linear $C_{1-10}$ alkyl group;
(3) a $C_{1-10}$ alkyl group consisting of a cyclic alkyl group and a linear alkyl group; or
(4) a $C_{1-8}$ alkyl group optionally substituted with a substituent selected from the group consisting of an optionally substituted carbamoyl group, an optionally substituted hydroxy group and an optionally substituted aromatic cyclic group;

$Q^1$ represents a $C_{1-4}$ alkyl group, which may optionally be substituted with a substituent selected from the group consisting of:

(1) an optionally substituted $C_{6-12}$ aromatic hydrocarbon group;
(2) an optionally substituted 5- to 14-membered aromatic heterocyclic group consisting of 1 to 7 carbon atoms and hetero atoms selected from the group consisting of nitrogen, oxygen and sulfur atoms;
(3) an optionally substituted $C_{8-14}$ aromatic fused-ring group;
(4) an optionally substituted 5- to 14-membered aromatic fused heterocyclic group consisting of 3 to 11 carbon atoms and hetero atoms selected from the group consisting of nitrogen, oxygen and sulfur atoms;
(5) an optionally substituted non-aromatic cyclic hydrocarbon group having carbon atoms not greater than 7; and

(6) an optionally substituted non-aromatic heterocyclic group having carbon atoms not greater than 7;

A represents:

(1) a nitrogen atom that is substituted with hydrogen atom or a $C_{1-3}$ alkyl group;
(2) a carbon atom that is substituted with a hydrogen atom or a $C_{1-3}$ alkyl group;
(3) O; or
(4) S;

A' represents:

(1) a carbon atom that may be substituted with a hydrogen atom, O, S, a halogen atom, an optionally halogenated $C_{1-3}$ alkyl group, a carbamoyl group or a hydroxy group;
(2) a nitrogen atom that may be substituted with a hydrogen atom or an optionally halogenated $C_{1-3}$ alkyl group,
(3) O; or
(4) S;

$Q^2$ represents:

(1) $CH_2$, CO, CS or $C=CH_2$, which may optionally be substituted with a $C_{0-4}$ alkyl group optionally substituted with a substituent selected from the group consisting of a carbamoyl group, a hydroxyl group, a $C_{1-3}$ alkoxy group, a halogen atom and an amino group;
(2) NH, which may optionally be substituted with a $C_{1-4}$ alkyl group optionally substituted with a substituent selected from the group consisting of a carbamoyl group and a hydroxyl group; or
(3) O;

Y represents:

(1) a group represented by formulae -CONH-, -CSNH-, $-CH_2NH-$,-NH-CO-, $-CH_2O-$, $-COCH_2-$, $-CH_2S-$, $-CSCH_2-$, $-CH_2SO-$, $-CH_2SO_2-$, -COO-,-CSO-, $-CH_2CH_2-$ or -CH=CH-, which may optionally be substituted with a substituent selected from the group consisting of a $C_{1-6}$ alkyl group, a hydroxyl group and a halogen atom;
(2) an optionally substituted $C_{6-7}$ aromatic hydrocarbon group;
(3) an optionally substituted 5- to 7-membered aromatic heterocyclic group consisting of 1 to 5 carbon atoms and hetero atoms selected from the group consisting of nitrogen, oxygen and sulfur atoms;
(4) an optionally substituted non-aromatic cyclic hydrocarbon group having not greater than 5 carbon atoms; or
(5) an optionally substituted non-aromatic heterocyclic group having not greater than 5 carbon atoms;

provided that when Y is (2), (3), (4) or (5), $Q^2$ may be a chemical bond; and the bonds between Y-$Q^2$, $Q^2$-A' and A'-A represent independently from each other, a single bond or a double bond;

XX9 represents Arg, Orn, Arg(Me) or Arg(asym$Me_2$); and
XX10 represents Phe, Trp, 2-naphthylalanine, 2-thienylalanine, tyrosine or 4-fluorophenylalanine].

[4] The metastin derivative modified with a polyethylene glycol or a salt thereof in accordance with any of

[1] to [3], wherein the molecular weight of the polyethylene glycol is 1 kDa to 1000 kDa.

[5] The metastin derivative modified with a polyethylene glycol or a salt thereof in accordance with any of [1] to [4], wherein -La-Lb-[Lc]j- is -CO-$CH_2CH_2$-CO-NH-$(CH_2)_5$-CO-, -$CH_2CH_2CH_2$-NH-$(CH_2)_5$-CO-, or -$CH_2CH_2CH_2CH_2CH_2$CO-NH-$(CH_2)_5$-CO-.

[6] PEGSK(CS)-CO-Acp-Tyr-Asn-Trp-Asn-Ser-Phe-Gly-Leu-Arg-Phe-$NH_2$,
PEG2K(CS)-CO-Acp-Tyr-Asn-Trp-Asn-Ser-Phe-Gly-Leu-Arg-Phe-$NH_2$,
PEG20K(CS)-CO-Acp-Tyr-Asn-Trp-Asn-Ser-Phe-Gly-Leu-Arg-Phe-$NH_2$,
PEG40K(AL)-$CH_2$-Acp-Tyr-Asn-Trp-Asn-Ser-Phe-Gly-Leu-Arg-Phe-$NH_2$,
PEG20K(HS)-CO-Acp-Tyr-Asn-Trp-Asn-Ser-Phe-Gly-Leu-Arg-Phe-$NH_2$,
PEG40K(HS)-CO-Acp-Tyr-Asn-Trp-Asn-Ser-Phe-Gly-Leu-Arg-Phe-$NH_2$,
PEG30K(AL)-$CH_2$-Acp-Tyr-Asn-Trp-Asn-Ser-Phe-Gly-Leu-Arg-Phe-$NH_2$,
PEG20K(CS)-CO-Acp-D-Tyr-D-Trp-Asn-Thr-Phe-AzaGly-Leu-Arg(Me)-Trp-$NH_2$,
PEG20K(CS)-CO-Acp-D-Tyr-Hyp-Asn-Thr-Phe-AzaGly-Leu-Arg(Me)-Trp-$NH_2$,
PEG20K(CS)-CO-Acp-D-Tyr-Glu-Asn-Thr-Phe(3F)-AzaGly-Leu-Arg(Me)-Trp-$NH_2$, or
PEG20K(CS)-CO-Acp-D-Tyr-Hyp-Asn-Thr-Phe(4F)-AzaGly-Leu-Arg(Me)-Trp-$NH_2$
(wherein the numbers and K mean kDa, which is the molecular weight of PEG, (CS) represents -$CO(CH_2)_2$-, (AL) represents -$(CH_2)_2$-, and (HS) represents -$(CH_2)_5$-.).

[7] A pharmaceutical comprising the metastin derivative modified with a polyethylene glycol or a salt thereof in accordance with [1] or a prodrug thereof.

[8] The pharmaceutical in accordance with [7], which is a cancer metastasis-inhibiting agent or a cancer proliferation-inhibiting agent.

[9] The pharmaceutical in accordance with [7], which is a pharmaceutical for preventing or treating cancer.

[10] The pharmaceutical in accordance with [7], which is a pharmaceutical for controlling placental function.

[11] The pharmaceutical in accordance with [7], which is a pharmaceutical for preventing or treating choriocarcinoma, hydatid mole, invasive mole, miscarriage, fetal hypoplasia, abnormal glucose metabolism, abnormal lipid metabolism or induction of delivery.

[12] The pharmaceutical in accordance with [7], which is a pharmaceutical for improving gonadal function.

[13] The pharmaceutical in accordance with [7], which is a pharmaceutical for preventing or treating hormone-dependent cancer, infertility, endometriosis, precocious puberty or hysteromyoma.

[14] The pharmaceutical in accordance with [7], which is a pharmaceutical for inducing or stimulating ovulation.

[15] The pharmaceutical in accordance with [7], which is a pharmaceutical for promoting gonadotropic hormone secretion or sex hormone secretion.

[16] The pharmaceutical in accordance with [7], which is a pharmaceutical for preventing or treating Alzheimer's disease, mild cognitive impairment or autism.

[17] The pharmaceutical in accordance with [13], which is a down-regulating agent for gonadotropic hormone or sex hormone.

[18] The pharmaceutical in accordance with [13], which is a down-regulating agent for human OT7T175 (metastin receptor) protein consisting of the amino acid sequence represented by SEQ ID NO: 9.

[19] The pharmaceutical in accordance with [17] or [18], which is a pharmaceutical for preventing or treating hormone-dependent cancer.

[20] A method for preventing or treating cancer, which comprises administering to a mammal, an effective dose of the metastin derivative modified with a polyethylene glycol or a salt thereof in accordance with [1].

[21] Use of the metastin derivative modified with a polyethylene glycol or a salt thereof in accordance with [1] to manufacture a pharmaceutical for preventing or treating cancer.

## Claims

1. A metastin derivative which is PEG(CS)-CO-Acp-D-Tyr-Hyp-Asn-Thr-Phe-AzaGly-Leu-Arg(Me)-Trp-$NH_2$ or a salt thereof, wherein the PEG has an average molecular weight of about 1000 daltons to about 40,000 daltons.

2. The metastin derivative of claim 1, wherein the PEG has an average molecular weight of about 2000 daltons to about 40,000 daltons.

**3.** A pharmaceutical comprising the metastin derivative according to claim 1 or 2.

**4.** The pharmaceutical according to claim 3, for use in the inhibition of cancer metastasis or cancer proliferation.

**5.** The pharmaceutical according to claim 3, for use in the prevention or treatment of cancer.

**6.** The pharmaceutical according to claim 3, for use in the control of placental function.

**7.** The pharmaceutical according to claim 3, for use in the prevention or treatment of choriocarcinoma, hydatid mole, invasive mole, miscarriage, fetal hypoplasia, abnormal glucose metabolism, abnormal lipid metabolism or induction of delivery.

**8.** The pharmaceutical according to claim 3, for use in the improvement of gonadal function.

**9.** The pharmaceutical according to claim 3, for use in the prevention or treatment of hormone-dependent cancer, infertility, endometriosis, precocious puberty or hysteromyoma.

**10.** The pharmaceutical according to claim 3, for use in the induction or stimulation of ovulation.

**11.** The pharmaceutical according to claim 3, for use in the promotion of gonadotropic hormone secretion or sex hormone secretion.

**12.** The pharmaceutical according to claim 3, for use in the prevention or treatment of Alzheimer's disease, mild cognitive impairment or autism.

**13.** The pharmaceutical according to claim 9, for use in the prevention or treatment of hormone-dependent cancer.

[Fig. 1]

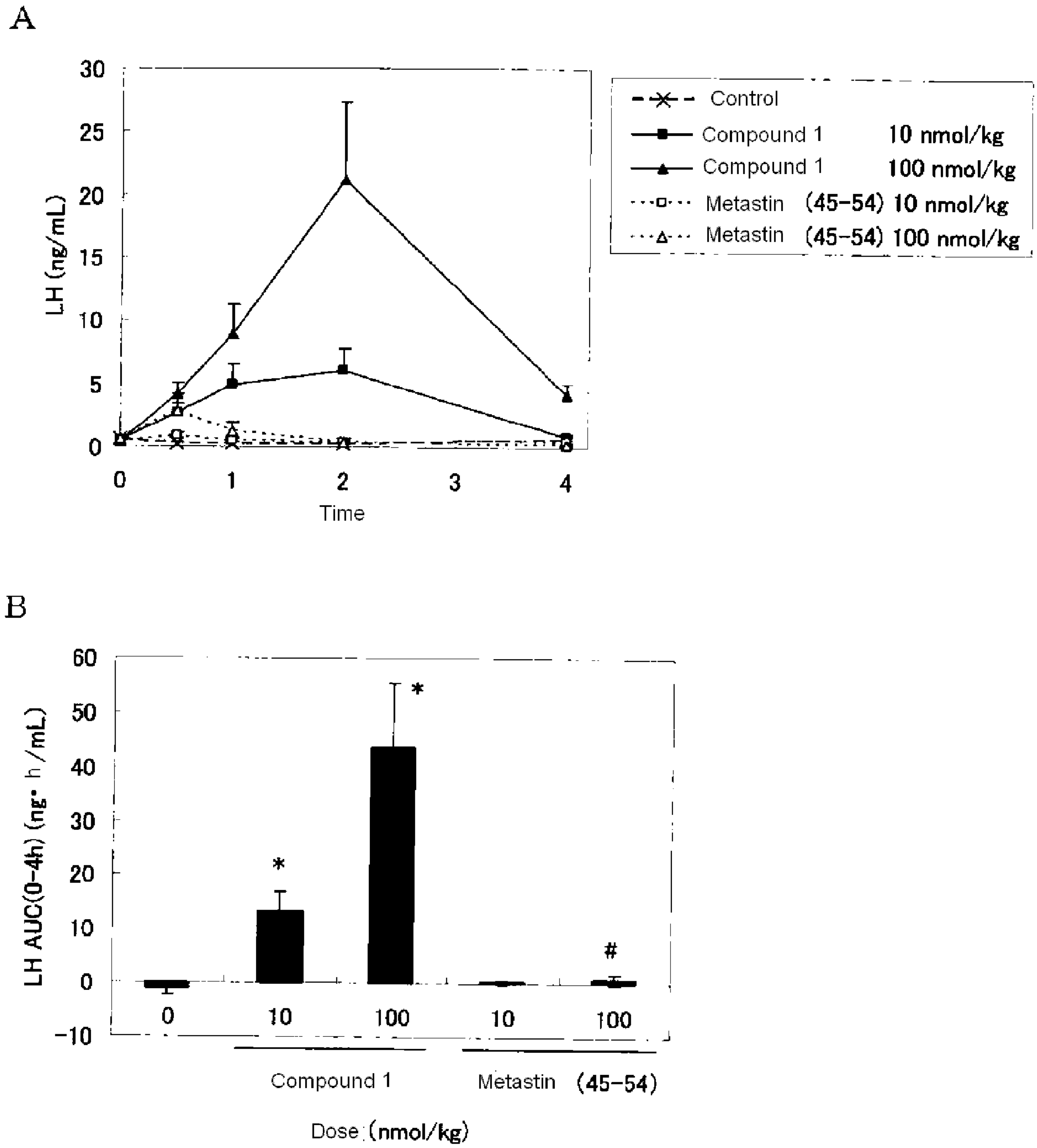
A
30
25
20
15
10
5
0
LH (ng/mL)
0
1
2
3
4
Time
Control
Compound 1 10 nmol/kg
Compound 1 100 nmol/kg
Metastin (45-54) 10 nmol/kg
Metastin (45-54) 100 nmol/kg
B
60
50
40
30
20
10
0
-10
LH AUC(0-4h) (ng・h/mL)
*
*
#
0
10
100
10
100
Compound 1
Metastin (45-54)
Dose (nmol/kg)

[Fig. 2]

A

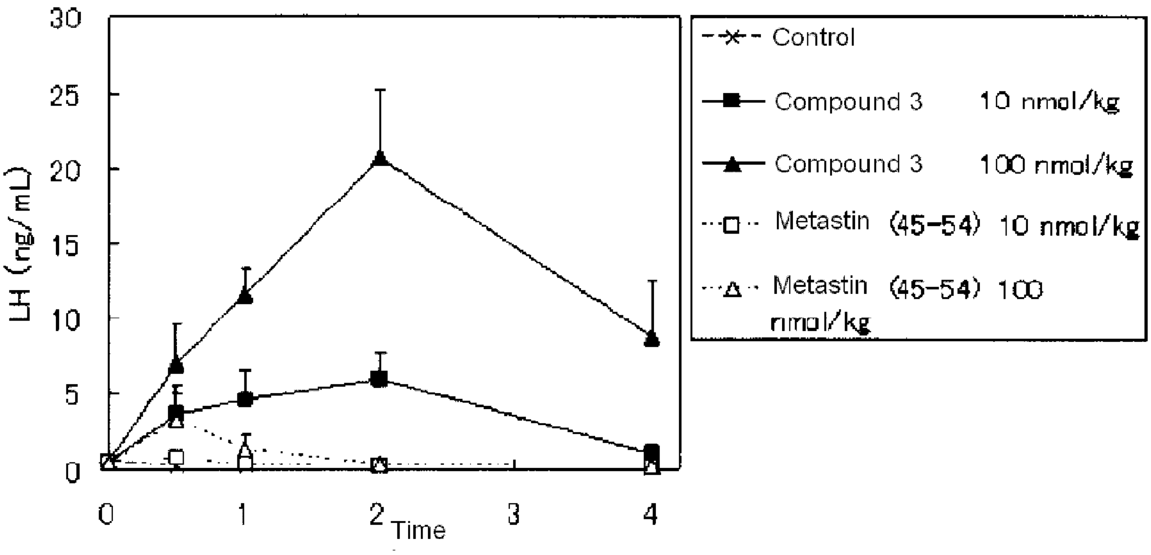

LH (ng/mL)
30
25
20
15
10
5
0
0
1
2
3
4
Time
Control
Compound 3 10 nmol/kg
Compound 3 100 nmol/kg
Metastin (45-54) 10 nmol/kg
Metastin (45-54) 100 nmol/kg


B

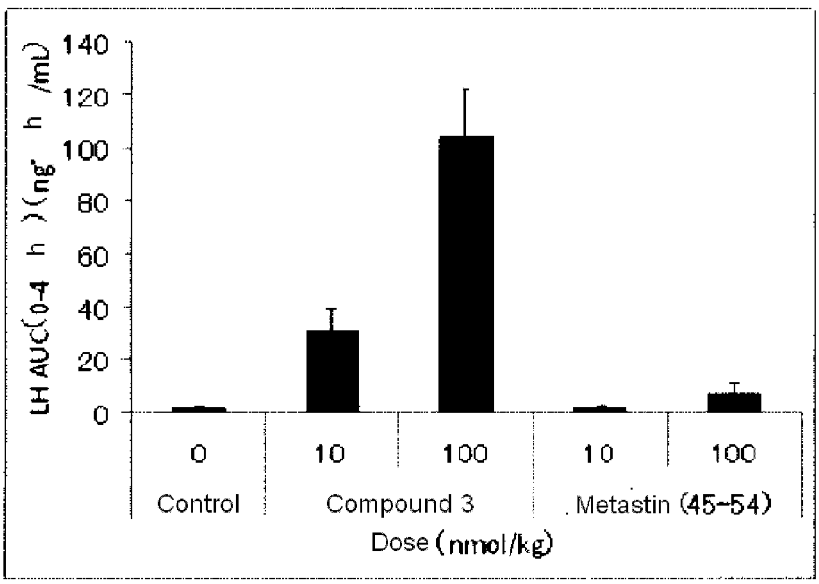

LH AUC(0-4 h)(ng·h/mL)
140
120
100
80
60
40
20
0
0
10
100
10
100
Control
Compound 3
Metastin (45-54)
Dose (nmol/kg)

[Fig. 3]

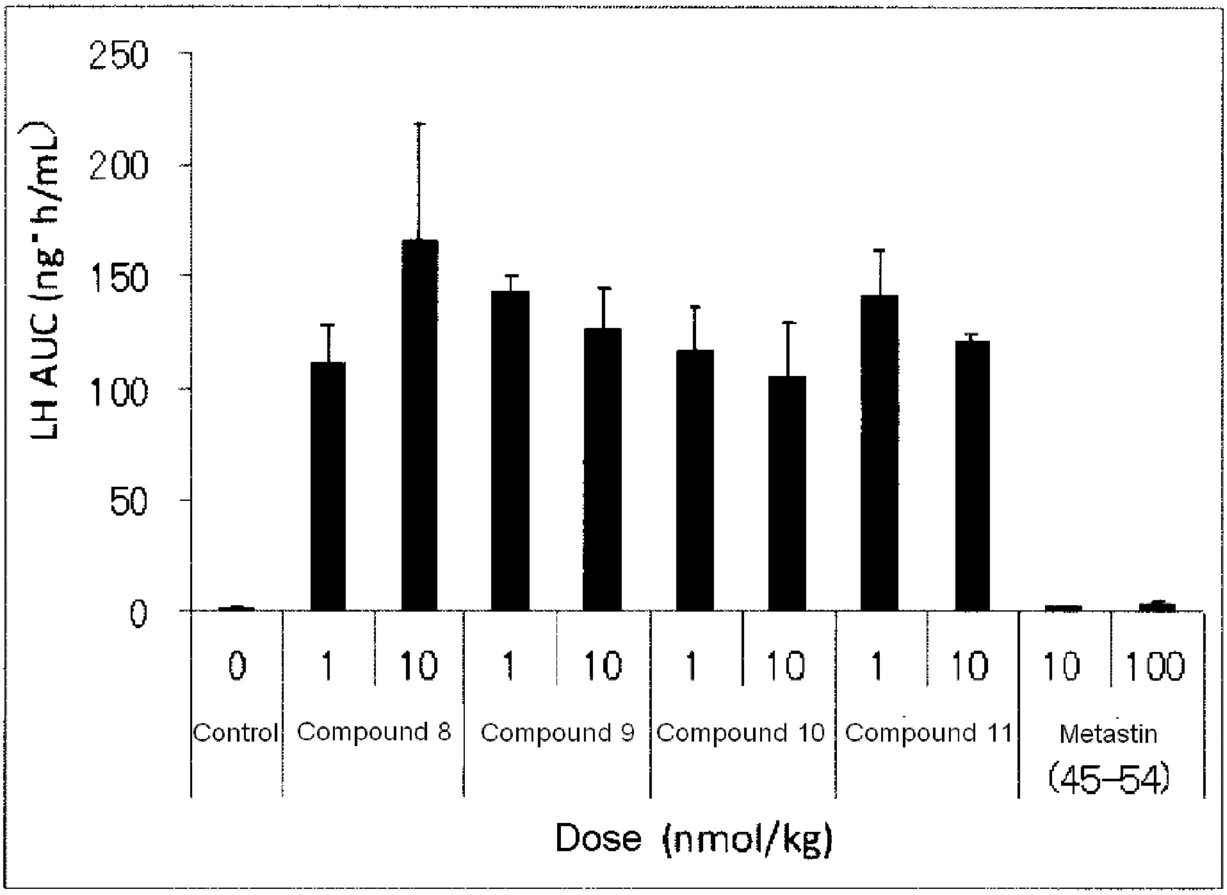

250
200
150
100
50
0
LH AUC (ng・h/mL)
0
1
10
1
10
1
10
1
10
10
100
Control
Compound 8
Compound 9
Compound 10
Compound 11
Metastin
(45-54)
Dose (nmol/kg)

[Fig. 4]

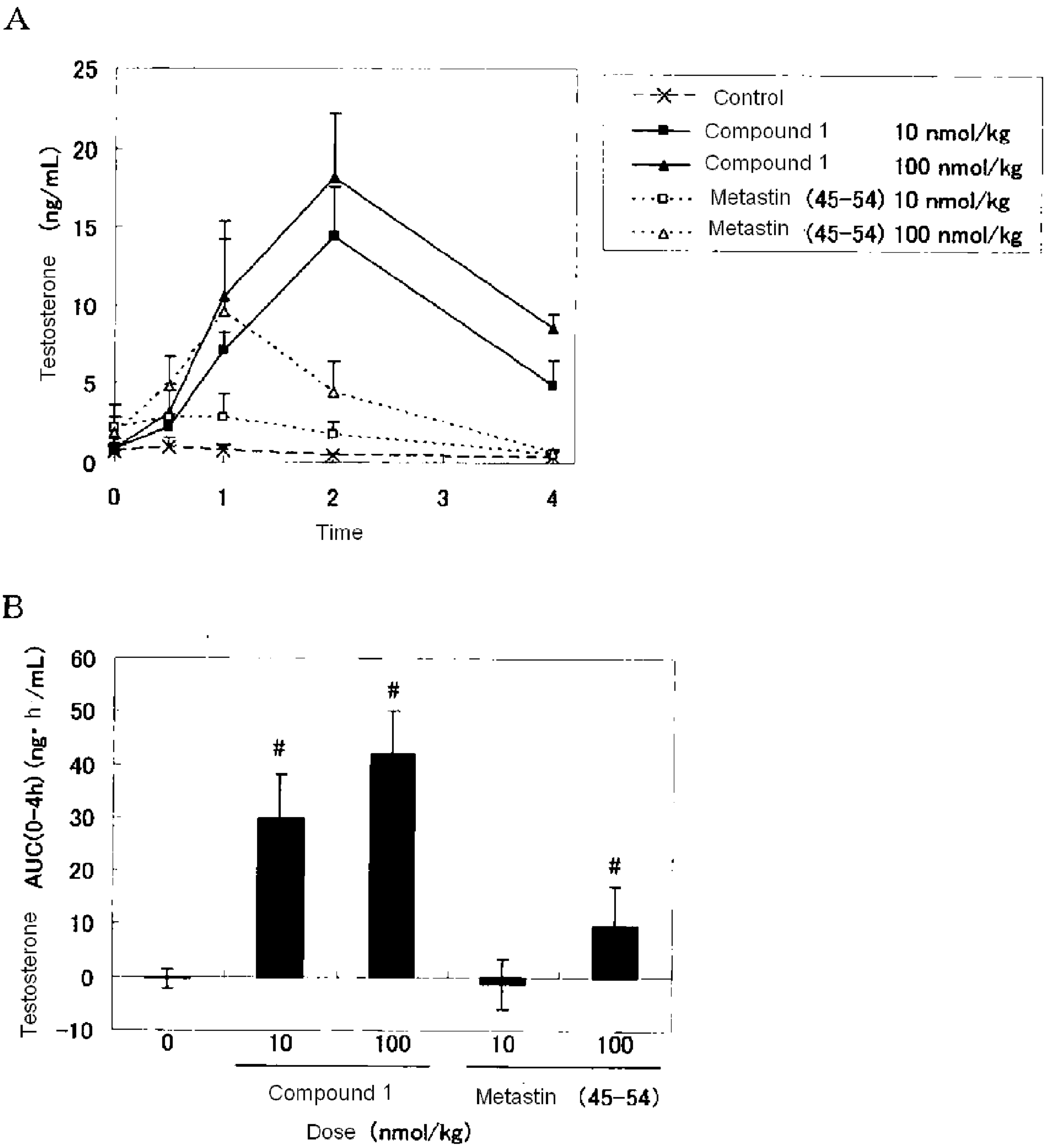
A
Testosterone (ng/mL)
25
20
15
10
5
0
0
1
2
3
4
Time
Control
Compound 1 10 nmol/kg
Compound 1 100 nmol/kg
Metastin (45-54) 10 nmol/kg
Metastin (45-54) 100 nmol/kg
B
Testosterone AUC(0-4h) (ng·h/mL)
60
50
40
30
20
10
0
-10
#
#
#
0
10
100
10
100
Compound 1
Metastin (45-54)
Dose (nmol/kg)

[Fig. 5]

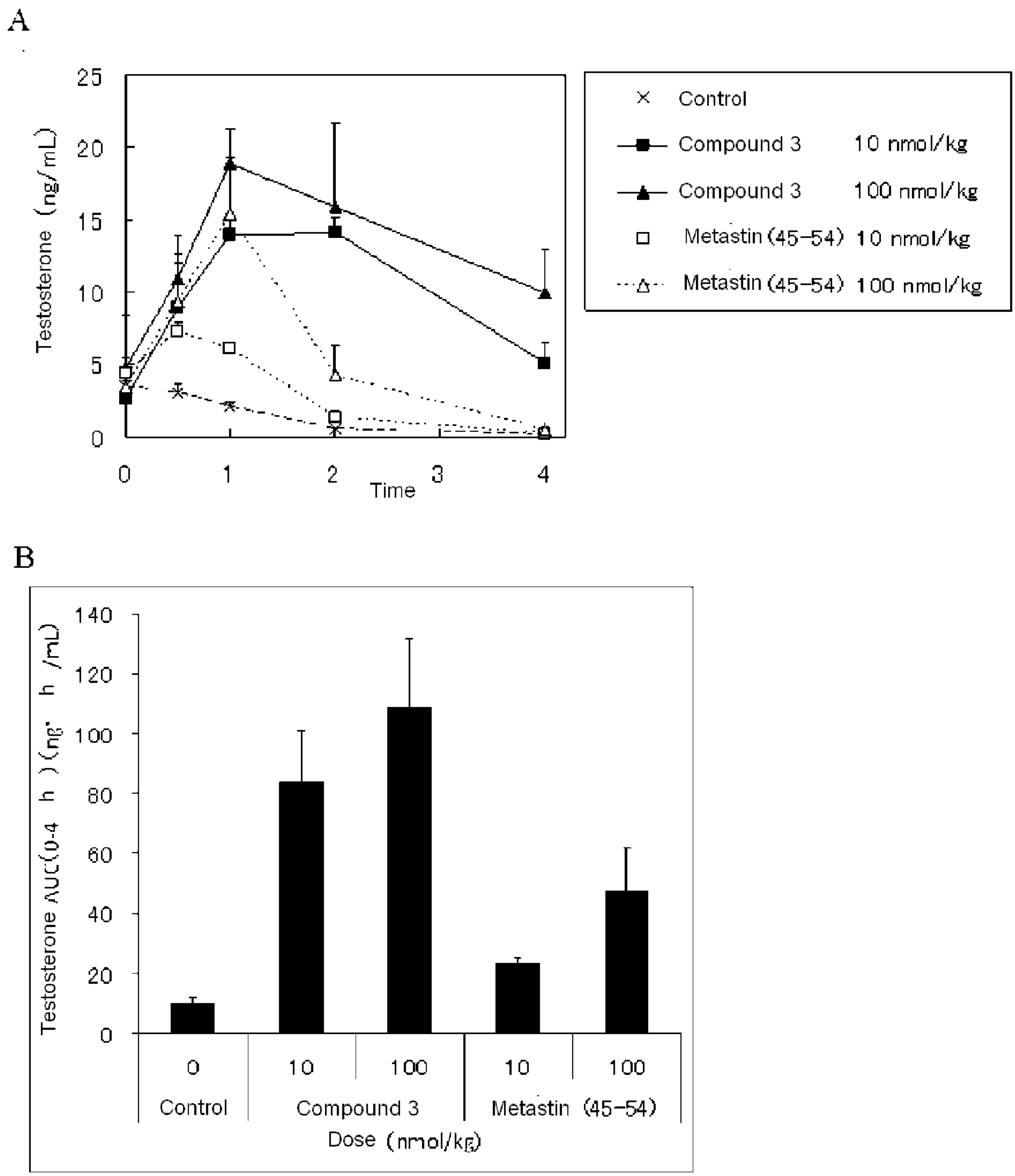
A
25
20
15
10
5
0
Testosterone (ng/mL)
0
1
2
3
4
Time
Control
Compound 3 10 nmol/kg
Compound 3 100 nmol/kg
Metastin (45-54) 10 nmol/kg
Metastin (45-54) 100 nmol/kg
B
140
120
100
80
60
40
20
0
Testosterone AUC(0-4 h) (ng・h/mL)
0
10
100
10
100
Control
Compound 3
Metastin (45-54)
Dose (nmol/kg)

[Fig. 6]

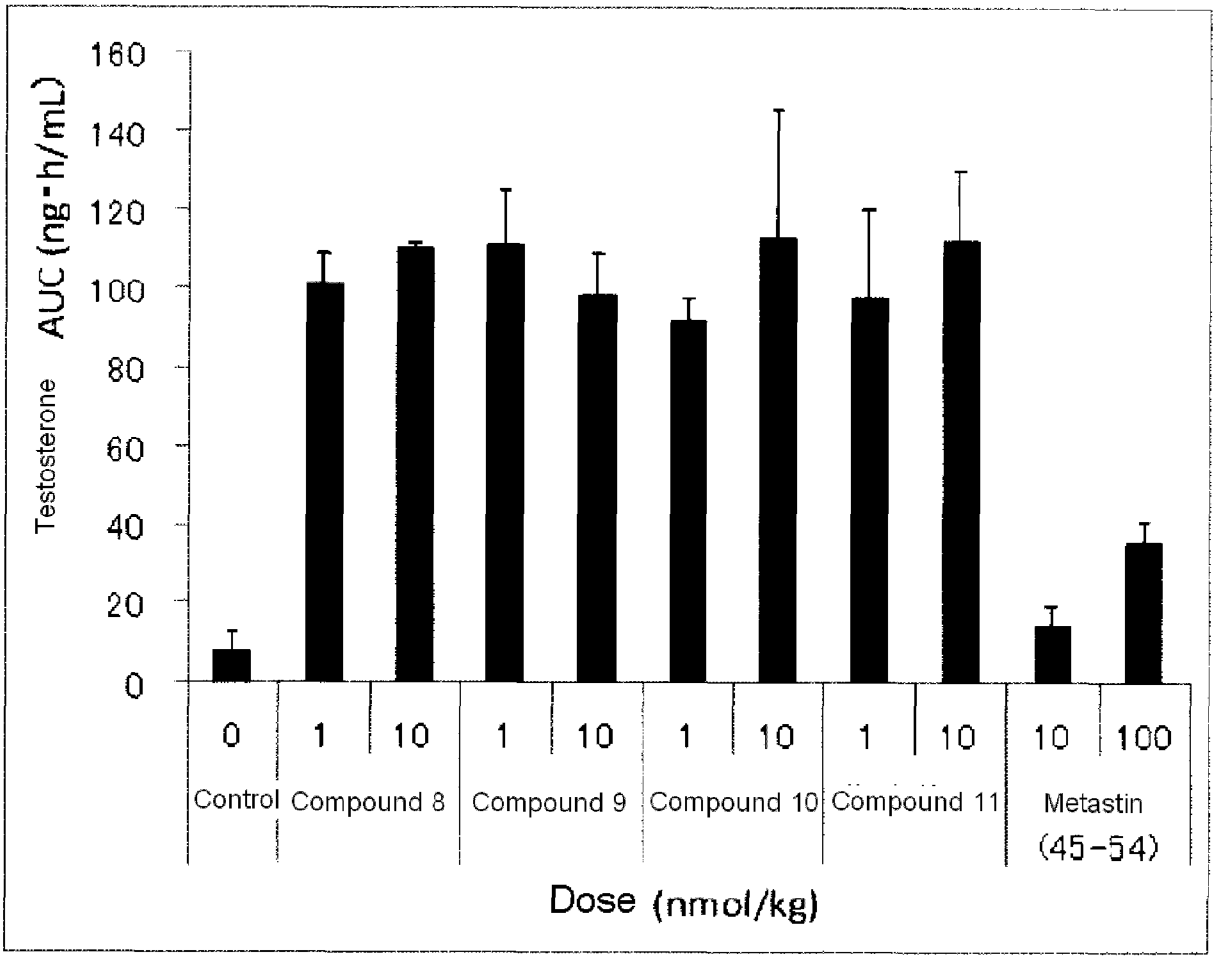
Testosterone AUC (ng·h/mL)
160
140
120
100
80
60
40
20
0
0
1
10
1
10
1
10
1
10
10
100
Control
Compound 8
Compound 9
Compound 10
Compound 11
Metastin (45-54)
Dose (nmol/kg)

Europäisches Patentamt
European Patent Office
Office européen des brevets

# EUROPEAN SEARCH REPORT

Application Number
EP 16 19 6765

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/117939 A2 (APPLIED RESEARCH SYSTEMS [NL]; CAMPBELL ROBERT K [US]; JIANG XULIANG [])<br>15 December 2005 (2005-12-15)<br>* the whole document *<br>* page 9 - page 15 *<br>* page 17 - page 20 * | 1,3-13 | INV.<br>C07K14/47<br>A61P3/06<br>A61P3/10<br>A61P5/24<br>A61P15/00<br>A61P15/06<br>A61P15/08<br>A61P25/28<br>A61P35/00<br>A61P35/04<br>C07K14/575 |
| X | WO 2007/084211 A2 (GEN HOSPITAL CORP [US]; BRIGHAM & WOMENS HOSPITAL [US]; UNIV PITTSBURG) 26 July 2007 (2007-07-26)<br>* the whole document *<br>* page 19 - page 20 *<br>* page 56 * | 1,3-13 | |
| A | ROBERTS M J ET AL: "Chemistry for peptide and protein PEGylation",<br>ADVANCED DRUG DELIVERY REVIEWS, ELSEVIER BV, AMSTERDAM, NL,<br>vol. 54, no. 4, 17 June 2002 (2002-06-17), pages 459-476, XP002293146,<br>ISSN: 0169-409X, DOI: 10.1016/S0169-409X(02)00022-4<br>* the whole document * | 1-13 | |
| | | | TECHNICAL FIELDS SEARCHED (IPC)<br>C07K<br>A61K |

The present search report has been drawn up for all claims

1

| Place of search | Date of completion of the search | Examiner |
|---|---|---|
| Munich | 7 June 2017 | Cervigni, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 19 6765

This annex lists the patent family members relating to the patent documents cited in the above-mentioned European search report. The members are as contained in the European Patent Office EDP file on The European Patent Office is in no way liable for these particulars which are merely given for the purpose of information.

07-06-2017

| Patent document cited in search report | Publication date | Patent family member(s) | Publication date |
|---|---|---|---|
| WO 2005117939 A2 | 15-12-2005 | AU 2005249373 A1 | 15-12-2005 |
| | | CA 2562676 A1 | 15-12-2005 |
| | | EP 1750513 A2 | 14-02-2007 |
| | | JP 2007533748 A | 22-11-2007 |
| | | WO 2005117939 A2 | 15-12-2005 |
| WO 2007084211 A2 | 26-07-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0024890 A
- WO 0175104 A
- WO 0285399 A
- WO 2004063221 A
- WO 2006001499 A
- WO 2007072997 A
- WO 2008050897 A
- JP 2008114480 A
- WO 2004060264 A
- WO 2007125619 A
- JP 200905810212 W
- JP 2009058102 W
- US 4343898 A


### Non-patent literature cited in the description


- **M. BODANSZKY ; M.A. ONDETTI.** Peptide Synthesis. Interscience Publishers, 1966
- **SCHROEDER ; LUEBKE.** The Peptide. Academic Press, 1965
- **NOBUO IZUMIYA et al.** Peptide Gosei-no-Kiso to Jikken (Basics and Experiments of Peptide Synthesis). Maruzen Co, 1975
- **HARUAKI YAJIMA ; SHUNPEI SAKAKIBARA ; SEIKAGAKU JIKKEN KOZA.** Biochemical Experiment) 1, Tanpakushitsu no Kagaku (Chemistry of Proteins). 1977, vol. IV, 205
- Zoku Iyakuhin no Kaihatsu (A sequel to Development of Pharmaceuticals). Peptide Synthesis. Hirokawa Shoten, vol. 14
- **ROBERTS, M. et al.** *Advanced Drug Delivery Reviews,* 2002, vol. 54, 457-476
- *International Journal of Peptide & Protein Research,* 1994, vol. 43, 127-138
- Iyakuhin no Kaihatsu (Pharmaceutical Research and Development). Bunshisekkei (Drug Design. Hirokawa Publishing Co, 1990, vol. 7, 163-198
- **J. SAMBROOK et al.** Molecular Cloning. Cold Spring Harbor Lab. Press, 1989

SEQUENCE LISTING

<110> Takeda Pharmaceutical Company Limited

<120> Metastin Derivatives and Its Use

<130> PCT09-0031

<150> JP 2008-196598
<151> 2008-07-30

<160> 22

<210> 1
<211> 54
<212> PRT
<213> Homo sapiens

<400> 1
Gly Thr Ser Leu Ser Pro Pro Pro Glu Ser Ser Gly Ser Arg Gln Gln
1 5 10 15
Pro Gly Leu Ser Ala Pro His Ser Arg Gln Ile Pro Ala Pro Gln Gly
20 25 30
Ala Val Leu Val Gln Arg Glu Lys Asp Leu Pro Asn Tyr Asn Trp Asn
35 40 45
Ser Phe Gly Leu Arg Phe
50

<210> 2
<211> 162
<212> DNA
<213> Homo sapiens

<400> 2
ggtacttctc tgtctccgcc gccggaatct tctggttctc gtcagcagcc gggtctgtct 60
gctccgcact ctcgtcagat cccggctccg cagggtgctg ttctggttca gcgtgaaaaa 120
gacctgccga actacaactg gaactctttc ggtctgcgtt tc 162

<210> 3
<211> 152
<212> PRT
<213> Mus musculus

<400> 3
Met Tyr Leu Arg Phe Gly Val Asp Val Cys Ser Leu Ser Pro Trp Lys
5 10 15
Glu Thr Val Asp Leu Pro Leu Pro Pro Arg Met Ile Ser Met Ala Ser
20 25 30
Trp Gln Leu Leu Leu Leu Leu Cys Val Ala Thr Tyr Gly Glu Pro Leu
35 40 45
Ala Lys Val Ala Pro Gly Ser Thr Gly Gln Gln Ser Gly Pro Gln Glu
50 55 60
Leu Val Asn Ala Trp Glu Lys Glu Ser Arg Tyr Ala Glu Ser Lys Pro
65 70 75 80
Gly Ser Ala Gly Leu Arg Ala Arg Arg Ser Ser Pro Cys Pro Pro Val
85 90 95
Glu Gly Pro Ala Gly Arg Gln Arg Pro Leu Cys Ala Ser Arg Ser Arg
100 105 110
Leu Ile Pro Ala Pro Arg Gly Ala Val Leu Val Gln Arg Glu Lys Asp
115 120 125
Leu Ser Thr Tyr Asn Trp Asn Ser Phe Gly Leu Arg Tyr Gly Arg Arg
130 135 140
Gln Ala Ala Arg Ala Ala Arg Gly

145 150

<210> 4
<211> 456
<212> DNA
<213> Mus musculus

<400> 4
atgtatctga gatttggcgt tgatgtctgc agcctgagtc cctggaagga gactgtagac 60
ctgccccttc ctcccagaat gatctcaatg gcttcttggc agctgctgct tctcctctgt 120
gtcgccacct atggggagcc gctggcaaaa gtgaagcctg gatccacagg ccagcagtcc 180
ggaccccagg aactcgttaa tgcctgggaa aaggaatcgc ggtatgcaga gagcaagcct 240
gggtctgcag ggctgcgcgc tcgtaggtcg tcgccatgcc cgccggttga gggccccgcg 300
gggcgccagc ggcccctgtg tgcctcccgc agtcgcctga tccctgcgcc ccgcggagcg 360
gtgctggtgc agcgggagaa ggacctgtcc acctacaact ggaactcctt cggcctgcgc 420
tacggcagga ggcaggcggc gcgggcagca cggggc 456

<210> 5
<211> 156
<212> PRT
<213> Mus musculus

<400> 5
Met Tyr Leu Arg Phe Gly Val Asp Val Cys Ser Leu Ser Pro Trp Lys
5 10 15
Glu Thr Val Asp Leu Pro Leu Pro Pro Arg Met Ile Ser Met Ala Ser
20 25 30
Trp Gln Leu Leu Leu Leu Leu Cys Val Ala Thr Tyr Gly Glu Pro Leu
35 40 45
Ala Lys Val Ala Pro Leu Val Lys Pro Gly Ser Thr Gly Gln Gln Ser
50 55 60
Gly Pro Gln Glu Leu Val Asn Ala Trp Glu Lys Glu Ser Arg Tyr Ala
65 70 75 80
Glu Ser Lys Pro Gly Ser Ala Gly Leu Arg Ala Arg Arg Ser Ser Pro
85 90 95
Cys Pro Pro Val Glu Gly Pro Ala Gly Arg Gln Arg Pro Leu Cys Ala
100 105 110
Ser Arg Ser Arg Leu Ile Pro Ala Pro Arg Gly Ala Val Leu Val Gln
115 120 125
Arg Glu Lys Asp Leu Ser Thr Tyr Asn Trp Asn Ser Phe Gly Leu Arg
130 135 140
Tyr Gly Arg Arg Gln Ala Ala Arg Ala Ala Arg Gly
145 150 155

<210> 6
<211> 468
<212> DNA
<213> Mus musculus

<400> 6
atgtatctga gatttggcgt tgatgtctgc agcctgagtc cctggaagga gactgtagac 60
ctgccccttc ctcccagaat gatctcaatg gcttcttggc agctgctgct tctcctctgt 120
gtcgccacct atggggagcc gctggcaaaa gtggcacctt tggtgaagcc tggatccaca 180
ggccagcagt ccggacccca ggaactcgtt aatgcctggg aaaaggaatc gcggtatgca 240
gagagcaagc ctgggtctgc agggctgcgc gctcgtaggt cgtcgccatg cccgccggtt 300
gagggccccg cggggcgcca gcggcccctg tgtgcctccc gcagtcgcct gatccctgcg 360
ccccgcggag cggtgctggt gcagcgggag aaggacctgt ccacctacaa ctggaactcc 420
ttcggcctgc gctacggcag gaggcaggcg gcgcgggcag cacggggc 468

<210> 7
<211> 130
<212> PRT
<213> Rattus sp.

<400> 7
Met Thr Ser Leu Ala Ser Trp Gln Leu Leu Leu Leu Leu Cys Val Ala
                5                   10                  15
Ser Phe Gly Glu Pro Leu Ala Lys Met Ala Pro Val Val Asn Pro Glu
            20                  25                  30
Pro Thr Gly Gln Gln Ser Gly Pro Gln Glu Leu Val Asn Ala Trp Gln
        35                  40                  45
Lys Gly Pro Arg Tyr Ala Glu Ser Lys Pro Gly Ala Ala Gly Leu Arg
    50                  55                  60
Ala Arg Arg Thr Ser Pro Cys Pro Pro Val Glu Asn Pro Thr Gly His
65                  70                  75                  80
Gln Arg Pro Pro Cys Ala Thr Arg Ser Arg Leu Ile Pro Ala Pro Arg
                85                  90                  95
Gly Ser Val Leu Val Gln Arg Glu Lys Asp Met Ser Ala Tyr Asn Trp
            100                 105                 110
Asn Ser Phe Gly Leu Arg Tyr Gly Arg Arg Gln Val Ala Arg Ala Ala
        115                 120                 125
Arg Gly
    130

<210> 8
<211> 390
<212> DNA
<213> Rattus sp.

<400> 8
atgacctcgc tggcttcttg gcagctgctg cttctcctct gtgtggcctc ttttggggag 60
ccactggcaa aaatggcacc tgtggtgaac cctgaaccca caggccaaca gtccggaccc 120
caggaactcg ttaatgcctg gcaaaagggc ccgcggtatg cagagagcaa gcctggggct 180
gcaggactgc gcgctcgccg aacatcgcca tgcccgccgg tggagaaccc cacggggcac 240
cagcggcccc cgtgtgccac ccgcagtcgc ctgatccctg cgccccgcgg atcggtgctg 300
gtgcagcgcg agaaggacat gtcagcctac aactggaact cctttggcct gcgctacggc 360
aggaggcagg tggcgcgggc ggcacggggc 390

<210> 9
<211> 398
<212> PRT
<213> Homo sapiens

<400> 9
Met His Thr Val Ala Thr Ser Gly Pro Asn Ala Ser Trp Gly Ala Pro
                5                   10                  15
Ala Asn Ala Ser Gly Cys Pro Gly Cys Gly Ala Asn Ala Ser Asp Gly
            20                  25                  30
Pro Val Pro Ser Pro Arg Ala Val Asp Ala Trp Leu Val Pro Leu Phe
        35                  40                  45
Phe Ala Ala Leu Met Leu Leu Gly Leu Val Gly Asn Ser Leu Val Ile
    50                  55                  60
Tyr Val Ile Cys Arg His Lys Pro Met Arg Thr Val Thr Asn Phe Tyr
65                  70                  75                  80
Ile Ala Asn Leu Ala Ala Thr Asp Val Thr Phe Leu Leu Cys Cys Val
                85                  90                  95
Pro Phe Thr Ala Leu Leu Tyr Pro Leu Pro Gly Trp Val Leu Gly Asp
            100                 105                 110
Phe Met Cys Lys Phe Val Asn Tyr Ile Gln Gln Val Ser Val Gln Ala
        115                 120                 125
Thr Cys Ala Thr Leu Thr Ala Met Ser Val Asp Arg Trp Tyr Val Thr
    130                 135                 140
Val Phe Pro Leu Arg Ala Leu His Arg Arg Thr Pro Arg Leu Ala Leu
145                 150                 155                 160
Ala Val Ser Leu Ser Ile Trp Val Gly Ser Ala Ala Val Ser Ala Pro
                165                 170                 175

Val Leu Ala Leu His Arg Leu Ser Pro Gly Pro Arg Ala Tyr Cys Ser
180 185 190
Glu Ala Phe Pro Ser Arg Ala Leu Glu Arg Ala Phe Ala Leu Tyr Asn
195 200 205
Leu Leu Ala Leu Tyr Leu Leu Pro Leu Leu Ala Thr Cys Ala Cys Tyr
210 215 220
Ala Ala Met Leu Arg His Leu Gly Arg Val Ala Val Arg Pro Ala Pro
225 230 235 240
Ala Asp Ser Ala Leu Gln Gly Gln Val Leu Ala Glu Arg Ala Gly Ala
245 250 255
Val Arg Ala Lys Val Ser Arg Leu Val Ala Ala Val Val Leu Leu Phe
260 265 270
Ala Ala Cys Trp Gly Pro Ile Gln Leu Phe Leu Val Leu Gln Ala Leu
275 280 285
Gly Pro Ala Gly Ser Trp His Pro Arg Ser Tyr Ala Ala Tyr Ala Leu
290 295 300
Lys Thr Trp Ala His Cys Met Ser Tyr Ser Asn Ser Ala Leu Asn Pro
305 310 315 320
Leu Leu Tyr Ala Phe Leu Gly Ser His Phe Arg Gln Ala Phe Arg Arg
325 330 335
Val Cys Pro Cys Ala Pro Arg Arg Pro Arg Arg Pro Arg Arg Pro Gly
340 345 350
Pro Ser Asp Pro Ala Ala Pro His Ala Glu Leu His Arg Leu Gly Ser
355 360 365
His Pro Ala Pro Ala Arg Ala Gln Lys Pro Gly Ser Ser Gly Leu Ala
370 375 380
Ala Arg Gly Leu Cys Val Leu Gly Glu Asp Asn Ala Pro Leu
385 390 395

<210> 10
<211> 1194
<212> DNA
<213> Homo sapiens

<400> 10
atgcacaccg tggctacgtc cggacccaac gcgtcctggg gggcaccggc caacgcctcc 60
ggctgcccgg gctgtggcgc caacgcctcg gacggcccag tcccttcgcc gcgggccgtg 120
gacgcctggc tcgtgccgct cttcttcgcg gcgctgatgc tgctgggcct ggtggggaac 180
tcgctggtca tctacgtcat ctgccgccac aagccgatgc ggaccgtgac caacttctac 240
atcgccaacc tggcggccac ggacgtgacc ttcctcctgt gctgcgtccc cttcacggcc 300
ctgctgtacc cgctgcccgg ctgggtgctg ggcgacttca tgtgcaagtt cgtcaactac 360
atccagcagg tctcggtgca ggccacgtgt gccactctga ccgccatgag tgtggaccgc 420
tggtacgtga cggtgttccc gttgcgcgcc ctgcaccgcc gcacgccccg cctggcgctg 480
gctgtcagcc tcagcatctg ggtaggctct gcggcggtgt ctgcgccggt gctcgccctg 540
caccgcctgt cacccgggcc gcgcgcctac tgcagtgagg ccttccccag ccgcgccctg 600
gagcgcgcct tcgcactgta caacctgctg gcgctgtacc tgctgccgct gctcgccacc 660
tgcgcctgct atgcggccat gctgcgccac ctgggccggg tcgccgtgcg ccccgcgccc 720
gccgatagcg ccctgcaggg gcaggtgctg gcagagcgcg caggcgccgt gcgggccaag 780
gtctcgcggc tggtggcggc cgtggtcctg ctcttcgccg cctgctgggg ccccatccag 840
ctgttcctgg tgctgcaggc gctgggcccc gcgggctcct ggcacccacg cagctacgcc 900
gcctacgcgc ttaagacctg ggctcactgc atgtcctaca gcaactccgc gctgaacccg 960
ctgctctacg ccttcctggg ctcgcacttc cgacaggcct tccgccgcgt ctgcccctgc 1020
gcgccgcgcc gcccccgccg cccccgccgg cccggaccct cggaccccgc agccccacac 1080
gcggagctgc accgcctggg gtcccacccg gcccccgcca gggcgcagaa gccagggagc 1140
agtgggctgg ccgcgcgcgg gctgtgcgtc ctgggggagg acaacgcccc tctc 1194

<210> 11
<211> 396
<212> PRT
<213> Rattus sp.

<400> 11
Met Ala Ala Glu Ala Thr Leu Gly Pro Asn Val Ser Trp Trp Ala Pro

```
                5                   10                  15
Ser Asn Ala Ser Gly Cys Pro Gly Cys Gly Val Asn Ala Ser Asp Gly
            20                  25                  30
Pro Gly Ser Ala Pro Arg Pro Leu Asp Ala Trp Leu Val Pro Leu Phe
        35                  40                  45
Phe Ala Ala Leu Met Leu Leu Gly Leu Val Gly Asn Ser Leu Val Ile
    50                  55                  60
Phe Val Ile Cys Arg His Lys His Met Gln Thr Val Thr Asn Phe Tyr
65                  70                  75                  80
Ile Ala Asn Leu Ala Ala Thr Asp Val Thr Phe Leu Leu Cys Cys Val
                85                  90                  95
Pro Phe Thr Ala Leu Leu Tyr Pro Leu Pro Thr Trp Val Leu Gly Asp
            100                 105                 110
Phe Met Cys Lys Phe Val Asn Tyr Ile Gln Gln Val Ser Val Gln Ala
        115                 120                 125
Thr Cys Ala Thr Leu Thr Ala Met Ser Val Asp Arg Trp Tyr Val Thr
    130                 135                 140
Val Phe Pro Leu Arg Ala Leu His Arg Arg Thr Pro Arg Leu Ala Leu
145                 150                 155                 160
Thr Val Ser Leu Ser Ile Trp Val Gly Ser Ala Ala Val Ser Ala Pro
                165                 170                 175
Val Leu Ala Leu His Arg Leu Ser Pro Gly Pro His Thr Tyr Cys Ser
            180                 185                 190
Glu Ala Phe Pro Ser Arg Ala Leu Glu Arg Ala Phe Ala Leu Tyr Asn
        195                 200                 205
Leu Leu Ala Leu Tyr Leu Leu Pro Leu Leu Ala Thr Cys Ala Cys Tyr
    210                 215                 220
Gly Ala Met Leu Arg His Leu Gly Arg Ala Ala Val Arg Pro Ala Pro
225                 230                 235                 240
Thr Asp Gly Ala Leu Gln Gly Gln Leu Leu Ala Gln Arg Ala Gly Ala
                245                 250                 255
Val Arg Thr Lys Val Ser Arg Leu Val Ala Ala Val Val Leu Leu Phe
            260                 265                 270
Ala Ala Cys Trp Gly Pro Ile Gln Leu Phe Leu Val Leu Gln Ala Leu
        275                 280                 285
Gly Pro Ser Gly Ala Trp His Pro Arg Ser Tyr Ala Ala Tyr Ala Leu
    290                 295                 300
Lys Ile Trp Ala His Cys Met Ser Tyr Ser Asn Ser Ala Leu Asn Pro
305                 310                 315                 320
Leu Leu Tyr Ala Phe Leu Gly Ser His Phe Arg Gln Ala Phe Cys Arg
                325                 330                 335
Val Cys Pro Cys Gly Pro Gln Arg Gln Arg Arg Pro His Ala Ser Ala
            340                 345                 350
His Ser Asp Arg Ala Ala Pro His Ser Val Pro His Ser Arg Ala Ala
        355                 360                 365
His Pro Val Arg Val Arg Thr Pro Glu Pro Gly Asn Pro Val Val Arg
    370                 375                 380
Ser Pro Ser Val Gln Asp Glu His Thr Ala Pro Leu
385                 390                 395
```

<210> 12
<211> 1188
<212> DNA
<213> Rattus sp.

<400> 12

```
atggccgcag aggcgacgtt gggtccgaac gtgagctggt gggctccgtc caacgcttcg     60
ggatgcccgg gctgcggtgt caatgcctcg gatggcccag gctccgcgcc aaggcccctg    120
gatgcctggc tggtgcccct gtttttcgct gccctaatgt tgctggggct agtcgggaac    180
tcactggtca tcttcgttat ctgccgccac aagcacatgc agaccgtcac caatttctac    240
atcgctaacc tggcggccac agatgtcact ttccttctgt gctgcgtacc cttcaccgcg    300
ctcctctatc cgctgcccac ctgggtgctg ggagacttca tgtgcaaatt cgtcaactac    360
atccagcagg tctcggtgca agccacatgt gccactttga cagccatgag tgtggaccgc    420
```

```
tggtacgtga ctgtgttccc gctgcgtgca cttcaccgcc gcactccgcg cctggccctg    480
actgtcagcc ttagcatctg ggtgggttcc gcagctgttt ccgccccggt gctggctctg    540
caccgcctgt cgcccgggcc tcacacctac tgcagtgagg cgtttcccag ccgtgccctg    600
gagcgcgctt tcgcgctcta caacctgctg gccctatacc tgctgccgct gctcgccacc    660
tgcgcctgct acggtgccat gctgcgccac ctgggccgcg ccgctgtacg ccccgcaccc    720
actgatggcg ccctgcaggg gcagctgcta gcacagcgcg ctggagcagt gcgcaccaag    780
gtctcccggc tggtggccgc tgtcgtcctg ctcttcgccg cctgctgggg cccgatccag    840
ctgttcctgg tgcttcaagc cctgggcccc tcgggggcct ggcaccctcg aagctatgcc    900
gcctacgcgc tcaagatctg ggctcactgc atgtcctaca gcaattctgc gctcaacccg    960
ctgctctatg ccttcctggg ttcccacttc agacaggcct tctgccgcgt gtgcccctgc   1020
ggcccgcaac gccagcgtcg gccccacgcg tcagcgcact cggaccgagc cgcaccccat   1080
agtgtgccgc acagccgggc tgcgcaccct gtccgggtca ggacccccga gcctgggaac   1140
cctgtggtgc gctcgccctc tgttcaggat gaacacactg ccccactc                1188
```

```
<210> 13
<211> 396
<212> PRT
<213> Mus musculus

<400> 13
Met Ala Thr Glu Ala Thr Leu Ala Pro Asn Val Thr Trp Trp Ala Pro
1               5                   10                  15
Ser Asn Ala Ser Gly Cys Pro Gly Cys Gly Val Asn Ala Ser Asp Asp
            20                  25                  30
Pro Gly Ser Ala Pro Arg Pro Leu Asp Ala Trp Leu Val Pro Leu Phe
        35                  40                  45
Phe Ala Thr Leu Met Leu Leu Gly Leu Val Gly Asn Ser Leu Val Ile
    50                  55                  60
Tyr Val Ile Cys Arg His Lys His Met Gln Thr Val Thr Asn Phe Tyr
65                  70                  75                  80
Ile Ala Asn Leu Ala Ala Thr Asp Val Thr Phe Leu Leu Cys Cys Val
                85                  90                  95
Pro Phe Thr Ala Leu Leu Tyr Pro Leu Pro Ala Trp Val Leu Gly Asp
            100                 105                 110
Phe Met Cys Lys Phe Val Asn Tyr Ile Gln Gln Val Ser Val Gln Ala
        115                 120                 125
Thr Cys Ala Thr Leu Thr Ala Met Ser Val Asp Arg Trp Tyr Val Thr
    130                 135                 140
Val Phe Pro Leu Arg Ala Leu His Arg Arg Thr Pro Arg Leu Ala Leu
145                 150                 155                 160
Ala Val Ser Leu Ser Ile Trp Val Gly Ser Ala Ala Val Ser Ala Pro
                165                 170                 175
Val Leu Ala Leu His Arg Leu Ser Pro Gly Pro Arg Thr Tyr Cys Ser
            180                 185                 190
Glu Ala Phe Pro Ser Arg Ala Leu Glu Arg Ala Phe Ala Leu Tyr Asn
        195                 200                 205
Leu Leu Ala Leu Tyr Leu Leu Pro Leu Leu Ala Thr Cys Ala Cys Tyr
    210                 215                 220
Gly Ala Met Leu Arg His Leu Gly Arg Ala Ala Val Arg Pro Ala Pro
225                 230                 235                 240
Thr Asp Gly Ala Leu Gln Gly Gln Leu Leu Ala Gln Arg Ala Gly Ala
                245                 250                 255
Val Arg Thr Lys Val Ser Arg Leu Val Ala Ala Val Val Leu Leu Phe
            260                 265                 270
Ala Ala Cys Trp Gly Pro Ile Gln Leu Phe Leu Val Leu Gln Ala Leu
        275                 280                 285
Gly Pro Ser Gly Ala Trp His Pro Arg Ser Tyr Ala Ala Tyr Ala Val
    290                 295                 300
Lys Ile Trp Ala His Cys Met Ser Tyr Ser Asn Ser Ala Leu Asn Pro
305                 310                 315                 320
Leu Leu Tyr Ala Phe Leu Gly Ser His Phe Arg Gln Ala Phe Cys Arg
                325                 330                 335
Val Cys Pro Cys Cys Arg Gln Arg Gln Arg Arg Pro His Thr Ser Ala
```

```
            340                 345                 350
His Ser Asp Arg Ala Ala Thr His Thr Val Pro His Ser Arg Ala Ala
        355                 360                 365
His Pro Val Arg Ile Arg Ser Pro Glu Pro Gly Asn Pro Val Val Arg
    370                 375                 380
Ser Pro Cys Ala Gln Ser Glu Arg Thr Ala Ser Leu
385                 390                 395

<210> 14
<211> 1188
<212> DNA
<213> Mus musculus

<400> 14
atggccaccg aggcgacatt ggctcccaat gtgacctggt gggctccgtc caacgcttca   60
ggatgcccag gctgcggtgt caacgcctcg gatgacccag gctctgcgcc aaggcccctg  120
gatgcctggc tggttcccct gtttttcgct acactcatgt tgcttgggct ggtcggaaac  180
tcattggtca tctacgttat ctgccgccac aagcacatgc agacagttac caacttctac  240
atcgctaacc tggctgccac agacgtcact ttcctactgt gctgcgtgcc cttcaccgca  300
ctcctctacc cgctgcccgc ctgggtgctg ggagacttca tgtgcaaatt cgtcaactac  360
atccagcagg tctcggtgca agccacatgt gccactctga cggccatgag tgtggaccgc  420
tggtatgtga ctgtgttccc gctgcgtgca cttcaccgcc gcactccgcg cctggccctg  480
gctgtcagcc tcagcatctg ggtggggtca gcagctgtgt ccgcccccggt gctggccctg 540
caccgcctgt cgccagggcc tcgcacctac tgcagcgagg cgtttcccag ccgcgccctg  600
gagcgcgcct tcgcgctcta caacctgctg gctctatatc tgctgccgct gctcgccacc  660
tgcgcctgct acggcgccat gctgcgccac ctgggccgtg cggctgtacg ccccgcaccc  720
actgacggcg ccctgcaggg acagctgcta gcacagcgcg ccggagcagt gcgcaccaag  780
gtctcccggc tggtggccgc tgtcgtcctg ctcttcgccg cctgctgggg cccgatccag  840
ctgttcctgg tgcttcaagc cctgggcccc tcgggggcct ggcaccctcg aagctatgcc  900
gcctacgcgg tcaagatctg ggctcactgc atgtcctaca gcaactcggc gctcaatccg  960
ctgctctatg ccttcctggg ttcacacttc agacaggcct tctgccgcgt gtgcccctgc 1020
tgccggcaac gccagcgccg gccccacacg tcagcgcact cggaccgagc tgcaactcac 1080
actgtgccgc acagccgtgc tgcgcaccct gtgcggatca ggagcccgga gcctgggaac 1140
cctgtggtgc gctcgccctg cgctcagagt gaacgcactg cctcactc              1188

<210> 15
<211> 15
<212> PRT
<213> Artificial

<220>
<223> the C-terminus of the polypeptide is amide (-CONH2) form

<400> 15
Lys Asp Leu Pro Asn Tyr Asn Trp Asn Ser Phe Gly Leu Arg Phe
1               5                   10                  15

<210> 16
<211> 10
<212> PRT
<213> Artificial

<220>
<223> the C-terminus of the polypeptide is amide (-CONH2) form

<400> 16
Tyr Asn Trp Asn Ser Phe Gly Leu Arg Phe
1               5                   10

<210> 17
<211> 9
<212> PRT
<213> Artificial
```

```
<220>
<223> the C-terminus of the polypeptide is amide (-CONH2) form

<400> 17
Asn Trp Asn Ser Phe Gly Leu Arg Phe
1               5               9

<210> 18
<211> 8
<212> PRT
<213> Artificial

<220>
<223> the C-terminus of the polypeptide is amide (-CONH2) form

<400> 18
Trp Asn Ser Phe Gly Leu Arg Phe
1               5           8

<210> 19
<211> 45
<212> DNA
<213> Homo sapiens

<400> 19
aaggacctgc cgaactacaa ctggaactcc ttcggcctgc gcttc                       45

<210> 20
<211> 30
<212> DNA
<213> Homo sapiens

<400> 20
tacaactgga actccttcgg cctgcgcttc                                        30

<210> 21
<211> 27
<212> DNA
<213> Homo sapiens

<400> 21
aactggaact ccttcggcct gcgcttc                                           27

<210> 22
<211> 24
<212> DNA
<213> Homo sapiens

<400> 22
tggaactcct tcggcctgcg cttc                                              24

4/12
```